(19) Europäisches Patentamt
European Patent Office
Office européen des brevets

(11) EP 1 820 861 A2

(12) **EUROPEAN PATENT APPLICATION**

(43) Date of publication:
**22.08.2007 Bulletin 2007/34**

(21) Application number: **07006973.7**

(22) Date of filing: **24.07.2001**

(51) Int Cl.:
***C12N 15/12*** *(2006.01)* ***C07K 14/705*** *(2006.01)*
***A01K 67/027*** *(2006.01)* ***A61K 38/16*** *(2006.01)*
***C07K 16/18*** *(2006.01)* ***C12N 5/10*** *(2006.01)*
***C12N 15/62*** *(2006.01)* ***G01N 33/50*** *(2006.01)*

(84) Designated Contracting States:
**AT BE CH CY DE DK ES FI FR GB GR IE IT LI LU MC NL PT SE TR**

(30) Priority: **02.08.2000 US 222504 P**
**28.11.2000 US 728787**

(62) Document number(s) of the earlier application(s) in accordance with Art. 76 EPC:
**01959147.8 / 1 307 554**

(71) Applicant: **Amgen Inc.**
**Thousand Oaks, CA 91320-1799 (US)**

(72) Inventors:
- **Welcher, Andrew A.**
**Ventura, CA 93001 (US)**
- **Elliott, Gary S.**
**Thousand Oaks, CA 91362 (US)**

(74) Representative: **Grünecker, Kinkeldey, Stockmair & Schwanhäusser Anwaltssozietät**
**Maximilianstrasse 58**
**80538 München (DE)**

Remarks:
This application was filed on 03 - 04 - 2007 as a divisional application to the application mentioned under INID code 62.

# (54) C3B/C4B complement receptor-like molecules and uses thereof

(57) Novel C3b/C4b CR-like polypeptides and nucleic acid molecules encoding the same. The invention also provides vectors, host cells, selective binding agents, and methods for producing C3b/C4b CR-like polypeptides. Also provided for are methods for the treatment, diagnosis, amelioration, or prevention of diseases with C3b/C4b CR-like polypeptides.

EP 1 820 861 A2

SEQUENCE LISTING

<110> AMGEN, Inc.

<120> C3B/C4B COMPLEMENT RECEPTOR-LIKE MOLECULES AND USES THEREOF

<130> 01017/37498

<140>

<141> 2001-07-23

<150> 09/728,787

<141> 2000-11-28

<150> US 60/222,504

<151> 2000-08-02

<160> 7

<170> PatentIn version 3.0

<210> 1

<211> 10673

<212> DNA

<213> Homo sapiens

<220>

<221> CDS

<222> (334)..(9540)

<400> 1

```
cctggggaag cctctcggtt ccaggaaaat gggatggttg attgccctaa attgattttt      60
taaaagaaaa ttcacgaatt ggcagccata gaatagagta atttctgtaa agcaccagtg     120
atagtgatgt ttgaatatta atataatgga ccagaggctg tacagtcttt gaaagagggt     180
```

```
cttgctacct atatatctag ggtttggctg tttaaagcag caagaccctc ctttcaggtg    240

gaagtcgatg tacttgttgc cttacctaaa agctttgaca tttctctttc ttgcaggctc    300

acgggatcca gtgttcctga cctcattgtg agc atg agc aac cag atg tgg cta      354
                                     Met Ser Asn Gln Met Trp Leu
                                     1               5

cat ctg cag tcg gat gat agc att ggc tca cct ggg ttt aaa gct gtt      402
His Leu Gln Ser Asp Asp Ser Ile Gly Ser Pro Gly Phe Lys Ala Val
        10                  15                  20

tac caa gaa att gaa aag gga ggg tgt ggg gat cct gga atc ccc gcc      450
Tyr Gln Glu Ile Glu Lys Gly Gly Cys Gly Asp Pro Gly Ile Pro Ala
    25                  30                  35

tat ggg aag cgg acg ggc agc agt ttc ctc cat gga gat aca ctc acc      498
Tyr Gly Lys Arg Thr Gly Ser Ser Phe Leu His Gly Asp Thr Leu Thr
40                  45                  50                  55

ttt gaa tgc ccg gcg gcc ttt gag ctg gtg ggg gag aga gtt atc acc      546
Phe Glu Cys Pro Ala Ala Phe Glu Leu Val Gly Glu Arg Val Ile Thr
                60                  65                  70

tgt cag cag aac aat cag tgg tct ggc aac aag ccc agc tgt gta ttt      594
Cys Gln Gln Asn Asn Gln Trp Ser Gly Asn Lys Pro Ser Cys Val Phe
            75                  80                  85

tca tgt ttc ttc aac ttt acg gca tca tct ggg att att ctg tca cca      642
Ser Cys Phe Phe Asn Phe Thr Ala Ser Ser Gly Ile Ile Leu Ser Pro
        90                  95                  100

aat tat cca gag gaa tat ggg aac aac atg aac tgt gtc tgg ttg att      690
Asn Tyr Pro Glu Glu Tyr Gly Asn Asn Met Asn Cys Val Trp Leu Ile
    105                 110                 115

atc tcg gag cca gga agt cga att cac cta atc ttt aat gat ttt gat      738
Ile Ser Glu Pro Gly Ser Arg Ile His Leu Ile Phe Asn Asp Phe Asp
120                 125                 130                 135

gtt gag cct caa ttt gac ttt ctc gcg gtc aag gat gat ggc att tct      786
Val Glu Pro Gln Phe Asp Phe Leu Ala Val Lys Asp Asp Gly Ile Ser
                140                 145                 150

gac ata act gtc ctg ggt act ttt tct ggc aat gaa gtg cct tcc cag      834
Asp Ile Thr Val Leu Gly Thr Phe Ser Gly Asn Glu Val Pro Ser Gln
            155                 160                 165

ctg gcc agc agt ggg cat ata gtt cgc ttg gaa ttt cag tct gac cat      882
Leu Ala Ser Ser Gly His Ile Val Arg Leu Glu Phe Gln Ser Asp His
        170                 175                 180

tcc act act ggc aga ggg ttc aac atc act tac acc aca ttt ggt cag      930
Ser Thr Thr Gly Arg Gly Phe Asn Ile Thr Tyr Thr Thr Phe Gly Gln
    185                 190                 195

aat gag tgc cat gat cct ggc att cct ata aac gga cga cgt ttt ggt      978
Asn Glu Cys His Asp Pro Gly Ile Pro Ile Asn Gly Arg Arg Phe Gly
200                 205                 210                 215

gac agg ttt cta ctc ggg agc tcg gtt tct ttc cac tgt gat gat ggc     1026
Asp Arg Phe Leu Leu Gly Ser Ser Val Ser Phe His Cys Asp Asp Gly
                220                 225                 230
```

```
ttt gtc aag acc cag gga tcc gag tcc att acc tgc ata ctg caa gac      1074
Phe Val Lys Thr Gln Gly Ser Glu Ser Ile Thr Cys Ile Leu Gln Asp
            235                 240                 245

ggg aac gtg gtc tgg agc tcc acc gtg ccc cgc tgt gaa gct cca tgt      1122
Gly Asn Val Val Trp Ser Ser Thr Val Pro Arg Cys Glu Ala Pro Cys
        250                 255                 260

ggt gga cat ctg aca gcg tcc agc gga gtc att ttg cct cct gga tgg      1170
Gly Gly His Leu Thr Ala Ser Ser Gly Val Ile Leu Pro Pro Gly Trp
    265                 270                 275

cca gga tat tat aag gat tct tta cat tgt gaa tgg ata att gaa gca      1218
Pro Gly Tyr Tyr Lys Asp Ser Leu His Cys Glu Trp Ile Ile Glu Ala
280                 285                 290                 295

aaa cca ggc cac tct atc aaa ata act ttt gac aga ttt cag aca gag      1266
Lys Pro Gly His Ser Ile Lys Ile Thr Phe Asp Arg Phe Gln Thr Glu
                300                 305                 310

gtc aat tat gac acc ttg gag gtc aga gat ggg cca gcc agt tcg tcc      1314
Val Asn Tyr Asp Thr Leu Glu Val Arg Asp Gly Pro Ala Ser Ser Ser
            315                 320                 325

cca ctg atc ggc gag tac cac ggc acc cag gca ccc cag ttc ctc atc      1362
Pro Leu Ile Gly Glu Tyr His Gly Thr Gln Ala Pro Gln Phe Leu Ile
        330                 335                 340

agc acc ggg aac ttc atg tac ctg cta ttc acc act gac aac agc cgc      1410
Ser Thr Gly Asn Phe Met Tyr Leu Leu Phe Thr Thr Asp Asn Ser Arg
    345                 350                 355

tcc agc atc ggc ttc ctc atc cac tat gag agt gtg acg ctt gag tcg      1458
Ser Ser Ile Gly Phe Leu Ile His Tyr Glu Ser Val Thr Leu Glu Ser
360                 365                 370                 375

gat tcc tgc ctg gac ccg ggc atc cct gtg aac grc cat cgc cac ggt      1506
Asp Ser Cys Leu Asp Pro Gly Ile Pro Val Asn Xaa His Arg His Gly
                380                 385                 390

gga gac ttt ggc atc agg tcc aca gtg act ttc agc tgt gac ccg ggg      1554
Gly Asp Phe Gly Ile Arg Ser Thr Val Thr Phe Ser Cys Asp Pro Gly
            395                 400                 405

tac aca cta agt gac gac gag ccc ctc gtc tgt gag agg aac cac cag      1602
Tyr Thr Leu Ser Asp Asp Glu Pro Leu Val Cys Glu Arg Asn His Gln
        410                 415                 420

tgg aac cac gcc ttg ccc agc tgc gac gct cta tgt gga ggc tac atc      1650
Trp Asn His Ala Leu Pro Ser Cys Asp Ala Leu Cys Gly Gly Tyr Ile
    425                 430                 435

caa ggg aag agt gga aca gtc ctt tct cct ggg ttt cca gat ttt tat      1698
Gln Gly Lys Ser Gly Thr Val Leu Ser Pro Gly Phe Pro Asp Phe Tyr
440                 445                 450                 455

cca aac tct cta aac ygc acg tgg acc att gaa gtg tct cat ggg aaa      1746
Pro Asn Ser Leu Asn Xaa Thr Trp Thr Ile Glu Val Ser His Gly Lys
                460                 465                 470

gga gtt caa atg atc ttt cac acc ttt cat ctt gag agt tcc cac gac      1794
Gly Val Gln Met Ile Phe His Thr Phe His Leu Glu Ser Ser His Asp
            475                 480                 485
```

```
tat tta ctg atc aca gag gat gga agt ttt tcc gag ccc gtt gcc agg      1842
Tyr Leu Leu Ile Thr Glu Asp Gly Ser Phe Ser Glu Pro Val Ala Arg
        490                 495                 500

ctc acc ggg tcg gtg ttg cct cat acg atc aag gca ggc ctg ttt gga      1890
Leu Thr Gly Ser Val Leu Pro His Thr Ile Lys Ala Gly Leu Phe Gly
    505                 510                 515

aac ttc act gcc cag ctt cgg ttt ata tca gac ttc tca att tcg tac      1938
Asn Phe Thr Ala Gln Leu Arg Phe Ile Ser Asp Phe Ser Ile Ser Tyr
520                 525                 530                 535

gag ggc ttc aat atc aca ttt tca gaa tat gac ctg gag cca tgt gat      1986
Glu Gly Phe Asn Ile Thr Phe Ser Glu Tyr Asp Leu Glu Pro Cys Asp
                540                 545                 550

gat cct gga gtc cct gcc ttc agc cga aga att ggt ttt cac ttt ggt      2034
Asp Pro Gly Val Pro Ala Phe Ser Arg Arg Ile Gly Phe His Phe Gly
            555                 560                 565

gtg gga gac tct ctg acg ttt tcc tgc ttc ctg gga tat cgt tta gaa      2082
Val Gly Asp Ser Leu Thr Phe Ser Cys Phe Leu Gly Tyr Arg Leu Glu
        570                 575                 580

ggt gcc rcc aag ctt acc tgc ctg ggt ggg ggc cgc cgt gtg tgg agt      2130
Gly Ala Xaa Lys Leu Thr Cys Leu Gly Gly Gly Arg Arg Val Trp Ser
    585                 590                 595

gca cct ctg cca agg tgt gtg gcc gaa tgt gga gca agt gtc aaa gga      2178
Ala Pro Leu Pro Arg Cys Val Ala Glu Cys Gly Ala Ser Val Lys Gly
600                 605                 610                 615

aat gaa gga aca tta ctg tct cca aat ttt cca tcc aat tat gat aat      2226
Asn Glu Gly Thr Leu Leu Ser Pro Asn Phe Pro Ser Asn Tyr Asp Asn
                620                 625                 630

aac cat gag tgt atc tat aaa ata gaa aca gaa gcc ggc aag ggc atc      2274
Asn His Glu Cys Ile Tyr Lys Ile Glu Thr Glu Ala Gly Lys Gly Ile
            635                 640                 645

cac ctt aga aca cga agc ttc cag ctg ttt gaa gga gat act cta aag      2322
His Leu Arg Thr Arg Ser Phe Gln Leu Phe Glu Gly Asp Thr Leu Lys
        650                 655                 660

gta tat gat gga aaa gac agt tcc tca cgt cca ctg ggc acg ttc act      2370
Val Tyr Asp Gly Lys Asp Ser Ser Ser Arg Pro Leu Gly Thr Phe Thr
    665                 670                 675

aaa aat gaa ctt ctg ggg ctg atc cta aac agc aca tcc aat cac ctr      2418
Lys Asn Glu Leu Leu Gly Leu Ile Leu Asn Ser Thr Ser Asn His Xaa
680                 685                 690                 695

tgg cta gag ttc aac acc aat gga tct gac acc gac caa ggt ttt caa      2466
Trp Leu Glu Phe Asn Thr Asn Gly Ser Asp Thr Asp Gln Gly Phe Gln
                700                 705                 710

ctc acc tat acc agt ttt gat ctg gta aaa tgt gag gat ccg ggc atc      2514
Leu Thr Tyr Thr Ser Phe Asp Leu Val Lys Cys Glu Asp Pro Gly Ile
            715                 720                 725
```

```
cct aac tac ggc tat agg atc cgt gat gaa ggc cac ttt acc gac act      2562
Pro Asn Tyr Gly Tyr Arg Ile Arg Asp Glu Gly His Phe Thr Asp Thr
        730                 735                 740

gta gtt ctg tac agt tgc aac ccg ggg tac gcc atg cat ggc agc aac      2610
Val Val Leu Tyr Ser Cys Asn Pro Gly Tyr Ala Met His Gly Ser Asn
    745                 750                 755

acc ctg acc tgt ttg agt gga gac agg aga gtg tgg gac aaa cca cta      2658
Thr Leu Thr Cys Leu Ser Gly Asp Arg Arg Val Trp Asp Lys Pro Leu
760                 765                 770                 775

cct tcg tgc ata gcg gaa tgt ggt ggt cag atc cat gca gcc aca tca      2706
Pro Ser Cys Ile Ala Glu Cys Gly Gly Gln Ile His Ala Ala Thr Ser
                780                 785                 790

gga cga ata ttg tcc cct ggc tat cca gct ccg tat gac aac aac ctc      2754
Gly Arg Ile Leu Ser Pro Gly Tyr Pro Ala Pro Tyr Asp Asn Asn Leu
            795                 800                 805

cac tgc acc tgg att ata gag gca gac cca gga aag acc att agc ctc      2802
His Cys Thr Trp Ile Ile Glu Ala Asp Pro Gly Lys Thr Ile Ser Leu
        810                 815                 820

cat ttc att gtt ttc gac acg gag atg gct cac gac atc ctc aag gtc      2850
His Phe Ile Val Phe Asp Thr Glu Met Ala His Asp Ile Leu Lys Val
    825                 830                 835

tgg gac ggg ccg gtg gac agt gac atc ctg ctg aag gag tgg agt ggc      2898
Trp Asp Gly Pro Val Asp Ser Asp Ile Leu Leu Lys Glu Trp Ser Gly
840                 845                 850                 855

tcc gcc ctt ccg gag gac atc cac agc acc ttc aac tca ctc acc ctg      2946
Ser Ala Leu Pro Glu Asp Ile His Ser Thr Phe Asn Ser Leu Thr Leu
                860                 865                 870

cag ttc gac agc gac ttc ttc atc agc aag tct ggc ttc tcc atc cag      2994
Gln Phe Asp Ser Asp Phe Phe Ile Ser Lys Ser Gly Phe Ser Ile Gln
            875                 880                 885

ttc tcc acc tca att gca gcc acc tgt aac gat cca ggt atg ccc caa      3042
Phe Ser Thr Ser Ile Ala Ala Thr Cys Asn Asp Pro Gly Met Pro Gln
        890                 895                 900

aat ggc acc cgc tat gga gac agc aga gag gct gga gac acc gtc aca      3090
Asn Gly Thr Arg Tyr Gly Asp Ser Arg Glu Ala Gly Asp Thr Val Thr
    905                 910                 915

ttc cag tgt gac cct ggc tat cag ctc caa gga caa gcc aaa atc acc      3138
Phe Gln Cys Asp Pro Gly Tyr Gln Leu Gln Gly Gln Ala Lys Ile Thr
920                 925                 930                 935

tgt gtg cag ctg aat aac cgg ttc ttt tgg caa cca gac cct cct aca      3186
Cys Val Gln Leu Asn Asn Arg Phe Phe Trp Gln Pro Asp Pro Pro Thr
                940                 945                 950

tgc ata gct gct tgt gga ggg aat ctg acg ggc cca gca ggt gtt att      3234
Cys Ile Ala Ala Cys Gly Gly Asn Leu Thr Gly Pro Ala Gly Val Ile
            955                 960                 965
```

ttg tca ccc aac tac cca cag ccg tat cct cct ggg aag gaa tgt gac 3282
Leu Ser Pro Asn Tyr Pro Gln Pro Tyr Pro Pro Gly Lys Glu Cys Asp
970 975 980

tgg aga gta aaa gtg aac ccg gac ttt gtc atc gcc ttg ata ttc aaa 3330
Trp Arg Val Lys Val Asn Pro Asp Phe Val Ile Ala Leu Ile Phe Lys
985 990 995

agt ttc aac atg gag ccc agc tat gac ttc cta cac atc tat gaa 3375
Ser Phe Asn Met Glu Pro Ser Tyr Asp Phe Leu His Ile Tyr Glu
1000 1005 1010

ggg gaa gat tcc aac agc ccc ctc att ggg agt tac cag ggc tct 3420
Gly Glu Asp Ser Asn Ser Pro Leu Ile Gly Ser Tyr Gln Gly Ser
1015 1020 1025

cag gcc cca gaa aga ata gag agt agc gga aac agc ctg ttt ctg 3465
Gln Ala Pro Glu Arg Ile Glu Ser Ser Gly Asn Ser Leu Phe Leu
1030 1035 1040

gca ttt cgg agt gat gcc tcc gtg ggc ctt tca ggg ttc gcc att 3510
Ala Phe Arg Ser Asp Ala Ser Val Gly Leu Ser Gly Phe Ala Ile
1045 1050 1055

gaa ttt aaa gag aaa cca cgg gaa gct tgt ttt gac cca gga aat 3555
Glu Phe Lys Glu Lys Pro Arg Glu Ala Cys Phe Asp Pro Gly Asn
1060 1065 1070

ata atg aat ggg aca aga gtt gga aca gac ttc aag ctt ggc tcc 3600
Ile Met Asn Gly Thr Arg Val Gly Thr Asp Phe Lys Leu Gly Ser
1075 1080 1085

acc atc acc tac cag tgt gac tct ggc tat aag att ctt gac ccc 3645
Thr Ile Thr Tyr Gln Cys Asp Ser Gly Tyr Lys Ile Leu Asp Pro
1090 1095 1100

tca tcc atc acc tgt gtg att ggg gct gat ggg aaa ccc tcc tgg 3690
Ser Ser Ile Thr Cys Val Ile Gly Ala Asp Gly Lys Pro Ser Trp
1105 1110 1115

gac caa gtg ctg ccc tcc tgc aat gct ccc tgt gga ggc cag tac 3735
Asp Gln Val Leu Pro Ser Cys Asn Ala Pro Cys Gly Gly Gln Tyr
1120 1125 1130

acg gga tca gaa ggg gta gtt tta tca cca aac tac ccc cat aat 3780
Thr Gly Ser Glu Gly Val Val Leu Ser Pro Asn Tyr Pro His Asn
1135 1140 1145

tac aca gct ggt caa ata tgc ctc tat tcc atc acg gta cca aag 3825
Tyr Thr Ala Gly Gln Ile Cys Leu Tyr Ser Ile Thr Val Pro Lys
1150 1155 1160

gaa ttc gtg gtc ttt gga cag ttt gcc tat ttc cag aca gcc ctg 3870
Glu Phe Val Val Phe Gly Gln Phe Ala Tyr Phe Gln Thr Ala Leu
1165 1170 1175

aat gat ttg gca gaa tta ttt gat gga acc cat gca cag gcc aga 3915
Asn Asp Leu Ala Glu Leu Phe Asp Gly Thr His Ala Gln Ala Arg
1180 1185 1190

ctt ctc agc tca ctc tcg ggg tct cac tca ggg gaa aca ttg ccc 3960
Leu Leu Ser Ser Leu Ser Gly Ser His Ser Gly Glu Thr Leu Pro
1195 1200 1205

```
ttg  gct acg tca aat caa  att ctg ctc cga ttc  agt gca aag agc      4005
Leu  Ala Thr Ser Asn Gln  Ile Leu Leu Arg Phe  Ser Ala Lys Ser
1210                 1215                 1220

ggt  gcc tct gcc cgc ggc  ttc cac ttc gtg tat  caa gct gtt cct      4050
Gly  Ala Ser Ala Arg Gly  Phe His Phe Val Tyr  Gln Ala Val Pro
1225                 1230                 1235

cgt  acc agt gac acc caa  tgc agc tct gtc ccc  gag ccc aga tac      4095
Arg  Thr Ser Asp Thr Gln  Cys Ser Ser Val Pro  Glu Pro Arg Tyr
1240                 1245                 1250

gga  agg aga att ggt tct  gag ttt tct gcc ggc  tcc atc gtc cga      4140
Gly  Arg Arg Ile Gly Ser  Glu Phe Ser Ala Gly  Ser Ile Val Arg
1255                 1260                 1265

ttc  gag trc aac ccg gga  tac ctg ctt cag ggt  tcc acg gcg ctc      4185
Phe  Glu Xaa Asn Pro Gly  Tyr Leu Leu Gln Gly  Ser Thr Ala Leu
1270                 1275                 1280

cac  tgc cag tcc gtg ccc  aac gcc ttg gca cag  tgg aac gac acg      4230
His  Cys Gln Ser Val Pro  Asn Ala Leu Ala Gln  Trp Asn Asp Thr
1285                 1290                 1295

atc  ccc agc tgt gtg gta  ccc tgc agt ggc aat  ttc act caa cga      4275
Ile  Pro Ser Cys Val Val  Pro Cys Ser Gly Asn  Phe Thr Gln Arg
1300                 1305                 1310

aga  ggt aca atc ctg tcc  ccc ggc tac cct gag  cca tac gga aac      4320
Arg  Gly Thr Ile Leu Ser  Pro Gly Tyr Pro Glu  Pro Tyr Gly Asn
1315                 1320                 1325

aac  ttg aac tgt ata tgg  aag atc ata gtt acg  gag ggc tcg gga      4365
Asn  Leu Asn Cys Ile Trp  Lys Ile Ile Val Thr  Glu Gly Ser Gly
1330                 1335                 1340

att  cag atc caa gtg atc  agt ttt gcc acg gag  cag aac tgg gac      4410
Ile  Gln Ile Gln Val Ile  Ser Phe Ala Thr Glu  Gln Asn Trp Asp
1345                 1350                 1355

tcc  ctt gag atc cac gat  ggt ggg gat gtg acc  gca ccc aga ctg      4455
Ser  Leu Glu Ile His Asp  Gly Gly Asp Val Thr  Ala Pro Arg Leu
1360                 1365                 1370

gga  agc ttc tca ggc acc  aca gta ccg gca ctg  ctg aac agt act      4500
Gly  Ser Phe Ser Gly Thr  Thr Val Pro Ala Leu  Leu Asn Ser Thr
1375                 1380                 1385

tcc  aac caa ctc tac ctg  cat ttc cag tct gac  att agt gtg gca      4545
Ser  Asn Gln Leu Tyr Leu  His Phe Gln Ser Asp  Ile Ser Val Ala
1390                 1395                 1400

gct  gct ggt ttc cac ctg  gaa tac aaa act gta  ggt ctt gct gca      4590
Ala  Ala Gly Phe His Leu  Glu Tyr Lys Thr Val  Gly Leu Ala Ala
1405                 1410                 1415

tgc  caa gaa cca gcc ctc  ccc agc aac agc atc  aaa atc gga gat      4635
Cys  Gln Glu Pro Ala Leu  Pro Ser Asn Ser Ile  Lys Ile Gly Asp
1420                 1425                 1430

cgg  tac atg gtg aac gac  gtg ctc tcc ttc cag  tgc gag ccc ggg      4680
Arg  Tyr Met Val Asn Asp  Val Leu Ser Phe Gln  Cys Glu Pro Gly
1435                 1440                 1445
```

```
tac acc ctg cag ggc cgt tcc cac att tcc tgt atg cca ggg acc      4725
Tyr Thr Leu Gln Gly Arg Ser His Ile Ser Cys Met Pro Gly Thr
1450                1455                1460

gtt cgc cgt tgg aac tat ccg tct ccc ctg tgc att gca acc tgt      4770
Val Arg Arg Trp Asn Tyr Pro Ser Pro Leu Cys Ile Ala Thr Cys
1465                1470                1475

gga ggg acg ctg agc acc ttg ggt ggt gtg atc ctg agc ccc ggc      4815
Gly Gly Thr Leu Ser Thr Leu Gly Gly Val Ile Leu Ser Pro Gly
1480                1485                1490

ttc cca ggt tct tac ccc aac aac tta gac tgc acc tgg agg atc      4860
Phe Pro Gly Ser Tyr Pro Asn Asn Leu Asp Cys Thr Trp Arg Ile
1495                1500                1505

tca tta ccc atc ggc tat ggt gca cat att cag ttt ctg aat ttt      4905
Ser Leu Pro Ile Gly Tyr Gly Ala His Ile Gln Phe Leu Asn Phe
1510                1515                1520

tct acc gaa gct aat cat gac ttc ctt gaa att caa aat gga cct      4950
Ser Thr Glu Ala Asn His Asp Phe Leu Glu Ile Gln Asn Gly Pro
1525                1530                1535

tac cac acc agc ccc atg att gga caa ttt agc ggc acg gat ctc      4995
Tyr His Thr Ser Pro Met Ile Gly Gln Phe Ser Gly Thr Asp Leu
1540                1545                1550

ccc gcg gcc ctg ctg agc aca acg cat gaa acc ctc atc cac ttt      5040
Pro Ala Ala Leu Leu Ser Thr Thr His Glu Thr Leu Ile His Phe
1555                1560                1565

tat agt gac cat tcg caa aac cgg caa gga ttt aaa ctt gct tac      5085
Tyr Ser Asp His Ser Gln Asn Arg Gln Gly Phe Lys Leu Ala Tyr
1570                1575                1580

caa gcc tat gaa tta cag aac tgt cca gat cca ccc cca ttt cag      5130
Gln Ala Tyr Glu Leu Gln Asn Cys Pro Asp Pro Pro Pro Phe Gln
1585                1590                1595

aat ggg tac atg atc aac tcg gat tac agc gtg ggg caa tca gta      5175
Asn Gly Tyr Met Ile Asn Ser Asp Tyr Ser Val Gly Gln Ser Val
1600                1605                1610

tct ttc gag tgt tat cct ggg tac att cta ata ggc cat cct gtc      5220
Ser Phe Glu Cys Tyr Pro Gly Tyr Ile Leu Ile Gly His Pro Val
1615                1620                1625

ctc act tgt cag cat ggg atc aac aga aac tgg aac tac cct ttt      5265
Leu Thr Cys Gln His Gly Ile Asn Arg Asn Trp Asn Tyr Pro Phe
1630                1635                1640

cca aga tgt gat gcc cct tgt ggg tac aac gta act tct cag aac      5310
Pro Arg Cys Asp Ala Pro Cys Gly Tyr Asn Val Thr Ser Gln Asn
1645                1650                1655

ggc acc atc tac tcc cct ggc ttt cct gat gag tat ccg atc ctg      5355
Gly Thr Ile Tyr Ser Pro Gly Phe Pro Asp Glu Tyr Pro Ile Leu
1660                1665                1670

aag gac tgc att tgg ctc atc acg gtg cct cca ggg cac gga gtt      5400
Lys Asp Cys Ile Trp Leu Ile Thr Val Pro Pro Gly His Gly Val
1675                1680                1685
```

```
tac  atc aac ttc acc ctg  tta cag acg gaa gct  gtc aac gat tac       5445
Tyr  Ile Asn Phe Thr Leu  Leu Gln Thr Glu Ala  Val Asn Asp Tyr
1690                 1695                 1700

att  gct gtt tgg gac ggt  ccc gat cag aac tca  ccc cag ctg gga       5490
Ile  Ala Val Trp Asp Gly  Pro Asp Gln Asn Ser  Pro Gln Leu Gly
1705                 1710                 1715

gtt  ttc agt ggc aac aca  gcc ctc gaa acg gcg  tat agc tcc acc       5535
Val  Phe Ser Gly Asn Thr  Ala Leu Glu Thr Ala  Tyr Ser Ser Thr
1720                 1725                 1730

aac  caa gtc ctg ctc aag  ttc cac agc gac ttt  tca aat gga ggc       5580
Asn  Gln Val Leu Leu Lys  Phe His Ser Asp Phe  Ser Asn Gly Gly
1735                 1740                 1745

ttc  ttt gtc ctc aat ttc  cac gca ttt cag ctc  aag aaa tgt caa       5625
Phe  Phe Val Leu Asn Phe  His Ala Phe Gln Leu  Lys Lys Cys Gln
1750                 1755                 1760

cct  ccc cca gcg gtt cca  cag gca gaa atg ctt  act gag gat gat       5670
Pro  Pro Pro Ala Val Pro  Gln Ala Glu Met Leu  Thr Glu Asp Asp
1765                 1770                 1775

gat  ttc gag ata gga gat  ttt gtg aag tac cag  tgc cac ccc ggg       5715
Asp  Phe Glu Ile Gly Asp  Phe Val Lys Tyr Gln  Cys His Pro Gly
1780                 1785                 1790

tac  acc ttg gtg ggg acc  gac att ctg act tgc  aag ctc agt tcc       5760
Tyr  Thr Leu Val Gly Thr  Asp Ile Leu Thr Cys  Lys Leu Ser Ser
1795                 1800                 1805

cag  ttg cag ttt gag ggt  tct ctc cca aca tgt  gaa gca caa tgc       5805
Gln  Leu Gln Phe Glu Gly  Ser Leu Pro Thr Cys  Glu Ala Gln Cys
1810                 1815                 1820

cca  gca aat gaa gtc cgg  act gga tca tcg gga  gtc att ctc agt       5850
Pro  Ala Asn Glu Val Arg  Thr Gly Ser Ser Gly  Val Ile Leu Ser
1825                 1830                 1835

cca  ggg tat ccg ggt aat  tat ttt aac tcc cag  act tgc tct tgg       5895
Pro  Gly Tyr Pro Gly Asn  Tyr Phe Asn Ser Gln  Thr Cys Ser Trp
1840                 1845                 1850

agt  att aaa gtg gaa cca  aac tac aac att acc  atc ttt gtg gac       5940
Ser  Ile Lys Val Glu Pro  Asn Tyr Asn Ile Thr  Ile Phe Val Asp
1855                 1860                 1865

aca  ttt caa agt gaa aag  cag ttt gat gca ctg  gaa gtg ttt gat       5985
Thr  Phe Gln Ser Glu Lys  Gln Phe Asp Ala Leu  Glu Val Phe Asp
1870                 1875                 1880

ggt  tct tct ggg caa agt  cct ctg cta gta gtc  tta agt ggg aat       6030
Gly  Ser Ser Gly Gln Ser  Pro Leu Leu Val Val  Leu Ser Gly Asn
1885                 1890                 1895

cat  act gaa caa tca aat  ttt aca agc agg agt  aat cag tta tat       6075
His  Thr Glu Gln Ser Asn  Phe Thr Ser Arg Ser  Asn Gln Leu Tyr
1900                 1905                 1910

ctc  cgc tgg tcc act gac  cat gcc acc agt aag  aaa gga ttc aag       6120
Leu  Arg Trp Ser Thr Asp  His Ala Thr Ser Lys  Lys Gly Phe Lys
1915                 1920                 1925
```

```
att cgc tat gca gca cct tac tgc agt ttg acc cac ccc ctg aag    6165
Ile Arg Tyr Ala Ala Pro Tyr Cys Ser Leu Thr His Pro Leu Lys
1930                1935                1940

aat ggg ggt att cta aac agg act gca gga gcg gtt gga agc aaa    6210
Asn Gly Gly Ile Leu Asn Arg Thr Ala Gly Ala Val Gly Ser Lys
1945                1950                1955

gtg cat tat ttt tgc aag cct gga tac cga atg gtc ggc cac agc    6255
Val His Tyr Phe Cys Lys Pro Gly Tyr Arg Met Val Gly His Ser
1960                1965                1970

aat gca acc tgt aga cga aac cca ctt ggc atg tac cag tgg gac    6300
Asn Ala Thr Cys Arg Arg Asn Pro Leu Gly Met Tyr Gln Trp Asp
1975                1980                1985

tcc ctc acg cca ctc tgc cag gct gtg tcc tgt gga atc cca gaa    6345
Ser Leu Thr Pro Leu Cys Gln Ala Val Ser Cys Gly Ile Pro Glu
1990                1995                2000

tcc cca gga aac ggt tca ttt acc ggg aac gag ttc act ttg gac    6390
Ser Pro Gly Asn Gly Ser Phe Thr Gly Asn Glu Phe Thr Leu Asp
2005                2010                2015

agt aaa gtg gtc tat gaa tgt cat gag ggc ttc aag ctt gaa tcc    6435
Ser Lys Val Val Tyr Glu Cys His Glu Gly Phe Lys Leu Glu Ser
2020                2025                2030

agc cag caa gca aca gcc gtg tgt caa gaa gat ggg ctg tgg agt    6480
Ser Gln Gln Ala Thr Ala Val Cys Gln Glu Asp Gly Leu Trp Ser
2035                2040                2045

aac aag ggg aag ccg ccc acg tgt aag ccg gtc gct tgc ccc agc    6525
Asn Lys Gly Lys Pro Pro Thr Cys Lys Pro Val Ala Cys Pro Ser
2050                2055                2060

att gaa gct cag ctc tca gaa cat gtc atc tgg agg ctg gtt tca    6570
Ile Glu Ala Gln Leu Ser Glu His Val Ile Trp Arg Leu Val Ser
2065                2070                2075

gga tcc ttg aat gag tac ggt gct caa gta ttg ctg agc tgc agt    6615
Gly Ser Leu Asn Glu Tyr Gly Ala Gln Val Leu Leu Ser Cys Ser
2080                2085                2090

cct ggt tac tac tta gaa ggc tgg agg ctc ctg cgg tgc cag gcc    6660
Pro Gly Tyr Tyr Leu Glu Gly Trp Arg Leu Leu Arg Cys Gln Ala
2095                2100                2105

aat ggg acg tgg aac ata gga gat gag agg cca agc tgt cga gtt    6705
Asn Gly Thr Trp Asn Ile Gly Asp Glu Arg Pro Ser Cys Arg Val
2110                2115                2120

atc tcg tgt gga agc ctt tcc ttt ccc cca aat ggc aac aag att    6750
Ile Ser Cys Gly Ser Leu Ser Phe Pro Pro Asn Gly Asn Lys Ile
2125                2130                2135

gga acg ttg aca gtt tat ggg gcc aca gct ata ttt acg tgc aac    6795
Gly Thr Leu Thr Val Tyr Gly Ala Thr Ala Ile Phe Thr Cys Asn
2140                2145                2150

acc ggc tac acg ctt gtg ggg tct cat gtc aga gag tgc ttg gca    6840
Thr Gly Tyr Thr Leu Val Gly Ser His Val Arg Glu Cys Leu Ala
2155                2160                2165
```

```
aat  ggg ctc tgg agc ggc  agc gaa act cga tgt  ctg gct ggc cac     6885
Asn  Gly Leu Trp Ser Gly  Ser Glu Thr Arg Cys  Leu Ala Gly His
2170                2175                 2180

tgc  ggt tcc cca gac ccg  att gtg aac ggt cac  att agt gga gat     6930
Cys  Gly Ser Pro Asp Pro  Ile Val Asn Gly His  Ile Ser Gly Asp
2185                2190                 2195

ggc  ttc agt tac aga gac  acg gtg gtt tac cag  tgc aat cct ggt     6975
Gly  Phe Ser Tyr Arg Asp  Thr Val Val Tyr Gln  Cys Asn Pro Gly
2200                2205                 2210

ttc  cgg ctt gtg gga act  tcc gtg agg ata tgc  ctg caa gac cac     7020
Phe  Arg Leu Val Gly Thr  Ser Val Arg Ile Cys  Leu Gln Asp His
2215                2220                 2225

aag  tgg tct gga caa acg  cct gtc tgt gtc ccc  atc aca tgt ggt     7065
Lys  Trp Ser Gly Gln Thr  Pro Val Cys Val Pro  Ile Thr Cys Gly
2230                2235                 2240

cac  cct gga aac cct gcc  cac gga ttc act aat  ggc agt gag ttc     7110
His  Pro Gly Asn Pro Ala  His Gly Phe Thr Asn  Gly Ser Glu Phe
2245                2250                 2255

aac  ctg aat gat gtc gtg  aat ttc acc tgc aac  acg ggc tat ttg     7155
Asn  Leu Asn Asp Val Val  Asn Phe Thr Cys Asn  Thr Gly Tyr Leu
2260                2265                 2270

ctg  cag ggc gtg tct cga  gcc cag tgt cgg agc  aac ggc cag tgg     7200
Leu  Gln Gly Val Ser Arg  Ala Gln Cys Arg Ser  Asn Gly Gln Trp
2275                2280                 2285

agt  agc cct ctg ccc acg  tgt cga gtg gtg aac  tgt tct gat cca     7245
Ser  Ser Pro Leu Pro Thr  Cys Arg Val Val Asn  Cys Ser Asp Pro
2290                2295                 2300

ggc  ttt gtg gaa aat gcc  att cgt cac ggg caa  cag aac ttc cct     7290
Gly  Phe Val Glu Asn Ala  Ile Arg His Gly Gln  Gln Asn Phe Pro
2305                2310                 2315

gag  agt ttt gag tat gga  atg agt atc ctg tac  cat tgc aag aag     7335
Glu  Ser Phe Glu Tyr Gly  Met Ser Ile Leu Tyr  His Cys Lys Lys
2320                2325                 2330

gga  ttt tac ttg ctg gga  tct tca gcc ttg acc  tgt atg gca aat     7380
Gly  Phe Tyr Leu Leu Gly  Ser Ser Ala Leu Thr  Cys Met Ala Asn
2335                2340                 2345

ggc  tta tgg gac cga tcc  ctg ccc aag tgt ttg  gct ata tcg tgt     7425
Gly  Leu Trp Asp Arg Ser  Leu Pro Lys Cys Leu  Ala Ile Ser Cys
2350                2355                 2360

gga  cac cca ggg gtc cct  gcc aac gcc gtc ctc  act gga gag ctg     7470
Gly  His Pro Gly Val Pro  Ala Asn Ala Val Leu  Thr Gly Glu Leu
2365                2370                 2375

ttt  acc tat ggc gcc gtc  gtg cac tac tcc tgc  aga ggg agc gag     7515
Phe  Thr Tyr Gly Ala Val  Val His Tyr Ser Cys  Arg Gly Ser Glu
2380                2385                 2390

agc  ctc ata ggc aac gac  acg aga gtg tgc cag  gaa gac agt cac     7560
Ser  Leu Ile Gly Asn Asp  Thr Arg Val Cys Gln  Glu Asp Ser His
2395                2400                 2405
```

```
tgg  agc ggg gca ctg ccc  cac tgc aca gga aat  aat cct gga ttc       7605
Trp  Ser Gly Ala Leu Pro  His Cys Thr Gly Asn  Asn Pro Gly Phe
2410                 2415                 2420

tgt  ggt gat ccg ggg acc  cca gca cat ggg tct  cgg ctt ggt gat       7650
Cys  Gly Asp Pro Gly Thr  Pro Ala His Gly Ser  Arg Leu Gly Asp
2425                 2430                 2435

gac  ttt aag aca aag agt  ctt ctc cgc ttc tcc  tgt gaa atg ggg       7695
Asp  Phe Lys Thr Lys Ser  Leu Leu Arg Phe Ser  Cys Glu Met Gly
2440                 2445                 2450

cac  cag ctg agg ggc tcc  cct gaa cgc acg tgt  ttg ctc aat ggg       7740
His  Gln Leu Arg Gly Ser  Pro Glu Arg Thr Cys  Leu Leu Asn Gly
2455                 2460                 2465

tca  tgg tca gga ctg cag  ccg gtg tgt gag gcc  gtg tcc tgt ggc       7785
Ser  Trp Ser Gly Leu Gln  Pro Val Cys Glu Ala  Val Ser Cys Gly
2470                 2475                 2480

aac  cct ggc aca ccc acc  aac gga atg att gtc  agt agt gat ggc       7830
Asn  Pro Gly Thr Pro Thr  Asn Gly Met Ile Val  Ser Ser Asp Gly
2485                 2490                 2495

att  ctg ttc tcc agc tcg  gtc atc tat gcc tgc  tgg gaa ggc tac       7875
Ile  Leu Phe Ser Ser Ser  Val Ile Tyr Ala Cys  Trp Glu Gly Tyr
2500                 2505                 2510

aag  acc tca ggg ctc atg  aca cgg cat tgc aca  gcc aat ggg acc       7920
Lys  Thr Ser Gly Leu Met  Thr Arg His Cys Thr  Ala Asn Gly Thr
2515                 2520                 2525

tgg  aca ggc act gct ccc  gac tgc aca att ata  agt tgt ggg gat       7965
Trp  Thr Gly Thr Ala Pro  Asp Cys Thr Ile Ile  Ser Cys Gly Asp
2530                 2535                 2540

cca  ggc aca cta gca aat  ggc atc cag ttt ggg  acc gac ttc acc       8010
Pro  Gly Thr Leu Ala Asn  Gly Ile Gln Phe Gly  Thr Asp Phe Thr
2545                 2550                 2555

ttc  aac aag act gtg agc  tat cag tgt aac cca  ggc tat gtc atg       8055
Phe  Asn Lys Thr Val Ser  Tyr Gln Cys Asn Pro  Gly Tyr Val Met
2560                 2565                 2570

gaa  gca gtc aca tcc gcc  act att cgc tgt acc  aaa gac ggc agg       8100
Glu  Ala Val Thr Ser Ala  Thr Ile Arg Cys Thr  Lys Asp Gly Arg
2575                 2580                 2585

tgg  aat ccg agc aaa cct  gtc tgc aaa gcc gtg  ctg tgt cct cag       8145
Trp  Asn Pro Ser Lys Pro  Val Cys Lys Ala Val  Leu Cys Pro Gln
2590                 2595                 2600

ccg  ccg ccg gtg cag aat  gga aca gtg gag gga  agt gat ttc cgc       8190
Pro  Pro Pro Val Gln Asn  Gly Thr Val Glu Gly  Ser Asp Phe Arg
2605                 2610                 2615

tgg  ggc tcc agc ata agt  tac agc tgc atg gac  ggt tac cag ctc       8235
Trp  Gly Ser Ser Ile Ser  Tyr Ser Cys Met Asp  Gly Tyr Gln Leu
2620                 2625                 2630

tct  cac tcc gcc atc ctc  tcc tgt gaa ggt cgc  ggg gtg tgg aaa       8280
Ser  His Ser Ala Ile Leu  Ser Cys Glu Gly Arg  Gly Val Trp Lys
2635                 2640                 2645
```

```
gga gag atc ccc cag tgt ctc cct gtg ttc tgc gga gac cct ggc      8325
Gly Glu Ile Pro Gln Cys Leu Pro Val Phe Cys Gly Asp Pro Gly
2650                2655                2660

atc ccc gca gaa ggg cga ctt agt ggg aaa agt ttc acc tat aag      8370
Ile Pro Ala Glu Gly Arg Leu Ser Gly Lys Ser Phe Thr Tyr Lys
2665                2670                2675

tcc gaa gtc ttc ttc cag tgc aaa tct cca ttt ata ctc gtg gga      8415
Ser Glu Val Phe Phe Gln Cys Lys Ser Pro Phe Ile Leu Val Gly
2680                2685                2690

tcc tcc aga aga gtc tgc caa gct gac ggc acg tgg agc ggc ata      8460
Ser Ser Arg Arg Val Cys Gln Ala Asp Gly Thr Trp Ser Gly Ile
2695                2700                2705

caa ccc acc tgc att gat cct gct cat aac acc tgc cca gac cct      8505
Gln Pro Thr Cys Ile Asp Pro Ala His Asn Thr Cys Pro Asp Pro
2710                2715                2720

ggt acg cca cac ttt gga ata cag aat agc tcc aga ggc tat gag      8550
Gly Thr Pro His Phe Gly Ile Gln Asn Ser Ser Arg Gly Tyr Glu
2725                2730                2735

gtt gga agc acg gtt ttt ttc agg tgc aga aaa ggc tac cat att      8595
Val Gly Ser Thr Val Phe Phe Arg Cys Arg Lys Gly Tyr His Ile
2740                2745                2750

caa ggt tcc acg act cgc acc tgc ctt gcc aat tta aca tgg agt      8640
Gln Gly Ser Thr Thr Arg Thr Cys Leu Ala Asn Leu Thr Trp Ser
2755                2760                2765

ggg ata cag acc gaa tgt ata cct cat gcc tgc aga cag cca gaa      8685
Gly Ile Gln Thr Glu Cys Ile Pro His Ala Cys Arg Gln Pro Glu
2770                2775                2780

acc ccg gca cac gcg gat gtg aga gcc atc gat ctt cct act ttc      8730
Thr Pro Ala His Ala Asp Val Arg Ala Ile Asp Leu Pro Thr Phe
2785                2790                2795

ggc tac acc tta gtg tac acc tgc cat cca ggc ttt ttc ctc gca      8775
Gly Tyr Thr Leu Val Tyr Thr Cys His Pro Gly Phe Phe Leu Ala
2800                2805                2810

ggg gga tct gag cac aga aca tgt aaa gca gac atg aaa tgg aca      8820
Gly Gly Ser Glu His Arg Thr Cys Lys Ala Asp Met Lys Trp Thr
2815                2820                2825

gga aag tcg cct gtg tgt aaa agt aaa gga gtg aga gaa gtt aat      8865
Gly Lys Ser Pro Val Cys Lys Ser Lys Gly Val Arg Glu Val Asn
2830                2835                2840

gaa aca gtt act aaa act cca gtt cct tca gat gtc ttt ttc gtc      8910
Glu Thr Val Thr Lys Thr Pro Val Pro Ser Asp Val Phe Phe Val
2845                2850                2855

aat tca ctg tgg aag ggg tat tat gaa tat tta ggg aaa aga caa      8955
Asn Ser Leu Trp Lys Gly Tyr Tyr Glu Tyr Leu Gly Lys Arg Gln
2860                2865                2870

ccc gcc act cta act gtt gac tgg ttc aat gca aca agc agt aag      9000
Pro Ala Thr Leu Thr Val Asp Trp Phe Asn Ala Thr Ser Ser Lys
2875                2880                2885
```

```
gtg  aat gcc acc ttc agc  gaa gcc tcg cca gtg  gag ctg aag ttg      9045
Val  Asn Ala Thr Phe Ser  Glu Ala Ser Pro Val  Glu Leu Lys Leu
2890                 2895                 2900

aca  ggc att tac aag aag  gag gag gcc cac tta  ctc ctg aaa gct      9090
Thr  Gly Ile Tyr Lys Lys  Glu Glu Ala His Leu  Leu Leu Lys Ala
2905                 2910                 2915

ttt  caa att aaa ggc cag  gca gat att ttt gta  agc aag ttc gaa      9135
Phe  Gln Ile Lys Gly Gln  Ala Asp Ile Phe Val  Ser Lys Phe Glu
2920                 2925                 2930

aat  gac aac tgg gga cta  gat ggt tat gtg tca  tct gga ctt gaa      9180
Asn  Asp Asn Trp Gly Leu  Asp Gly Tyr Val Ser  Ser Gly Leu Glu
2935                 2940                 2945

aga  gga gga ttt act ttt  caa ggt gac att cat  gga aaa gac ttt      9225
Arg  Gly Gly Phe Thr Phe  Gln Gly Asp Ile His  Gly Lys Asp Phe
2950                 2955                 2960

gga  aaa ttt aag cta gaa  agg caa gat cct tta  aac cca gat caa      9270
Gly  Lys Phe Lys Leu Glu  Arg Gln Asp Pro Leu  Asn Pro Asp Gln
2965                 2970                 2975

gac  tct tcc agt cat tac  cac ggc acc agc agt  ggc tct gtg gcg      9315
Asp  Ser Ser Ser His Tyr  His Gly Thr Ser Ser  Gly Ser Val Ala
2980                 2985                 2990

gct  gcc att ctg gtt cct  ttc ttt gct cta att  tta tca ggg ttt      9360
Ala  Ala Ile Leu Val Pro  Phe Phe Ala Leu Ile  Leu Ser Gly Phe
2995                 3000                 3005

gca  ttt tac ctc tac aaa  cac aga acg aga cca  aaa gtt caa tac      9405
Ala  Phe Tyr Leu Tyr Lys  His Arg Thr Arg Pro  Lys Val Gln Tyr
3010                 3015                 3020

aat  ggc tat gct ggg cat  gaa aac agc aat gga  caa gca tcg ttt      9450
Asn  Gly Tyr Ala Gly His  Glu Asn Ser Asn Gly  Gln Ala Ser Phe
3025                 3030                 3035

gaa  aac ccc atg tat gat  aca aac tta aaa ccc  aca gaa gcc aag      9495
Glu  Asn Pro Met Tyr Asp  Thr Asn Leu Lys Pro  Thr Glu Ala Lys
3040                 3045                 3050

gct  gtg agg ttt gac aca  act ctg aac aca gtc  tgt aca gtg gta      9540
Ala  Val Arg Phe Asp Thr  Thr Leu Asn Thr Val  Cys Thr Val Val
3055                 3060                 3065

tagccctcag tgccccaaca ggactgattc atagccatac ctctgatgga caagcagtga   9600
ttcctttggt gccatatacc actctcccyt ccactctggc tttactgcag cgatcttcaa   9660
ccttgtctac tggcataagt gcagcgggga tctctactca aatgtgtcag ggtcttctac   9720
ggatcaaact acacatgcgt tttcattcca aaagtgggtt ctaaatgcct ggctgcatct   9780
gtatgaaatc aaggcacact ccaggaagac tgccacgtcg cgccaacacg tcatactcaa   9840
trcctcagac tttcatattt ctgtgttgct gagatgcctt tcaatgcaat cgtctgggct   9900
cgtggatatg tccctcaggt gcggtgacag aatggtggca ccacgatatg tgttctcttg   9960
tgttgttttt ccttttttaaa cccccatgaa cacgaatact ctgaaaaaaa taaaaagctt  10020
```

tctggaagaa gacacctttc tgatagaggc tcacacctac aaatgcttca ctctgtcctt 10080

ccgagacctg acaagctttg aggacctcac agctcccctg tgtgttcatc tctagggatg 10140

tttgcaattt cccagtcagc tgttctgtcg cagaatgttt aatgcacaat tttttgcact 10200

agtgtgttat gaatgactaa gattctgata aaaaaaataa attatttaca cagggtttat 10260

acacactatc cattgtatat aagcattatt tcatattatc aagctaaaca ttcccccatc 10320

agcttagttg gagtgttagg gaaaagtatt cctagatatg gcacagattt taaaaggaaa 10380

tacagtattg acgagattta ttttattatt gcttcaatta gctccattta cgtgttgaat 10440

tcattgaaga ggtccaatga gaaaaaaaca gaagcctcct tatttcacac gttttcctcc 10500

tttagtacca tcctcatcca attactgtct ctctgatact acttaatagc aggggtttg 10560

cagaaatttc tgtttgccat gtaaaactgt gaatagtaat ttattttaga tagtcgatga 10620

acttgtgggt tttagctcac aatgcagcct tccctttgc agtgtttttt ttt 10673

<210> 2

<211> 3069

<212> PRT

<213> Homo sapiens

<400> 2

```
Met Ser Asn Gln Met Trp Leu His Leu Gln Ser Asp Asp Ser Ile Gly
1               5                   10                  15

Ser Pro Gly Phe Lys Ala Val Tyr Gln Glu Ile Glu Lys Gly Gly Cys
            20                  25                  30

Gly Asp Pro Gly Ile Pro Ala Tyr Gly Lys Arg Thr Gly Ser Ser Phe
        35                  40                  45

Leu His Gly Asp Thr Leu Thr Phe Glu Cys Pro Ala Ala Phe Glu Leu
    50                  55                  60

Val Gly Glu Arg Val Ile Thr Cys Gln Gln Asn Asn Gln Trp Ser Gly
65                  70                  75                  80

Asn Lys Pro Ser Cys Val Phe Ser Cys Phe Phe Asn Phe Thr Ala Ser
                85                  90                  95

Ser Gly Ile Ile Leu Ser Pro Asn Tyr Pro Glu Glu Tyr Gly Asn Asn
            100                 105                 110
```

```
Met Asn Cys Val Trp Leu Ile Ile Ser Glu Pro Gly Ser Arg Ile His
        115                 120                 125

Leu Ile Phe Asn Asp Phe Asp Val Glu Pro Gln Phe Asp Phe Leu Ala
    130                 135                 140

Val Lys Asp Asp Gly Ile Ser Asp Ile Thr Val Leu Gly Thr Phe Ser
145                 150                 155                 160

Gly Asn Glu Val Pro Ser Gln Leu Ala Ser Ser Gly His Ile Val Arg
                165                 170                 175

Leu Glu Phe Gln Ser Asp His Ser Thr Thr Gly Arg Gly Phe Asn Ile
            180                 185                 190

Thr Tyr Thr Thr Phe Gly Gln Asn Glu Cys His Asp Pro Gly Ile Pro
        195                 200                 205

Ile Asn Gly Arg Arg Phe Gly Asp Arg Phe Leu Leu Gly Ser Ser Val
    210                 215                 220

Ser Phe His Cys Asp Asp Gly Phe Val Lys Thr Gln Gly Ser Glu Ser
225                 230                 235                 240

Ile Thr Cys Ile Leu Gln Asp Gly Asn Val Val Trp Ser Ser Thr Val
                245                 250                 255

Pro Arg Cys Glu Ala Pro Cys Gly Gly His Leu Thr Ala Ser Ser Gly
            260                 265                 270

Val Ile Leu Pro Pro Gly Trp Pro Gly Tyr Tyr Lys Asp Ser Leu His
        275                 280                 285

Cys Glu Trp Ile Ile Glu Ala Lys Pro Gly His Ser Ile Lys Ile Thr
    290                 295                 300

Phe Asp Arg Phe Gln Thr Glu Val Asn Tyr Asp Thr Leu Glu Val Arg
305                 310                 315                 320

Asp Gly Pro Ala Ser Ser Ser Pro Leu Ile Gly Glu Tyr His Gly Thr
                325                 330                 335

Gln Ala Pro Gln Phe Leu Ile Ser Thr Gly Asn Phe Met Tyr Leu Leu
            340                 345                 350

Phe Thr Thr Asp Asn Ser Arg Ser Ser Ile Gly Phe Leu Ile His Tyr
        355                 360                 365
```

Glu Ser Val Thr Leu Glu Ser Asp Ser Cys Leu Asp Pro Gly Ile Pro
370 375 380

Val Asn Xaa His Arg His Gly Gly Asp Phe Gly Ile Arg Ser Thr Val
385 390 395 400

Thr Phe Ser Cys Asp Pro Gly Tyr Thr Leu Ser Asp Asp Glu Pro Leu
405 410 415

Val Cys Glu Arg Asn His Gln Trp Asn His Ala Leu Pro Ser Cys Asp
420 425 430

Ala Leu Cys Gly Gly Tyr Ile Gln Gly Lys Ser Gly Thr Val Leu Ser
435 440 445

Pro Gly Phe Pro Asp Phe Tyr Pro Asn Ser Leu Asn Xaa Thr Trp Thr
450 455 460

Ile Glu Val Ser His Gly Lys Gly Val Gln Met Ile Phe His Thr Phe
465 470 475 480

His Leu Glu Ser Ser His Asp Tyr Leu Leu Ile Thr Glu Asp Gly Ser
485 490 495

Phe Ser Glu Pro Val Ala Arg Leu Thr Gly Ser Val Leu Pro His Thr
500 505 510

Ile Lys Ala Gly Leu Phe Gly Asn Phe Thr Ala Gln Leu Arg Phe Ile
515 520 525

Ser Asp Phe Ser Ile Ser Tyr Glu Gly Phe Asn Ile Thr Phe Ser Glu
530 535 540

Tyr Asp Leu Glu Pro Cys Asp Asp Pro Gly Val Pro Ala Phe Ser Arg
545 550 555 560

Arg Ile Gly Phe His Phe Gly Val Gly Asp Ser Leu Thr Phe Ser Cys
565 570 575

Phe Leu Gly Tyr Arg Leu Glu Gly Ala Xaa Lys Leu Thr Cys Leu Gly
580 585 590

Gly Gly Arg Arg Val Trp Ser Ala Pro Leu Pro Arg Cys Val Ala Glu
595 600 605

Cys Gly Ala Ser Val Lys Gly Asn Glu Gly Thr Leu Leu Ser Pro Asn
610 615 620

Phe Pro Ser Asn Tyr Asp Asn Asn His Glu Cys Ile Tyr Lys Ile Glu
625 630 635 640

Thr Glu Ala Gly Lys Gly Ile His Leu Arg Thr Arg Ser Phe Gln Leu
645 650 655

Phe Glu Gly Asp Thr Leu Lys Val Tyr Asp Gly Lys Asp Ser Ser Ser
660 665 670

Arg Pro Leu Gly Thr Phe Thr Lys Asn Glu Leu Leu Gly Leu Ile Leu
675 680 685

Asn Ser Thr Ser Asn His Xaa Trp Leu Glu Phe Asn Thr Asn Gly Ser
690 695 700

Asp Thr Asp Gln Gly Phe Gln Leu Thr Tyr Thr Ser Phe Asp Leu Val
705 710 715 720

Lys Cys Glu Asp Pro Gly Ile Pro Asn Tyr Gly Tyr Arg Ile Arg Asp
725 730 735

Glu Gly His Phe Thr Asp Thr Val Val Leu Tyr Ser Cys Asn Pro Gly
740 745 750

Tyr Ala Met His Gly Ser Asn Thr Leu Thr Cys Leu Ser Gly Asp Arg
755 760 765

Arg Val Trp Asp Lys Pro Leu Pro Ser Cys Ile Ala Glu Cys Gly Gly
770 775 780

Gln Ile His Ala Ala Thr Ser Gly Arg Ile Leu Ser Pro Gly Tyr Pro
785 790 795 800

Ala Pro Tyr Asp Asn Asn Leu His Cys Thr Trp Ile Ile Glu Ala Asp
805 810 815

Pro Gly Lys Thr Ile Ser Leu His Phe Ile Val Phe Asp Thr Glu Met
820 825 830

Ala His Asp Ile Leu Lys Val Trp Asp Gly Pro Val Asp Ser Asp Ile
835 840 845

Leu Leu Lys Glu Trp Ser Gly Ser Ala Leu Pro Glu Asp Ile His Ser
850 855 860

Thr Phe Asn Ser Leu Thr Leu Gln Phe Asp Ser Asp Phe Phe Ile Ser
865 870 875 880

Lys Ser Gly Phe Ser Ile Gln Phe Ser Thr Ser Ile Ala Ala Thr Cys
885 890 895

Asn Asp Pro Gly Met Pro Gln Asn Gly Thr Arg Tyr Gly Asp Ser Arg
900 905 910

Glu Ala Gly Asp Thr Val Thr Phe Gln Cys Asp Pro Gly Tyr Gln Leu
915 920 925

Gln Gly Gln Ala Lys Ile Thr Cys Val Gln Leu Asn Asn Arg Phe Phe
930 935 940

Trp Gln Pro Asp Pro Pro Thr Cys Ile Ala Ala Cys Gly Gly Asn Leu
945 950 955 960

Thr Gly Pro Ala Gly Val Ile Leu Ser Pro Asn Tyr Pro Gln Pro Tyr
965 970 975

Pro Pro Gly Lys Glu Cys Asp Trp Arg Val Lys Val Asn Pro Asp Phe
980 985 990

Val Ile Ala Leu Ile Phe Lys Ser Phe Asn Met Glu Pro Ser Tyr Asp
995 1000 1005

Phe Leu His Ile Tyr Glu Gly Glu Asp Ser Asn Ser Pro Leu Ile
1010 1015 1020

Gly Ser Tyr Gln Gly Ser Gln Ala Pro Glu Arg Ile Glu Ser Ser
1025 1030 1035

Gly Asn Ser Leu Phe Leu Ala Phe Arg Ser Asp Ala Ser Val Gly
1040 1045 1050

Leu Ser Gly Phe Ala Ile Glu Phe Lys Glu Lys Pro Arg Glu Ala
1055 1060 1065

Cys Phe Asp Pro Gly Asn Ile Met Asn Gly Thr Arg Val Gly Thr
1070 1075 1080

Asp Phe Lys Leu Gly Ser Thr Ile Thr Tyr Gln Cys Asp Ser Gly
1085 1090 1095

Tyr Lys Ile Leu Asp Pro Ser Ser Ile Thr Cys Val Ile Gly Ala
1100 1105 1110

Asp Gly Lys Pro Ser Trp Asp Gln Val Leu Pro Ser Cys Asn Ala
1115 1120 1125

Pro Cys Gly Gly Gln Tyr Thr Gly Ser Glu Gly Val Val Leu Ser
1130 1135 1140

Pro Asn Tyr Pro His Asn Tyr Thr Ala Gly Gln Ile Cys Leu Tyr
1145 1150 1155

Ser Ile Thr Val Pro Lys Glu Phe Val Val Phe Gly Gln Phe Ala
1160 1165 1170

Tyr Phe Gln Thr Ala Leu Asn Asp Leu Ala Glu Leu Phe Asp Gly
1175 1180 1185

Thr His Ala Gln Ala Arg Leu Leu Ser Ser Leu Ser Gly Ser His
1190 1195 1200

Ser Gly Glu Thr Leu Pro Leu Ala Thr Ser Asn Gln Ile Leu Leu
1205 1210 1215

Arg Phe Ser Ala Lys Ser Gly Ala Ser Ala Arg Gly Phe His Phe
1220 1225 1230

Val Tyr Gln Ala Val Pro Arg Thr Ser Asp Thr Gln Cys Ser Ser
1235 1240 1245

Val Pro Glu Pro Arg Tyr Gly Arg Arg Ile Gly Ser Glu Phe Ser
1250 1255 1260

Ala Gly Ser Ile Val Arg Phe Glu Xaa Asn Pro Gly Tyr Leu Leu
1265 1270 1275

Gln Gly Ser Thr Ala Leu His Cys Gln Ser Val Pro Asn Ala Leu
1280 1285 1290

Ala Gln Trp Asn Asp Thr Ile Pro Ser Cys Val Val Pro Cys Ser
1295 1300 1305

Gly Asn Phe Thr Gln Arg Arg Gly Thr Ile Leu Ser Pro Gly Tyr
1310 1315 1320

Pro Glu Pro Tyr Gly Asn Asn Leu Asn Cys Ile Trp Lys Ile Ile
1325 1330 1335

Val Thr Glu Gly Ser Gly Ile Gln Ile Gln Val Ile Ser Phe Ala
1340 1345 1350

Thr Glu Gln Asn Trp Asp Ser Leu Glu Ile His Asp Gly Gly Asp
1355 1360 1365

Val Thr Ala Pro Arg Leu Gly Ser Phe Ser Gly Thr Thr Val Pro
1370 1375 1380

Ala Leu Leu Asn Ser Thr Ser Asn Gln Leu Tyr Leu His Phe Gln
1385 1390 1395

Ser Asp Ile Ser Val Ala Ala Ala Gly Phe His Leu Glu Tyr Lys
1400 1405 1410

Thr Val Gly Leu Ala Ala Cys Gln Glu Pro Ala Leu Pro Ser Asn
1415 1420 1425

Ser Ile Lys Ile Gly Asp Arg Tyr Met Val Asn Asp Val Leu Ser
1430 1435 1440

Phe Gln Cys Glu Pro Gly Tyr Thr Leu Gln Gly Arg Ser His Ile
1445 1450 1455

Ser Cys Met Pro Gly Thr Val Arg Arg Trp Asn Tyr Pro Ser Pro
1460 1465 1470

Leu Cys Ile Ala Thr Cys Gly Gly Thr Leu Ser Thr Leu Gly Gly
1475 1480 1485

Val Ile Leu Ser Pro Gly Phe Pro Gly Ser Tyr Pro Asn Asn Leu
1490 1495 1500

Asp Cys Thr Trp Arg Ile Ser Leu Pro Ile Gly Tyr Gly Ala His
1505 1510 1515

Ile Gln Phe Leu Asn Phe Ser Thr Glu Ala Asn His Asp Phe Leu
1520 1525 1530

Glu Ile Gln Asn Gly Pro Tyr His Thr Ser Pro Met Ile Gly Gln
1535 1540 1545

Phe Ser Gly Thr Asp Leu Pro Ala Ala Leu Leu Ser Thr Thr His
1550 1555 1560

Glu Thr Leu Ile His Phe Tyr Ser Asp His Ser Gln Asn Arg Gln
1565 1570 1575

Gly Phe Lys Leu Ala Tyr Gln Ala Tyr Glu Leu Gln Asn Cys Pro
1580 1585 1590

Asp Pro Pro Pro Phe Gln Asn Gly Tyr Met Ile Asn Ser Asp Tyr
1595 1600 1605

Ser Val Gly Gln Ser Val Ser Phe Glu Cys Tyr Pro Gly Tyr Ile
1610 1615 1620

Leu Ile Gly His Pro Val Leu Thr Cys Gln His Gly Ile Asn Arg
1625 1630 1635

Asn Trp Asn Tyr Pro Phe Pro Arg Cys Asp Ala Pro Cys Gly Tyr
1640 1645 1650

Asn Val Thr Ser Gln Asn Gly Thr Ile Tyr Ser Pro Gly Phe Pro
1655 1660 1665

Asp Glu Tyr Pro Ile Leu Lys Asp Cys Ile Trp Leu Ile Thr Val
1670 1675 1680

Pro Pro Gly His Gly Val Tyr Ile Asn Phe Thr Leu Leu Gln Thr
1685 1690 1695

Glu Ala Val Asn Asp Tyr Ile Ala Val Trp Asp Gly Pro Asp Gln
1700 1705 1710

Asn Ser Pro Gln Leu Gly Val Phe Ser Gly Asn Thr Ala Leu Glu
1715 1720 1725

Thr Ala Tyr Ser Ser Thr Asn Gln Val Leu Leu Lys Phe His Ser
1730 1735 1740

Asp Phe Ser Asn Gly Gly Phe Phe Val Leu Asn Phe His Ala Phe
1745 1750 1755

Gln Leu Lys Lys Cys Gln Pro Pro Pro Ala Val Pro Gln Ala Glu
1760 1765 1770

Met Leu Thr Glu Asp Asp Asp Phe Glu Ile Gly Asp Phe Val Lys
1775 1780 1785

Tyr Gln Cys His Pro Gly Tyr Thr Leu Val Gly Thr Asp Ile Leu
1790 1795 1800

Thr Cys Lys Leu Ser Ser Gln Leu Gln Phe Glu Gly Ser Leu Pro
1805 1810 1815

Thr Cys Glu Ala Gln Cys Pro Ala Asn Glu Val Arg Thr Gly Ser
1820 1825 1830

Ser Gly Val Ile Leu Ser Pro Gly Tyr Pro Gly Asn Tyr Phe Asn
1835 1840 1845

Ser Gln Thr Cys Ser Trp Ser Ile Lys Val Glu Pro Asn Tyr Asn
1850 1855 1860

Ile Thr Ile Phe Val Asp Thr Phe Gln Ser Glu Lys Gln Phe Asp
1865 1870 1875

Ala Leu Glu Val Phe Asp Gly Ser Ser Gly Gln Ser Pro Leu Leu
1880 1885 1890

Val Val Leu Ser Gly Asn His Thr Glu Gln Ser Asn Phe Thr Ser
1895 1900 1905

Arg Ser Asn Gln Leu Tyr Leu Arg Trp Ser Thr Asp His Ala Thr
1910 1915 1920

Ser Lys Lys Gly Phe Lys Ile Arg Tyr Ala Ala Pro Tyr Cys Ser
1925 1930 1935

Leu Thr His Pro Leu Lys Asn Gly Gly Ile Leu Asn Arg Thr Ala
1940 1945 1950

Gly Ala Val Gly Ser Lys Val His Tyr Phe Cys Lys Pro Gly Tyr
1955 1960 1965

Arg Met Val Gly His Ser Asn Ala Thr Cys Arg Arg Asn Pro Leu
1970 1975 1980

Gly Met Tyr Gln Trp Asp Ser Leu Thr Pro Leu Cys Gln Ala Val
1985 1990 1995

Ser Cys Gly Ile Pro Glu Ser Pro Gly Asn Gly Ser Phe Thr Gly
2000 2005 2010

Asn Glu Phe Thr Leu Asp Ser Lys Val Val Tyr Glu Cys His Glu
2015 2020 2025

Gly Phe Lys Leu Glu Ser Ser Gln Gln Ala Thr Ala Val Cys Gln
2030 2035 2040

Glu Asp Gly Leu Trp Ser Asn Lys Gly Lys Pro Pro Thr Cys Lys
2045 2050 2055

Pro Val Ala Cys Pro Ser Ile Glu Ala Gln Leu Ser Glu His Val
2060 2065 2070

Ile Trp Arg Leu Val Ser Gly Ser Leu Asn Glu Tyr Gly Ala Gln
2075 2080 2085

Val Leu Leu Ser Cys Ser Pro Gly Tyr Tyr Leu Glu Gly Trp Arg
2090 2095 2100

Leu Leu Arg Cys Gln Ala Asn Gly Thr Trp Asn Ile Gly Asp Glu
2105 2110 2115

Arg Pro Ser Cys Arg Val Ile Ser Cys Gly Ser Leu Ser Phe Pro
2120 2125 2130

Pro Asn Gly Asn Lys Ile Gly Thr Leu Thr Val Tyr Gly Ala Thr
2135 2140 2145

Ala Ile Phe Thr Cys Asn Thr Gly Tyr Thr Leu Val Gly Ser His
2150 2155 2160

Val Arg Glu Cys Leu Ala Asn Gly Leu Trp Ser Gly Ser Glu Thr
2165 2170 2175

Arg Cys Leu Ala Gly His Cys Gly Ser Pro Asp Pro Ile Val Asn
2180 2185 2190

Gly His Ile Ser Gly Asp Gly Phe Ser Tyr Arg Asp Thr Val Val
2195 2200 2205

Tyr Gln Cys Asn Pro Gly Phe Arg Leu Val Gly Thr Ser Val Arg
2210 2215 2220

Ile Cys Leu Gln Asp His Lys Trp Ser Gly Gln Thr Pro Val Cys
2225 2230 2235

Val Pro Ile Thr Cys Gly His Pro Gly Asn Pro Ala His Gly Phe
2240 2245 2250

Thr Asn Gly Ser Glu Phe Asn Leu Asn Asp Val Val Asn Phe Thr
2255 2260 2265

Cys Asn Thr Gly Tyr Leu Leu Gln Gly Val Ser Arg Ala Gln Cys
2270 2275 2280

Arg Ser Asn Gly Gln Trp Ser Ser Pro Leu Pro Thr Cys Arg Val
2285 2290 2295

Val Asn Cys Ser Asp Pro Gly Phe Val Glu Asn Ala Ile Arg His
2300 2305 2310

Gly Gln Gln Asn Phe Pro Glu Ser Phe Glu Tyr Gly Met Ser Ile
2315 2320 2325

Leu Tyr His Cys Lys Lys Gly Phe Tyr Leu Leu Gly Ser Ser Ala
2330 2335 2340

Leu Thr Cys Met Ala Asn Gly Leu Trp Asp Arg Ser Leu Pro Lys
2345 2350 2355

Cys Leu Ala Ile Ser Cys Gly His Pro Gly Val Pro Ala Asn Ala
2360 2365 2370

Val Leu Thr Gly Glu Leu Phe Thr Tyr Gly Ala Val Val His Tyr
2375 2380 2385

Ser Cys Arg Gly Ser Glu Ser Leu Ile Gly Asn Asp Thr Arg Val
2390 2395 2400

Cys Gln Glu Asp Ser His Trp Ser Gly Ala Leu Pro His Cys Thr
2405 2410 2415

Gly Asn Asn Pro Gly Phe Cys Gly Asp Pro Gly Thr Pro Ala His
2420 2425 2430

Gly Ser Arg Leu Gly Asp Asp Phe Lys Thr Lys Ser Leu Leu Arg
2435 2440 2445

Phe Ser Cys Glu Met Gly His Gln Leu Arg Gly Ser Pro Glu Arg
2450 2455 2460

Thr Cys Leu Leu Asn Gly Ser Trp Ser Gly Leu Gln Pro Val Cys
2465 2470 2475

Glu Ala Val Ser Cys Gly Asn Pro Gly Thr Pro Thr Asn Gly Met
2480 2485 2490

Ile Val Ser Ser Asp Gly Ile Leu Phe Ser Ser Ser Val Ile Tyr
2495 2500 2505

Ala Cys Trp Glu Gly Tyr Lys Thr Ser Gly Leu Met Thr Arg His
2510 2515 2520

Cys Thr Ala Asn Gly Thr Trp Thr Gly Thr Ala Pro Asp Cys Thr
2525 2530 2535

Ile Ile Ser Cys Gly Asp Pro Gly Thr Leu Ala Asn Gly Ile Gln
2540 2545 2550

Phe Gly Thr Asp Phe Thr Phe Asn Lys Thr Val Ser Tyr Gln Cys
2555 2560 2565

```
Asn Pro  Gly Tyr Val Met Glu  Ala Val Thr Ser Ala  Thr Ile Arg
    2570                 2575                 2580

Cys Thr  Lys Asp Gly Arg Trp  Asn Pro Ser Lys Pro  Val Cys Lys
    2585                 2590                 2595

Ala Val  Leu Cys Pro Gln Pro  Pro Pro Val Gln Asn  Gly Thr Val
    2600                 2605                 2610

Glu Gly  Ser Asp Phe Arg Trp  Gly Ser Ser Ile Ser  Tyr Ser Cys
    2615                 2620                 2625

Met Asp  Gly Tyr Gln Leu Ser  His Ser Ala Ile Leu  Ser Cys Glu
    2630                 2635                 2640

Gly Arg  Gly Val Trp Lys Gly  Glu Ile Pro Gln Cys  Leu Pro Val
    2645                 2650                 2655

Phe Cys  Gly Asp Pro Gly Ile  Pro Ala Glu Gly Arg  Leu Ser Gly
    2660                 2665                 2670

Lys Ser  Phe Thr Tyr Lys Ser  Glu Val Phe Phe Gln  Cys Lys Ser
    2675                 2680                 2685

Pro Phe  Ile Leu Val Gly Ser  Ser Arg Arg Val Cys  Gln Ala Asp
    2690                 2695                 2700

Gly Thr  Trp Ser Gly Ile Gln  Pro Thr Cys Ile Asp  Pro Ala His
    2705                 2710                 2715

Asn Thr  Cys Pro Asp Pro Gly  Thr Pro His Phe Gly  Ile Gln Asn
    2720                 2725                 2730

Ser Ser  Arg Gly Tyr Glu Val  Gly Ser Thr Val Phe  Phe Arg Cys
    2735                 2740                 2745

Arg Lys  Gly Tyr His Ile Gln  Gly Ser Thr Thr Arg  Thr Cys Leu
    2750                 2755                 2760

Ala Asn  Leu Thr Trp Ser Gly  Ile Gln Thr Glu Cys  Ile Pro His
    2765                 2770                 2775

Ala Cys  Arg Gln Pro Glu Thr  Pro Ala His Ala Asp  Val Arg Ala
    2780                 2785                 2790

Ile Asp  Leu Pro Thr Phe Gly  Tyr Thr Leu Val Tyr  Thr Cys His
    2795                 2800                 2805
```

Pro Gly Phe Phe Leu Ala Gly Gly Ser Glu His Arg Thr Cys Lys
2810 2815 2820

Ala Asp Met Lys Trp Thr Gly Lys Ser Pro Val Cys Lys Ser Lys
2825 2830 2835

Gly Val Arg Glu Val Asn Glu Thr Val Thr Lys Thr Pro Val Pro
2840 2845 2850

Ser Asp Val Phe Phe Val Asn Ser Leu Trp Lys Gly Tyr Tyr Glu
2855 2860 2865

Tyr Leu Gly Lys Arg Gln Pro Ala Thr Leu Thr Val Asp Trp Phe
2870 2875 2880

Asn Ala Thr Ser Ser Lys Val Asn Ala Thr Phe Ser Glu Ala Ser
2885 2890 2895

Pro Val Glu Leu Lys Leu Thr Gly Ile Tyr Lys Lys Glu Glu Ala
2900 2905 2910

His Leu Leu Leu Lys Ala Phe Gln Ile Lys Gly Gln Ala Asp Ile
2915 2920 2925

Phe Val Ser Lys Phe Glu Asn Asp Asn Trp Gly Leu Asp Gly Tyr
2930 2935 2940

Val Ser Ser Gly Leu Glu Arg Gly Gly Phe Thr Phe Gln Gly Asp
2945 2950 2955

Ile His Gly Lys Asp Phe Gly Lys Phe Lys Leu Glu Arg Gln Asp
2960 2965 2970

Pro Leu Asn Pro Asp Gln Asp Ser Ser Ser His Tyr His Gly Thr
2975 2980 2985

Ser Ser Gly Ser Val Ala Ala Ala Ile Leu Val Pro Phe Phe Ala
2990 2995 3000

Leu Ile Leu Ser Gly Phe Ala Phe Tyr Leu Tyr Lys His Arg Thr
3005 3010 3015

Arg Pro Lys Val Gln Tyr Asn Gly Tyr Ala Gly His Glu Asn Ser
3020 3025 3030

Asn Gly Gln Ala Ser Phe Glu Asn Pro Met Tyr Asp Thr Asn Leu
3035 3040 3045

```
Lys Pro  Thr Glu Ala Lys Ala  Val Arg Phe Asp Thr  Thr Leu Asn
    3050                 3055                 3060

Thr Val  Cys Thr Val Val
    3065
```

<210> 3

<211> 12525

<212> DNA

<213> Rattus rattus

<220>

<221> CDS

<222> (1)..(9285)

<223> N = any amino acid

<400> 3

```
gat gcc ggg aag gtg ggg gac acc aga tcc gtc ttg tac gtg ctt aca      48
Asp Ala Gly Lys Val Gly Asp Thr Arg Ser Val Leu Tyr Val Leu Thr
1               5                   10                  15

ggc tcc agt gtc cct gac ctc atc gtg agc atg agc aat cag atg tgg      96
Gly Ser Ser Val Pro Asp Leu Ile Val Ser Met Ser Asn Gln Met Trp
            20                  25                  30

ctc cac ctg cag tca gac gac agc att ggt tcc cca gga ttt aaa gct     144
Leu His Leu Gln Ser Asp Asp Ser Ile Gly Ser Pro Gly Phe Lys Ala
        35                  40                  45

gtg tac caa gaa atc gag aag gga ggc tgc ggg gac cct ggc atc cca     192
Val Tyr Gln Glu Ile Glu Lys Gly Gly Cys Gly Asp Pro Gly Ile Pro
    50                  55                  60

gcc tac ggg aag cgg act ggc agc agc ttc ttg cac ggg gac acg ctc     240
Ala Tyr Gly Lys Arg Thr Gly Ser Ser Phe Leu His Gly Asp Thr Leu
65                  70                  75                  80

acc ttt gag tgc cag gca gct ttt gag ctg gta gga gag aga gtg att     288
Thr Phe Glu Cys Gln Ala Ala Phe Glu Leu Val Gly Glu Arg Val Ile
                85                  90                  95

acg tgc cag aga aac aac cag tgg tcc ggc aac aag cca agc tgt gtg     336
Thr Cys Gln Arg Asn Asn Gln Trp Ser Gly Asn Lys Pro Ser Cys Val
            100                 105                 110

ttt tca tgt ttc ttc aac ttc acg gcg tcc tct ggg atc atc ctg tcg     384
Phe Ser Cys Phe Phe Asn Phe Thr Ala Ser Ser Gly Ile Ile Leu Ser
        115                 120                 125
```

```
cca aac tat cct gag gaa tat ggc aac aac atg aat tgt gtg tgg ttg       432
Pro Asn Tyr Pro Glu Glu Tyr Gly Asn Asn Met Asn Cys Val Trp Leu
    130                 135                 140

att ata tct gag ccc ggg agc cgg att cac ctc atc ttc aat gat ttc       480
Ile Ile Ser Glu Pro Gly Ser Arg Ile His Leu Ile Phe Asn Asp Phe
145                 150                 155                 160

gat gtg gag cct cag ttt gac ttc ctt gcg gtc aaa gat gat ggg att       528
Asp Val Glu Pro Gln Phe Asp Phe Leu Ala Val Lys Asp Asp Gly Ile
                165                 170                 175

tct gac atc aca gtc ctc ggg act ttc tct ggc aat gag gtg cct gca       576
Ser Asp Ile Thr Val Leu Gly Thr Phe Ser Gly Asn Glu Val Pro Ala
            180                 185                 190

cag ctg gcc ngc agt gga cac ata gta cgc ctg gag ttt cag tcc gat       624
Gln Leu Ala Xaa Ser Gly His Ile Val Arg Leu Glu Phe Gln Ser Asp
        195                 200                 205

cac tct acc acg ggc aga ggg ttc aac atc ata tac acc aca ttt ggt       672
His Ser Thr Thr Gly Arg Gly Phe Asn Ile Ile Tyr Thr Thr Phe Gly
    210                 215                 220

cag aac gag tgt cat gac cct ggg atc cct gtg aat gga cgg cgc ttt       720
Gln Asn Glu Cys His Asp Pro Gly Ile Pro Val Asn Gly Arg Arg Phe
225                 230                 235                 240

gga gac agg ttt ctg ctg gga agt tct gtg tcc ttc cac tgt gat gat       768
Gly Asp Arg Phe Leu Leu Gly Ser Ser Val Ser Phe His Cys Asp Asp
                245                 250                 255

ggc ttt gtg aag act cag ggt tct gag tct atc aca tgc atc ttg caa       816
Gly Phe Val Lys Thr Gln Gly Ser Glu Ser Ile Thr Cys Ile Leu Gln
            260                 265                 270

gat gga aac gtg gtc tgg agc tct act gtc cct cgc tgt gaa gct cct       864
Asp Gly Asn Val Val Trp Ser Ser Thr Val Pro Arg Cys Glu Ala Pro
        275                 280                 285

tgt ggt ggg cat ctg aca gct tct agt ggg gtc ata tta cct cca gga       912
Cys Gly Gly His Leu Thr Ala Ser Ser Gly Val Ile Leu Pro Pro Gly
    290                 295                 300

tgg cca gga tat tac aaa gat tct tta aat tgc gaa tgg gtc att gaa       960
Trp Pro Gly Tyr Tyr Lys Asp Ser Leu Asn Cys Glu Trp Val Ile Glu
305                 310                 315                 320

gcc aaa cca gga cat tcc atc aaa ata aca ttt gac agg ttc cag aca      1008
Ala Lys Pro Gly His Ser Ile Lys Ile Thr Phe Asp Arg Phe Gln Thr
                325                 330                 335

gaa gtc aat tat gat act ctg gaa gtc cgg gat ggg cca acc agc tca      1056
Glu Val Asn Tyr Asp Thr Leu Glu Val Arg Asp Gly Pro Thr Ser Ser
            340                 345                 350

tcc cca ctg att ggg gag tac cat ggc acc cag gct cca cag ttc ctc      1104
Ser Pro Leu Ile Gly Glu Tyr His Gly Thr Gln Ala Pro Gln Phe Leu
        355                 360                 365

atc agc aca ggg aac tac atg tac ctg ctg ttt acc act gac agc agc      1152
Ile Ser Thr Gly Asn Tyr Met Tyr Leu Leu Phe Thr Thr Asp Ser Ser
    370                 375                 380
```

```
cgc gct agt gtt ggc ttc ctc atc cac tat gag agt gtg act ctt gaa      1200
Arg Ala Ser Val Gly Phe Leu Ile His Tyr Glu Ser Val Thr Leu Glu
385                 390                 395                 400

tct gac tcc tgt ctg gac ccg ggc atc cct gta aat ggt cat cgg cat      1248
Ser Asp Ser Cys Leu Asp Pro Gly Ile Pro Val Asn Gly His Arg His
                405                 410                 415

ggc agt aac ttt ggt atc aga tct aca gtg acc ttc agc tgt gac cct      1296
Gly Ser Asn Phe Gly Ile Arg Ser Thr Val Thr Phe Ser Cys Asp Pro
            420                 425                 430

ggg tac acg ctc agt gat gac gat ccc ctc atc tgt gag aag aac cat      1344
Gly Tyr Thr Leu Ser Asp Asp Asp Pro Leu Ile Cys Glu Lys Asn His
        435                 440                 445

cag tgg aac cac gcc ttg ccc agc tgt gat gcc ctg tgt gga ggc tac      1392
Gln Trp Asn His Ala Leu Pro Ser Cys Asp Ala Leu Cys Gly Gly Tyr
    450                 455                 460

atc cat gga aag agt ggg act gtt ctt tca cca gga ttt cca gac ttt      1440
Ile His Gly Lys Ser Gly Thr Val Leu Ser Pro Gly Phe Pro Asp Phe
465                 470                 475                 480

tat cca aac tct ctg aac tgt aca tgg acc att gaa gtc tct cat ggc      1488
Tyr Pro Asn Ser Leu Asn Cys Thr Trp Thr Ile Glu Val Ser His Gly
                485                 490                 495

aag gga gtg cag atg aat ttc cac acc ttt cac ctt gaa agt tcc cac      1536
Lys Gly Val Gln Met Asn Phe His Thr Phe His Leu Glu Ser Ser His
            500                 505                 510

gac tat ttg ctg atc aca gag gat ggg agt ttc tca gag ccg gta gcc      1584
Asp Tyr Leu Leu Ile Thr Glu Asp Gly Ser Phe Ser Glu Pro Val Ala
        515                 520                 525

agg ctc act ggg tcg gtc ctg cct cac acc att aag gct ggc ttg ttt      1632
Arg Leu Thr Gly Ser Val Leu Pro His Thr Ile Lys Ala Gly Leu Phe
    530                 535                 540

gga aac ttc act gcg caa ctc agg ttc atc tct gac ttc tcc atc tcc      1680
Gly Asn Phe Thr Ala Gln Leu Arg Phe Ile Ser Asp Phe Ser Ile Ser
545                 550                 555                 560

tat gaa ggc ttc aac att acg ttt gca gaa tat gac cta gaa ccc tgt      1728
Tyr Glu Gly Phe Asn Ile Thr Phe Ala Glu Tyr Asp Leu Glu Pro Cys
                565                 570                 575

gat gac cct gga gtc cct gcc tac agt cgc aga att ggg ttc cag ttc      1776
Asp Asp Pro Gly Val Pro Ala Tyr Ser Arg Arg Ile Gly Phe Gln Phe
            580                 585                 590

ggt gtg ggt gac acc ctg gct ttc acc tgc ttc cag gga tac cgc tta      1824
Gly Val Gly Asp Thr Leu Ala Phe Thr Cys Phe Gln Gly Tyr Arg Leu
        595                 600                 605

gaa ggt gca acc aag ctt acc tgc ctg ggt ggg gga cgc cga gtg tgg      1872
Glu Gly Ala Thr Lys Leu Thr Cys Leu Gly Gly Gly Arg Arg Val Trp
    610                 615                 620

agt gca cct ctg cca agg tgt gtg gct gaa tgt gga gca agc gtc aaa      1920
Ser Ala Pro Leu Pro Arg Cys Val Ala Glu Cys Gly Ala Ser Val Lys
625                 630                 635                 640
```

```
gga aat gaa gga aca tta ctc tct cca aat ttc cca tcc aat tat gat      1968
Gly Asn Glu Gly Thr Leu Leu Ser Pro Asn Phe Pro Ser Asn Tyr Asp
                645                 650                 655

aat aac cat gag tgt atc tat aaa ata gaa aca gaa gcc gga aag ggg      2016
Asn Asn His Glu Cys Ile Tyr Lys Ile Glu Thr Glu Ala Gly Lys Gly
            660                 665                 670

atc cat ctc aga gcc cga acc ttc caa ctc ttc gaa gga gac act cta      2064
Ile His Leu Arg Ala Arg Thr Phe Gln Leu Phe Glu Gly Asp Thr Leu
        675                 680                 685

aag gtt tat gat gga aag gac agc tcc tcg agg tca ctg gga gtc ttc      2112
Lys Val Tyr Asp Gly Lys Asp Ser Ser Ser Arg Ser Leu Gly Val Phe
    690                 695                 700

aca aga agt gaa ctg atg ggg ctg gtg cta aac agc acc tcc aac cac      2160
Thr Arg Ser Glu Leu Met Gly Leu Val Leu Asn Ser Thr Ser Asn His
705                 710                 715                 720

ctg agg ctg gag ttc aac tct aac ggg tca gat acc gcc caa ggc ttc      2208
Leu Arg Leu Glu Phe Asn Ser Asn Gly Ser Asp Thr Ala Gln Gly Phe
                725                 730                 735

cag ctc acc tac acc agt ttt gac cta gtg aaa tgt gag gat cca ggc      2256
Gln Leu Thr Tyr Thr Ser Phe Asp Leu Val Lys Cys Glu Asp Pro Gly
            740                 745                 750

atc cct aac tat ggc tac agg atc cga gat gat ggt cac ttc aca gac      2304
Ile Pro Asn Tyr Gly Tyr Arg Ile Arg Asp Asp Gly His Phe Thr Asp
        755                 760                 765

act gtg gtt ctc tac agc tgc aac cca ggc tac gca atg cat ggc agc      2352
Thr Val Val Leu Tyr Ser Cys Asn Pro Gly Tyr Ala Met His Gly Ser
    770                 775                 780

agt acc ctg acc tgc ctg agt ggg gac cga agg gtg tgg gac aaa cct      2400
Ser Thr Leu Thr Cys Leu Ser Gly Asp Arg Arg Val Trp Asp Lys Pro
785                 790                 795                 800

atg cct tcc tgt gtg gcg gaa tgt ggt ggt ctc gtc cat gca gcc aca      2448
Met Pro Ser Cys Val Ala Glu Cys Gly Gly Leu Val His Ala Ala Thr
                805                 810                 815

tca gga cgc ata ctc tct cct ggc tac cct gcc cca tat gac aac aac      2496
Ser Gly Arg Ile Leu Ser Pro Gly Tyr Pro Ala Pro Tyr Asp Asn Asn
            820                 825                 830

ctt cat tgc act tgg acc ata gag gct gat cct ggc aag acc ayc agc      2544
Leu His Cys Thr Trp Thr Ile Glu Ala Asp Pro Gly Lys Thr Xaa Ser
        835                 840                 845

ctc cat ttc att gtg ttt gac act gaa acg gcg cac gac atc ctc aag      2592
Leu His Phe Ile Val Phe Asp Thr Glu Thr Ala His Asp Ile Leu Lys
    850                 855                 860

gtc tgg gat ggt cca gtg gac agc aac atc ctg ctg aag gag tgg agc      2640
Val Trp Asp Gly Pro Val Asp Ser Asn Ile Leu Leu Lys Glu Trp Ser
865                 870                 875                 880

ggc tcg gcc ctt cct gag gac atc cac agc acc ttc aac tcg ctc acc      2688
Gly Ser Ala Leu Pro Glu Asp Ile His Ser Thr Phe Asn Ser Leu Thr
                885                 890                 895
```

```
ctg cag ttc gat agt gac ttc ttc atc agc aag tcc ggc ttc tcc atc      2736
Leu Gln Phe Asp Ser Asp Phe Phe Ile Ser Lys Ser Gly Phe Ser Ile
            900                 905                 910

cag ttc tct act tcc att gca tcc acc tgc aat gac cct ggg atg cct      2784
Gln Phe Ser Thr Ser Ile Ala Ser Thr Cys Asn Asp Pro Gly Met Pro
        915                 920                 925

cag aat gga acc cgc tat ggt gac agc cgg gaa cct gga gac acc atc      2832
Gln Asn Gly Thr Arg Tyr Gly Asp Ser Arg Glu Pro Gly Asp Thr Ile
    930                 935                 940

acc ttc cag tgt gac cct gga tac cag ctc caa ggg caa gcc aag atc      2880
Thr Phe Gln Cys Asp Pro Gly Tyr Gln Leu Gln Gly Gln Ala Lys Ile
945                 950                 955                 960

act tgt gtg cag ctt aac aac cgc ttc ttc tgg caa cca gac cct ccg      2928
Thr Cys Val Gln Leu Asn Asn Arg Phe Phe Trp Gln Pro Asp Pro Pro
                965                 970                 975

tca tgc ata gct gct tgt ggt ggg aat ctg aca ggc cct gct gga gtg      2976
Ser Cys Ile Ala Ala Cys Gly Gly Asn Leu Thr Gly Pro Ala Gly Val
            980                 985                 990

att tta tcc cca aac tac cca cag  cca tac cct cct ggg  aag gag tgt    3024
Ile Leu Ser Pro Asn Tyr Pro Gln  Pro Tyr Pro Pro Gly  Lys Glu Cys
        995                 1000                1005

gac tgg  aga att aag gtg aac  cca gac ttt gtc att  gcc tta ata      3069
Asp Trp  Arg Ile Lys Val Asn  Pro Asp Phe Val Ile  Ala Leu Ile
    1010                1015                1020

ttc aaa  agt ttt agc atg gag  cca agt tac gac ttc  ctg cat atc      3114
Phe Lys  Ser Phe Ser Met Glu  Pro Ser Tyr Asp Phe  Leu His Ile
    1025                1030                1035

tat gaa  ggg aag gac tcc aac  agc cca ctg atc gga  agc ttc cag      3159
Tyr Glu  Gly Lys Asp Ser Asn  Ser Pro Leu Ile Gly  Ser Phe Gln
    1040                1045                1050

ggt tct  caa gcc cca gag agg  att gag agc agt ggt  aac agc ctc      3204
Gly Ser  Gln Ala Pro Glu Arg  Ile Glu Ser Ser Gly  Asn Ser Leu
    1055                1060                1065

ttc ctg  gca ttc agg agt gat  gcc tct gtt ggc ctg  tcc ggg ttt      3249
Phe Leu  Ala Phe Arg Ser Asp  Ala Ser Val Gly Leu  Ser Gly Phe
    1070                1075                1080

gcc att  gaa ttt aaa gag aaa  cca cgg gaa gct tgc  ttt gac cct      3294
Ala Ile  Glu Phe Lys Glu Lys  Pro Arg Glu Ala Cys  Phe Asp Pro
    1085                1090                1095

ggg aac  ata atg aac ggg aca  agg att gga acg gac  ttt aag ctg      3339
Gly Asn  Ile Met Asn Gly Thr  Arg Ile Gly Thr Asp  Phe Lys Leu
    1100                1105                1110

ggc tct  aca gtt acc tat caa  tgt gac tct ggt tac  aag att gtg      3384
Gly Ser  Thr Val Thr Tyr Gln  Cys Asp Ser Gly Tyr  Lys Ile Val
    1115                1120                1125

gat ccc  tca tcc att gag tgt  gtg aca ggg gct gat  ggg aag ccg      3429
Asp Pro  Ser Ser Ile Glu Cys  Val Thr Gly Ala Asp  Gly Lys Pro
    1130                1135                1140
```

```
tcc tgg  gac cgg gca ctg cct  gcc tgc caa gca ccc  tgt gga ggc     3474
Ser Trp  Asp Arg Ala Leu Pro  Ala Cys Gln Ala Pro  Cys Gly Gly
    1145                 1150                 1155

caa tac  atg ggc tcg gag ggg  gta gtt ttg tca cca  aac tac cct     3519
Gln Tyr  Met Gly Ser Glu Gly  Val Val Leu Ser Pro  Asn Tyr Pro
    1160                 1165                 1170

cat aac  tac acg gct ggg cag  ata tgc atc tat tcc  atc acg gtg     3564
His Asn  Tyr Thr Ala Gly Gln  Ile Cys Ile Tyr Ser  Ile Thr Val
    1175                 1180                 1185

ccc aag  gaa ttt gtg gtg ttt  gga cag ttt gcc tat  ttc cag act     3609
Pro Lys  Glu Phe Val Val Phe  Gly Gln Phe Ala Tyr  Phe Gln Thr
    1190                 1195                 1200

gcg ctg  aac gac ttg gca gaa  ttg ttt gat gga acc  cat cct cag     3654
Ala Leu  Asn Asp Leu Ala Glu  Leu Phe Asp Gly Thr  His Pro Gln
    1205                 1210                 1215

gcc agg  ctt ctc agt tct ctc  tct ggt tcc cat tca  ggt gaa aca     3699
Ala Arg  Leu Leu Ser Ser Leu  Ser Gly Ser His Ser  Gly Glu Thr
    1220                 1225                 1230

ctc ccg  ctg gct aca tcc aat  cag att ctg ctt cgc  ttc agc gca     3744
Leu Pro  Leu Ala Thr Ser Asn  Gln Ile Leu Leu Arg  Phe Ser Ala
    1235                 1240                 1245

aag agc  gga gct tct gca cgg  ggt ttc cac ttc gtc  tac caa gcc     3789
Lys Ser  Gly Ala Ser Ala Arg  Gly Phe His Phe Val  Tyr Gln Ala
    1250                 1255                 1260

gtc cca  cgc acc agt gac acg  cag tgc agc tcc gtc  cct gag ccc     3834
Val Pro  Arg Thr Ser Asp Thr  Gln Cys Ser Ser Val  Pro Glu Pro
    1265                 1270                 1275

aga tat  ggg aga agg att ggt  tct gag ttc tct gca  ggc tcc atc     3879
Arg Tyr  Gly Arg Arg Ile Gly  Ser Glu Phe Ser Ala  Gly Ser Ile
    1280                 1285                 1290

gtc cga  ttc gag tgc aac cca  ggt tac ctg ctg caa  ggc tcc aca     3924
Val Arg  Phe Glu Cys Asn Pro  Gly Tyr Leu Leu Gln  Gly Ser Thr
    1295                 1300                 1305

gcc atc  cgt tgt cag tct gtg  cca aac gct ttg gcc  cag tgg aat     3969
Ala Ile  Arg Cys Gln Ser Val  Pro Asn Ala Leu Ala  Gln Trp Asn
    1310                 1315                 1320

gac acc  atc cca agc tgt gta  gtt cca tgc agt ggc  aat ttc act     4014
Asp Thr  Ile Pro Ser Cys Val  Val Pro Cys Ser Gly  Asn Phe Thr
    1325                 1330                 1335

cag aga  aga ggg aca atc tta  tct cca ggc tac cct  gag ccc tat     4059
Gln Arg  Arg Gly Thr Ile Leu  Ser Pro Gly Tyr Pro  Glu Pro Tyr
    1340                 1345                 1350

ggg aac  aac ctg aac tgt gta  tgg aag atc ata gta  tcg gag ggc     4104
Gly Asn  Asn Leu Asn Cys Val  Trp Lys Ile Ile Val  Ser Glu Gly
    1355                 1360                 1365

tca ggg  atc cag atc caa gtg  att agc ttt gcc acg  gag cag aac     4149
Ser Gly  Ile Gln Ile Gln Val  Ile Ser Phe Ala Thr  Glu Gln Asn
    1370                 1375                 1380
```

```
tgg gac  tcc ctg gag atc cat  gac gga gga gac atg  acg gcc ccc      4194
Trp Asp  Ser Leu Glu Ile His  Asp Gly Gly Asp Met  Thr Ala Pro
    1385                 1390                 1395

aga ctg  ggc agc ttc tca ggt  acc aca gtg ccc gca  ctg ctg aat      4239
Arg Leu  Gly Ser Phe Ser Gly  Thr Thr Val Pro Ala  Leu Leu Asn
    1400                 1405                 1410

agc acc  tcc aac cag ctc tgc  ctg cac ttc cag tcg  gac atc agt      4284
Ser Thr  Ser Asn Gln Leu Cys  Leu His Phe Gln Ser  Asp Ile Ser
    1415                 1420                 1425

gtt gcc  gct gcg ggc ttt cac  ctg gaa tac aaa acg  gtg ggt ctg      4329
Val Ala  Ala Ala Gly Phe His  Leu Glu Tyr Lys Thr  Val Gly Leu
    1430                 1435                 1440

gct gcg  tgc cag gaa cct gct  ctc ccg agc aac ggc  atc aag ata      4374
Ala Ala  Cys Gln Glu Pro Ala  Leu Pro Ser Asn Gly  Ile Lys Ile
    1445                 1450                 1455

gga gac  cgc tat atg gtg aac  gat gtg ctg tcc ttc  cag tgc gag      4419
Gly Asp  Arg Tyr Met Val Asn  Asp Val Leu Ser Phe  Gln Cys Glu
    1460                 1465                 1470

cct ggg  tac acc ttg cag ggc  cgc tca cac att tct  tgt atg ccg      4464
Pro Gly  Tyr Thr Leu Gln Gly  Arg Ser His Ile Ser  Cys Met Pro
    1475                 1480                 1485

gga act  gta cgt cgc tgg aac  tat cct tcc cct ctg  tgc att gcc      4509
Gly Thr  Val Arg Arg Trp Asn  Tyr Pro Ser Pro Leu  Cys Ile Ala
    1490                 1495                 1500

acc tgt  ggt ggg aca ctg acc  agc atg agt gga gtg  atc ctg agc      4554
Thr Cys  Gly Gly Thr Leu Thr  Ser Met Ser Gly Val  Ile Leu Ser
    1505                 1510                 1515

cca ggc  ttc cca ggg tca tac  ccc aac aac ctg gac  tgc acc tgg      4599
Pro Gly  Phe Pro Gly Ser Tyr  Pro Asn Asn Leu Asp  Cys Thr Trp
    1520                 1525                 1530

aag ata  tcc ctg ccc att ggc  tat ggt gca cat atc  caa ttt ctg      4644
Lys Ile  Ser Leu Pro Ile Gly  Tyr Gly Ala His Ile  Gln Phe Leu
    1535                 1540                 1545

aat ttc  tca act gaa gcc aac  cat gac tac ctg gag  atc cag aat      4689
Asn Phe  Ser Thr Glu Ala Asn  His Asp Tyr Leu Glu  Ile Gln Asn
    1550                 1555                 1560

ggc cct  tac cac agt agt cca  atg atg gga cag ttc  agt ggc cct      4734
Gly Pro  Tyr His Ser Ser Pro  Met Met Gly Gln Phe  Ser Gly Pro
    1565                 1570                 1575

gac ctg  cct gcg tca ctg ctg  agc acc aca cat gaa  acc ctc atc      4779
Asp Leu  Pro Ala Ser Leu Leu  Ser Thr Thr His Glu  Thr Leu Ile
    1580                 1585                 1590

cgc ttc  tat agt gac cac tca  cag aac cga caa gga  ttt aaa ctc      4824
Arg Phe  Tyr Ser Asp His Ser  Gln Asn Arg Gln Gly  Phe Lys Leu
    1595                 1600                 1605

agt tac  caa gct tat gag tta  cag aac tgc ccg gac  cca ccc gca      4869
Ser Tyr  Gln Ala Tyr Glu Leu  Gln Asn Cys Pro Asp  Pro Pro Ala
    1610                 1615                 1620
```

```
ttc cag aat ggg ttc atg atc aac tcc gat tac agc gtg ggc cag       4914
Phe Gln Asn Gly Phe Met Ile Asn Ser Asp Tyr Ser Val Gly Gln
    1625                1630                1635

tcg atc tca ttt gag tgc tac ccg ggc tac atc ttg cta ggc cac       4959
Ser Ile Ser Phe Glu Cys Tyr Pro Gly Tyr Ile Leu Leu Gly His
    1640                1645                1650

cct gtg ctc acc tgc cag cat ggc act gac agg aac tgg aac tac       5004
Pro Val Leu Thr Cys Gln His Gly Thr Asp Arg Asn Trp Asn Tyr
    1655                1660                1665

cct ttc cca cgg tgt gac gct ccc tgt ggg tat aat gtg aca tca       5049
Pro Phe Pro Arg Cys Asp Ala Pro Cys Gly Tyr Asn Val Thr Ser
    1670                1675                1680

cag aat ggc acc att tat tcc cct ggg ttc cca gac gag tat cca       5094
Gln Asn Gly Thr Ile Tyr Ser Pro Gly Phe Pro Asp Glu Tyr Pro
    1685                1690                1695

att ctg aag gac tgc ctg tgg ctg gtc act gtc cct cca gga cat       5139
Ile Leu Lys Asp Cys Leu Trp Leu Val Thr Val Pro Pro Gly His
    1700                1705                1710

gga gtg tac atc aac ttc acc ttg ctg cag act gag gct gta aat       5184
Gly Val Tyr Ile Asn Phe Thr Leu Leu Gln Thr Glu Ala Val Asn
    1715                1720                1725

gac tac atc gct gtg tgg gat ggt cct gac cag aac tcg cct cag       5229
Asp Tyr Ile Ala Val Trp Asp Gly Pro Asp Gln Asn Ser Pro Gln
    1730                1735                1740

ctc ggg gtc ttc agt gga aac act gcc ctc gag aca gca tac agc       5274
Leu Gly Val Phe Ser Gly Asn Thr Ala Leu Glu Thr Ala Tyr Ser
    1745                1750                1755

tcc acc aac cag gtc ttg ctc aaa ttc cac agc gat ttc tcc aat       5319
Ser Thr Asn Gln Val Leu Leu Lys Phe His Ser Asp Phe Ser Asn
    1760                1765                1770

gga ggc ttc ttt gtc ctc aat ttt cat gca ttt caa ctg aag agg       5364
Gly Gly Phe Phe Val Leu Asn Phe His Ala Phe Gln Leu Lys Arg
    1775                1780                1785

tgc ccg cct cct cca gta gtg ccg cag gct gac ctg ctt aca gaa       5409
Cys Pro Pro Pro Pro Val Val Pro Gln Ala Asp Leu Leu Thr Glu
    1790                1795                1800

gat gaa gac ttt gaa ata ggg gac ttc gta aag tac cag tgc cat       5454
Asp Glu Asp Phe Glu Ile Gly Asp Phe Val Lys Tyr Gln Cys His
    1805                1810                1815

cca ggg tac acg ctg ttg gga agt gac acc ctg aca tgc aag ctc       5499
Pro Gly Tyr Thr Leu Leu Gly Ser Asp Thr Leu Thr Cys Lys Leu
    1820                1825                1830

agc tca cag cta ttg ttc caa ggc tct cca cct acc tgt gaa gca       5544
Ser Ser Gln Leu Leu Phe Gln Gly Ser Pro Pro Thr Cys Glu Ala
    1835                1840                1845

caa tgc cca gcc aat gaa gtg cga aca gag tct tct ggg gtg att       5589
Gln Cys Pro Ala Asn Glu Val Arg Thr Glu Ser Ser Gly Val Ile
    1850                1855                1860
```

```
ctc agt cct ggg tac cca ggc aac tat ttt aac tcc cag aca tgt      5634
Leu Ser Pro Gly Tyr Pro Gly Asn Tyr Phe Asn Ser Gln Thr Cys
    1865                1870                1875

gct tgg agt att aaa gtg gag cca aac ttt aac att acg ctc ttt      5679
Ala Trp Ser Ile Lys Val Glu Pro Asn Phe Asn Ile Thr Leu Phe
    1880                1885                1890

gtg gac acc ttt caa agt gaa aag caa ttt gat gca ctg gaa gta      5724
Val Asp Thr Phe Gln Ser Glu Lys Gln Phe Asp Ala Leu Glu Val
    1895                1900                1905

ttt gat ggt tct tct ggg caa agt cct ttg tta gtg gtc tta agt      5769
Phe Asp Gly Ser Ser Gly Gln Ser Pro Leu Leu Val Val Leu Ser
    1910                1915                1920

ggg aac cac act gaa cag tcc aat ttt acc agc aga agt aac cat      5814
Gly Asn His Thr Glu Gln Ser Asn Phe Thr Ser Arg Ser Asn His
    1925                1930                1935

ctg tac ctc cgc tgg tcc aca gat cat gca acc agc aag aaa gga      5859
Leu Tyr Leu Arg Trp Ser Thr Asp His Ala Thr Ser Lys Lys Gly
    1940                1945                1950

ttc aag att cgc tat gca gct cct tac tgc agc ctc acc tct aca      5904
Phe Lys Ile Arg Tyr Ala Ala Pro Tyr Cys Ser Leu Thr Ser Thr
    1955                1960                1965

ctc aag aat ggt ggc gtt tta aat aaa acc gca ggc gcc ctg ggg      5949
Leu Lys Asn Gly Gly Val Leu Asn Lys Thr Ala Gly Ala Leu Gly
    1970                1975                1980

agc aag gtg cag tat ttc tgc aag cct gga tat cga atg att ggc      5994
Ser Lys Val Gln Tyr Phe Cys Lys Pro Gly Tyr Arg Met Ile Gly
    1985                1990                1995

cac agc aac gcc acc tgc agg cgg aac cca gtg ggc gtg tac cag      6039
His Ser Asn Ala Thr Cys Arg Arg Asn Pro Val Gly Val Tyr Gln
    2000                2005                2010

tgg gac tcg atg gca ccg ctt tgc cag gct gtg tcc tgt gga att      6084
Trp Asp Ser Met Ala Pro Leu Cys Gln Ala Val Ser Cys Gly Ile
    2015                2020                2025

cca gag gct cca gga aat ggc tcg ttc aca ggc aat gag ttc acc      6129
Pro Glu Ala Pro Gly Asn Gly Ser Phe Thr Gly Asn Glu Phe Thr
    2030                2035                2040

tta gac agt aaa gtg act tat gaa tgt aat gaa ggc ttc aag ctg      6174
Leu Asp Ser Lys Val Thr Tyr Glu Cys Asn Glu Gly Phe Lys Leu
    2045                2050                2055

gat gcc agt cag caa gcc act gct gtg tgt caa gaa gat ggc ctg      6219
Asp Ala Ser Gln Gln Ala Thr Ala Val Cys Gln Glu Asp Gly Leu
    2060                2065                2070

tgg agc aac aga gga aag cca ccc acg tgc aaa ccg gtg ccc tgc      6264
Trp Ser Asn Arg Gly Lys Pro Pro Thr Cys Lys Pro Val Pro Cys
    2075                2080                2085

ccc agc atc gaa ggc cag ctg tca gag cac gtg ctc tgg agg ctg      6309
Pro Ser Ile Glu Gly Gln Leu Ser Glu His Val Leu Trp Arg Leu
    2090                2095                2100
```

```
gtt tcg gga tca ttg aat gaa tat gga gct caa gtt ctc ctc agc      6354
Val Ser Gly Ser Leu Asn Glu Tyr Gly Ala Gln Val Leu Leu Ser
    2105                2110                2115

tgt agt cct ggc tac ttc ttg cag ggt cag agg ctg ttg cag tgc      6399
Cys Ser Pro Gly Tyr Phe Leu Gln Gly Gln Arg Leu Leu Gln Cys
    2120                2125                2130

caa gcc aat ggg acc tgg aac act gag gag gac aga ccc aga tgt      6444
Gln Ala Asn Gly Thr Trp Asn Thr Glu Glu Asp Arg Pro Arg Cys
    2135                2140                2145

aaa gtc atc tcc tgt gga agc ctg tcc ttt ccc cca aat ggt aac      6489
Lys Val Ile Ser Cys Gly Ser Leu Ser Phe Pro Pro Asn Gly Asn
    2150                2155                2160

aag ata ggg acg ctc act atg tat gga gcc acc gcc atc ttt acc      6534
Lys Ile Gly Thr Leu Thr Met Tyr Gly Ala Thr Ala Ile Phe Thr
    2165                2170                2175

tgc aat acc ggc tac aca ctt gta ggc tcc cat gtc cgg gag tgc      6579
Cys Asn Thr Gly Tyr Thr Leu Val Gly Ser His Val Arg Glu Cys
    2180                2185                2190

ttg gcc aat ggt ctc tgg agc gga tct gaa aca agg tgc ctg gcg      6624
Leu Ala Asn Gly Leu Trp Ser Gly Ser Glu Thr Arg Cys Leu Ala
    2195                2200                2205

ggt cat tgt ggc tct cca gac ccc att gtg aat ggc cat atc agt      6669
Gly His Cys Gly Ser Pro Asp Pro Ile Val Asn Gly His Ile Ser
    2210                2215                2220

ggc gat ggc ttc agc tac agg gac aca gtg gtc tac caa tgc aac      6714
Gly Asp Gly Phe Ser Tyr Arg Asp Thr Val Val Tyr Gln Cys Asn
    2225                2230                2235

cct ggg ttt cga ctc gta ggc acg tct gtg agg att tgc ctg cag      6759
Pro Gly Phe Arg Leu Val Gly Thr Ser Val Arg Ile Cys Leu Gln
    2240                2245                2250

gac cac aag tgg tcg ggg cag acc ccc gtt tgc gtc ccc atc aca      6804
Asp His Lys Trp Ser Gly Gln Thr Pro Val Cys Val Pro Ile Thr
    2255                2260                2265

tgt gga cac cct gga aac cct gcc cat ggc ctc acc aac ggc agc      6849
Cys Gly His Pro Gly Asn Pro Ala His Gly Leu Thr Asn Gly Ser
    2270                2275                2280

gag ttc aac ctg aat gac ctt gtg aat ttc acc tgc cat acg ggc      6894
Glu Phe Asn Leu Asn Asp Leu Val Asn Phe Thr Cys His Thr Gly
    2285                2290                2295

tac ctg ctg cag ggt gcc tcc cga gcc caa tgt cgg agc aac ggc      6939
Tyr Leu Leu Gln Gly Ala Ser Arg Ala Gln Cys Arg Ser Asn Gly
    2300                2305                2310

cag tgg agc agc ccc ttg cct atc tgc cga gtg gtg aac tgt tcc      6984
Gln Trp Ser Ser Pro Leu Pro Ile Cys Arg Val Val Asn Cys Ser
    2315                2320                2325

gat cct gga ttt gtg gaa aat gca gtt cgc cac ggg caa cag aac      7029
Asp Pro Gly Phe Val Glu Asn Ala Val Arg His Gly Gln Gln Asn
    2330                2335                2340
```

```
ttt cca gag agt ttc gag tat ggg aca agt gtg atg tat cac tgc    7074
Phe Pro Glu Ser Phe Glu Tyr Gly Thr Ser Val Met Tyr His Cys
    2345                2350                2355

aag aag ggg ttc tac cta ctg ggc tct tct gcc ctg acc tgc atg    7119
Lys Lys Gly Phe Tyr Leu Leu Gly Ser Ser Ala Leu Thr Cys Met
    2360                2365                2370

gca agt ggc ttg tgg gac cgc tcc tta ccc aag tgt ctg gct ata    7164
Ala Ser Gly Leu Trp Asp Arg Ser Leu Pro Lys Cys Leu Ala Ile
    2375                2380                2385

tca tgt ggg cat cct ggg gtc ccc gct aat gct gtc ctg act gga    7209
Ser Cys Gly His Pro Gly Val Pro Ala Asn Ala Val Leu Thr Gly
    2390                2395                2400

gaa ttg ttt aca ttt gga gcc aca gtt cag tac tcc tgc aaa ggg    7254
Glu Leu Phe Thr Phe Gly Ala Thr Val Gln Tyr Ser Cys Lys Gly
    2405                2410                2415

ggc cag att ctc aca ggc aat agc aca aga gtc tgc caa gaa gac    7299
Gly Gln Ile Leu Thr Gly Asn Ser Thr Arg Val Cys Gln Glu Asp
    2420                2425                2430

agt cac tgg agt gga tcc ctt ccc cat tgt tca gga aat agt cct    7344
Ser His Trp Ser Gly Ser Leu Pro His Cys Ser Gly Asn Ser Pro
    2435                2440                2445

gga ttt tgt ggt gat cca ggg acc cca gca cat ggg tct cgt ctt    7389
Gly Phe Cys Gly Asp Pro Gly Thr Pro Ala His Gly Ser Arg Leu
    2450                2455                2460

ggg gat gag ttt aag aca aag agt ctt ttg cga ttc tcc tgt gag    7434
Gly Asp Glu Phe Lys Thr Lys Ser Leu Leu Arg Phe Ser Cys Glu
    2465                2470                2475

atg ggc cac cag ctg cgg ggt tct gca gag cgc aca tgc ctg gtg    7479
Met Gly His Gln Leu Arg Gly Ser Ala Glu Arg Thr Cys Leu Val
    2480                2485                2490

aat ggg tcc tgg tca gga gtc cag cct gtg tgt gag gcc gtg tcc    7524
Asn Gly Ser Trp Ser Gly Val Gln Pro Val Cys Glu Ala Val Ser
    2495                2500                2505

tgt gga aac cct ggc acc cct acc aat ggg atg atc ctc agc agc    7569
Cys Gly Asn Pro Gly Thr Pro Thr Asn Gly Met Ile Leu Ser Ser
    2510                2515                2520

gat gga atc ctc ttc tcc agc tct gtc atc tat gcc tgc tgg gaa    7614
Asp Gly Ile Leu Phe Ser Ser Ser Val Ile Tyr Ala Cys Trp Glu
    2525                2530                2535

ggc tac aag acc tcg ggg ctc atg acg cgg cac tgc aca gcg aac    7659
Gly Tyr Lys Thr Ser Gly Leu Met Thr Arg His Cys Thr Ala Asn
    2540                2545                2550

ggg aca tgg aca ggc aca gcc cct gac tgt aca atc atc agc tgt    7704
Gly Thr Trp Thr Gly Thr Ala Pro Asp Cys Thr Ile Ile Ser Cys
    2555                2560                2565

ggt gat cct ggc aca ctg ccc aat ggc atc cag ttt ggg aca gac    7749
Gly Asp Pro Gly Thr Leu Pro Asn Gly Ile Gln Phe Gly Thr Asp
    2570                2575                2580
```

ttc act ttc aac aag acc gtg agc tat cag tgc aac cct ggc tac 7794
Phe Thr Phe Asn Lys Thr Val Ser Tyr Gln Cys Asn Pro Gly Tyr
2585 2590 2595

ctg atg gag ccc cca aca tca ccc acc atc cgc tgc acc aaa gat 7839
Leu Met Glu Pro Pro Thr Ser Pro Thr Ile Arg Cys Thr Lys Asp
2600 2605 2610

ggt aca tgg aat cag acc cgg ccc ctc tgc aaa gct gtt cta tgc 7884
Gly Thr Trp Asn Gln Thr Arg Pro Leu Cys Lys Ala Val Leu Cys
2615 2620 2625

agc cag cct ccc tca gtg cca aac gga aag gtg gag ggg tca gac 7929
Ser Gln Pro Pro Ser Val Pro Asn Gly Lys Val Glu Gly Ser Asp
2630 2635 2640

ttc cga tgg ggt gcc agc ata agc tac agt tgt gtg gat ggc tac 7974
Phe Arg Trp Gly Ala Ser Ile Ser Tyr Ser Cys Val Asp Gly Tyr
2645 2650 2655

cag ctc tcc cac tcg gcc atc ctg tcc tgt gaa ggg cgt gga gta 8019
Gln Leu Ser His Ser Ala Ile Leu Ser Cys Glu Gly Arg Gly Val
2660 2665 2670

tgg aaa gga gaa gtc cct cag tgc ttg cct gtg ttc tgt ggc gat 8064
Trp Lys Gly Glu Val Pro Gln Cys Leu Pro Val Phe Cys Gly Asp
2675 2680 2685

cca ggc act cca gca gag gga cgg ctc agt ggg aaa agc ttc acc 8109
Pro Gly Thr Pro Ala Glu Gly Arg Leu Ser Gly Lys Ser Phe Thr
2690 2695 2700

ttt aag tct gag gtc ttc atc cag tgc aaa ccc cca ttt gtg tta 8154
Phe Lys Ser Glu Val Phe Ile Gln Cys Lys Pro Pro Phe Val Leu
2705 2710 2715

gtg ggt tcc tcg agg aga acc tgc cag gcc gat ggg atg tgg agt 8199
Val Gly Ser Ser Arg Arg Thr Cys Gln Ala Asp Gly Met Trp Ser
2720 2725 2730

ggc atc cag ccc act tgt ata gat cca gcc cac acc gct tgc cca 8244
Gly Ile Gln Pro Thr Cys Ile Asp Pro Ala His Thr Ala Cys Pro
2735 2740 2745

gac ccc ggc act ccc cac ttt gga ata cag aat agc tcg aaa gga 8289
Asp Pro Gly Thr Pro His Phe Gly Ile Gln Asn Ser Ser Lys Gly
2750 2755 2760

tac gag gtt gga agc act gtg ttc ttc aga tgt aga aaa ggt tac 8334
Tyr Glu Val Gly Ser Thr Val Phe Phe Arg Cys Arg Lys Gly Tyr
2765 2770 2775

cac atc caa ggc tcc act acc cgg acc tgt ctt gcc aac ctc acg 8379
His Ile Gln Gly Ser Thr Thr Arg Thr Cys Leu Ala Asn Leu Thr
2780 2785 2790

tgg agt gga atc cag aca gag tgc atc ccc cat gcc tgc cgg cag 8424
Trp Ser Gly Ile Gln Thr Glu Cys Ile Pro His Ala Cys Arg Gln
2795 2800 2805

cca gag acc cca gcg cat gca gat gtg aga gcc atc gat ctt cca 8469
Pro Glu Thr Pro Ala His Ala Asp Val Arg Ala Ile Asp Leu Pro
2810 2815 2820

```
gct ttt  ggc tac acc tta gtc  tac acc tgt cat cca  gga ttt ttc       8514
Ala Phe  Gly Tyr Thr Leu Val  Tyr Thr Cys His Pro  Gly Phe Phe
    2825                 2830                 2835

ctt gct  ggc gga tct gag cac  agg acg tgt aaa gca  gac atg aaa       8559
Leu Ala  Gly Gly Ser Glu His  Arg Thr Cys Lys Ala  Asp Met Lys
    2840                 2845                 2850

tgg aca  gga aag tca cct gtt  tgt aaa agt aaa gga  gtg aga gaa       8604
Trp Thr  Gly Lys Ser Pro Val  Cys Lys Ser Lys Gly  Val Arg Glu
    2855                 2860                 2865

gtt aat  gaa aca gtt act aaa  act cca gtt cct tct  gat gta ttt       8649
Val Asn  Glu Thr Val Thr Lys  Thr Pro Val Pro Ser  Asp Val Phe
    2870                 2875                 2880

ttc atc  aac tcg gtg tgg aag  gga tat tat gaa tat  tta ggc aag       8694
Phe Ile  Asn Ser Val Trp Lys  Gly Tyr Tyr Glu Tyr  Leu Gly Lys
    2885                 2890                 2895

aga cag  ccg gcg act ctc act  gtg gac tgg ttt aat  gca acc agc       8739
Arg Gln  Pro Ala Thr Leu Thr  Val Asp Trp Phe Asn  Ala Thr Ser
    2900                 2905                 2910

agc aag  gtc aat gcg acc ttc  acc gca gcc tca cag  gtg cag ctg       8784
Ser Lys  Val Asn Ala Thr Phe  Thr Ala Ala Ser Gln  Val Gln Leu
    2915                 2920                 2925

gag ctg  aca ggg gtc tac aag  aag gaa gag gcc cac  ctg ctt ctg       8829
Glu Leu  Thr Gly Val Tyr Lys  Lys Glu Glu Ala His  Leu Leu Leu
    2930                 2935                 2940

aaa gcc  ttt cat atc aaa ggc  cca gca gat att ttt  gta agc aag       8874
Lys Ala  Phe His Ile Lys Gly  Pro Ala Asp Ile Phe  Val Ser Lys
    2945                 2950                 2955

ttt gaa  aat gac aac tgg gga  ctc gat ggt tat gta  tcc tca gga       8919
Phe Glu  Asn Asp Asn Trp Gly  Leu Asp Gly Tyr Val  Ser Ser Gly
    2960                 2965                 2970

ctt gag  aga gga gga ttc tcc  ttt cag ggt gat ata  cat gga aaa       8964
Leu Glu  Arg Gly Gly Phe Ser  Phe Gln Gly Asp Ile  His Gly Lys
    2975                 2980                 2985

gac ttc  ggg aag ttc aag ctg  gaa aga caa gat cct  tcc aac tct       9009
Asp Phe  Gly Lys Phe Lys Leu  Glu Arg Gln Asp Pro  Ser Asn Ser
    2990                 2995                 3000

gat gca  gat tct tca aat cat  tac cag ggc acc agc  agt ggc tct       9054
Asp Ala  Asp Ser Ser Asn His  Tyr Gln Gly Thr Ser  Ser Gly Ser
    3005                 3010                 3015

gtg gca  gct gcg att ctc gtc  ccc ttc ttc gct cta  att cta tca       9099
Val Ala  Ala Ala Ile Leu Val  Pro Phe Phe Ala Leu  Ile Leu Ser
    3020                 3025                 3030

ggg ttt  gca ttt tac ctc tac  aaa cac aga aca aga  cca aaa gtt       9144
Gly Phe  Ala Phe Tyr Leu Tyr  Lys His Arg Thr Arg  Pro Lys Val
    3035                 3040                 3045

caa tac  aat ggc tat gct ggc  cat gaa aac agt aat  gga caa gct       9189
Gln Tyr  Asn Gly Tyr Ala Gly  His Glu Asn Ser Asn  Gly Gln Ala
    3050                 3055                 3060
```

```
tca ttt gaa aac ccc atg tat gat aca aac tta aaa ccc aca gag      9234
Ser Phe Glu Asn Pro Met Tyr Asp Thr Asn Leu Lys Pro Thr Glu
    3065                3070                3075

gcc aag gct gtg agg ttt gac acg act ctg aac aca gtg tgt aca      9279
Ala Lys Ala Val Arg Phe Asp Thr Thr Leu Asn Thr Val Cys Thr
    3080                3085                3090

gtg gta tagccctcag tgccccctag gaccgactca tagccatacc tctgatggac   9335
Val Val
    3095

aagcagtaaa atcctttggt gccatatacc accccccttct actcttacct tgctgcagca   9395

acgttggcca tcgtctgctg gcataacgca gtgggaatgt cttctccatc atgcccgagt   9455

cttctgagga tcaaattgca aatacacctt catctggaaa gtggcttata aaaagcccgg   9515

ttgctgcatc caccagaaat caagaccccg acaacagcga gggcaaggaa gactgcagag   9575

tctcccagac cggtggtact taatgcctct gacttttgtg tctctgtgtg gccaggatgc   9635

ctttggtgta gtcttctgag cacaccgata catccctcag gtgcggcgac aacatggtag   9695

ccacttgatg tgtgttttgt gtttttctgt tttcttttca accctatcca ctggacatga   9755

attctttaca aaagaaaagc cttcctggag aagacgcctt ctggaaaatg cacacacaga   9815

cgctttgctt ctgccctgcc tgagacagga gctctccgga tcttcaggct ccactgggcg   9875

tccatcagcc actagggatg tttgcagatc tcacagtcag agctggtcca tcccagagtt   9935

ttttgatgct caacattttg cactagtgtg tcaagaatga ctaagtctga tttctaaaca   9995

aactatttcc acagggtttg tatccactat acattgtaca tacgcatttt ctcataccgt  10055

attctcaagc aaatgatgcc actgtcagtt aagtttggga tgcaaaggaa ggtctccccg  10115

gatacagaac agattttgaa aaggagatag tgctagtaat gctgaagaag ttactctttt  10175

aattgcttct gttggccaca ttttcatgtc aaattcattg cctacttcca gtggtggaaa  10235

tgaagcccgt gtattcccct tggtatcccc ccacttcatg tgcatacgac tattgtctac  10295

accatactaa tcaataacag gggctccag caatgtctgt tttccatgta cagatgtgaa  10355

tagtaattta ttttaggtag ctcatgaact cagttcacag tgaagtcttc ccttccggat  10415

tgtttccttt ctctgttttg taacatcacc ctcccagaat gcattgagag tctatctcac  10475

agccacaccc aagctcagag gaatcgaaag ggaaatcaaa gaagtccaaa tcagaatcgg  10535

aagggcaggc accgctcgca caccctcatg atgatctgtt ttatagatta tttgcctttc  10595

tgcaaaaaaa aaatcattac agtgattttt gaaacattaa aattccttac tgatagacta  10655

tctattgtga tatatataag ataggtggta tggccaacag ggataaaata aacagcctaa  10715

agacaaggca gggctagaga aatgtctgta agaaatttca aagagaagat catgtttatt  10775

tttatttata tttgtttgat aaaagtattt ttggaaatat aatgcttatt ttattatttg  10835

acgtttcatg cacagtccac gtggtaaaaa tcccccccttt gtacatccca gatttgcact  10895
```

gatacatggg tcaggatgtc atgctgatgt tctgtttgct gtggtgacta cattcatgcc 10955

tagctttaag acaggtggat ctgtctatct acatgatgtt taaatgcagg acttcccaga 11015

ggacagtggg taacggaaca tggcttgctt gcggctttgg aagttcagca ttctgagcgt 11075

tccagaggcc cggctgggct ccctccttct agcccactgt tcttgcaagg gctgtctgtt 11135

gtgtgccagg gctcctgact tcttctgctg acactctgtc cactgggttc catattccag 11195

gactccatgt cctaggaaag agttttgaca taggttcctc cagccaagcc gacacacatc 11255

cacggggttc ctctgggctc cacagaggtt cttcattggc tccctgggat aaattcagat 11315

gatgtcagca agagtgtgct tctataccac acattgagcc aaaacaaaac agagaacgtc 11375

agaaggtcca cggaccagag tgcgcaaggg agaacagggt tactatatat attagatgta 11435

tataaaaaca cacacacaaa catatatata ttgtacatat ctaagtttga gtcactcaga 11495

ctaggtgcaa aatgctgact ttggagtcta aactaacgtc tctgtcccca catccctggc 11555

ctctttcctg gccagttaca ttaagaagac ttgacttaga cagggcatac atacatgcaa 11615

ggaaccacat catcagacca gtgtcgtttt cctttgtgtg caaactgacc tacagctacc 11675

agactgcatc atggtattta aaaccaacat acaatattga gcggcactct cagttgagag 11735

cctagctcaa tccttcctag gannnnnnnn nnnnnnnnnn nnnnnnnnnn nnnnnnnnnn 11795

nnnnnnnnnn nnnnnnnnnn nnnnnnnnnn nnncaccagg tctcagaggc attgaagacc 11855

tagcaggaca gtcaggaaca ccttcctcag tgaggtctag acttttccct gaagcgccca 11915

gagcacagtg aggagtcacg ctctatgaat gacaggttat gtgctttgaa gctgttcaac 11975

tgttgcttgt ctttgcccat cttgccttca ggctagctgc aataattttt ttcttctgta 12035

aaatattttg taaacaataa caacaacaac aaaagctatt ataaaaaggg agaaaagaaa 12095

gctggcatta tgatcaggaa aaccatccat tcttgctgcc ccccccctcc tgtctccacc 12155

acacgctgct gtcacaacgt aggtgcggaa gaccttttttg tacagagata tatttttttat 12215

gaagaatttg taaaattatt aaatatgctg taattttttg attaatgtag gtaaattgtt 12275

aaaaaataaa tgtttttaca atatgaaact gtaattttcc cccataatgt aacattaccc 12335

tctctagctg attttcagtt ccaatcctat tcgaacatgt attaatatta aggcggcctg 12395

ttaaaatgaa cagtatcttt tttttgtca aaaaaaatta taaagagagt gtaacataac 12455

ctgtgtaatg ccacctatct ttaaagcaaa tcagagttct aattaaatat ttaattttag 12515

atttcaaaaa 12525

<210> 4

<211> 3095

<212> PRT

<213> Rattus rattus

<400> 4

```
Asp Ala Gly Lys Val Gly Asp Thr Arg Ser Val Leu Tyr Val Leu Thr
1               5                   10                  15

Gly Ser Ser Val Pro Asp Leu Ile Val Ser Met Ser Asn Gln Met Trp
            20                  25                  30

Leu His Leu Gln Ser Asp Asp Ser Ile Gly Ser Pro Gly Phe Lys Ala
        35                  40                  45

Val Tyr Gln Glu Ile Glu Lys Gly Gly Cys Gly Asp Pro Gly Ile Pro
    50                  55                  60

Ala Tyr Gly Lys Arg Thr Gly Ser Ser Phe Leu His Gly Asp Thr Leu
65                  70                  75                  80

Thr Phe Glu Cys Gln Ala Ala Phe Glu Leu Val Gly Glu Arg Val Ile
                85                  90                  95

Thr Cys Gln Arg Asn Asn Gln Trp Ser Gly Asn Lys Pro Ser Cys Val
            100                 105                 110

Phe Ser Cys Phe Phe Asn Phe Thr Ala Ser Ser Gly Ile Ile Leu Ser
        115                 120                 125

Pro Asn Tyr Pro Glu Glu Tyr Gly Asn Asn Met Asn Cys Val Trp Leu
    130                 135                 140

Ile Ile Ser Glu Pro Gly Ser Arg Ile His Leu Ile Phe Asn Asp Phe
145                 150                 155                 160

Asp Val Glu Pro Gln Phe Asp Phe Leu Ala Val Lys Asp Asp Gly Ile
                165                 170                 175

Ser Asp Ile Thr Val Leu Gly Thr Phe Ser Gly Asn Glu Val Pro Ala
            180                 185                 190

Gln Leu Ala Xaa Ser Gly His Ile Val Arg Leu Glu Phe Gln Ser Asp
        195                 200                 205

His Ser Thr Thr Gly Arg Gly Phe Asn Ile Ile Tyr Thr Thr Phe Gly
    210                 215                 220
```

Gln Asn Glu Cys His Asp Pro Gly Ile Pro Val Asn Gly Arg Arg Phe
225 230 235 240

Gly Asp Arg Phe Leu Leu Gly Ser Ser Val Ser Phe His Cys Asp Asp
245 250 255

Gly Phe Val Lys Thr Gln Gly Ser Glu Ser Ile Thr Cys Ile Leu Gln
260 265 270

Asp Gly Asn Val Val Trp Ser Ser Thr Val Pro Arg Cys Glu Ala Pro
275 280 285

Cys Gly Gly His Leu Thr Ala Ser Ser Gly Val Ile Leu Pro Pro Gly
290 295 300

Trp Pro Gly Tyr Tyr Lys Asp Ser Leu Asn Cys Glu Trp Val Ile Glu
305 310 315 320

Ala Lys Pro Gly His Ser Ile Lys Ile Thr Phe Asp Arg Phe Gln Thr
325 330 335

Glu Val Asn Tyr Asp Thr Leu Glu Val Arg Asp Gly Pro Thr Ser Ser
340 345 350

Ser Pro Leu Ile Gly Glu Tyr His Gly Thr Gln Ala Pro Gln Phe Leu
355 360 365

Ile Ser Thr Gly Asn Tyr Met Tyr Leu Leu Phe Thr Thr Asp Ser Ser
370 375 380

Arg Ala Ser Val Gly Phe Leu Ile His Tyr Glu Ser Val Thr Leu Glu
385 390 395 400

Ser Asp Ser Cys Leu Asp Pro Gly Ile Pro Val Asn Gly His Arg His
405 410 415

Gly Ser Asn Phe Gly Ile Arg Ser Thr Val Thr Phe Ser Cys Asp Pro
420 425 430

Gly Tyr Thr Leu Ser Asp Asp Asp Pro Leu Ile Cys Glu Lys Asn His
435 440 445

Gln Trp Asn His Ala Leu Pro Ser Cys Asp Ala Leu Cys Gly Gly Tyr
450 455 460

Ile His Gly Lys Ser Gly Thr Val Leu Ser Pro Gly Phe Pro Asp Phe
465 470 475 480

Tyr Pro Asn Ser Leu Asn Cys Thr Trp Thr Ile Glu Val Ser His Gly
485 490 495

Lys Gly Val Gln Met Asn Phe His Thr Phe His Leu Glu Ser Ser His
500 505 510

Asp Tyr Leu Leu Ile Thr Glu Asp Gly Ser Phe Ser Glu Pro Val Ala
515 520 525

Arg Leu Thr Gly Ser Val Leu Pro His Thr Ile Lys Ala Gly Leu Phe
530 535 540

Gly Asn Phe Thr Ala Gln Leu Arg Phe Ile Ser Asp Phe Ser Ile Ser
545 550 555 560

Tyr Glu Gly Phe Asn Ile Thr Phe Ala Glu Tyr Asp Leu Glu Pro Cys
565 570 575

Asp Asp Pro Gly Val Pro Ala Tyr Ser Arg Arg Ile Gly Phe Gln Phe
580 585 590

Gly Val Gly Asp Thr Leu Ala Phe Thr Cys Phe Gln Gly Tyr Arg Leu
595 600 605

Glu Gly Ala Thr Lys Leu Thr Cys Leu Gly Gly Gly Arg Arg Val Trp
610 615 620

Ser Ala Pro Leu Pro Arg Cys Val Ala Glu Cys Gly Ala Ser Val Lys
625 630 635 640

Gly Asn Glu Gly Thr Leu Leu Ser Pro Asn Phe Pro Ser Asn Tyr Asp
645 650 655

Asn Asn His Glu Cys Ile Tyr Lys Ile Glu Thr Glu Ala Gly Lys Gly
660 665 670

Ile His Leu Arg Ala Arg Thr Phe Gln Leu Phe Glu Gly Asp Thr Leu
675 680 685

Lys Val Tyr Asp Gly Lys Asp Ser Ser Ser Arg Ser Leu Gly Val Phe
690 695 700

Thr Arg Ser Glu Leu Met Gly Leu Val Leu Asn Ser Thr Ser Asn His
705 710 715 720

Leu Arg Leu Glu Phe Asn Ser Asn Gly Ser Asp Thr Ala Gln Gly Phe
725 730 735

Gln Leu Thr Tyr Thr Ser Phe Asp Leu Val Lys Cys Glu Asp Pro Gly
740 745 750

Ile Pro Asn Tyr Gly Tyr Arg Ile Arg Asp Asp Gly His Phe Thr Asp
755 760 765

Thr Val Val Leu Tyr Ser Cys Asn Pro Gly Tyr Ala Met His Gly Ser
770 775 780

Ser Thr Leu Thr Cys Leu Ser Gly Asp Arg Arg Val Trp Asp Lys Pro
785 790 795 800

Met Pro Ser Cys Val Ala Glu Cys Gly Gly Leu Val His Ala Ala Thr
805 810 815

Ser Gly Arg Ile Leu Ser Pro Gly Tyr Pro Ala Pro Tyr Asp Asn Asn
820 825 830

Leu His Cys Thr Trp Thr Ile Glu Ala Asp Pro Gly Lys Thr Xaa Ser
835 840 845

Leu His Phe Ile Val Phe Asp Thr Glu Thr Ala His Asp Ile Leu Lys
850 855 860

Val Trp Asp Gly Pro Val Asp Ser Asn Ile Leu Leu Lys Glu Trp Ser
865 870 875 880

Gly Ser Ala Leu Pro Glu Asp Ile His Ser Thr Phe Asn Ser Leu Thr
885 890 895

Leu Gln Phe Asp Ser Asp Phe Phe Ile Ser Lys Ser Gly Phe Ser Ile
900 905 910

Gln Phe Ser Thr Ser Ile Ala Ser Thr Cys Asn Asp Pro Gly Met Pro
915 920 925

Gln Asn Gly Thr Arg Tyr Gly Asp Ser Arg Glu Pro Gly Asp Thr Ile
930 935 940

Thr Phe Gln Cys Asp Pro Gly Tyr Gln Leu Gln Gly Gln Ala Lys Ile
945 950 955 960

Thr Cys Val Gln Leu Asn Asn Arg Phe Phe Trp Gln Pro Asp Pro Pro
965 970 975

Ser Cys Ile Ala Ala Cys Gly Gly Asn Leu Thr Gly Pro Ala Gly Val
980 985 990

Ile Leu Ser Pro Asn Tyr Pro Gln Pro Tyr Pro Pro Gly Lys Glu Cys
995 1000 1005

Asp Trp Arg Ile Lys Val Asn Pro Asp Phe Val Ile Ala Leu Ile
1010 1015 1020

Phe Lys Ser Phe Ser Met Glu Pro Ser Tyr Asp Phe Leu His Ile
1025 1030 1035

Tyr Glu Gly Lys Asp Ser Asn Ser Pro Leu Ile Gly Ser Phe Gln
1040 1045 1050

Gly Ser Gln Ala Pro Glu Arg Ile Glu Ser Ser Gly Asn Ser Leu
1055 1060 1065

Phe Leu Ala Phe Arg Ser Asp Ala Ser Val Gly Leu Ser Gly Phe
1070 1075 1080

Ala Ile Glu Phe Lys Glu Lys Pro Arg Glu Ala Cys Phe Asp Pro
1085 1090 1095

Gly Asn Ile Met Asn Gly Thr Arg Ile Gly Thr Asp Phe Lys Leu
1100 1105 1110

Gly Ser Thr Val Thr Tyr Gln Cys Asp Ser Gly Tyr Lys Ile Val
1115 1120 1125

Asp Pro Ser Ser Ile Glu Cys Val Thr Gly Ala Asp Gly Lys Pro
1130 1135 1140

Ser Trp Asp Arg Ala Leu Pro Ala Cys Gln Ala Pro Cys Gly Gly
1145 1150 1155

Gln Tyr Met Gly Ser Glu Gly Val Val Leu Ser Pro Asn Tyr Pro
1160 1165 1170

His Asn Tyr Thr Ala Gly Gln Ile Cys Ile Tyr Ser Ile Thr Val
1175 1180 1185

Pro Lys Glu Phe Val Val Phe Gly Gln Phe Ala Tyr Phe Gln Thr
1190 1195 1200

Ala Leu Asn Asp Leu Ala Glu Leu Phe Asp Gly Thr His Pro Gln
1205 1210 1215

Ala Arg Leu Leu Ser Ser Leu Ser Gly Ser His Ser Gly Glu Thr
1220 1225 1230

Leu Pro Leu Ala Thr Ser Asn Gln Ile Leu Leu Arg Phe Ser Ala
1235 1240 1245

Lys Ser Gly Ala Ser Ala Arg Gly Phe His Phe Val Tyr Gln Ala
1250 1255 1260

Val Pro Arg Thr Ser Asp Thr Gln Cys Ser Ser Val Pro Glu Pro
1265 1270 1275

Arg Tyr Gly Arg Arg Ile Gly Ser Glu Phe Ser Ala Gly Ser Ile
1280 1285 1290

Val Arg Phe Glu Cys Asn Pro Gly Tyr Leu Leu Gln Gly Ser Thr
1295 1300 1305

Ala Ile Arg Cys Gln Ser Val Pro Asn Ala Leu Ala Gln Trp Asn
1310 1315 1320

Asp Thr Ile Pro Ser Cys Val Val Pro Cys Ser Gly Asn Phe Thr
1325 1330 1335

Gln Arg Arg Gly Thr Ile Leu Ser Pro Gly Tyr Pro Glu Pro Tyr
1340 1345 1350

Gly Asn Asn Leu Asn Cys Val Trp Lys Ile Ile Val Ser Glu Gly
1355 1360 1365

Ser Gly Ile Gln Ile Gln Val Ile Ser Phe Ala Thr Glu Gln Asn
1370 1375 1380

Trp Asp Ser Leu Glu Ile His Asp Gly Gly Asp Met Thr Ala Pro
1385 1390 1395

Arg Leu Gly Ser Phe Ser Gly Thr Thr Val Pro Ala Leu Leu Asn
1400 1405 1410

Ser Thr Ser Asn Gln Leu Cys Leu His Phe Gln Ser Asp Ile Ser
1415 1420 1425

Val Ala Ala Ala Gly Phe His Leu Glu Tyr Lys Thr Val Gly Leu
1430 1435 1440

Ala Ala Cys Gln Glu Pro Ala Leu Pro Ser Asn Gly Ile Lys Ile
1445 1450 1455

Gly Asp Arg Tyr Met Val Asn Asp Val Leu Ser Phe Gln Cys Glu
1460 1465 1470

Pro Gly Tyr Thr Leu Gln Gly Arg Ser His Ile Ser Cys Met Pro
1475 1480 1485

Gly Thr Val Arg Arg Trp Asn Tyr Pro Ser Pro Leu Cys Ile Ala
1490 1495 1500

Thr Cys Gly Gly Thr Leu Thr Ser Met Ser Gly Val Ile Leu Ser
1505 1510 1515

Pro Gly Phe Pro Gly Ser Tyr Pro Asn Asn Leu Asp Cys Thr Trp
1520 1525 1530

Lys Ile Ser Leu Pro Ile Gly Tyr Gly Ala His Ile Gln Phe Leu
1535 1540 1545

Asn Phe Ser Thr Glu Ala Asn His Asp Tyr Leu Glu Ile Gln Asn
1550 1555 1560

Gly Pro Tyr His Ser Ser Pro Met Met Gly Gln Phe Ser Gly Pro
1565 1570 1575

Asp Leu Pro Ala Ser Leu Leu Ser Thr Thr His Glu Thr Leu Ile
1580 1585 1590

Arg Phe Tyr Ser Asp His Ser Gln Asn Arg Gln Gly Phe Lys Leu
1595 1600 1605

Ser Tyr Gln Ala Tyr Glu Leu Gln Asn Cys Pro Asp Pro Pro Ala
1610 1615 1620

Phe Gln Asn Gly Phe Met Ile Asn Ser Asp Tyr Ser Val Gly Gln
1625 1630 1635

Ser Ile Ser Phe Glu Cys Tyr Pro Gly Tyr Ile Leu Leu Gly His
1640 1645 1650

Pro Val Leu Thr Cys Gln His Gly Thr Asp Arg Asn Trp Asn Tyr
1655 1660 1665

Pro Phe Pro Arg Cys Asp Ala Pro Cys Gly Tyr Asn Val Thr Ser
1670 1675 1680

Gln Asn Gly Thr Ile Tyr Ser Pro Gly Phe Pro Asp Glu Tyr Pro
1685 1690 1695

Ile Leu Lys Asp Cys Leu Trp Leu Val Thr Val Pro Pro Gly His
1700 1705 1710

Gly Val Tyr Ile Asn Phe Thr Leu Leu Gln Thr Glu Ala Val Asn
1715 1720 1725

Asp Tyr Ile Ala Val Trp Asp Gly Pro Asp Gln Asn Ser Pro Gln
1730 1735 1740

Leu Gly Val Phe Ser Gly Asn Thr Ala Leu Glu Thr Ala Tyr Ser
1745 1750 1755

Ser Thr Asn Gln Val Leu Leu Lys Phe His Ser Asp Phe Ser Asn
1760 1765 1770

Gly Gly Phe Phe Val Leu Asn Phe His Ala Phe Gln Leu Lys Arg
1775 1780 1785

Cys Pro Pro Pro Pro Val Val Pro Gln Ala Asp Leu Leu Thr Glu
1790 1795 1800

Asp Glu Asp Phe Glu Ile Gly Asp Phe Val Lys Tyr Gln Cys His
1805 1810 1815

Pro Gly Tyr Thr Leu Leu Gly Ser Asp Thr Leu Thr Cys Lys Leu
1820 1825 1830

Ser Ser Gln Leu Leu Phe Gln Gly Ser Pro Pro Thr Cys Glu Ala
1835 1840 1845

Gln Cys Pro Ala Asn Glu Val Arg Thr Glu Ser Ser Gly Val Ile
1850 1855 1860

Leu Ser Pro Gly Tyr Pro Gly Asn Tyr Phe Asn Ser Gln Thr Cys
1865 1870 1875

Ala Trp Ser Ile Lys Val Glu Pro Asn Phe Asn Ile Thr Leu Phe
1880 1885 1890

Val Asp Thr Phe Gln Ser Glu Lys Gln Phe Asp Ala Leu Glu Val
1895 1900 1905

Phe Asp Gly Ser Ser Gly Gln Ser Pro Leu Leu Val Val Leu Ser
1910 1915 1920

Gly Asn His Thr Glu Gln Ser Asn Phe Thr Ser Arg Ser Asn His
1925 1930 1935

Leu Tyr Leu Arg Trp Ser Thr Asp His Ala Thr Ser Lys Lys Gly
1940 1945 1950

Phe Lys Ile Arg Tyr Ala Ala Pro Tyr Cys Ser Leu Thr Ser Thr
1955 1960 1965

Leu Lys Asn Gly Gly Val Leu Asn Lys Thr Ala Gly Ala Leu Gly
1970 1975 1980

Ser Lys Val Gln Tyr Phe Cys Lys Pro Gly Tyr Arg Met Ile Gly
1985 1990 1995

His Ser Asn Ala Thr Cys Arg Arg Asn Pro Val Gly Val Tyr Gln
2000 2005 2010

Trp Asp Ser Met Ala Pro Leu Cys Gln Ala Val Ser Cys Gly Ile
2015 2020 2025

Pro Glu Ala Pro Gly Asn Gly Ser Phe Thr Gly Asn Glu Phe Thr
2030 2035 2040

Leu Asp Ser Lys Val Thr Tyr Glu Cys Asn Glu Gly Phe Lys Leu
2045 2050 2055

Asp Ala Ser Gln Gln Ala Thr Ala Val Cys Gln Glu Asp Gly Leu
2060 2065 2070

Trp Ser Asn Arg Gly Lys Pro Pro Thr Cys Lys Pro Val Pro Cys
2075 2080 2085

Pro Ser Ile Glu Gly Gln Leu Ser Glu His Val Leu Trp Arg Leu
2090 2095 2100

Val Ser Gly Ser Leu Asn Glu Tyr Gly Ala Gln Val Leu Leu Ser
2105 2110 2115

Cys Ser Pro Gly Tyr Phe Leu Gln Gly Gln Arg Leu Leu Gln Cys
2120 2125 2130

Gln Ala Asn Gly Thr Trp Asn Thr Glu Glu Asp Arg Pro Arg Cys
2135 2140 2145

Lys Val Ile Ser Cys Gly Ser Leu Ser Phe Pro Pro Asn Gly Asn
2150 2155 2160

Lys Ile Gly Thr Leu Thr Met Tyr Gly Ala Thr Ala Ile Phe Thr
2165 2170 2175

Cys Asn Thr Gly Tyr Thr Leu Val Gly Ser His Val Arg Glu Cys
2180 2185 2190

Leu Ala Asn Gly Leu Trp Ser Gly Ser Glu Thr Arg Cys Leu Ala
2195 2200 2205

Gly His Cys Gly Ser Pro Asp Pro Ile Val Asn Gly His Ile Ser
2210 2215 2220

Gly Asp Gly Phe Ser Tyr Arg Asp Thr Val Val Tyr Gln Cys Asn
2225 2230 2235

Pro Gly Phe Arg Leu Val Gly Thr Ser Val Arg Ile Cys Leu Gln
2240 2245 2250

Asp His Lys Trp Ser Gly Gln Thr Pro Val Cys Val Pro Ile Thr
2255 2260 2265

Cys Gly His Pro Gly Asn Pro Ala His Gly Leu Thr Asn Gly Ser
2270 2275 2280

Glu Phe Asn Leu Asn Asp Leu Val Asn Phe Thr Cys His Thr Gly
2285 2290 2295

Tyr Leu Leu Gln Gly Ala Ser Arg Ala Gln Cys Arg Ser Asn Gly
2300 2305 2310

Gln Trp Ser Ser Pro Leu Pro Ile Cys Arg Val Val Asn Cys Ser
2315 2320 2325

Asp Pro Gly Phe Val Glu Asn Ala Val Arg His Gly Gln Gln Asn
2330 2335 2340

Phe Pro Glu Ser Phe Glu Tyr Gly Thr Ser Val Met Tyr His Cys
2345 2350 2355

Lys Lys Gly Phe Tyr Leu Leu Gly Ser Ser Ala Leu Thr Cys Met
2360 2365 2370

Ala Ser Gly Leu Trp Asp Arg Ser Leu Pro Lys Cys Leu Ala Ile
2375 2380 2385

Ser Cys Gly His Pro Gly Val Pro Ala Asn Ala Val Leu Thr Gly
2390 2395 2400

Glu Leu Phe Thr Phe Gly Ala Thr Val Gln Tyr Ser Cys Lys Gly
2405 2410 2415

Gly Gln Ile Leu Thr Gly Asn Ser Thr Arg Val Cys Gln Glu Asp
2420 2425 2430

Ser His Trp Ser Gly Ser Leu Pro His Cys Ser Gly Asn Ser Pro
2435 2440 2445

Gly Phe Cys Gly Asp Pro Gly Thr Pro Ala His Gly Ser Arg Leu
2450 2455 2460

Gly Asp Glu Phe Lys Thr Lys Ser Leu Leu Arg Phe Ser Cys Glu
2465 2470 2475

Met Gly His Gln Leu Arg Gly Ser Ala Glu Arg Thr Cys Leu Val
2480 2485 2490

Asn Gly Ser Trp Ser Gly Val Gln Pro Val Cys Glu Ala Val Ser
2495 2500 2505

Cys Gly Asn Pro Gly Thr Pro Thr Asn Gly Met Ile Leu Ser Ser
2510 2515 2520

Asp Gly Ile Leu Phe Ser Ser Ser Val Ile Tyr Ala Cys Trp Glu
2525 2530 2535

Gly Tyr Lys Thr Ser Gly Leu Met Thr Arg His Cys Thr Ala Asn
2540 2545 2550

Gly Thr Trp Thr Gly Thr Ala Pro Asp Cys Thr Ile Ile Ser Cys
2555 2560 2565

Gly Asp Pro Gly Thr Leu Pro Asn Gly Ile Gln Phe Gly Thr Asp
2570 2575 2580

Phe Thr Phe Asn Lys Thr Val Ser Tyr Gln Cys Asn Pro Gly Tyr
2585 2590 2595

Leu Met Glu Pro Pro Thr Ser Pro Thr Ile Arg Cys Thr Lys Asp
2600 2605 2610

Gly Thr Trp Asn Gln Thr Arg Pro Leu Cys Lys Ala Val Leu Cys
2615 2620 2625

Ser Gln Pro Pro Ser Val Pro Asn Gly Lys Val Glu Gly Ser Asp
2630 2635 2640

Phe Arg Trp Gly Ala Ser Ile Ser Tyr Ser Cys Val Asp Gly Tyr
2645 2650 2655

Gln Leu Ser His Ser Ala Ile Leu Ser Cys Glu Gly Arg Gly Val
2660 2665 2670

Trp Lys Gly Glu Val Pro Gln Cys Leu Pro Val Phe Cys Gly Asp
2675 2680 2685

Pro Gly Thr Pro Ala Glu Gly Arg Leu Ser Gly Lys Ser Phe Thr
2690 2695 2700

Phe Lys Ser Glu Val Phe Ile Gln Cys Lys Pro Pro Phe Val Leu
2705 2710 2715

Val Gly Ser Ser Arg Arg Thr Cys Gln Ala Asp Gly Met Trp Ser
2720 2725 2730

Gly Ile Gln Pro Thr Cys Ile Asp Pro Ala His Thr Ala Cys Pro
2735 2740 2745

Asp Pro Gly Thr Pro His Phe Gly Ile Gln Asn Ser Ser Lys Gly
2750 2755 2760

Tyr Glu Val Gly Ser Thr Val Phe Phe Arg Cys Arg Lys Gly Tyr
2765 2770 2775

His Ile Gln Gly Ser Thr Thr Arg Thr Cys Leu Ala Asn Leu Thr
2780 2785 2790

Trp Ser Gly Ile Gln Thr Glu Cys Ile Pro His Ala Cys Arg Gln
2795 2800 2805

Pro Glu Thr Pro Ala His Ala Asp Val Arg Ala Ile Asp Leu Pro
2810 2815 2820

Ala Phe Gly Tyr Thr Leu Val Tyr Thr Cys His Pro Gly Phe Phe
2825 2830 2835

Leu Ala Gly Gly Ser Glu His Arg Thr Cys Lys Ala Asp Met Lys
2840 2845 2850

Trp Thr Gly Lys Ser Pro Val Cys Lys Ser Lys Gly Val Arg Glu
2855 2860 2865

Val Asn Glu Thr Val Thr Lys Thr Pro Val Pro Ser Asp Val Phe
2870 2875 2880

Phe Ile Asn Ser Val Trp Lys Gly Tyr Tyr Glu Tyr Leu Gly Lys
2885 2890 2895

Arg Gln Pro Ala Thr Leu Thr Val Asp Trp Phe Asn Ala Thr Ser
2900 2905 2910

Ser Lys Val Asn Ala Thr Phe Thr Ala Ala Ser Gln Val Gln Leu
2915 2920 2925

Glu Leu Thr Gly Val Tyr Lys Lys Glu Glu Ala His Leu Leu Leu
2930 2935 2940

Lys Ala Phe His Ile Lys Gly Pro Ala Asp Ile Phe Val Ser Lys
2945 2950 2955

Phe Glu Asn Asp Asn Trp Gly Leu Asp Gly Tyr Val Ser Ser Gly
2960 2965 2970

Leu Glu Arg Gly Gly Phe Ser Phe Gln Gly Asp Ile His Gly Lys
2975 2980 2985

Asp Phe Gly Lys Phe Lys Leu Glu Arg Gln Asp Pro Ser Asn Ser
2990 2995 3000

Asp Ala Asp Ser Ser Asn His Tyr Gln Gly Thr Ser Ser Gly Ser
3005 3010 3015

Val Ala Ala Ala Ile Leu Val Pro Phe Phe Ala Leu Ile Leu Ser
3020 3025 3030

Gly Phe Ala Phe Tyr Leu Tyr Lys His Arg Thr Arg Pro Lys Val
3035 3040 3045

Gln Tyr Asn Gly Tyr Ala Gly His Glu Asn Ser Asn Gly Gln Ala
3050 3055 3060

Ser Phe Glu Asn Pro Met Tyr Asp Thr Asn Leu Lys Pro Thr Glu
3065 3070 3075

Ala Lys Ala Val Arg Phe Asp Thr Thr Leu Asn Thr Val Cys Thr
3080 3085 3090

Val Val
3095

<210> 5

<211> 2527

<212> PRT

<213> Homo sapiens

<220>

<221> misc

<222> (684)..(684)

<223> X = amino acid

<220>

<221> misc

<222> (1134)..(1134)

<223> X = amino acid

<400> 5

```
Lys Ser Cys Arg Asn Pro Pro Asp Pro Val Asn Gly Met Val His Val
1               5                   10                  15

Ile Lys Gly Ile Gln Phe Gly Ser Gln Ile Lys Tyr Ser Cys Thr Lys
            20                  25                  30

Gly Tyr Arg Leu Ile Gly Ser Ser Ser Ala Thr Cys Ile Ile Ser Gly
        35                  40                  45

Asp Thr Gln Asn Cys Pro Asp Pro Pro Pro Phe Gln Asn Gly Tyr Met
    50                  55                  60

Ile Asn Ser Asp Tyr Ser Val Gly Gln Ser Val Ser Phe Glu Cys Tyr
65                  70                  75                  80

Pro Gly Tyr Ile Leu Ile Gly His Pro Val Leu Thr Cys Gln His Gly
                85                  90                  95

Ile Asn Val Ile Trp Asp Asn Glu Thr Pro Ile Cys Asp Arg Ile Pro
            100                 105                 110

Cys Gly Leu Pro Pro Thr Ile Thr Asn Gly Asp Phe Ile Ser Thr Asn
        115                 120                 125

Arg Glu Asn Phe His Tyr Gly Ser Val Val Thr Tyr Arg Cys Asn Pro
    130                 135                 140

Gly Arg Asn Trp Asn Tyr Pro Phe Pro Arg Cys Asp Ala Pro Cys Gly
145                 150                 155                 160

Tyr Asn Val Thr Ser Gln Asn Gly Thr Ile Tyr Ser Pro Gly Phe Pro
                165                 170                 175

Asp Glu Tyr Pro Ile Leu Lys Asp Cys Ile Trp Leu Ile Thr Val Pro
            180                 185                 190

Pro Gly Ser Gly Gly Arg Lys Val Phe Glu Leu Val Gly Glu Pro Ser
        195                 200                 205

Ile Tyr Cys Thr Ser Asn Asp Asp Gln Val Gly Ile Trp Ser Gly Pro
    210                 215                 220
```

Ala Pro Gln Cys Ile Ile Pro Asn Lys Cys Thr Pro Pro Asn Val Glu
225 230 235 240

Asn His Gly Val Tyr Ile Asn Phe Thr Leu Leu Gln Thr Glu Ala Val
245 250 255

Asn Asp Tyr Ile Ala Val Trp Asp Gly Pro Asp Gln Asn Ser Pro Gln
260 265 270

Leu Gly Val Phe Ser Gly Asn Thr Ala Leu Glu Thr Gly Ile Leu Val
275 280 285

Ser Asp Asn Arg Ser Leu Phe Ser Leu Asn Glu Val Val Glu Phe Arg
290 295 300

Cys Gln Pro Gly Phe Val Met Lys Gly Pro Arg Arg Val Lys Cys Gln
305 310 315 320

Ala Leu Asn Lys Trp Glu Pro Glu Leu Pro Ser Cys Ser Arg Ala Tyr
325 330 335

Ser Ser Thr Asn Gln Val Leu Leu Lys Phe His Ser Asp Phe Ser Asn
340 345 350

Gly Gly Phe Phe Val Leu Asn Phe His Ala Phe Gln Leu Lys Val Cys
355 360 365

Gln Pro Pro Pro Asp Val Leu His Ala Glu Arg Thr Gln Arg Asp Lys
370 375 380

Asp Asn Phe Ser Pro Gly Gln Glu Val Phe Tyr Ser Cys Glu Pro Gly
385 390 395 400

Tyr Asp Leu Arg Gly Ala Ala Ser Met Arg Cys Thr Pro Gln Lys Cys
405 410 415

Gln Pro Pro Pro Ala Val Pro Gln Ala Glu Met Leu Thr Glu Asp Asp
420 425 430

Asp Phe Glu Ile Gly Asp Phe Val Lys Tyr Gln Cys His Pro Gly Tyr
435 440 445

Thr Leu Val Gly Thr Asp Ile Leu Thr Cys Lys Leu Ser Ser Gln Gly
450 455 460

Asp Trp Ser Pro Ala Ala Pro Thr Cys Glu Val Lys Ser Cys Asp Asp
465 470 475 480

Phe Met Gly Gln Leu Leu Asn Gly Arg Leu Gln Phe Glu Gly Ser Leu
485 490 495

Pro Thr Cys Glu Ala Gln Cys Pro Ala Asn Glu Val Arg Thr Gly Ser
500 505 510

Ser Gly Val Ile Leu Ser Pro Gly Tyr Pro Gly Asn Tyr Phe Asn Ser
515 520 525

Gln Thr Cys Ser Trp Ser Ile Lys Val Glu Pro Asn Leu Gln Leu Gly
530 535 540

Ala Lys Val Asp Phe Val Cys Asp Glu Gly Phe Gln Leu Lys Gly Ser
545 550 555 560

```
Ser Ala Ser Tyr Cys Val Leu Ala Gly Met Glu Ser Asn Tyr Asn Ile
                565                 570                 575

Thr Ile Phe Val Asp Thr Phe Gln Ser Glu Lys Gln Phe Asp Ala Leu
            580                 585                 590

Glu Val Phe Asp Gly Ser Ser Gly Gln Ser Pro Leu Leu Val Val Leu
        595                 600                 605

Ser Gly Asn His Thr Glu Gln Ser Asn Phe Thr Ser Arg Ser Leu Trp
    610                 615                 620

Asn Ser Ser Val Pro Val Cys Glu Gln Ile Phe Cys Pro Ser Pro Pro
625                 630                 635                 640

Val Ile Pro Asn Gly Arg His Thr Gly Lys Pro Leu Glu Val Phe Pro
                645                 650                 655

Phe Gly Lys Asn Gln Leu Tyr Leu Arg Trp Ser Thr Asp His Ala Thr
            660                 665                 670

Ser Lys Lys Gly Phe Lys Ile Arg Tyr Ala Ala Pro Tyr Cys Ser Leu
        675                 680                 685

Thr His Pro Leu Lys Asn Gly Gly Ile Leu Asn Arg Thr Ala Gly Ala
    690                 695                 700

Val Gly Ser Ala Val Asn Tyr Thr Cys Asp Pro His Pro Asp Arg Gly
705                 710                 715                 720

Thr Ser Phe Asp Leu Ile Gly Glu Ser Thr Ile Arg Cys Thr Ser Asp
                725                 730                 735

Pro Gln Gly Asn Gly Val Trp Ser Ser Pro Ala Pro Arg Cys Gly Ile
            740                 745                 750

Leu Gly His Cys Gln Lys Val His Tyr Phe Cys Lys Pro Gly Tyr Arg
        755                 760                 765

Met Val Gly His Ser Asn Ala Thr Cys Arg Arg Asn Pro Leu Gly Met
    770                 775                 780

Tyr Gln Trp Asp Ser Leu Thr Pro Leu Cys Gln Ala Val Ser Cys Gly
785                 790                 795                 800

Ala Pro Asp His Phe Leu Phe Ala Lys Leu Lys Thr Gln Thr Asn Ala
                805                 810                 815

Ser Asp Phe Pro Ile Gly Thr Ser Leu Lys Tyr Glu Cys Arg Pro Glu
            820                 825                 830

Tyr Tyr Gly Arg Pro Phe Ser Ile Thr Cys Leu Asp Asn Leu Val Ile
        835                 840                 845

Pro Glu Ser Pro Gly Asn Gly Ser Phe Thr Gly Asn Glu Phe Thr Leu
    850                 855                 860

Asp Ser Lys Val Val Tyr Glu Cys His Glu Gly Phe Lys Leu Glu Ser
865                 870                 875                 880

Ser Gln Gln Ala Thr Ala Val Cys Gln Glu Asp Gly Leu Trp Ser Ser
                885                 890                 895
```

```
Pro Lys Asp Val Cys Lys Arg Lys Ser Cys Lys Thr Pro Pro Asp Pro
                900                 905                 910

Val Asn Gly Met Val His Val Ile Thr Asp Ile Gln Val Gly Ser Arg
            915                 920                 925

Ile Asn Tyr Ser Cys Thr Thr Trp Ser Asn Lys Gly Lys Pro Pro Thr
    930                 935                 940

Cys Lys Pro Val Ala Cys Pro Ser Ile Glu Ala Gln Leu Ser Glu His
945                 950                 955                 960

Val Ile Trp Arg Leu Val Ser Gly Ser Leu Asn Glu Tyr Gly Ala Gln
                965                 970                 975

Val Leu Leu Ser Cys Ser Pro Gly His Arg Leu Ile Gly His Ser Ser
            980                 985                 990

Ala Glu Cys Ile Leu Ser Gly Asn Ala Ala His Trp Ser Thr Lys Pro
        995                 1000                1005

Pro Ile Cys Gln Arg Ile Pro Cys Gly Leu Pro Pro Thr Ile Ala
    1010                1015                1020

Asn Gly Asp Phe Ile Ser Thr Asn Gly Tyr Tyr Leu Glu Gly Trp
    1025                1030                1035

Arg Leu Leu Arg Cys Gln Ala Asn Gly Thr Trp Asn Ile Gly Asp
    1040                1045                1050

Glu Arg Pro Ser Cys Arg Val Ile Ser Cys Gly Ser Leu Ser Phe
    1055                1060                1065

Pro Pro Asn Gly Asn Lys Ile Gly Thr Leu Arg Glu Asn Phe His
    1070                1075                1080

Tyr Gly Ser Val Val Thr Tyr Arg Cys Asn Pro Gly Ser Gly Gly
    1085                1090                1095

Arg Lys Val Phe Glu Leu Val Gly Glu Pro Ser Ile Tyr Cys Thr
    1100                1105                1110

Ser Asn Asp Asp Gln Val Gly Ile Trp Ser Gly Pro Ala Pro Gln
    1115                1120                1125

Thr Val Tyr Gly Ala Thr Ala Ile Phe Thr Cys Asn Thr Gly Tyr
    1130                1135                1140

Thr Leu Val Gly Ser His Val Arg Glu Cys Leu Ala Asn Gly Leu
    1145                1150                1155

Trp Ser Gly Ser Glu Thr Arg Cys Ile Xaa Pro Asn Lys Cys Thr
    1160                1165                1170

Pro Pro Asn Val Glu Asn Gly Ile Leu Val Ser Asp Asn Arg Ser
    1175                1180                1185

Leu Phe Ser Leu Asn Glu Val Val Glu Phe Arg Cys Gln Pro Gly
    1190                1195                1200

Phe Val Met Lys Gly Pro Arg Arg Val Lys Cys Gln Cys Leu Ala
    1205                1210                1215
```

Gly His Cys Gly Ser Pro Asp Pro Ile Val Asn Gly His Ile Ser
1220 1225 1230

Gly Asp Gly Phe Ser Tyr Arg Asp Thr Val Val Tyr Gln Cys Asn
1235 1240 1245

Pro Gly Phe Arg Leu Val Gly Thr Ser Val Arg Ile Cys Leu Ala
1250 1255 1260

Leu Asn Lys Trp Glu Pro Glu Leu Pro Ser Cys Ser Arg Val Cys
1265 1270 1275

Gln Pro Pro Pro Asp Val Leu His Ala Glu Arg Thr Gln Arg Asp
1280 1285 1290

Lys Asp Asn Phe Ser Pro Gly Gln Glu Val Phe Tyr Ser Cys Glu
1295 1300 1305

Pro Gly Tyr Gln Asp His Lys Trp Ser Gly Gln Thr Pro Val Cys
1310 1315 1320

Val Pro Ile Thr Cys Gly His Pro Gly Asn Pro Ala His Gly Phe
1325 1330 1335

Thr Asn Gly Ser Glu Phe Asn Leu Asn Asp Val Val Asn Phe Thr
1340 1345 1350

Cys Asn Thr Gly Tyr Asp Leu Arg Gly Ala Ala Ser Met Arg Cys
1355 1360 1365

Thr Pro Gln Gly Asp Trp Ser Pro Ala Ala Pro Thr Cys Glu Val
1370 1375 1380

Lys Ser Cys Asp Asp Phe Met Gly Gln Leu Leu Asn Gly Arg Val
1385 1390 1395

Leu Phe Pro Val Asn Leu Gln Leu Leu Gln Gly Val Ser Arg Ala
1400 1405 1410

Gln Cys Arg Ser Asn Gly Gln Trp Ser Ser Pro Leu Pro Thr Cys
1415 1420 1425

Arg Val Val Asn Cys Ser Asp Pro Gly Phe Val Glu Asn Ala Ile
1430 1435 1440

Arg His Gly Gln Gln Asn Phe Pro Glu Ser Phe Glu Leu Gly Ala
1445 1450 1455

Lys Val Asp Phe Val Cys Asp Glu Gly Phe Gln Leu Lys Gly Ser
1460 1465 1470

Ser Ala Ser Tyr Cys Val Leu Ala Gly Met Glu Ser Leu Trp Asn
1475 1480 1485

Ser Ser Val Pro Val Cys Glu Gln Ile Phe Cys Pro Ser Pro Pro
1490 1495 1500

Val Ile Tyr Gly Met Ser Ile Leu Tyr His Cys Lys Lys Gly Phe
1505 1510 1515

Tyr Leu Leu Gly Ser Ser Ala Leu Thr Cys Met Ala Asn Gly Leu
1520 1525 1530

Trp Asp Arg Ser Leu Pro Lys Cys Leu Ala Ile Ser Cys Gly His
1535 1540 1545

Pro Gly Val Pro Pro Asn Gly Arg His Thr Gly Lys Pro Leu Glu
1550 1555 1560

Val Phe Pro Phe Gly Lys Thr Val Asn Tyr Thr Cys Asp Pro His
1565 1570 1575

Pro Asp Arg Gly Thr Ser Phe Asp Leu Ile Gly Glu Ser Thr Ile
1580 1585 1590

Arg Cys Thr Ser Asp Pro Gln Gly Asn Ala Asn Ala Val Leu Thr
1595 1600 1605

Gly Glu Leu Phe Thr Tyr Gly Ala Val Val His Tyr Ser Cys Arg
1610 1615 1620

Gly Ser Glu Ser Leu Ile Gly Asn Asp Thr Arg Val Cys Gln Glu
1625 1630 1635

Asp Ser His Gly Val Trp Ser Ser Pro Ala Pro Arg Cys Gly Ile
1640 1645 1650

Leu Gly His Cys Gln Ala Pro Asp His Phe Leu Phe Ala Lys Leu
1655 1660 1665

Lys Thr Gln Thr Asn Ala Ser Asp Phe Pro Ile Gly Thr Ser Leu
1670 1675 1680

Lys Tyr Glu Trp Ser Gly Ala Leu Pro His Cys Thr Gly Asn Asn
1685 1690 1695

Pro Gly Phe Cys Gly Asp Pro Gly Thr Pro Ala His Gly Ser Arg
1700 1705 1710

Leu Gly Asp Asp Phe Lys Thr Lys Ser Leu Leu Arg Phe Ser Cys
1715 1720 1725

Arg Pro Glu Tyr Tyr Gly Arg Pro Phe Ser Ile Thr Cys Leu Asp
1730 1735 1740

Asn Leu Val Trp Ser Ser Pro Lys Asp Val Cys Lys Arg Lys Ser
1745 1750 1755

Cys Lys Thr Pro Pro Asp Pro Val Asn Gly Met Val His Val Ile
1760 1765 1770

Thr Asp Cys Glu Met Gly His Gln Leu Arg Gly Ser Pro Glu Arg
1775 1780 1785

Thr Cys Leu Leu Asn Gly Ser Trp Ser Gly Leu Gln Pro Val Cys
1790 1795 1800

Glu Ala Val Ser Cys Gly Asn Pro Gly Thr Pro Thr Asn Gly Met
1805 1810 1815

Ile Val Ser Ser Asp Gly Ile Gln Val Gly Ser Arg Ile Asn Tyr
1820 1825 1830

Ser Cys Thr Thr Gly His Arg Leu Ile Gly His Ser Ser Ala Glu
1835 1840 1845

| | | | | | | | | | | | | | | |
|---|---|---|---|---|---|---|---|---|---|---|---|---|---|---|
| Cys | Ile | Leu | Ser | Gly | Asn | Ala | Ala | His | Trp | Ser | Thr | Lys | Pro | Pro |
| | 1850 | | | | | 1855 | | | | | 1860 | | | |
| Ile | Cys | Gln | Arg | Ile | Pro | Cys | Gly | Leu | Pro | Pro | Ile | Leu | Phe | Ser |
| | 1865 | | | | | 1870 | | | | | 1875 | | | |
| Ser | Ser | Val | Ile | Tyr | Ala | Cys | Trp | Glu | Gly | Tyr | Lys | Thr | Ser | Gly |
| | 1880 | | | | | 1885 | | | | | 1890 | | | |
| Leu | Met | Thr | Arg | His | Cys | Thr | Ala | Asn | Gly | Thr | Trp | Thr | Gly | Thr |
| | 1895 | | | | | 1900 | | | | | 1905 | | | |
| Ala | Pro | Asp | Cys | Thr | Ile | Ile | Ser | Cys | Gly | Asp | Pro | Gly | Thr | Ile |
| | 1910 | | | | | 1915 | | | | | 1920 | | | |
| Ala | Asn | Gly | Asp | Phe | Ile | Ser | Thr | Asn | Arg | Glu | Asn | Phe | His | Tyr |
| | 1925 | | | | | 1930 | | | | | 1935 | | | |
| Gly | Ser | Val | Val | Thr | Tyr | Arg | Cys | Asn | Pro | Gly | Ser | Gly | Gly | Arg |
| | 1940 | | | | | 1945 | | | | | 1950 | | | |
| Lys | Val | Phe | Glu | Leu | Val | Gly | Glu | Pro | Ser | Ile | Tyr | Cys | Thr | Ser |
| | 1955 | | | | | 1960 | | | | | 1965 | | | |
| Asn | Asp | Asp | Thr | Leu | Ala | Asn | Gly | Ile | Gln | Phe | Gly | Thr | Asp | Phe |
| | 1970 | | | | | 1975 | | | | | 1980 | | | |
| Thr | Phe | Asn | Lys | Thr | Val | Ser | Tyr | Gln | Cys | Asn | Pro | Gly | Tyr | Val |
| | 1985 | | | | | 1990 | | | | | 1995 | | | |
| Met | Glu | Ala | Val | Thr | Ser | Ala | Thr | Ile | Arg | Cys | Thr | Lys | Asp | Gln |
| | 2000 | | | | | 2005 | | | | | 2010 | | | |
| Val | Gly | Ile | Trp | Ser | Gly | Pro | Ala | Pro | Gln | Cys | Ile | Xaa | Pro | Asn |
| | 2015 | | | | | 2020 | | | | | 2025 | | | |
| Lys | Cys | Thr | Pro | Pro | Asn | Val | Glu | Asn | Gly | Ile | Leu | Val | Ser | Asp |
| | 2030 | | | | | 2035 | | | | | 2040 | | | |
| Asn | Arg | Ser | Leu | Phe | Ser | Leu | Asn | Glu | Val | Val | Glu | Phe | Arg | Cys |
| | 2045 | | | | | 2050 | | | | | 2055 | | | |
| Gln | Pro | Gly | Phe | Gly | Arg | Trp | Asn | Pro | Ser | Lys | Pro | Val | Cys | Lys |
| | 2060 | | | | | 2065 | | | | | 2070 | | | |
| Ala | Val | Leu | Cys | Pro | Gln | Pro | Pro | Pro | Val | Gln | Asn | Gly | Thr | Val |
| | 2075 | | | | | 2080 | | | | | 2085 | | | |
| Glu | Gly | Ser | Asp | Phe | Arg | Trp | Gly | Ser | Ser | Ile | Ser | Tyr | Ser | Cys |
| | 2090 | | | | | 2095 | | | | | 2100 | | | |
| Met | Asp | Gly | Tyr | Val | Met | Lys | Gly | Pro | Arg | Arg | Val | Lys | Cys | Gln |
| | 2105 | | | | | 2110 | | | | | 2115 | | | |
| Ala | Leu | Asn | Lys | Trp | Glu | Pro | Glu | Leu | Pro | Ser | Cys | Ser | Arg | Val |
| | 2120 | | | | | 2125 | | | | | 2130 | | | |
| Cys | Gln | Pro | Pro | Pro | Asp | Val | Leu | His | Ala | Glu | Arg | Thr | Gln | Arg |
| | 2135 | | | | | 2140 | | | | | 2145 | | | |
| Asp | Lys | Asp | Asn | Phe | Ser | Pro | Gly | Gln | Leu | Ser | His | Ser | Ala | Ile |
| | 2150 | | | | | 2155 | | | | | 2160 | | | |

Leu Ser Cys Glu Gly Arg Gly Val Trp Lys Gly Glu Ile Pro Gln
2165 2170 2175

Cys Leu Pro Val Phe Cys Gly Asp Pro Gly Ile Pro Ala Glu Gly
2180 2185 2190

Arg Leu Ser Gly Lys Ser Phe Thr Tyr Lys Gln Glu Val Phe Tyr
2195 2200 2205

Ser Cys Glu Pro Gly Tyr Asp Leu Arg Gly Ala Ala Ser Met Arg
2210 2215 2220

Cys Thr Pro Gln Gly Asp Trp Ser Pro Ala Ala Pro Thr Cys Glu
2225 2230 2235

Val Lys Ser Cys Asp Asp Phe Met Gly Gln Leu Leu Ser Glu Val
2240 2245 2250

Phe Phe Gln Cys Lys Ser Pro Phe Ile Leu Val Gly Ser Ser Arg
2255 2260 2265

Arg Val Cys Gln Ala Asp Gly Thr Trp Ser Gly Ile Gln Pro Thr
2270 2275 2280

Cys Ile Asp Pro Ala His Asn Thr Cys Pro Asp Pro Gly Thr Pro
2285 2290 2295

His Asn Gly Arg Val Leu Phe Pro Val Asn Leu Gln Leu Gly Ala
2300 2305 2310

Lys Val Asp Phe Val Cys Asp Glu Gly Phe Gln Leu Lys Gly Ser
2315 2320 2325

Ser Ala Ser Tyr Cys Val Leu Ala Gly Met Glu Ser Leu Trp Asn
2330 2335 2340

Ser Ser Val Pro Val Cys Phe Gly Ile Gln Asn Ser Ser Arg Gly
2345 2350 2355

Tyr Glu Val Gly Ser Thr Val Phe Phe Arg Cys Arg Lys Gly Tyr
2360 2365 2370

His Ile Gln Gly Ser Thr Thr Arg Thr Cys Leu Ala Asn Leu Thr
2375 2380 2385

Trp Ser Gly Ile Gln Thr Glu Cys Glu Gln Ile Phe Cys Pro Ser
2390 2395 2400

Pro Pro Val Ile Pro Asn Gly Arg His Thr Gly Lys Pro Leu Glu
2405 2410 2415

Val Phe Pro Phe Gly Lys Ala Val Asn Tyr Thr Cys Asp Pro His
2420 2425 2430

Pro Asp Arg Gly Thr Ser Phe Asp Leu Ile Gly Glu Ser Ile Pro
2435 2440 2445

His Ala Cys Arg Gln Pro Glu Thr Pro Ala His Ala Asp Val Arg
2450 2455 2460

Ala Ile Asp Leu Pro Thr Phe Gly Tyr Thr Leu Val Tyr Thr Cys
2465 2470 2475

```
His Pro Gly Phe Phe Leu Ala Gly Gly Ser Thr Ile Arg Cys Thr
    2480                2485                2490

Ser Asp Pro Gln Gly Asn Gly Val Trp Ser Ser Pro Ala Pro Arg
    2495                2500                2505

Cys Glu His Arg Thr Cys Lys Ala Asp Met Lys Trp Thr Gly Lys
    2510                2515                2520

Ser Pro Val Cys
    2525
```

<210> 6

<211> 10433

<212> DNA

<213> Homo sapiens

<220>

<221> CDS

<222> (1)..(9300)

<400> 6

```
acc ctg acg gtt ggt gat gct ggg aag gtg gga gac acc aga tcg gtc      48
Thr Leu Thr Val Gly Asp Ala Gly Lys Val Gly Asp Thr Arg Ser Val
1               5                   10                  15

ttg tac gtg ctc acg gga tcc agt gtt cct gac ctc att gtg agc atg      96
Leu Tyr Val Leu Thr Gly Ser Ser Val Pro Asp Leu Ile Val Ser Met
            20                  25                  30

agc aac cag atg tgg cta cat ctg cag tcg gat gat agc att ggc tca     144
Ser Asn Gln Met Trp Leu His Leu Gln Ser Asp Asp Ser Ile Gly Ser
        35                  40                  45

cct ggg ttt aaa gct gtt tac caa gaa att gaa aag gga ggg tgt ggg     192
Pro Gly Phe Lys Ala Val Tyr Gln Glu Ile Glu Lys Gly Gly Cys Gly
    50                  55                  60

gat cct gga atc ccc gcc tat ggg aag cgg acg ggc agc agt ttc ctc     240
Asp Pro Gly Ile Pro Ala Tyr Gly Lys Arg Thr Gly Ser Ser Phe Leu
65                  70                  75                  80

cat gga gat aca ctc acc ttt gaa tgc ccg gcg gcc ttt gag ctg gtg     288
His Gly Asp Thr Leu Thr Phe Glu Cys Pro Ala Ala Phe Glu Leu Val
                85                  90                  95

ggg gag aga gtt atc acc tgt cag cag aac aat cag tgg tct ggc aac     336
Gly Glu Arg Val Ile Thr Cys Gln Gln Asn Asn Gln Trp Ser Gly Asn
            100                 105                 110

aag ccc agc tgt gta ttt tca tgt ttc ttc aac ttt acg gca tca tct     384
Lys Pro Ser Cys Val Phe Ser Cys Phe Phe Asn Phe Thr Ala Ser Ser
        115                 120                 125
```

```
ggg att att ctg tca cca aat tat cca gag gaa tat ggg aac aac atg      432
Gly Ile Ile Leu Ser Pro Asn Tyr Pro Glu Glu Tyr Gly Asn Asn Met
    130                 135                 140

aac tgt gtc tgg ttg att atc tcg gag cca gga agt cga att cac cta      480
Asn Cys Val Trp Leu Ile Ile Ser Glu Pro Gly Ser Arg Ile His Leu
145                 150                 155                 160

atc ttt aat gat ttt gat gtt gag cct caa ttt gac ttt ctc gcg gtc      528
Ile Phe Asn Asp Phe Asp Val Glu Pro Gln Phe Asp Phe Leu Ala Val
                165                 170                 175

aag gat gat ggc att tct gac ata act gtc ctg ggt act ttt tct ggc      576
Lys Asp Asp Gly Ile Ser Asp Ile Thr Val Leu Gly Thr Phe Ser Gly
            180                 185                 190

aat gaa gtg cct tcc cag ctg gcc agc agt ggg cat ata gtt cgc ttg      624
Asn Glu Val Pro Ser Gln Leu Ala Ser Ser Gly His Ile Val Arg Leu
        195                 200                 205

gaa ttt cag tct gac cat tcc act act ggc aga ggg ttc aac atc act      672
Glu Phe Gln Ser Asp His Ser Thr Thr Gly Arg Gly Phe Asn Ile Thr
    210                 215                 220

tac acc aca ttt ggt cag aat gag tgc cat gat cct ggc att cct ata      720
Tyr Thr Thr Phe Gly Gln Asn Glu Cys His Asp Pro Gly Ile Pro Ile
225                 230                 235                 240

aac gga cga cgt ttt ggt gac agg ttt cta ctc ggg agc tcg gtt tct      768
Asn Gly Arg Arg Phe Gly Asp Arg Phe Leu Leu Gly Ser Ser Val Ser
                245                 250                 255

ttc cac tgt gat gat ggc ttt gtc aag acc cag gga tcc gag tcc att      816
Phe His Cys Asp Asp Gly Phe Val Lys Thr Gln Gly Ser Glu Ser Ile
            260                 265                 270

acc tgc ata ctg caa gac ggg aac gtg gtc tgg agc tcc acc gtg ccc      864
Thr Cys Ile Leu Gln Asp Gly Asn Val Val Trp Ser Ser Thr Val Pro
        275                 280                 285

cgc tgt gaa gct cca tgt ggt gga cat ctg aca gcg tcc agc gga gtc      912
Arg Cys Glu Ala Pro Cys Gly Gly His Leu Thr Ala Ser Ser Gly Val
    290                 295                 300

att ttg cct cct gga tgg cca gga tat tat aag gat tct tta cat tgt      960
Ile Leu Pro Pro Gly Trp Pro Gly Tyr Tyr Lys Asp Ser Leu His Cys
305                 310                 315                 320

gaa tgg ata att gaa gca aaa cca ggc cac tct atc aaa ata act ttt     1008
Glu Trp Ile Ile Glu Ala Lys Pro Gly His Ser Ile Lys Ile Thr Phe
                325                 330                 335

gac aga ttt cag aca gag gtc aat tat gac acc ttg gag gtc aga gat     1056
Asp Arg Phe Gln Thr Glu Val Asn Tyr Asp Thr Leu Glu Val Arg Asp
            340                 345                 350

ggg cca gcc agt tcg tcc cca ctg atc ggc gag tac cac ggc acc cag     1104
Gly Pro Ala Ser Ser Ser Pro Leu Ile Gly Glu Tyr His Gly Thr Gln
        355                 360                 365

gca ccc cag ttc ctc atc agc acc ggg aac ttc atg tac ctg cta ttc     1152
Ala Pro Gln Phe Leu Ile Ser Thr Gly Asn Phe Met Tyr Leu Leu Phe
    370                 375                 380
```

```
acc act gac aac agc cgc tcc agc atc ggc ttc ctc atc cac tat gag      1200
Thr Thr Asp Asn Ser Arg Ser Ser Ile Gly Phe Leu Ile His Tyr Glu
385                 390                 395                 400

agt gtg acg ctt gag tcg gat tcc tgc ctg gac ccg ggc atc cct gtg      1248
Ser Val Thr Leu Glu Ser Asp Ser Cys Leu Asp Pro Gly Ile Pro Val
                405                 410                 415

aac grc cat cgc cac ggt gga gac ttt ggc atc agg tcc aca gtg act      1296
Asn Xaa His Arg His Gly Gly Asp Phe Gly Ile Arg Ser Thr Val Thr
                420                 425                 430

ttc agc tgt gac ccg ggg tac aca cta agt gac gac gag ccc ctc gtc      1344
Phe Ser Cys Asp Pro Gly Tyr Thr Leu Ser Asp Asp Glu Pro Leu Val
        435                 440                 445

tgt gag agg aac cac cag tgg aac cac gcc ttg ccc agc tgc gac gct      1392
Cys Glu Arg Asn His Gln Trp Asn His Ala Leu Pro Ser Cys Asp Ala
    450                 455                 460

cta tgt gga ggc tac atc caa ggg aag agt gga aca gtc ctt tct cct      1440
Leu Cys Gly Gly Tyr Ile Gln Gly Lys Ser Gly Thr Val Leu Ser Pro
465                 470                 475                 480

ggg ttt cca gat ttt tat cca aac tct cta aac ygc acg tgg acc att      1488
Gly Phe Pro Asp Phe Tyr Pro Asn Ser Leu Asn Xaa Thr Trp Thr Ile
                485                 490                 495

gaa gtg tct cat ggg aaa gga gtt caa atg atc ttt cac acc ttt cat      1536
Glu Val Ser His Gly Lys Gly Val Gln Met Ile Phe His Thr Phe His
            500                 505                 510

ctt gag agt tcc cac gac tat tta ctg atc aca gag gat gga agt ttt      1584
Leu Glu Ser Ser His Asp Tyr Leu Leu Ile Thr Glu Asp Gly Ser Phe
        515                 520                 525

tcc gag ccc gtt gcc agg ctc acc ggg tcg gtg ttg cct cat acg atc      1632
Ser Glu Pro Val Ala Arg Leu Thr Gly Ser Val Leu Pro His Thr Ile
    530                 535                 540

aag gca ggc ctg ttt gga aac ttc act gcc cag ctt cgg ttt ata tca      1680
Lys Ala Gly Leu Phe Gly Asn Phe Thr Ala Gln Leu Arg Phe Ile Ser
545                 550                 555                 560

gac ttc tca att tcg tac gag ggc ttc aat atc aca ttt tca gaa tat      1728
Asp Phe Ser Ile Ser Tyr Glu Gly Phe Asn Ile Thr Phe Ser Glu Tyr
                565                 570                 575

gac ctg gag cca tgt gat gat cct gga gtc cct gcc ttc agc cga aga      1776
Asp Leu Glu Pro Cys Asp Asp Pro Gly Val Pro Ala Phe Ser Arg Arg
            580                 585                 590

att ggt ttt cac ttt ggt gtg gga gac tct ctg acg ttt tcc tgc ttc      1824
Ile Gly Phe His Phe Gly Val Gly Asp Ser Leu Thr Phe Ser Cys Phe
        595                 600                 605

ctg gga tat cgt tta gaa ggt gcc rcc aag ctt acc tgc ctg ggt ggg      1872
Leu Gly Tyr Arg Leu Glu Gly Ala Xaa Lys Leu Thr Cys Leu Gly Gly
    610                 615                 620

ggc cgc cgt gtg tgg agt gca cct ctg cca agg tgt gtg gcc gaa tgt      1920
Gly Arg Arg Val Trp Ser Ala Pro Leu Pro Arg Cys Val Ala Glu Cys
625                 630                 635                 640
```

```
gga gca agt gtc aaa gga aat gaa gga aca tta ctg tct cca aat ttt      1968
Gly Ala Ser Val Lys Gly Asn Glu Gly Thr Leu Leu Ser Pro Asn Phe
                645                 650                 655

cca tcc aat tat gat aat aac cat gag tgt atc tat aaa ata gaa aca      2016
Pro Ser Asn Tyr Asp Asn Asn His Glu Cys Ile Tyr Lys Ile Glu Thr
            660                 665                 670

gaa gcc ggc aag ggc atc cac ctt aga aca cga agc ttc cag ctg ttt      2064
Glu Ala Gly Lys Gly Ile His Leu Arg Thr Arg Ser Phe Gln Leu Phe
        675                 680                 685

gaa gga gat act cta aag gta tat gat gga aaa gac agt tcc tca cgt      2112
Glu Gly Asp Thr Leu Lys Val Tyr Asp Gly Lys Asp Ser Ser Ser Arg
    690                 695                 700

cca ctg ggc acg ttc act aaa aat gaa ctt ctg ggg ctg atc cta aac      2160
Pro Leu Gly Thr Phe Thr Lys Asn Glu Leu Leu Gly Leu Ile Leu Asn
705                 710                 715                 720

agc aca tcc aat cac ctr tgg cta gag ttc aac acc aat gga tct gac      2208
Ser Thr Ser Asn His Xaa Trp Leu Glu Phe Asn Thr Asn Gly Ser Asp
                725                 730                 735

acc gac caa ggt ttt caa ctc acc tat acc agt ttt gat ctg gta aaa      2256
Thr Asp Gln Gly Phe Gln Leu Thr Tyr Thr Ser Phe Asp Leu Val Lys
            740                 745                 750

tgt gag gat ccg ggc atc cct aac tac ggc tat agg atc cgt gat gaa      2304
Cys Glu Asp Pro Gly Ile Pro Asn Tyr Gly Tyr Arg Ile Arg Asp Glu
        755                 760                 765

ggc cac ttt acc gac act gta gtt ctg tac agt tgc aac ccg ggg tac      2352
Gly His Phe Thr Asp Thr Val Val Leu Tyr Ser Cys Asn Pro Gly Tyr
    770                 775                 780

gcc atg cat ggc agc aac acc ctg acc tgt ttg agt gga gac agg aga      2400
Ala Met His Gly Ser Asn Thr Leu Thr Cys Leu Ser Gly Asp Arg Arg
785                 790                 795                 800

gtg tgg gac aaa cca cta cct tcg tgc ata gcg gaa tgt ggt ggt cag      2448
Val Trp Asp Lys Pro Leu Pro Ser Cys Ile Ala Glu Cys Gly Gly Gln
                805                 810                 815

atc cat gca gcc aca tca gga cga ata ttg tcc cct ggc tat cca gct      2496
Ile His Ala Ala Thr Ser Gly Arg Ile Leu Ser Pro Gly Tyr Pro Ala
            820                 825                 830

ccg tat gac aac aac ctc cac tgc acc tgg att ata gag gca gac cca      2544
Pro Tyr Asp Asn Asn Leu His Cys Thr Trp Ile Ile Glu Ala Asp Pro
        835                 840                 845

gga aag acc att agc ctc cat ttc att gtt ttc gac acg gag atg gct      2592
Gly Lys Thr Ile Ser Leu His Phe Ile Val Phe Asp Thr Glu Met Ala
    850                 855                 860

cac gac atc ctc aag gtc tgg gac ggg ccg gtg gac agt gac atc ctg      2640
His Asp Ile Leu Lys Val Trp Asp Gly Pro Val Asp Ser Asp Ile Leu
865                 870                 875                 880

ctg aag gag tgg agt ggc tcc gcc ctt ccg gag gac atc cac agc acc      2688
Leu Lys Glu Trp Ser Gly Ser Ala Leu Pro Glu Asp Ile His Ser Thr
                885                 890                 895
```

```
ttc aac tca ctc acc ctg cag ttc gac agc gac ttc ttc atc agc aag      2736
Phe Asn Ser Leu Thr Leu Gln Phe Asp Ser Asp Phe Phe Ile Ser Lys
            900                 905                 910

tct ggc ttc tcc atc cag ttc tcc acc tca att gca gcc acc tgt aac      2784
Ser Gly Phe Ser Ile Gln Phe Ser Thr Ser Ile Ala Ala Thr Cys Asn
        915                 920                 925

gat cca ggt atg ccc caa aat ggc acc cgc tat gga gac agc aga gag      2832
Asp Pro Gly Met Pro Gln Asn Gly Thr Arg Tyr Gly Asp Ser Arg Glu
    930                 935                 940

gct gga gac acc gtc aca ttc cag tgt gac cct ggc tat cag ctc caa      2880
Ala Gly Asp Thr Val Thr Phe Gln Cys Asp Pro Gly Tyr Gln Leu Gln
945                 950                 955                 960

gga caa gcc aaa atc acc tgt gtg cag ctg aat aac cgg ttc ttt tgg      2928
Gly Gln Ala Lys Ile Thr Cys Val Gln Leu Asn Asn Arg Phe Phe Trp
                965                 970                 975

caa cca gac cct cct aca tgc ata gct gct tgt gga ggg aat ctg acg      2976
Gln Pro Asp Pro Pro Thr Cys Ile Ala Ala Cys Gly Gly Asn Leu Thr
            980                 985                 990

ggc cca gca ggt gtt att ttg tca  ccc aac tac cca cag  ccg tat cct    3024
Gly Pro Ala Gly Val Ile Leu Ser  Pro Asn Tyr Pro Gln  Pro Tyr Pro
        995                 1000                 1005

cct ggg  aag gaa tgt gac tgg  aga gta aaa gtg aac  ccg gac ttt      3069
Pro Gly  Lys Glu Cys Asp Trp  Arg Val Lys Val Asn  Pro Asp Phe
    1010                 1015                 1020

gtc atc  gcc ttg ata ttc aaa  agt ttc aac atg gag  ccc agc tat      3114
Val Ile  Ala Leu Ile Phe Lys  Ser Phe Asn Met Glu  Pro Ser Tyr
    1025                 1030                 1035

gac ttc  cta cac atc tat gaa  ggg gaa gat tcc aac  agc ccc ctc      3159
Asp Phe  Leu His Ile Tyr Glu  Gly Glu Asp Ser Asn  Ser Pro Leu
    1040                 1045                 1050

att ggg  agt tac cag ggc tct  cag gcc cca gaa aga  ata gag agt      3204
Ile Gly  Ser Tyr Gln Gly Ser  Gln Ala Pro Glu Arg  Ile Glu Ser
    1055                 1060                 1065

agc gga  aac agc ctg ttt ctg  gca ttt cgg agt gat  gcc tcc gtg      3249
Ser Gly  Asn Ser Leu Phe Leu  Ala Phe Arg Ser Asp  Ala Ser Val
    1070                 1075                 1080

ggc ctt  tca ggg ttc gcc att  gaa ttt aaa gag aaa  cca cgg gaa      3294
Gly Leu  Ser Gly Phe Ala Ile  Glu Phe Lys Glu Lys  Pro Arg Glu
    1085                 1090                 1095

gct tgt  ttt gac cca gga aat  ata atg aat ggg aca  aga gtt gga      3339
Ala Cys  Phe Asp Pro Gly Asn  Ile Met Asn Gly Thr  Arg Val Gly
    1100                 1105                 1110

aca gac  ttc aag ctt ggc tcc  acc atc acc tac cag  tgt gac tct      3384
Thr Asp  Phe Lys Leu Gly Ser  Thr Ile Thr Tyr Gln  Cys Asp Ser
    1115                 1120                 1125

ggc tat  aag att ctt gac ccc  tca tcc atc acc tgt  gtg att ggg      3429
Gly Tyr  Lys Ile Leu Asp Pro  Ser Ser Ile Thr Cys  Val Ile Gly
    1130                 1135                 1140
```

```
gct gat  ggg aaa ccc tcc tgg  gac caa gtg ctg ccc  tcc tgc aat      3474
Ala Asp  Gly Lys Pro Ser Trp  Asp Gln Val Leu Pro  Ser Cys Asn
    1145                 1150                 1155

gct ccc  tgt gga ggc cag tac  acg gga tca gaa ggg  gta gtt tta      3519
Ala Pro  Cys Gly Gly Gln Tyr  Thr Gly Ser Glu Gly  Val Val Leu
    1160                 1165                 1170

tca cca  aac tac ccc cat aat  tac aca gct ggt caa  ata tgc ctc      3564
Ser Pro  Asn Tyr Pro His Asn  Tyr Thr Ala Gly Gln  Ile Cys Leu
    1175                 1180                 1185

tat tcc  atc acg gta cca aag  gaa ttc gtg gtc ttt  gga cag ttt      3609
Tyr Ser  Ile Thr Val Pro Lys  Glu Phe Val Val Phe  Gly Gln Phe
    1190                 1195                 1200

gcc tat  ttc cag aca gcc ctg  aat gat ttg gca gaa  tta ttt gat      3654
Ala Tyr  Phe Gln Thr Ala Leu  Asn Asp Leu Ala Glu  Leu Phe Asp
    1205                 1210                 1215

gga acc  cat gca cag gcc aga  ctt ctc agc tca ctc  tcg ggg tct      3699
Gly Thr  His Ala Gln Ala Arg  Leu Leu Ser Ser Leu  Ser Gly Ser
    1220                 1225                 1230

cac tca  ggg gaa aca ttg ccc  ttg gct acg tca aat  caa att ctg      3744
His Ser  Gly Glu Thr Leu Pro  Leu Ala Thr Ser Asn  Gln Ile Leu
    1235                 1240                 1245

ctc cga  ttc agt gca aag agc  ggt gcc tct gcc cgc  ggc ttc cac      3789
Leu Arg  Phe Ser Ala Lys Ser  Gly Ala Ser Ala Arg  Gly Phe His
    1250                 1255                 1260

ttc gtg  tat caa gct gtt cct  cgt acc agt gac acc  caa tgc agc      3834
Phe Val  Tyr Gln Ala Val Pro  Arg Thr Ser Asp Thr  Gln Cys Ser
    1265                 1270                 1275

tct gtc  ccc gag ccc aga tac  gga agg aga att ggt  tct gag ttt      3879
Ser Val  Pro Glu Pro Arg Tyr  Gly Arg Arg Ile Gly  Ser Glu Phe
    1280                 1285                 1290

tct gcc  ggc tcc atc gtc cga  ttc gag trc aac ccg  gga tac ctg      3924
Ser Ala  Gly Ser Ile Val Arg  Phe Glu Xaa Asn Pro  Gly Tyr Leu
    1295                 1300                 1305

ctt cag  ggt tcc acg gcg ctc  cac tgc cag tcc gtg  ccc aac gcc      3969
Leu Gln  Gly Ser Thr Ala Leu  His Cys Gln Ser Val  Pro Asn Ala
    1310                 1315                 1320

ttg gca  cag tgg aac gac acg  atc ccc agc tgt gtg  gta ccc tgc      4014
Leu Ala  Gln Trp Asn Asp Thr  Ile Pro Ser Cys Val  Val Pro Cys
    1325                 1330                 1335

agt ggc  aat ttc act caa cga  aga ggt aca atc ctg  tcc ccc ggc      4059
Ser Gly  Asn Phe Thr Gln Arg  Arg Gly Thr Ile Leu  Ser Pro Gly
    1340                 1345                 1350

tac cct  gag cca tac gga aac  aac ttg aac tgt ata  tgg aag atc      4104
Tyr Pro  Glu Pro Tyr Gly Asn  Asn Leu Asn Cys Ile  Trp Lys Ile
    1355                 1360                 1365

ata gtt  acg gag ggc tcg gga  att cag atc caa gtg  atc agt ttt      4149
Ile Val  Thr Glu Gly Ser Gly  Ile Gln Ile Gln Val  Ile Ser Phe
    1370                 1375                 1380
```

```
gcc acg gag cag aac tgg gac tcc ctt gag atc cac gat ggt ggg     4194
Ala Thr Glu Gln Asn Trp Asp Ser Leu Glu Ile His Asp Gly Gly
    1385                1390                1395

gat gtg acc gca ccc aga ctg gga agc ttc tca ggc acc aca gta     4239
Asp Val Thr Ala Pro Arg Leu Gly Ser Phe Ser Gly Thr Thr Val
    1400                1405                1410

ccg gca ctg ctg aac agt act tcc aac caa ctc tac ctg cat ttc     4284
Pro Ala Leu Leu Asn Ser Thr Ser Asn Gln Leu Tyr Leu His Phe
    1415                1420                1425

cag tct gac att agt gtg gca gct gct ggt ttc cac ctg gaa tac     4329
Gln Ser Asp Ile Ser Val Ala Ala Ala Gly Phe His Leu Glu Tyr
    1430                1435                1440

aaa act gta ggt ctt gct gca tgc caa gaa cca gcc ctc ccc agc     4374
Lys Thr Val Gly Leu Ala Ala Cys Gln Glu Pro Ala Leu Pro Ser
    1445                1450                1455

aac agc atc aaa atc gga gat cgg tac atg gtg aac gac gtg ctc     4419
Asn Ser Ile Lys Ile Gly Asp Arg Tyr Met Val Asn Asp Val Leu
    1460                1465                1470

tcc ttc cag tgc gag ccc ggg tac acc ctg cag ggc cgt tcc cac     4464
Ser Phe Gln Cys Glu Pro Gly Tyr Thr Leu Gln Gly Arg Ser His
    1475                1480                1485

att tcc tgt atg cca ggg acc gtt cgc cgt tgg aac tat ccg tct     4509
Ile Ser Cys Met Pro Gly Thr Val Arg Arg Trp Asn Tyr Pro Ser
    1490                1495                1500

ccc ctg tgc att gca acc tgt gga ggg acg ctg agc acc ttg ggt     4554
Pro Leu Cys Ile Ala Thr Cys Gly Gly Thr Leu Ser Thr Leu Gly
    1505                1510                1515

ggt gtg atc ctg agc ccc ggc ttc cca ggt tct tac ccc aac aac     4599
Gly Val Ile Leu Ser Pro Gly Phe Pro Gly Ser Tyr Pro Asn Asn
    1520                1525                1530

tta gac tgc acc tgg agg atc tca tta ccc atc ggc tat ggt gca     4644
Leu Asp Cys Thr Trp Arg Ile Ser Leu Pro Ile Gly Tyr Gly Ala
    1535                1540                1545

cat att cag ttt ctg aat ttt tct acc gaa gct aat cat gac ttc     4689
His Ile Gln Phe Leu Asn Phe Ser Thr Glu Ala Asn His Asp Phe
    1550                1555                1560

ctt gaa att caa aat gga cct tac cac acc agc ccc atg att gga     4734
Leu Glu Ile Gln Asn Gly Pro Tyr His Thr Ser Pro Met Ile Gly
    1565                1570                1575

caa ttt agc ggc acg gat ctc ccc gcg gcc ctg ctg agc aca acg     4779
Gln Phe Ser Gly Thr Asp Leu Pro Ala Ala Leu Leu Ser Thr Thr
    1580                1585                1590

cat gaa acc ctc atc cac ttt tat agt gac cat tcg caa aac cgg     4824
His Glu Thr Leu Ile His Phe Tyr Ser Asp His Ser Gln Asn Arg
    1595                1600                1605

caa gga ttt aaa ctt gct tac caa gcc tat gaa tta cag aac tgt     4869
Gln Gly Phe Lys Leu Ala Tyr Gln Ala Tyr Glu Leu Gln Asn Cys
    1610                1615                1620
```

```
cca gat  cca ccc cca ttt cag  aat ggg tac atg atc  aac tcg gat      4914
Pro Asp  Pro Pro Pro Phe Gln  Asn Gly Tyr Met Ile  Asn Ser Asp
    1625                 1630                 1635

tac agc  gtg ggg caa tca gta  tct ttc gag tgt tat  cct ggg tac      4959
Tyr Ser  Val Gly Gln Ser Val  Ser Phe Glu Cys Tyr  Pro Gly Tyr
    1640                 1645                 1650

att cta  ata ggc cat cct gtc  ctc act tgt cag cat  ggg atc aac      5004
Ile Leu  Ile Gly His Pro Val  Leu Thr Cys Gln His  Gly Ile Asn
    1655                 1660                 1665

aga aac  tgg aac tac cct ttt  cca aga tgt gat gcc  cct tgt ggg      5049
Arg Asn  Trp Asn Tyr Pro Phe  Pro Arg Cys Asp Ala  Pro Cys Gly
    1670                 1675                 1680

tac aac  gta act tct cag aac  ggc acc atc tac tcc  cct ggc ttt      5094
Tyr Asn  Val Thr Ser Gln Asn  Gly Thr Ile Tyr Ser  Pro Gly Phe
    1685                 1690                 1695

cct gat  gag tat ccg atc ctg  aag gac tgc att tgg  ctc atc acg      5139
Pro Asp  Glu Tyr Pro Ile Leu  Lys Asp Cys Ile Trp  Leu Ile Thr
    1700                 1705                 1710

gtg cct  cca ggg cac gga gtt  tac atc aac ttc acc  ctg tta cag      5184
Val Pro  Pro Gly His Gly Val  Tyr Ile Asn Phe Thr  Leu Leu Gln
    1715                 1720                 1725

acg gaa  gct gtc aac gat tac  att gct gtt tgg gac  ggt ccc gat      5229
Thr Glu  Ala Val Asn Asp Tyr  Ile Ala Val Trp Asp  Gly Pro Asp
    1730                 1735                 1740

cag aac  tca ccc cag ctg gga  gtt ttc agt ggc aac  aca gcc ctc      5274
Gln Asn  Ser Pro Gln Leu Gly  Val Phe Ser Gly Asn  Thr Ala Leu
    1745                 1750                 1755

gaa acg  gcg tat agc tcc acc  aac caa gtc ctg ctc  aag ttc cac      5319
Glu Thr  Ala Tyr Ser Ser Thr  Asn Gln Val Leu Leu  Lys Phe His
    1760                 1765                 1770

agc gac  ttt tca aat gga ggc  ttc ttt gtc ctc aat  ttc cac gca      5364
Ser Asp  Phe Ser Asn Gly Gly  Phe Phe Val Leu Asn  Phe His Ala
    1775                 1780                 1785

ttt cag  ctc aag aaa tgt caa  cct ccc cca gcg gtt  cca cag gca      5409
Phe Gln  Leu Lys Lys Cys Gln  Pro Pro Pro Ala Val  Pro Gln Ala
    1790                 1795                 1800

gaa atg  ctt act gag gat gat  gat ttc gag ata gga  gat ttt gtg      5454
Glu Met  Leu Thr Glu Asp Asp  Asp Phe Glu Ile Gly  Asp Phe Val
    1805                 1810                 1815

aag tac  cag tgc cac ccc ggg  tac acc ttg gtg ggg  acc gac att      5499
Lys Tyr  Gln Cys His Pro Gly  Tyr Thr Leu Val Gly  Thr Asp Ile
    1820                 1825                 1830

ctg act  tgc aag ctc agt tcc  cag ttg cag ttt gag  ggt tct ctc      5544
Leu Thr  Cys Lys Leu Ser Ser  Gln Leu Gln Phe Glu  Gly Ser Leu
    1835                 1840                 1845

cca aca  tgt gaa gca caa tgc  cca gca aat gaa gtc  cgg act gga      5589
Pro Thr  Cys Glu Ala Gln Cys  Pro Ala Asn Glu Val  Arg Thr Gly
    1850                 1855                 1860
```

```
tca tcg  gga gtc att ctc agt  cca ggg tat ccg ggt  aat tat ttt      5634
Ser Ser  Gly Val Ile Leu Ser  Pro Gly Tyr Pro Gly  Asn Tyr Phe
    1865                 1870                 1875

aac tcc  cag act tgc tct tgg  agt att aaa gtg gaa  cca aac tac      5679
Asn Ser  Gln Thr Cys Ser Trp  Ser Ile Lys Val Glu  Pro Asn Tyr
    1880                 1885                 1890

aac att  acc atc ttt gtg gac  aca ttt caa agt gaa  aag cag ttt      5724
Asn Ile  Thr Ile Phe Val Asp  Thr Phe Gln Ser Glu  Lys Gln Phe
    1895                 1900                 1905

gat gca  ctg gaa gtg ttt gat  ggt tct tct ggg caa  agt cct ctg      5769
Asp Ala  Leu Glu Val Phe Asp  Gly Ser Ser Gly Gln  Ser Pro Leu
    1910                 1915                 1920

cta gta  gtc tta agt ggg aat  cat act gaa caa tca  aat ttt aca      5814
Leu Val  Val Leu Ser Gly Asn  His Thr Glu Gln Ser  Asn Phe Thr
    1925                 1930                 1935

agc agg  agt aat cag tta tat  ctc cgc tgg tcc act  gac cat gcc      5859
Ser Arg  Ser Asn Gln Leu Tyr  Leu Arg Trp Ser Thr  Asp His Ala
    1940                 1945                 1950

acc agt  aag aaa gga ttc aag  att cgc tat gca gca  cct tac tgc      5904
Thr Ser  Lys Lys Gly Phe Lys  Ile Arg Tyr Ala Ala  Pro Tyr Cys
    1955                 1960                 1965

agt ttg  acc cac ccc ctg aag  aat ggg ggt att cta  aac agg act      5949
Ser Leu  Thr His Pro Leu Lys  Asn Gly Gly Ile Leu  Asn Arg Thr
    1970                 1975                 1980

gca gga  gcg gtt gga agc aaa  gtg cat tat ttt tgc  aag cct gga      5994
Ala Gly  Ala Val Gly Ser Lys  Val His Tyr Phe Cys  Lys Pro Gly
    1985                 1990                 1995

tac cga  atg gtc ggc cac agc  aat gca acc tgt aga  cga aac cca      6039
Tyr Arg  Met Val Gly His Ser  Asn Ala Thr Cys Arg  Arg Asn Pro
    2000                 2005                 2010

ctt ggc  atg tac cag tgg gac  tcc ctc acg cca ctc  tgc cag gct      6084
Leu Gly  Met Tyr Gln Trp Asp  Ser Leu Thr Pro Leu  Cys Gln Ala
    2015                 2020                 2025

gtg tcc  tgt gga atc cca gaa  tcc cca gga aac ggt  tca ttt acc      6129
Val Ser  Cys Gly Ile Pro Glu  Ser Pro Gly Asn Gly  Ser Phe Thr
    2030                 2035                 2040

ggg aac  gag ttc act ttg gac  agt aaa gtg gtc tat  gaa tgt cat      6174
Gly Asn  Glu Phe Thr Leu Asp  Ser Lys Val Val Tyr  Glu Cys His
    2045                 2050                 2055

gag ggc  ttc aag ctt gaa tcc  agc cag caa gca aca  gcc gtg tgt      6219
Glu Gly  Phe Lys Leu Glu Ser  Ser Gln Gln Ala Thr  Ala Val Cys
    2060                 2065                 2070

caa gaa  gat ggg ctg tgg agt  aac aag ggg aag ccg  ccc acg tgt      6264
Gln Glu  Asp Gly Leu Trp Ser  Asn Lys Gly Lys Pro  Pro Thr Cys
    2075                 2080                 2085

aag ccg  gtc gct tgc ccc agc  att gaa gct cag ctc  tca gaa cat      6309
Lys Pro  Val Ala Cys Pro Ser  Ile Glu Ala Gln Leu  Ser Glu His
    2090                 2095                 2100
```

```
gtc atc  tgg agg ctg gtt tca  gga tcc ttg aat gag  tac ggt gct      6354
Val Ile  Trp Arg Leu Val Ser  Gly Ser Leu Asn Glu  Tyr Gly Ala
    2105                 2110                 2115

caa gta  ttg ctg agc tgc agt  cct ggt tac tac tta  gaa ggc tgg      6399
Gln Val  Leu Leu Ser Cys Ser  Pro Gly Tyr Tyr Leu  Glu Gly Trp
    2120                 2125                 2130

agg ctc  ctg cgg tgc cag gcc  aat ggg acg tgg aac  ata gga gat      6444
Arg Leu  Leu Arg Cys Gln Ala  Asn Gly Thr Trp Asn  Ile Gly Asp
    2135                 2140                 2145

gag agg  cca agc tgt cga gtt  atc tcg tgt gga agc  ctt tcc ttt      6489
Glu Arg  Pro Ser Cys Arg Val  Ile Ser Cys Gly Ser  Leu Ser Phe
    2150                 2155                 2160

ccc cca  aat ggc aac aag att  gga acg ttg aca gtt  tat ggg gcc      6534
Pro Pro  Asn Gly Asn Lys Ile  Gly Thr Leu Thr Val  Tyr Gly Ala
    2165                 2170                 2175

aca gct  ata ttt acg tgc aac  acc ggc tac acg ctt  gtg ggg tct      6579
Thr Ala  Ile Phe Thr Cys Asn  Thr Gly Tyr Thr Leu  Val Gly Ser
    2180                 2185                 2190

cat gtc  aga gag tgc ttg gca  aat ggg ctc tgg agc  ggc agc gaa      6624
His Val  Arg Glu Cys Leu Ala  Asn Gly Leu Trp Ser  Gly Ser Glu
    2195                 2200                 2205

act cga  tgt ctg gct ggc cac  tgc ggt tcc cca gac  ccg att gtg      6669
Thr Arg  Cys Leu Ala Gly His  Cys Gly Ser Pro Asp  Pro Ile Val
    2210                 2215                 2220

aac ggt  cac att agt gga gat  ggc ttc agt tac aga  gac acg gtg      6714
Asn Gly  His Ile Ser Gly Asp  Gly Phe Ser Tyr Arg  Asp Thr Val
    2225                 2230                 2235

gtt tac  cag tgc aat cct ggt  ttc cgg ctt gtg gga  act tcc gtg      6759
Val Tyr  Gln Cys Asn Pro Gly  Phe Arg Leu Val Gly  Thr Ser Val
    2240                 2245                 2250

agg ata  tgc ctg caa gac cac  aag tgg tct gga caa  acg cct gtc      6804
Arg Ile  Cys Leu Gln Asp His  Lys Trp Ser Gly Gln  Thr Pro Val
    2255                 2260                 2265

tgt gtc  ccc atc aca tgt ggt  cac cct gga aac cct  gcc cac gga      6849
Cys Val  Pro Ile Thr Cys Gly  His Pro Gly Asn Pro  Ala His Gly
    2270                 2275                 2280

ttc act  aat ggc agt gag ttc  aac ctg aat gat gtc  gtg aat ttc      6894
Phe Thr  Asn Gly Ser Glu Phe  Asn Leu Asn Asp Val  Val Asn Phe
    2285                 2290                 2295

acc tgc  aac acg ggc tat ttg  ctg cag ggc gtg tct  cga gcc cag      6939
Thr Cys  Asn Thr Gly Tyr Leu  Leu Gln Gly Val Ser  Arg Ala Gln
    2300                 2305                 2310

tgt cgg  agc aac ggc cag tgg  agt agc cct ctg ccc  acg tgt cga      6984
Cys Arg  Ser Asn Gly Gln Trp  Ser Ser Pro Leu Pro  Thr Cys Arg
    2315                 2320                 2325

gtg gtg  aac tgt tct gat cca  ggc ttt gtg gaa aat  gcc att cgt      7029
Val Val  Asn Cys Ser Asp Pro  Gly Phe Val Glu Asn  Ala Ile Arg
    2330                 2335                 2340
```

cac ggg caa cag aac ttc cct gag agt ttt gag tat gga atg agt 7074
His Gly Gln Gln Asn Phe Pro Glu Ser Phe Glu Tyr Gly Met Ser
2345 2350 2355

atc ctg tac cat tgc aag aag gga ttt tac ttg ctg gga tct tca 7119
Ile Leu Tyr His Cys Lys Lys Gly Phe Tyr Leu Leu Gly Ser Ser
2360 2365 2370

gcc ttg acc tgt atg gca aat ggc tta tgg gac cga tcc ctg ccc 7164
Ala Leu Thr Cys Met Ala Asn Gly Leu Trp Asp Arg Ser Leu Pro
2375 2380 2385

aag tgt ttg gct ata tcg tgt gga cac cca ggg gtc cct gcc aac 7209
Lys Cys Leu Ala Ile Ser Cys Gly His Pro Gly Val Pro Ala Asn
2390 2395 2400

gcc gtc ctc act gga gag ctg ttt acc tat ggc gcc gtc gtg cac 7254
Ala Val Leu Thr Gly Glu Leu Phe Thr Tyr Gly Ala Val Val His
2405 2410 2415

tac tcc tgc aga ggg agc gag agc ctc ata ggc aac gac acg aga 7299
Tyr Ser Cys Arg Gly Ser Glu Ser Leu Ile Gly Asn Asp Thr Arg
2420 2425 2430

gtg tgc cag gaa gac agt cac tgg agc ggg gca ctg ccc cac tgc 7344
Val Cys Gln Glu Asp Ser His Trp Ser Gly Ala Leu Pro His Cys
2435 2440 2445

aca gga aat aat cct gga ttc tgt ggt gat ccg ggg acc cca gca 7389
Thr Gly Asn Asn Pro Gly Phe Cys Gly Asp Pro Gly Thr Pro Ala
2450 2455 2460

cat ggg tct cgg ctt ggt gat gac ttt aag aca aag agt ctt ctc 7434
His Gly Ser Arg Leu Gly Asp Asp Phe Lys Thr Lys Ser Leu Leu
2465 2470 2475

cgc ttc tcc tgt gaa atg ggg cac cag ctg agg ggc tcc cct gaa 7479
Arg Phe Ser Cys Glu Met Gly His Gln Leu Arg Gly Ser Pro Glu
2480 2485 2490

cgc acg tgt ttg ctc aat ggg tca tgg tca gga ctg cag ccg gtg 7524
Arg Thr Cys Leu Leu Asn Gly Ser Trp Ser Gly Leu Gln Pro Val
2495 2500 2505

tgt gag gcc gtg tcc tgt ggc aac cct ggc aca ccc acc aac gga 7569
Cys Glu Ala Val Ser Cys Gly Asn Pro Gly Thr Pro Thr Asn Gly
2510 2515 2520

atg att gtc agt agt gat ggc att ctg ttc tcc agc tcg gtc atc 7614
Met Ile Val Ser Ser Asp Gly Ile Leu Phe Ser Ser Ser Val Ile
2525 2530 2535

tat gcc tgc tgg gaa ggc tac aag acc tca ggg ctc atg aca cgg 7659
Tyr Ala Cys Trp Glu Gly Tyr Lys Thr Ser Gly Leu Met Thr Arg
2540 2545 2550

cat tgc aca gcc aat ggg acc tgg aca ggc act gct ccc gac tgc 7704
His Cys Thr Ala Asn Gly Thr Trp Thr Gly Thr Ala Pro Asp Cys
2555 2560 2565

aca att ata agt tgt ggg gat cca ggc aca cta gca aat ggc atc 7749
Thr Ile Ile Ser Cys Gly Asp Pro Gly Thr Leu Ala Asn Gly Ile
2570 2575 2580

```
cag ttt ggg acc gac ttc acc ttc aac aag act gtg agc tat cag       7794
Gln Phe Gly Thr Asp Phe Thr Phe Asn Lys Thr Val Ser Tyr Gln
    2585                2590                2595

tgt aac cca ggc tat gtc atg gaa gca gtc aca tcc gcc act att       7839
Cys Asn Pro Gly Tyr Val Met Glu Ala Val Thr Ser Ala Thr Ile
    2600                2605                2610

cgc tgt acc aaa gac ggc agg tgg aat ccg agc aaa cct gtc tgc       7884
Arg Cys Thr Lys Asp Gly Arg Trp Asn Pro Ser Lys Pro Val Cys
    2615                2620                2625

aaa gcc gtg ctg tgt cct cag ccg ccg ccg gtg cag aat gga aca       7929
Lys Ala Val Leu Cys Pro Gln Pro Pro Pro Val Gln Asn Gly Thr
    2630                2635                2640

gtg gag gga agt gat ttc cgc tgg ggc tcc agc ata agt tac agc       7974
Val Glu Gly Ser Asp Phe Arg Trp Gly Ser Ser Ile Ser Tyr Ser
    2645                2650                2655

tgc atg gac ggt tac cag ctc tct cac tcc gcc atc ctc tcc tgt       8019
Cys Met Asp Gly Tyr Gln Leu Ser His Ser Ala Ile Leu Ser Cys
    2660                2665                2670

gaa ggt cgc ggg gtg tgg aaa gga gag atc ccc cag tgt ctc cct       8064
Glu Gly Arg Gly Val Trp Lys Gly Glu Ile Pro Gln Cys Leu Pro
    2675                2680                2685

gtg ttc tgc gga gac cct ggc atc ccc gca gaa ggg cga ctt agt       8109
Val Phe Cys Gly Asp Pro Gly Ile Pro Ala Glu Gly Arg Leu Ser
    2690                2695                2700

ggg aaa agt ttc acc tat aag tcc gaa gtc ttc ttc cag tgc aaa       8154
Gly Lys Ser Phe Thr Tyr Lys Ser Glu Val Phe Phe Gln Cys Lys
    2705                2710                2715

tct cca ttt ata ctc gtg gga tcc tcc aga aga gtc tgc caa gct       8199
Ser Pro Phe Ile Leu Val Gly Ser Ser Arg Arg Val Cys Gln Ala
    2720                2725                2730

gac ggc acg tgg agc ggc ata caa ccc acc tgc att gat cct gct       8244
Asp Gly Thr Trp Ser Gly Ile Gln Pro Thr Cys Ile Asp Pro Ala
    2735                2740                2745

cat aac acc tgc cca gac cct ggt acg cca cac ttt gga ata cag       8289
His Asn Thr Cys Pro Asp Pro Gly Thr Pro His Phe Gly Ile Gln
    2750                2755                2760

aat agc tcc aga ggc tat gag gtt gga agc acg gtt ttt ttc agg       8334
Asn Ser Ser Arg Gly Tyr Glu Val Gly Ser Thr Val Phe Phe Arg
    2765                2770                2775

tgc aga aaa ggc tac cat att caa ggt tcc acg act cgc acc tgc       8379
Cys Arg Lys Gly Tyr His Ile Gln Gly Ser Thr Thr Arg Thr Cys
    2780                2785                2790

ctt gcc aat tta aca tgg agt ggg ata cag acc gaa tgt ata cct       8424
Leu Ala Asn Leu Thr Trp Ser Gly Ile Gln Thr Glu Cys Ile Pro
    2795                2800                2805

cat gcc tgc aga cag cca gaa acc ccg gca cac gcg gat gtg aga       8469
His Ala Cys Arg Gln Pro Glu Thr Pro Ala His Ala Asp Val Arg
    2810                2815                2820
```

```
gcc atc gat ctt cct act ttc ggc tac acc tta gtg tac acc tgc     8514
Ala Ile Asp Leu Pro Thr Phe Gly Tyr Thr Leu Val Tyr Thr Cys
    2825                2830                2835

cat cca ggc ttt ttc ctc gca ggg gga tct gag cac aga aca tgt     8559
His Pro Gly Phe Phe Leu Ala Gly Gly Ser Glu His Arg Thr Cys
    2840                2845                2850

aaa gca gac atg aaa tgg aca gga aag tcg cct gtg tgt aaa agt     8604
Lys Ala Asp Met Lys Trp Thr Gly Lys Ser Pro Val Cys Lys Ser
    2855                2860                2865

aaa gga gtg aga gaa gtt aat gaa aca gtt act aaa act cca gtt     8649
Lys Gly Val Arg Glu Val Asn Glu Thr Val Thr Lys Thr Pro Val
    2870                2875                2880

cct tca gat gtc ttt ttc gtc aat tca ctg tgg aag ggg tat tat     8694
Pro Ser Asp Val Phe Phe Val Asn Ser Leu Trp Lys Gly Tyr Tyr
    2885                2890                2895

gaa tat tta ggg aaa aga caa ccc gcc act cta act gtt gac tgg     8739
Glu Tyr Leu Gly Lys Arg Gln Pro Ala Thr Leu Thr Val Asp Trp
    2900                2905                2910

ttc aat gca aca agc agt aag gtg aat gcc acc ttc agc gaa gcc     8784
Phe Asn Ala Thr Ser Ser Lys Val Asn Ala Thr Phe Ser Glu Ala
    2915                2920                2925

tcg cca gtg gag ctg aag ttg aca ggc att tac aag aag gag gag     8829
Ser Pro Val Glu Leu Lys Leu Thr Gly Ile Tyr Lys Lys Glu Glu
    2930                2935                2940

gcc cac tta ctc ctg aaa gct ttt caa att aaa ggc cag gca gat     8874
Ala His Leu Leu Leu Lys Ala Phe Gln Ile Lys Gly Gln Ala Asp
    2945                2950                2955

att ttt gta agc aag ttc gaa aat gac aac tgg gga cta gat ggt     8919
Ile Phe Val Ser Lys Phe Glu Asn Asp Asn Trp Gly Leu Asp Gly
    2960                2965                2970

tat gtg tca tct gga ctt gaa aga gga gga ttt act ttt caa ggt     8964
Tyr Val Ser Ser Gly Leu Glu Arg Gly Gly Phe Thr Phe Gln Gly
    2975                2980                2985

gac att cat gga aaa gac ttt gga aaa ttt aag cta gaa agg caa     9009
Asp Ile His Gly Lys Asp Phe Gly Lys Phe Lys Leu Glu Arg Gln
    2990                2995                3000

gat cct tta aac cca gat caa gac tct tcc agt cat tac cac ggc     9054
Asp Pro Leu Asn Pro Asp Gln Asp Ser Ser Ser His Tyr His Gly
    3005                3010                3015

acc agc agt ggc tct gtg gcg gct gcc att ctg gtt cct ttc ttt     9099
Thr Ser Ser Gly Ser Val Ala Ala Ala Ile Leu Val Pro Phe Phe
    3020                3025                3030

gct cta att tta tca ggg ttt gca ttt tac ctc tac aaa cac aga     9144
Ala Leu Ile Leu Ser Gly Phe Ala Phe Tyr Leu Tyr Lys His Arg
    3035                3040                3045

acg aga cca aaa gtt caa tac aat ggc tat gct ggg cat gaa aac     9189
Thr Arg Pro Lys Val Gln Tyr Asn Gly Tyr Ala Gly His Glu Asn
    3050                3055                3060
```

```
agc aat  gga caa gca tcg ttt  gaa aac ccc atg tat  gat aca aac      9234
Ser Asn  Gly Gln Ala Ser Phe  Glu Asn Pro Met Tyr  Asp Thr Asn
    3065                3070                 3075

tta aaa  ccc aca gaa gcc aag  gct gtg agg ttt gac  aca act ctg      9279
Leu Lys  Pro Thr Glu Ala Lys  Ala Val Arg Phe Asp  Thr Thr Leu
    3080                3085                 3090

aac aca  gtc tgt aca gtg gta  tagccctcag tgccccaaca ggactgattc      9330
Asn Thr  Val Cys Thr Val Val
    3095                3100

atagccatac ctctgatgga caagcagtga ttcctttggt gccatatacc actctcccyt   9390

ccactctggc tttactgcag cgatcttcaa ccttgtctac tggcataagt gcagcgggga   9450

tctctactca aatgtgtcag ggtcttctac ggatcaaact acacatgcgt tttcattcca   9510

aaagtgggtt ctaaatgcct ggctgcatct gtatgaaatc aaggcacact ccaggaagac   9570

tgccacgtcg cgccaacacg tcatactcaa trcctcagac tttcatattt ctgtgttgct   9630

gagatgcctt tcaatgcaat cgtctgggct cgtggatatg tccctcaggt gcggtgacag   9690

aatggtggca ccacgatatg tgttctcttg tgttgttttt cctttttaaa cccccatgaa   9750

cacgaatact ctgaaaaaaa taaaaagctt tctggaagaa gacacctttc tgatagaggc   9810

tcacacctac aaatgcttca ctctgtcctt ccgagacctg acaagctttg aggacctcac   9870

agctcccctg tgtgttcatc tctagggatg tttgcaattt cccagtcagc tgttctgtcg   9930

cagaatgttt aatgcacaat tttttgcact agtgtgttat gaatgactaa gattctgata   9990

aaaaaaataa attatttaca cagggtttat acacactatc cattgtatat aagcattatt  10050

tcatattatc aagctaaaca ttcccccatc agcttagttg gagtgttagg gaaaagtatt  10110

cctagatatg gcacagattt taaaaggaaa tacagtattg acgagattta ttttattatt  10170

gcttcaatta gctccattta cgtgttgaat tcattgaaga ggtccaatga gaaaaaaaca  10230

gaagcctcct tatttcacac gttttcctcc tttagtacca tcctcatcca attactgtct  10290

ctctgatact acttaatagc agggggtttg cagaaatttc tgtttgccat gtaaaactgt  10350

gaatagtaat ttattttaga tagtcgatga acttgtgggt tttagctcac aatgcagcct  10410

tccctttgc agtgtttttt ttt                                          10433
```

<210> 7

<211> 3100

<212> PRT

<213> Homo sapiens

<400> 7

```
Thr Leu Thr Val Gly Asp Ala Gly Lys Val Gly Asp Thr Arg Ser Val
1               5                   10                  15

Leu Tyr Val Leu Thr Gly Ser Ser Val Pro Asp Leu Ile Val Ser Met
            20                  25                  30

Ser Asn Gln Met Trp Leu His Leu Gln Ser Asp Asp Ser Ile Gly Ser
        35                  40                  45

Pro Gly Phe Lys Ala Val Tyr Gln Glu Ile Glu Lys Gly Gly Cys Gly
    50                  55                  60

Asp Pro Gly Ile Pro Ala Tyr Gly Lys Arg Thr Gly Ser Ser Phe Leu
65                  70                  75                  80

His Gly Asp Thr Leu Thr Phe Glu Cys Pro Ala Ala Phe Glu Leu Val
                85                  90                  95

Gly Glu Arg Val Ile Thr Cys Gln Gln Asn Asn Gln Trp Ser Gly Asn
            100                 105                 110

Lys Pro Ser Cys Val Phe Ser Cys Phe Phe Asn Phe Thr Ala Ser Ser
        115                 120                 125

Gly Ile Ile Leu Ser Pro Asn Tyr Pro Glu Glu Tyr Gly Asn Asn Met
    130                 135                 140

Asn Cys Val Trp Leu Ile Ile Ser Glu Pro Gly Ser Arg Ile His Leu
145                 150                 155                 160

Ile Phe Asn Asp Phe Asp Val Glu Pro Gln Phe Asp Phe Leu Ala Val
                165                 170                 175

Lys Asp Asp Gly Ile Ser Asp Ile Thr Val Leu Gly Thr Phe Ser Gly
            180                 185                 190

Asn Glu Val Pro Ser Gln Leu Ala Ser Ser Gly His Ile Val Arg Leu
        195                 200                 205

Glu Phe Gln Ser Asp His Ser Thr Thr Gly Arg Gly Phe Asn Ile Thr
    210                 215                 220

Tyr Thr Thr Phe Gly Gln Asn Glu Cys His Asp Pro Gly Ile Pro Ile
225                 230                 235                 240

Asn Gly Arg Arg Phe Gly Asp Arg Phe Leu Leu Gly Ser Ser Val Ser
                245                 250                 255
```

```
Phe His Cys Asp Asp Gly Phe Val Lys Thr Gln Gly Ser Glu Ser Ile
            260                 265                 270

Thr Cys Ile Leu Gln Asp Gly Asn Val Val Trp Ser Ser Thr Val Pro
        275                 280                 285

Arg Cys Glu Ala Pro Cys Gly Gly His Leu Thr Ala Ser Ser Gly Val
    290                 295                 300

Ile Leu Pro Pro Gly Trp Pro Gly Tyr Tyr Lys Asp Ser Leu His Cys
305                 310                 315                 320

Glu Trp Ile Ile Glu Ala Lys Pro Gly His Ser Ile Lys Ile Thr Phe
                325                 330                 335

Asp Arg Phe Gln Thr Glu Val Asn Tyr Asp Thr Leu Glu Val Arg Asp
            340                 345                 350

Gly Pro Ala Ser Ser Ser Pro Leu Ile Gly Glu Tyr His Gly Thr Gln
        355                 360                 365

Ala Pro Gln Phe Leu Ile Ser Thr Gly Asn Phe Met Tyr Leu Leu Phe
    370                 375                 380

Thr Thr Asp Asn Ser Arg Ser Ser Ile Gly Phe Leu Ile His Tyr Glu
385                 390                 395                 400

Ser Val Thr Leu Glu Ser Asp Ser Cys Leu Asp Pro Gly Ile Pro Val
                405                 410                 415

Asn Xaa His Arg His Gly Gly Asp Phe Gly Ile Arg Ser Thr Val Thr
            420                 425                 430

Phe Ser Cys Asp Pro Gly Tyr Thr Leu Ser Asp Asp Glu Pro Leu Val
        435                 440                 445

Cys Glu Arg Asn His Gln Trp Asn His Ala Leu Pro Ser Cys Asp Ala
    450                 455                 460

Leu Cys Gly Gly Tyr Ile Gln Gly Lys Ser Gly Thr Val Leu Ser Pro
465                 470                 475                 480

Gly Phe Pro Asp Phe Tyr Pro Asn Ser Leu Asn Xaa Thr Trp Thr Ile
                485                 490                 495

Glu Val Ser His Gly Lys Gly Val Gln Met Ile Phe His Thr Phe His
            500                 505                 510
```

```
Leu Glu Ser Ser His Asp Tyr Leu Leu Ile Thr Glu Asp Gly Ser Phe
        515                 520                 525

Ser Glu Pro Val Ala Arg Leu Thr Gly Ser Val Leu Pro His Thr Ile
    530                 535                 540

Lys Ala Gly Leu Phe Gly Asn Phe Thr Ala Gln Leu Arg Phe Ile Ser
545                 550                 555                 560

Asp Phe Ser Ile Ser Tyr Glu Gly Phe Asn Ile Thr Phe Ser Glu Tyr
                565                 570                 575

Asp Leu Glu Pro Cys Asp Asp Pro Gly Val Pro Ala Phe Ser Arg Arg
            580                 585                 590

Ile Gly Phe His Phe Gly Val Gly Asp Ser Leu Thr Phe Ser Cys Phe
        595                 600                 605

Leu Gly Tyr Arg Leu Glu Gly Ala Xaa Lys Leu Thr Cys Leu Gly Gly
    610                 615                 620

Gly Arg Arg Val Trp Ser Ala Pro Leu Pro Arg Cys Val Ala Glu Cys
625                 630                 635                 640

Gly Ala Ser Val Lys Gly Asn Glu Gly Thr Leu Leu Ser Pro Asn Phe
                645                 650                 655

Pro Ser Asn Tyr Asp Asn Asn His Glu Cys Ile Tyr Lys Ile Glu Thr
            660                 665                 670

Glu Ala Gly Lys Gly Ile His Leu Arg Thr Arg Ser Phe Gln Leu Phe
        675                 680                 685

Glu Gly Asp Thr Leu Lys Val Tyr Asp Gly Lys Asp Ser Ser Ser Arg
    690                 695                 700

Pro Leu Gly Thr Phe Thr Lys Asn Glu Leu Leu Gly Leu Ile Leu Asn
705                 710                 715                 720

Ser Thr Ser Asn His Xaa Trp Leu Glu Phe Asn Thr Asn Gly Ser Asp
                725                 730                 735

Thr Asp Gln Gly Phe Gln Leu Thr Tyr Thr Ser Phe Asp Leu Val Lys
            740                 745                 750

Cys Glu Asp Pro Gly Ile Pro Asn Tyr Gly Tyr Arg Ile Arg Asp Glu
        755                 760                 765
```

Gly His Phe Thr Asp Thr Val Val Leu Tyr Ser Cys Asn Pro Gly Tyr
770 775 780

Ala Met His Gly Ser Asn Thr Leu Thr Cys Leu Ser Gly Asp Arg Arg
785 790 795 800

Val Trp Asp Lys Pro Leu Pro Ser Cys Ile Ala Glu Cys Gly Gly Gln
805 810 815

Ile His Ala Ala Thr Ser Gly Arg Ile Leu Ser Pro Gly Tyr Pro Ala
820 825 830

Pro Tyr Asp Asn Asn Leu His Cys Thr Trp Ile Ile Glu Ala Asp Pro
835 840 845

Gly Lys Thr Ile Ser Leu His Phe Ile Val Phe Asp Thr Glu Met Ala
850 855 860

His Asp Ile Leu Lys Val Trp Asp Gly Pro Val Asp Ser Asp Ile Leu
865 870 875 880

Leu Lys Glu Trp Ser Gly Ser Ala Leu Pro Glu Asp Ile His Ser Thr
885 890 895

Phe Asn Ser Leu Thr Leu Gln Phe Asp Ser Asp Phe Phe Ile Ser Lys
900 905 910

Ser Gly Phe Ser Ile Gln Phe Ser Thr Ser Ile Ala Ala Thr Cys Asn
915 920 925

Asp Pro Gly Met Pro Gln Asn Gly Thr Arg Tyr Gly Asp Ser Arg Glu
930 935 940

Ala Gly Asp Thr Val Thr Phe Gln Cys Asp Pro Gly Tyr Gln Leu Gln
945 950 955 960

Gly Gln Ala Lys Ile Thr Cys Val Gln Leu Asn Asn Arg Phe Phe Trp
965 970 975

Gln Pro Asp Pro Pro Thr Cys Ile Ala Ala Cys Gly Gly Asn Leu Thr
980 985 990

Gly Pro Ala Gly Val Ile Leu Ser Pro Asn Tyr Pro Gln Pro Tyr Pro
995 1000 1005

Pro Gly Lys Glu Cys Asp Trp Arg Val Lys Val Asn Pro Asp Phe
1010 1015 1020

Val Ile Ala Leu Ile Phe Lys Ser Phe Asn Met Glu Pro Ser Tyr
1025 1030 1035

Asp Phe Leu His Ile Tyr Glu Gly Glu Asp Ser Asn Ser Pro Leu
1040 1045 1050

Ile Gly Ser Tyr Gln Gly Ser Gln Ala Pro Glu Arg Ile Glu Ser
1055 1060 1065

Ser Gly Asn Ser Leu Phe Leu Ala Phe Arg Ser Asp Ala Ser Val
1070 1075 1080

Gly Leu Ser Gly Phe Ala Ile Glu Phe Lys Glu Lys Pro Arg Glu
1085 1090 1095

Ala Cys Phe Asp Pro Gly Asn Ile Met Asn Gly Thr Arg Val Gly
1100 1105 1110

Thr Asp Phe Lys Leu Gly Ser Thr Ile Thr Tyr Gln Cys Asp Ser
1115 1120 1125

Gly Tyr Lys Ile Leu Asp Pro Ser Ser Ile Thr Cys Val Ile Gly
1130 1135 1140

Ala Asp Gly Lys Pro Ser Trp Asp Gln Val Leu Pro Ser Cys Asn
1145 1150 1155

Ala Pro Cys Gly Gly Gln Tyr Thr Gly Ser Glu Gly Val Val Leu
1160 1165 1170

Ser Pro Asn Tyr Pro His Asn Tyr Thr Ala Gly Gln Ile Cys Leu
1175 1180 1185

Tyr Ser Ile Thr Val Pro Lys Glu Phe Val Val Phe Gly Gln Phe
1190 1195 1200

Ala Tyr Phe Gln Thr Ala Leu Asn Asp Leu Ala Glu Leu Phe Asp
1205 1210 1215

Gly Thr His Ala Gln Ala Arg Leu Leu Ser Ser Leu Ser Gly Ser
1220 1225 1230

His Ser Gly Glu Thr Leu Pro Leu Ala Thr Ser Asn Gln Ile Leu
1235 1240 1245

Leu Arg Phe Ser Ala Lys Ser Gly Ala Ser Ala Arg Gly Phe His
1250 1255 1260

Phe Val Tyr Gln Ala Val Pro Arg Thr Ser Asp Thr Gln Cys Ser
1265 1270 1275

Ser Val Pro Glu Pro Arg Tyr Gly Arg Arg Ile Gly Ser Glu Phe
1280 1285 1290

Ser Ala Gly Ser Ile Val Arg Phe Glu Xaa Asn Pro Gly Tyr Leu
1295 1300 1305

Leu Gln Gly Ser Thr Ala Leu His Cys Gln Ser Val Pro Asn Ala
1310 1315 1320

Leu Ala Gln Trp Asn Asp Thr Ile Pro Ser Cys Val Val Pro Cys
1325 1330 1335

Ser Gly Asn Phe Thr Gln Arg Arg Gly Thr Ile Leu Ser Pro Gly
1340 1345 1350

Tyr Pro Glu Pro Tyr Gly Asn Asn Leu Asn Cys Ile Trp Lys Ile
1355 1360 1365

Ile Val Thr Glu Gly Ser Gly Ile Gln Ile Gln Val Ile Ser Phe
1370 1375 1380

Ala Thr Glu Gln Asn Trp Asp Ser Leu Glu Ile His Asp Gly Gly
1385 1390 1395

Asp Val Thr Ala Pro Arg Leu Gly Ser Phe Ser Gly Thr Thr Val
1400 1405 1410

Pro Ala Leu Leu Asn Ser Thr Ser Asn Gln Leu Tyr Leu His Phe
1415 1420 1425

Gln Ser Asp Ile Ser Val Ala Ala Ala Gly Phe His Leu Glu Tyr
1430 1435 1440

Lys Thr Val Gly Leu Ala Ala Cys Gln Glu Pro Ala Leu Pro Ser
1445 1450 1455

Asn Ser Ile Lys Ile Gly Asp Arg Tyr Met Val Asn Asp Val Leu
1460 1465 1470

Ser Phe Gln Cys Glu Pro Gly Tyr Thr Leu Gln Gly Arg Ser His
1475 1480 1485

Ile Ser Cys Met Pro Gly Thr Val Arg Arg Trp Asn Tyr Pro Ser
1490 1495 1500

Pro Leu Cys Ile Ala Thr Cys Gly Gly Thr Leu Ser Thr Leu Gly
1505 1510 1515

Gly Val Ile Leu Ser Pro Gly Phe Pro Gly Ser Tyr Pro Asn Asn
1520 1525 1530

Leu Asp Cys Thr Trp Arg Ile Ser Leu Pro Ile Gly Tyr Gly Ala
1535 1540 1545

His Ile Gln Phe Leu Asn Phe Ser Thr Glu Ala Asn His Asp Phe
1550 1555 1560

Leu Glu Ile Gln Asn Gly Pro Tyr His Thr Ser Pro Met Ile Gly
1565 1570 1575

Gln Phe Ser Gly Thr Asp Leu Pro Ala Ala Leu Leu Ser Thr Thr
1580 1585 1590

His Glu Thr Leu Ile His Phe Tyr Ser Asp His Ser Gln Asn Arg
1595 1600 1605

Gln Gly Phe Lys Leu Ala Tyr Gln Ala Tyr Glu Leu Gln Asn Cys
1610 1615 1620

Pro Asp Pro Pro Pro Phe Gln Asn Gly Tyr Met Ile Asn Ser Asp
1625 1630 1635

Tyr Ser Val Gly Gln Ser Val Ser Phe Glu Cys Tyr Pro Gly Tyr
1640 1645 1650

Ile Leu Ile Gly His Pro Val Leu Thr Cys Gln His Gly Ile Asn
1655 1660 1665

Arg Asn Trp Asn Tyr Pro Phe Pro Arg Cys Asp Ala Pro Cys Gly
1670 1675 1680

Tyr Asn Val Thr Ser Gln Asn Gly Thr Ile Tyr Ser Pro Gly Phe
1685 1690 1695

Pro Asp Glu Tyr Pro Ile Leu Lys Asp Cys Ile Trp Leu Ile Thr
1700 1705 1710

Val Pro Pro Gly His Gly Val Tyr Ile Asn Phe Thr Leu Leu Gln
1715 1720 1725

Thr Glu Ala Val Asn Asp Tyr Ile Ala Val Trp Asp Gly Pro Asp
1730 1735 1740

```
Gln Asn Ser Pro Gln Leu Gly Val Phe Ser Gly Asn Thr Ala Leu
    1745                1750                1755

Glu Thr Ala Tyr Ser Ser Thr Asn Gln Val Leu Leu Lys Phe His
    1760                1765                1770

Ser Asp Phe Ser Asn Gly Gly Phe Phe Val Leu Asn Phe His Ala
    1775                1780                1785

Phe Gln Leu Lys Lys Cys Gln Pro Pro Pro Ala Val Pro Gln Ala
    1790                1795                1800

Glu Met Leu Thr Glu Asp Asp Asp Phe Glu Ile Gly Asp Phe Val
    1805                1810                1815

Lys Tyr Gln Cys His Pro Gly Tyr Thr Leu Val Gly Thr Asp Ile
    1820                1825                1830

Leu Thr Cys Lys Leu Ser Ser Gln Leu Gln Phe Glu Gly Ser Leu
    1835                1840                1845

Pro Thr Cys Glu Ala Gln Cys Pro Ala Asn Glu Val Arg Thr Gly
    1850                1855                1860

Ser Ser Gly Val Ile Leu Ser Pro Gly Tyr Pro Gly Asn Tyr Phe
    1865                1870                1875

Asn Ser Gln Thr Cys Ser Trp Ser Ile Lys Val Glu Pro Asn Tyr
    1880                1885                1890

Asn Ile Thr Ile Phe Val Asp Thr Phe Gln Ser Glu Lys Gln Phe
    1895                1900                1905

Asp Ala Leu Glu Val Phe Asp Gly Ser Ser Gly Gln Ser Pro Leu
    1910                1915                1920

Leu Val Val Leu Ser Gly Asn His Thr Glu Gln Ser Asn Phe Thr
    1925                1930                1935

Ser Arg Ser Asn Gln Leu Tyr Leu Arg Trp Ser Thr Asp His Ala
    1940                1945                1950

Thr Ser Lys Lys Gly Phe Lys Ile Arg Tyr Ala Ala Pro Tyr Cys
    1955                1960                1965

Ser Leu Thr His Pro Leu Lys Asn Gly Gly Ile Leu Asn Arg Thr
    1970                1975                1980
```

Ala Gly Ala Val Gly Ser Lys Val His Tyr Phe Cys Lys Pro Gly
1985 1990 1995

Tyr Arg Met Val Gly His Ser Asn Ala Thr Cys Arg Arg Asn Pro
2000 2005 2010

Leu Gly Met Tyr Gln Trp Asp Ser Leu Thr Pro Leu Cys Gln Ala
2015 2020 2025

Val Ser Cys Gly Ile Pro Glu Ser Pro Gly Asn Gly Ser Phe Thr
2030 2035 2040

Gly Asn Glu Phe Thr Leu Asp Ser Lys Val Val Tyr Glu Cys His
2045 2050 2055

Glu Gly Phe Lys Leu Glu Ser Ser Gln Gln Ala Thr Ala Val Cys
2060 2065 2070

Gln Glu Asp Gly Leu Trp Ser Asn Lys Gly Lys Pro Pro Thr Cys
2075 2080 2085

Lys Pro Val Ala Cys Pro Ser Ile Glu Ala Gln Leu Ser Glu His
2090 2095 2100

Val Ile Trp Arg Leu Val Ser Gly Ser Leu Asn Glu Tyr Gly Ala
2105 2110 2115

Gln Val Leu Leu Ser Cys Ser Pro Gly Tyr Tyr Leu Glu Gly Trp
2120 2125 2130

Arg Leu Leu Arg Cys Gln Ala Asn Gly Thr Trp Asn Ile Gly Asp
2135 2140 2145

Glu Arg Pro Ser Cys Arg Val Ile Ser Cys Gly Ser Leu Ser Phe
2150 2155 2160

Pro Pro Asn Gly Asn Lys Ile Gly Thr Leu Thr Val Tyr Gly Ala
2165 2170 2175

Thr Ala Ile Phe Thr Cys Asn Thr Gly Tyr Thr Leu Val Gly Ser
2180 2185 2190

His Val Arg Glu Cys Leu Ala Asn Gly Leu Trp Ser Gly Ser Glu
2195 2200 2205

Thr Arg Cys Leu Ala Gly His Cys Gly Ser Pro Asp Pro Ile Val
2210 2215 2220

Asn Gly His Ile Ser Gly Asp Gly Phe Ser Tyr Arg Asp Thr Val
2225 2230 2235

Val Tyr Gln Cys Asn Pro Gly Phe Arg Leu Val Gly Thr Ser Val
2240 2245 2250

Arg Ile Cys Leu Gln Asp His Lys Trp Ser Gly Gln Thr Pro Val
2255 2260 2265

Cys Val Pro Ile Thr Cys Gly His Pro Gly Asn Pro Ala His Gly
2270 2275 2280

Phe Thr Asn Gly Ser Glu Phe Asn Leu Asn Asp Val Val Asn Phe
2285 2290 2295

Thr Cys Asn Thr Gly Tyr Leu Leu Gln Gly Val Ser Arg Ala Gln
2300 2305 2310

Cys Arg Ser Asn Gly Gln Trp Ser Ser Pro Leu Pro Thr Cys Arg
2315 2320 2325

Val Val Asn Cys Ser Asp Pro Gly Phe Val Glu Asn Ala Ile Arg
2330 2335 2340

His Gly Gln Gln Asn Phe Pro Glu Ser Phe Glu Tyr Gly Met Ser
2345 2350 2355

Ile Leu Tyr His Cys Lys Lys Gly Phe Tyr Leu Leu Gly Ser Ser
2360 2365 2370

Ala Leu Thr Cys Met Ala Asn Gly Leu Trp Asp Arg Ser Leu Pro
2375 2380 2385

Lys Cys Leu Ala Ile Ser Cys Gly His Pro Gly Val Pro Ala Asn
2390 2395 2400

Ala Val Leu Thr Gly Glu Leu Phe Thr Tyr Gly Ala Val Val His
2405 2410 2415

Tyr Ser Cys Arg Gly Ser Glu Ser Leu Ile Gly Asn Asp Thr Arg
2420 2425 2430

Val Cys Gln Glu Asp Ser His Trp Ser Gly Ala Leu Pro His Cys
2435 2440 2445

Thr Gly Asn Asn Pro Gly Phe Cys Gly Asp Pro Gly Thr Pro Ala
2450 2455 2460

His Gly Ser Arg Leu Gly Asp Asp Phe Lys Thr Lys Ser Leu Leu
2465 2470 2475

Arg Phe Ser Cys Glu Met Gly His Gln Leu Arg Gly Ser Pro Glu
2480 2485 2490

Arg Thr Cys Leu Leu Asn Gly Ser Trp Ser Gly Leu Gln Pro Val
2495 2500 2505

Cys Glu Ala Val Ser Cys Gly Asn Pro Gly Thr Pro Thr Asn Gly
2510 2515 2520

Met Ile Val Ser Ser Asp Gly Ile Leu Phe Ser Ser Ser Val Ile
2525 2530 2535

Tyr Ala Cys Trp Glu Gly Tyr Lys Thr Ser Gly Leu Met Thr Arg
2540 2545 2550

His Cys Thr Ala Asn Gly Thr Trp Thr Gly Thr Ala Pro Asp Cys
2555 2560 2565

Thr Ile Ile Ser Cys Gly Asp Pro Gly Thr Leu Ala Asn Gly Ile
2570 2575 2580

Gln Phe Gly Thr Asp Phe Thr Phe Asn Lys Thr Val Ser Tyr Gln
2585 2590 2595

Cys Asn Pro Gly Tyr Val Met Glu Ala Val Thr Ser Ala Thr Ile
2600 2605 2610

Arg Cys Thr Lys Asp Gly Arg Trp Asn Pro Ser Lys Pro Val Cys
2615 2620 2625

Lys Ala Val Leu Cys Pro Gln Pro Pro Pro Val Gln Asn Gly Thr
2630 2635 2640

Val Glu Gly Ser Asp Phe Arg Trp Gly Ser Ser Ile Ser Tyr Ser
2645 2650 2655

Cys Met Asp Gly Tyr Gln Leu Ser His Ser Ala Ile Leu Ser Cys
2660 2665 2670

Glu Gly Arg Gly Val Trp Lys Gly Glu Ile Pro Gln Cys Leu Pro
2675 2680 2685

Val Phe Cys Gly Asp Pro Gly Ile Pro Ala Glu Gly Arg Leu Ser
2690 2695 2700

Gly Lys Ser Phe Thr Tyr Lys Ser Glu Val Phe Phe Gln Cys Lys
2705 2710 2715

Ser Pro Phe Ile Leu Val Gly Ser Ser Arg Arg Val Cys Gln Ala
2720 2725 2730

Asp Gly Thr Trp Ser Gly Ile Gln Pro Thr Cys Ile Asp Pro Ala
2735 2740 2745

His Asn Thr Cys Pro Asp Pro Gly Thr Pro His Phe Gly Ile Gln
2750 2755 2760

Asn Ser Ser Arg Gly Tyr Glu Val Gly Ser Thr Val Phe Phe Arg
2765 2770 2775

Cys Arg Lys Gly Tyr His Ile Gln Gly Ser Thr Thr Arg Thr Cys
2780 2785 2790

Leu Ala Asn Leu Thr Trp Ser Gly Ile Gln Thr Glu Cys Ile Pro
2795 2800 2805

His Ala Cys Arg Gln Pro Glu Thr Pro Ala His Ala Asp Val Arg
2810 2815 2820

Ala Ile Asp Leu Pro Thr Phe Gly Tyr Thr Leu Val Tyr Thr Cys
2825 2830 2835

His Pro Gly Phe Phe Leu Ala Gly Gly Ser Glu His Arg Thr Cys
2840 2845 2850

Lys Ala Asp Met Lys Trp Thr Gly Lys Ser Pro Val Cys Lys Ser
2855 2860 2865

Lys Gly Val Arg Glu Val Asn Glu Thr Val Thr Lys Thr Pro Val
2870 2875 2880

Pro Ser Asp Val Phe Phe Val Asn Ser Leu Trp Lys Gly Tyr Tyr
2885 2890 2895

Glu Tyr Leu Gly Lys Arg Gln Pro Ala Thr Leu Thr Val Asp Trp
2900 2905 2910

Phe Asn Ala Thr Ser Ser Lys Val Asn Ala Thr Phe Ser Glu Ala
2915 2920 2925

Ser Pro Val Glu Leu Lys Leu Thr Gly Ile Tyr Lys Lys Glu Glu
2930 2935 2940

```
Ala His  Leu Leu Leu Lys Ala  Phe Gln Ile Lys Gly  Gln Ala Asp
    2945                 2950                 2955

Ile Phe  Val Ser Lys Phe Glu  Asn Asp Asn Trp Gly  Leu Asp Gly
    2960                 2965                 2970

Tyr Val  Ser Ser Gly Leu Glu  Arg Gly Gly Phe Thr  Phe Gln Gly
    2975                 2980                 2985

Asp Ile  His Gly Lys Asp Phe  Gly Lys Phe Lys Leu  Glu Arg Gln
    2990                 2995                 3000

Asp Pro  Leu Asn Pro Asp Gln  Asp Ser Ser Ser His  Tyr His Gly
    3005                 3010                 3015

Thr Ser  Ser Gly Ser Val Ala  Ala Ala Ile Leu Val  Pro Phe Phe
    3020                 3025                 3030

Ala Leu  Ile Leu Ser Gly Phe  Ala Phe Tyr Leu Tyr  Lys His Arg
    3035                 3040                 3045

Thr Arg  Pro Lys Val Gln Tyr  Asn Gly Tyr Ala Gly  His Glu Asn
    3050                 3055                 3060

Ser Asn  Gly Gln Ala Ser Phe  Glu Asn Pro Met Tyr  Asp Thr Asn
    3065                 3070                 3075

Leu Lys  Pro Thr Glu Ala Lys  Ala Val Arg Phe Asp  Thr Thr Leu
    3080                 3085                 3090

Asn Thr  Val Cys Thr Val Val
    3095                 3100
```

## Description

This application claims the benefit of U.S. Provisional Application No. 60/222,504, filed August 2, 2000 and U.S. Application No. 09/728,787 filed November 28, 2000, which are hereby incorporated by reference.

### Field of the Invention

The present invention relates to novel C3b/C4b Complement Receptor-like polypeptides and nucleic acid molecules encoding the same. The invention also relates to vectors, host cells, pharmaceutical compositions, selective binding agents and methods for producing C3b/C4b Complement Receptor-like polypeptides. Also provided for are methods for the diagnosis, treatment, amelioration, and/or prevention of diseases associated with C3b/C4b Complement Receptor-like polypeptides.

### Background of the Invention

Technical advances in the identification, cloning, expression and manipulation of nucleic acid molecules and the deciphering of the human genome have greatly accelerated the discovery of novel therapeutics. Rapid nucleic acid sequencing techniques can now generate sequence information at unprecedented rates and, coupled with computational analyses, allow the assembly of overlapping sequences into partial and entire genomes and the identification of polypeptide-encoding regions. A comparison of a predicted amino acid sequence against a database compilation of known amino acid sequences allows one to determine the extent of homology to previously identified sequences and/or structural landmarks. The cloning and expression of a polypeptide-encoding region of a nucleic acid molecule provides a polypeptide product for structural and functional analyses. The manipulation of nucleic acid molecules and encoded polypeptides may confer advantageous properties on a product for use as a therapeutic.

Despite the significant technical advances in genome research over the past decade, the potential for the development of novel therapeutics based on the human genome is still largely unrealized. Many genes encoding potentially beneficial polypeptide therapeutics, or those encoding polypeptides, which may act as "targets" for therapeutic molecules, have still not been identified.

Accordingly, it is an object of the invention to identify novel polypeptides and nucleic acid molecules encoding the same, which have diagnostic or therapeutic benefit.

### Summary of the Invention

The present invention relates to novel C3b/C4b Complement Receptor-like nucleic acid molecules and encoded polypeptides.

The invention provides for an isolated nucleic acid molecule comprising a nucleotide sequence selected from the group consisting of:

(a) the nucleotide sequence as set forth in SEQ ID NO:1, SEQ ID NO:3, or SEQ ID NO:6;

(b) a nucleotide sequence encoding the polypeptide as set forth in SEQ ID NO:2, SEQ ID NO:4, or SEQ ID NO:7;

(c) a nucleotide sequence which hybridizes under moderately or highly stringent conditions to the complement of (a) or (b), wherein the encoded polypeptide has an activity of the polypeptide as set forth in SEQ ID NO:2, SEQ ID NO:4, or SEQ ID NO:7; and

(d) a nucleotide sequence complementary to any of (a)-(c).

The invention also provides for an isolated nucleic acid molecule comprising a nucleotide sequence selected from the group consisting of:

(a) a nucleotide sequence encoding a polypeptide that is at least about 70, 75, 80, 85, 90, 95, 96, 97, 98, or 99 percent identical to the polypeptide as set forth in SEQ ID NO: 2 , SEQ ID NO: 4 , or SEQ ID NO:7, wherein the polypeptide has an activity of the polypeptide as set forth in SEQ ID NO:2, SEQ ID NO:4, or SEQ ID NO:7;

(b) a nucleotide sequence encoding an allelic variant or splice variant of the nucleotide sequence as set forth in SEQ ID NO:1, SEQ ID NO:3, or SEQ ID NO:6, wherein the encoded polypeptide has an activity of the polypeptide as set forth in SEQ ID NO:2, SEQ ID NO:4, or SEQ ID NO:7;

(c) a nucleotide sequence of SEQ ID NO:1, SEQ ID NO:3, or SEQ ID NO:6, (a), or (b) encoding a polypeptide fragment of at least about 25 amino acid residues, wherein the polypeptide has an activity of the polypeptide as set forth in SEQ ID NO:2, SEQ ID NO:4, or SEQ ID NO:7;

(d) a nucleotide sequence of SEQ ID NO:1, SEQ ID NO:3, or SEQ ID NO:6, or (a)-(c) comprising a fragment of at least about 16 nucleotides;

(e) a nucleotide sequence which hybridizes under moderately or highly stringent conditions to the complement of any of (a)-(d), wherein the polypeptide has an activity of the polypeptide as set forth in SEQ ID NO:2, SEQ ID NO: 4, or SEQ ID NO:7; and

(f) a nucleotide sequence complementary to any of (a) - (e).

The invention further provides for an isolated nucleic acid molecule comprising a nucleotide sequence selected from the group consisting of:

(a) a nucleotide sequence encoding a polypeptide as set forth in SEQ ID NO:2, SEQ ID NO:4, or SEQ ID NO:7, with at least one conservative amino acid substitution, wherein the polypeptide has an activity of the polypeptide as set forth in SEQ ID NO:2, SEQ ID NO:4, or SEQ ID NO:7;

(b) a nucleotide sequence encoding a polypeptide as set forth in SEQ ID NO:2, SEQ ID NO:4, or SEQ ID NO:7 with at least one amino acid insertion, wherein the polypeptide has an activity of the polypeptide as set forth in SEQ ID NO:2, SEQ ID NO:4, or SEQ ID NO:7;

(c) a nucleotide sequence encoding a polypeptide as set forth in SEQ ID NO:2, SEQ ID NO:4, or SEQ ID NO:7 with at least one amino acid deletion, wherein the polypeptide has an activity of the polypeptide as set forth in SEQ ID NO:2, SEQ ID NO:4, or SEQ ID NO:7;

(d) a nucleotide sequence encoding a polypeptide as set forth in SEQ ID NO:2, SEQ ID NO:4, or SEQ ID NO:7 which has a C- and/or N- terminal truncation, wherein the polypeptide has an activity of the polypeptide as set forth in SEQ ID NO:2, SEQ ID NO:4, or SEQ ID NO:7;

(e) a nucleotide sequence encoding a polypeptide as set forth in SEQ ID NO:2, SEQ ID NO:4, or SEQ ID NO:7 with at least one modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2, SEQ ID NO:4, or SEQ ID NO:7;

(f) a nucleotide sequence of (a)-(e) comprising a fragment of at least about 16 nucleotides;

(g) a nucleotide sequence which hybridizes under moderately or highly stringent conditions to the complement of any of (a)-(f), wherein the polypeptide has an activity of the polypeptide as set forth in SEQ ID NO:2, SEQ ID NO: 4, or SEQ ID NO:7; and

(h) a nucleotide sequence complementary to any of (a) - (e).

The invention also provides for an isolated polypeptide comprising the amino acid sequence selected from the group consisting of:

(a) an amino acid sequence of the mature C3b/C4b Complement Receptor-like polypeptide wherein the polypeptide comprises the amino acid sequence contained within SEQ ID NO:2, SEQ ID NO:4, or SEQ ID NO:7, and optionally further comprises an amino-terminal methionine;

(b) an amino acid sequence for an ortholog of SEQ ID NO:2, SEQ ID NO:4, or SEQ ID NO:7, wherein the encoded polypeptide has an activity of the polypeptide as set forth,in SEQ ID NO:2, SEQ ID NO:4, or SEQ ID NO:7;

(c) an amino acid sequence that is at least about 70, 80, 85, 90, 95, 96, 97, 98, or 99 percent identical to the amino acid sequence of SEQ ID NO:2, SEQ ID NO:4, or SEQ ID NO:7, wherein the polypeptide has an activity of the polypeptide as set forth in SEQ ID NO:2, SEQ ID NO:4, or SEQ ID NO:7;

(d) a fragment of the amino acid sequence set forth in SEQ ID NO:2, SEQ ID NO:4, or SEQ ID NO:7 comprising at least about 25 amino acid residues, wherein the polypeptide has an activity of the polypeptide as set forth in SEQ ID NO:2, SEQ ID NO:4, or SEQ ID NO:7;

(e) an amino acid sequence for an allelic variant or splice variant of either the amino acid sequence as set forth in SEQ ID NO:2, SEQ ID NO:4, or SEQ ID NO:7, or at least one of (a)-(c) wherein the polypeptide has an activity of the polypeptide as set forth in SEQ ID NO:2, SEQ ID NO:4, or SEQ ID NO:7.

The invention further provides for an isolated polypeptide comprising the amino acid sequence selected from the group consisting of:

(a) the amino acid sequence as set forth in SEQ ID NO:2, SEQ ID NO:4, or SEQ ID NO:7 with at least one conservative amino acid substitution, wherein the polypeptide has an activity of the polypeptide as set forth in SEQ ID NO:2, SEQ ID NO:4, or SEQ ID NO:7;

(b) the amino acid sequence as set forth in SEQ ID NO:2, SEQ ID NO:4, or SEQ ID NO:7 with at least one amino acid insertion, wherein the polypeptide has an activity of the polypeptide as set forth in SEQ ID NO:2, SEQ ID NO: 4, or SEQ ID NO:7;

(c) the amino acid sequence as set forth in SEQ ID NO:2, SEQ ID NO:4, or SEQ ID NO:7 with at least one amino acid deletion, wherein the polypeptide has an activity of the polypeptide as set forth in SEQ ID NO:2, SEQ ID NO: 4, or SEQ ID NO:7;

(d) the amino acid sequence as set forth in SEQ ID NO:2, SEQ ID NO:4, or SEQ ID NO:7 which has a C- and/or N-terminal truncation, wherein the polypeptide has an activity of the polypeptide as set forth in SEQ ID NO:2, SEQ ID NO:4, or SEQ ID NO:7; and

(e) the amino acid sequence as set forth in SEQ ID NO:2, SEQ ID NO:4, or SEQ ID NO:7, with at least one modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2, SEQ ID NO:4, or SEQ ID NO:7.

Also provided are fusion polypeptides comprising the amino acid sequences of (a)-(e) above.

The present invention also provides for an expression vector comprising the isolated nucleic acid molecules as set forth herein, recombinant host cells comprising recombinant nucleic acid molecules as set forth herein, and a method of producing a C3b/C4b Complement Receptor-like polypeptide comprising culturing the host cells and optionally isolating the polypeptide so produced.

A transgenic non-human animal comprising a nucleic acid molecule encoding a C3b/C4b Complement Receptor-like polypeptide is also encompassed by the invention. The C3b/C4b Complement Receptor-like nucleic acid molecules are introduced into the animal in a manner that allows expression and increased levels of the C3b/C4b Complement Receptor-like polypeptide, which may include increased circulating levels. The transgenic non-human animal is preferably a mammal.

Also provided are derivatives of the C3b/C4b Complement Receptor-like polypeptides of the present invention.

Additionally provided are selective binding agents such as antibodies and peptides capable of specifically binding the C3b/C4b Complement Receptor-like polypeptides of the invention. Such antibodies and peptides may be agonistic or antagonistic.

Pharmaceutical compositions comprising the nucleotides, polypeptides, or selective binding agents of the present invention and one or more pharmaceutically acceptable formulation agents are also encompassed by the invention. The pharmaceutical compositions are used to provide therapeutically effective amounts of the nucleotides or polypeptides of the present invention. The invention is also directed to methods of using the polypeptides, nucleic acid molecules, and selective binding agents.

The C3b/C4b Complement Receptor-like polypeptides and nucleic acid molecules of the present invention may be used to treat, prevent, ameliorate, and/or detect diseases and disorders, including those recited herein.

The present invention also provides a method of assaying test molecules to identify a test molecule which binds to a C3b/C4b Complement Receptor-like polypeptide. The method comprises contacting a C3b/C4b Complement Receptor-like polypeptide with a test molecule and determining the extent of binding of the test molecule to the polypeptide. The method further comprises determining whether such test molecules are agonists or antagonists of a C3b/C4b Complement Receptor-like polypeptide. The present invention further provides a method of testing the impact of molecules on the expression of C3b/C4b Complement Receptor-like polypeptide or on the activity of C3b/C4b Complement Receptor-like polypeptide.

Methods of regulating expression and modulating (i.e., increasing or decreasing) levels of a C3b/C4b Complement Receptor-like polypeptide are also encompassed by the invention. One method comprises administering to an animal a nucleic acid molecule encoding a C3b/C4b Complement Receptor-like polypeptide. In another method, a nucleic acid molecule comprising elements that regulate or modulate the expression of a C3b/C4b Complement Receptor-like polypeptide may be administered. Examples of these methods include gene therapy, cell therapy, and anti-sense therapy as further described herein.

The C3b/C4b Complement Receptor-like polypeptide can be used for identifying ligands thereof. Various forms of "expression cloning" have been used for cloning ligands for receptors. *See e.g.,* Davis et al., Cell, 87:1161-1169 (1996). These and other C3b/C4b Complement Receptor-like ligand cloning experiments are described in greater detail herein. Isolation of the C3b/C4b Complement Receptor-like ligand(s) allows for the identification or development of novel agonists and/or antagonists of the C3b/C4b Complement Receptor-like signaling pathway. Such agonists and antagonists include C3b/C4b Complement Receptor-like ligand(s), anti-C3b/C4b Complement Receptor-like ligand antibodies and derivatives thereof, small molecules, or antisense oligonucleotides, any of which can be used for potentially treating one or more diseases or disorders, including those recited herein.

## Brief Description of the Figures

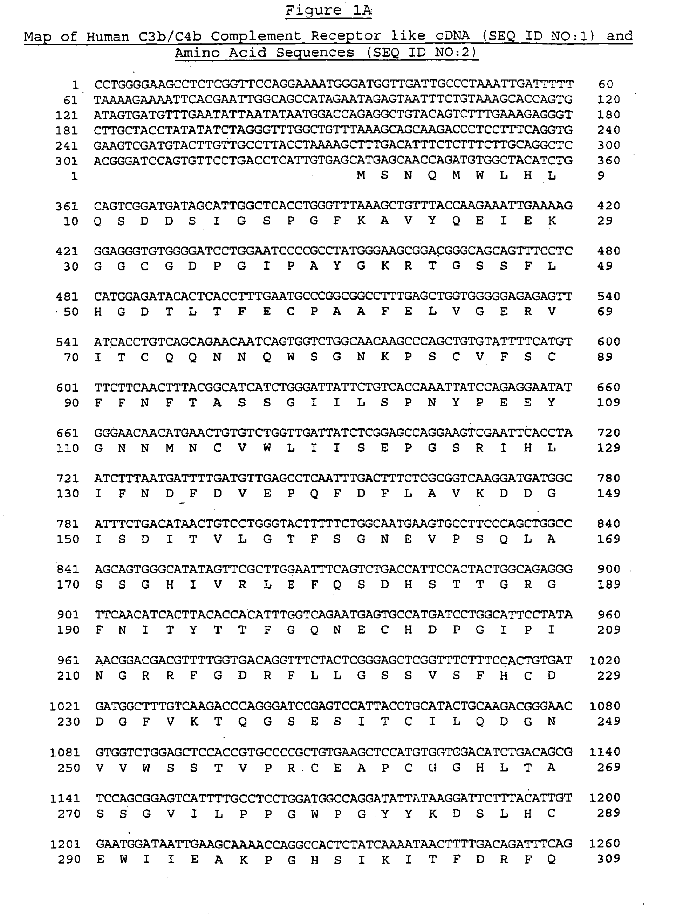

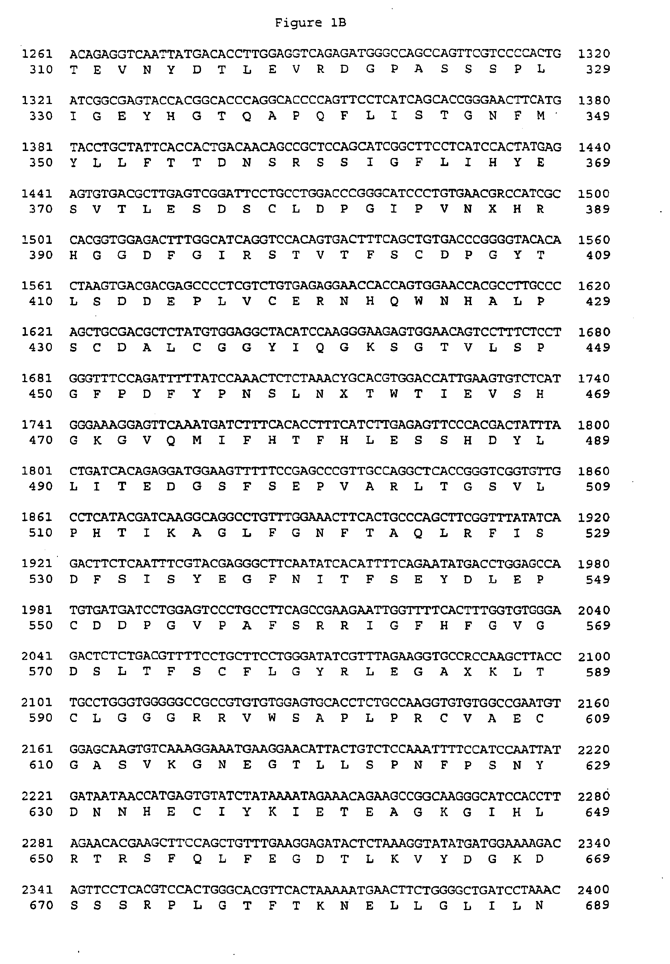

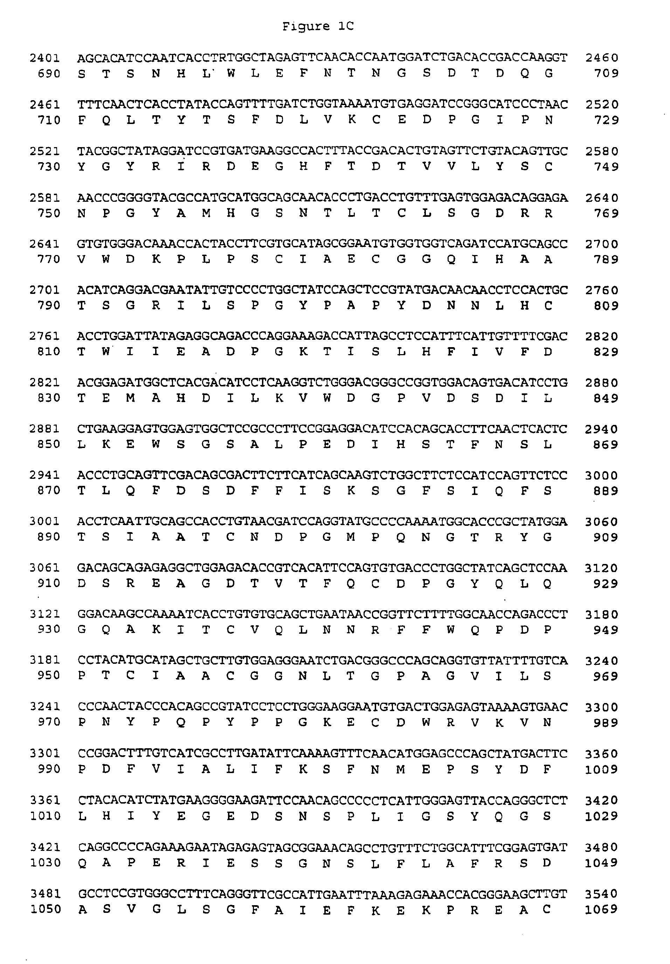

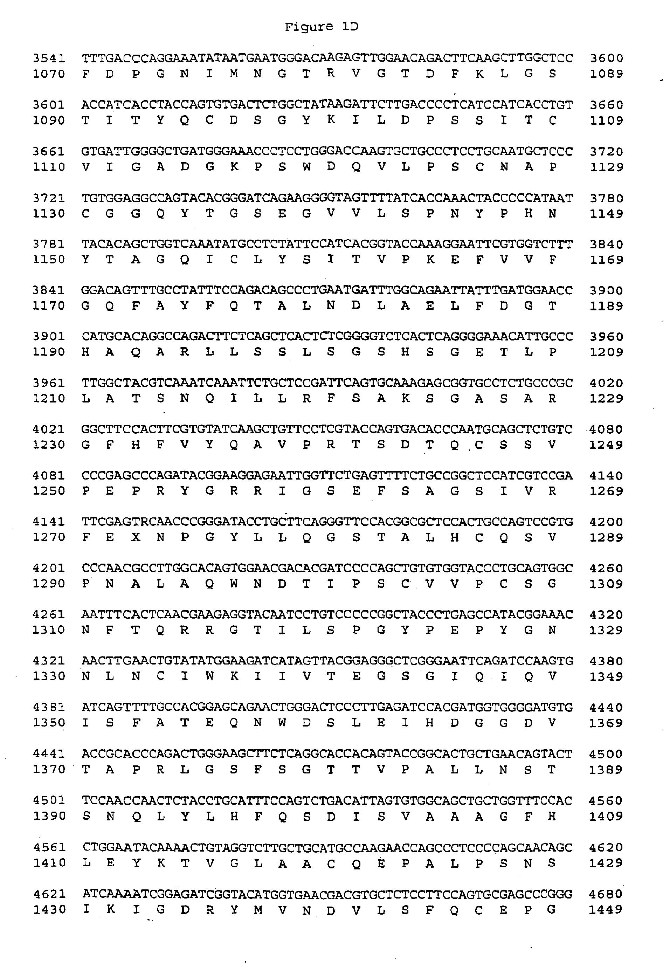

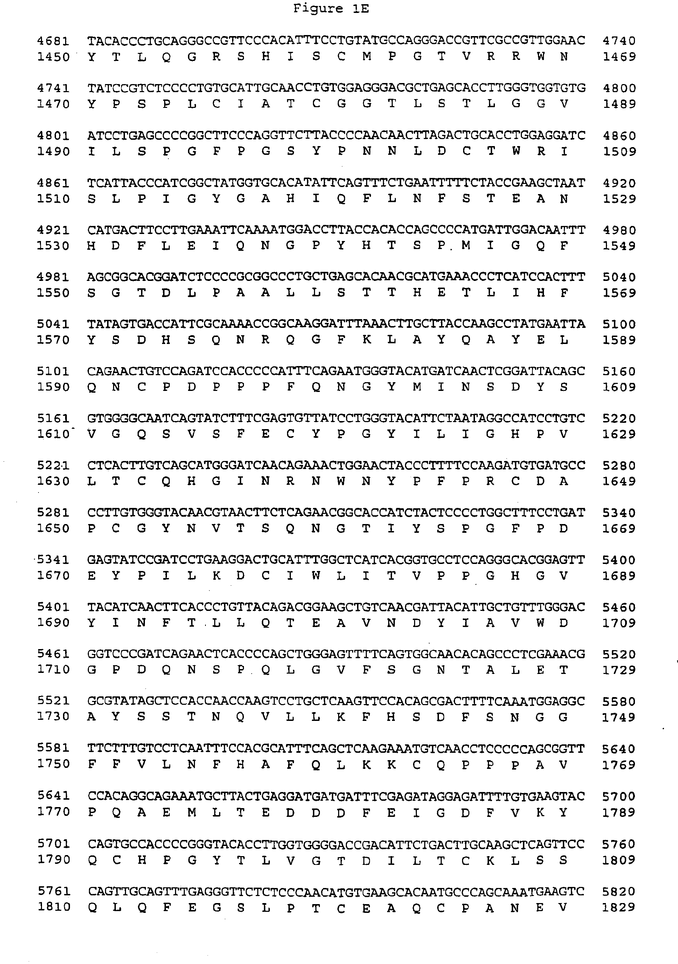

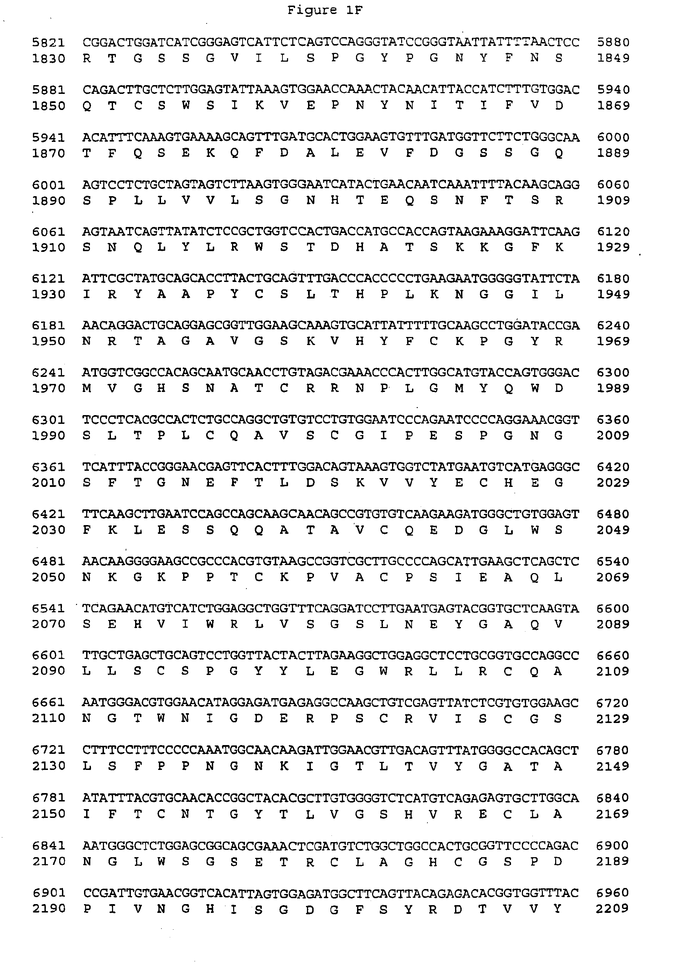

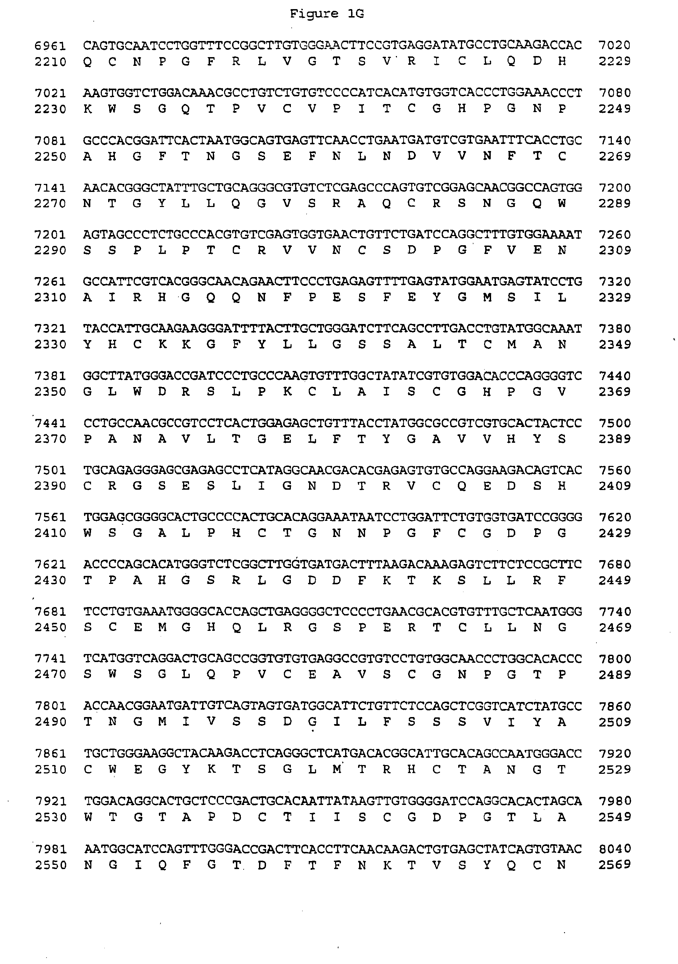

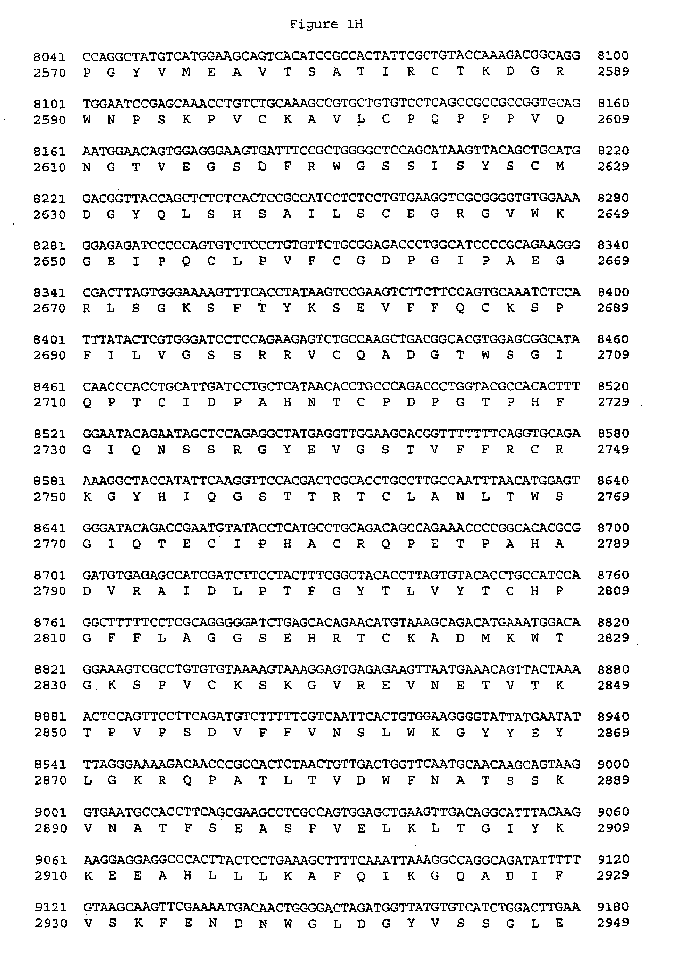

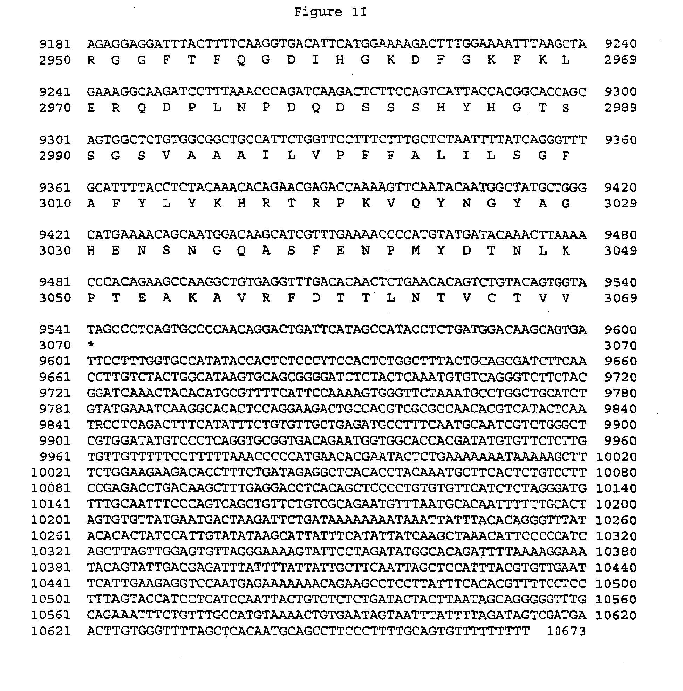

Figure 1 depicts a nucleic acid sequence (SEQ ID NO:1) encoding human C3b/C4b Complement Receptor-like polypeptide. Also depicted is the amino acid sequence (SEQ ID NO:2) of human C3B/C4b Complement Receptor-like polypeptide.

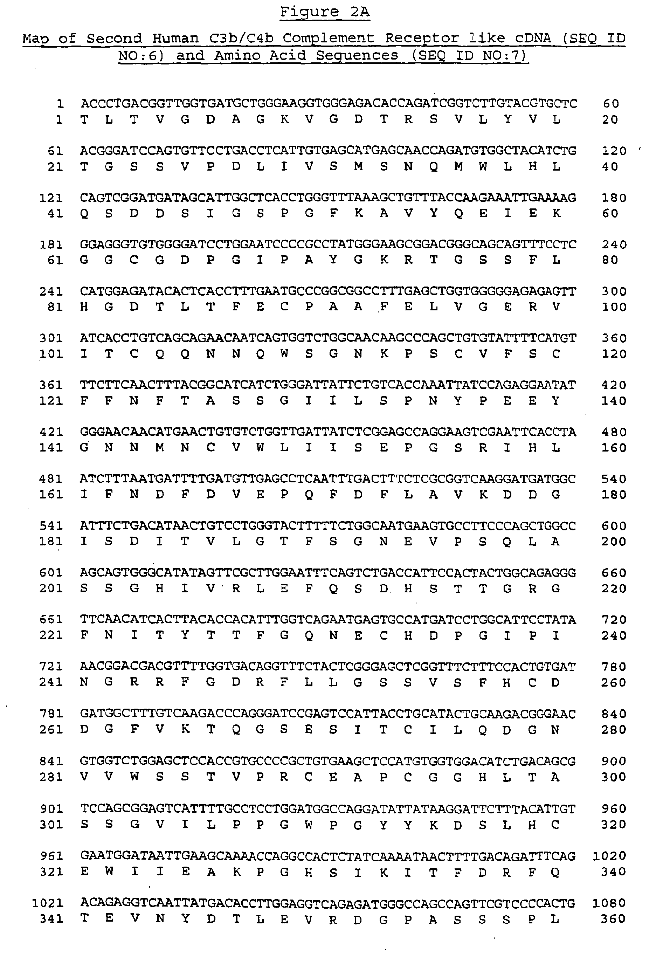

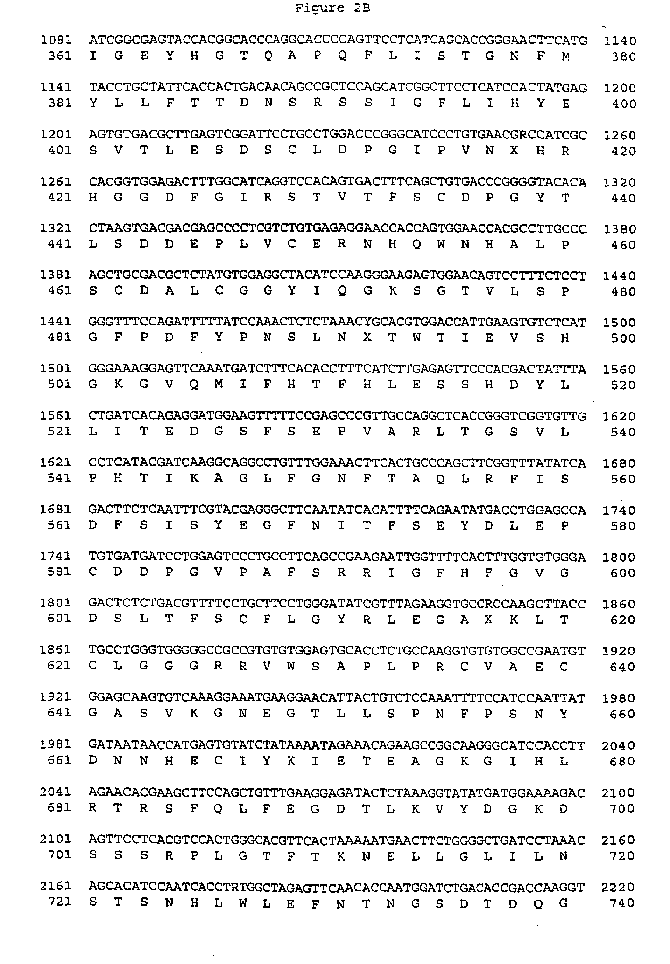

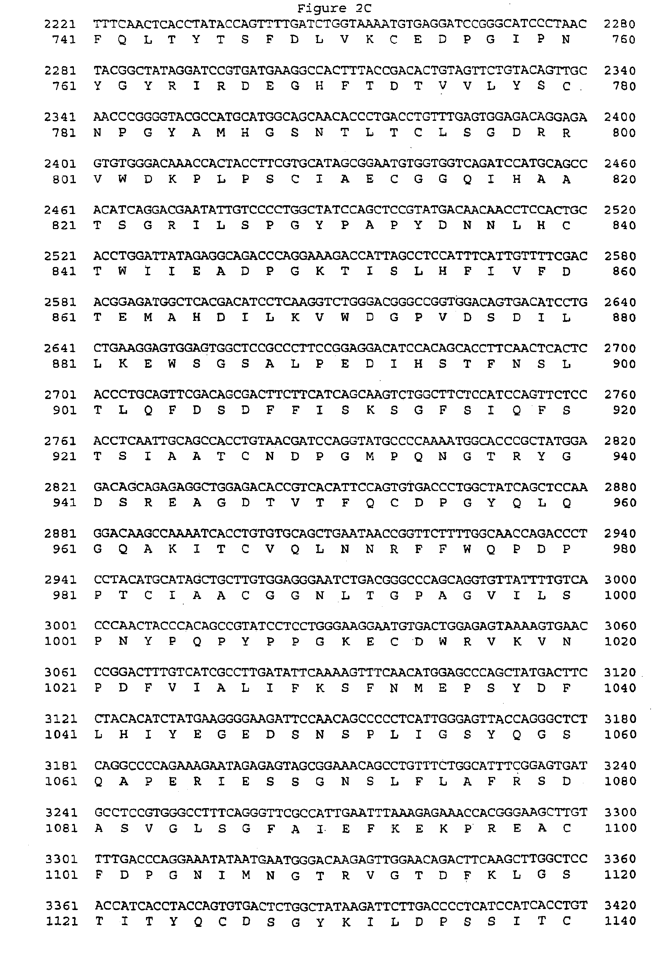

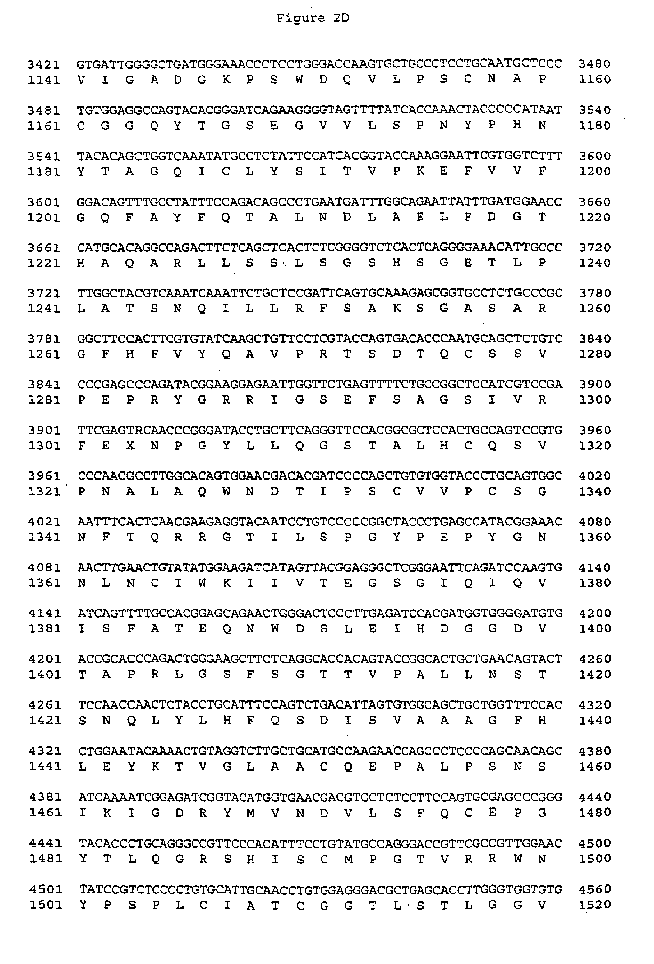

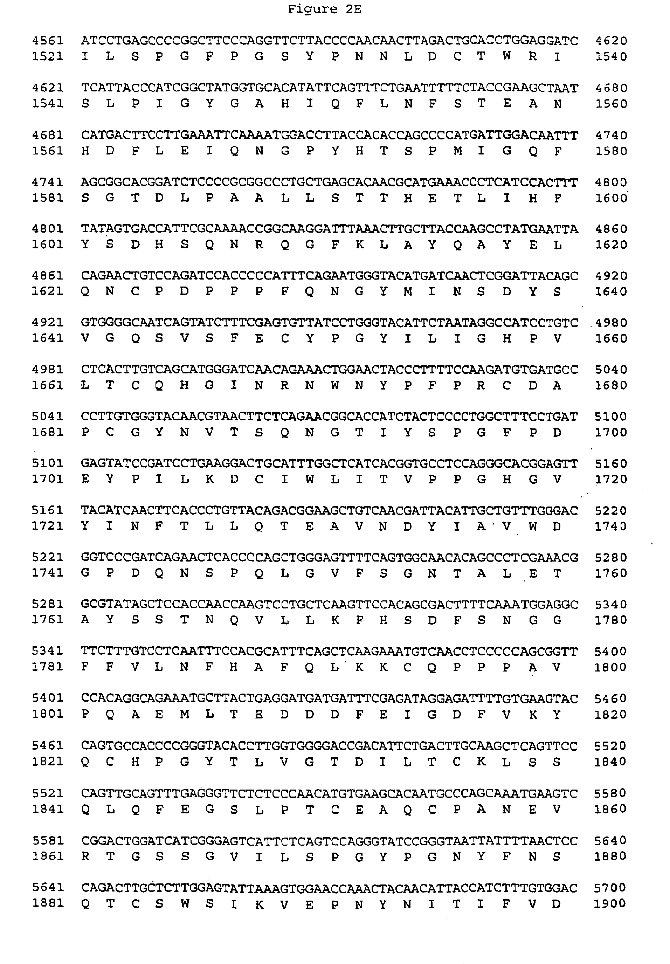

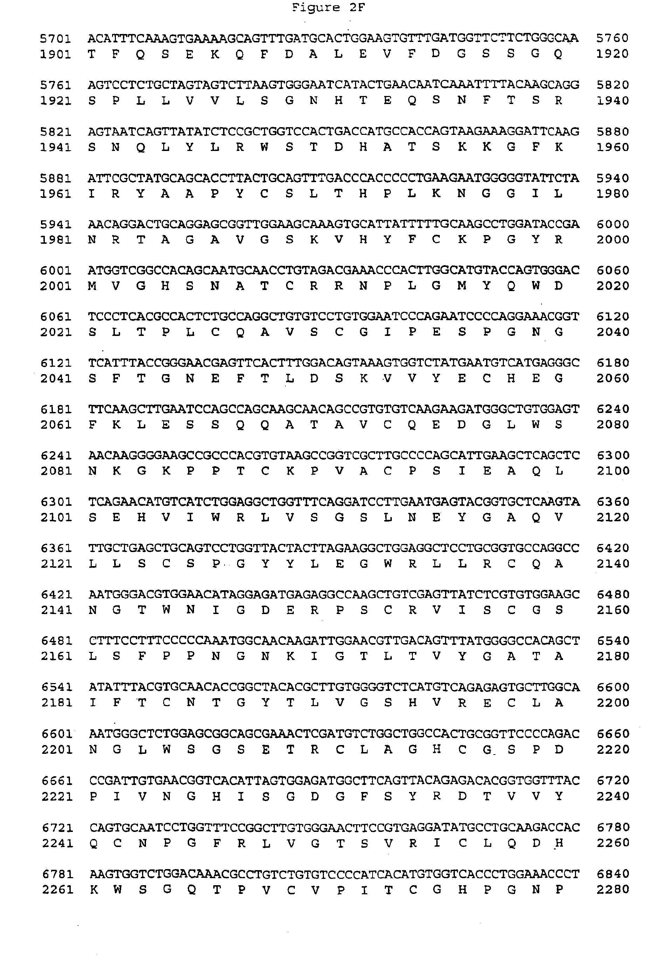

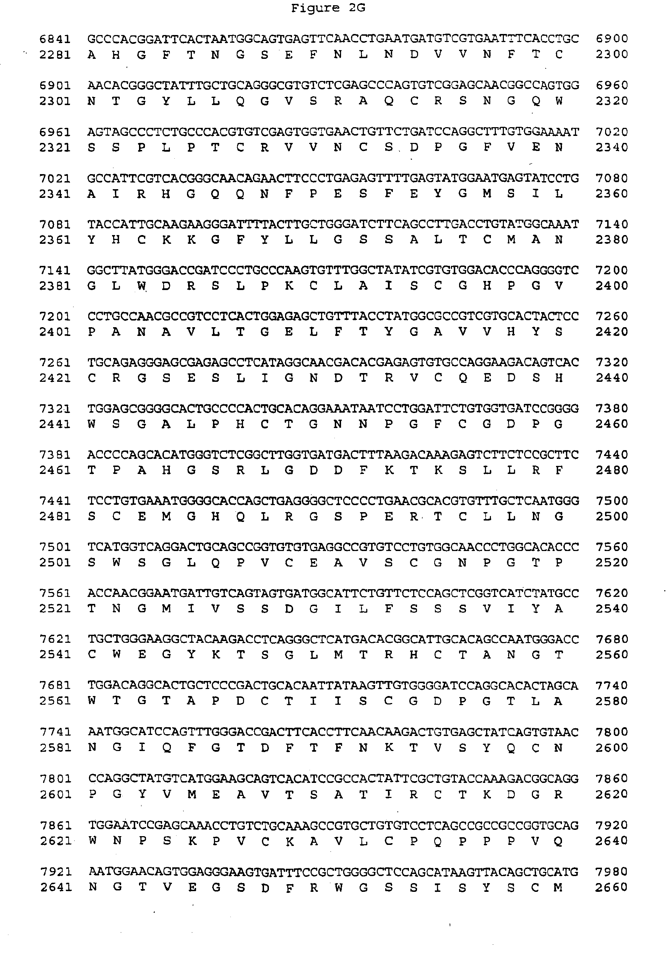

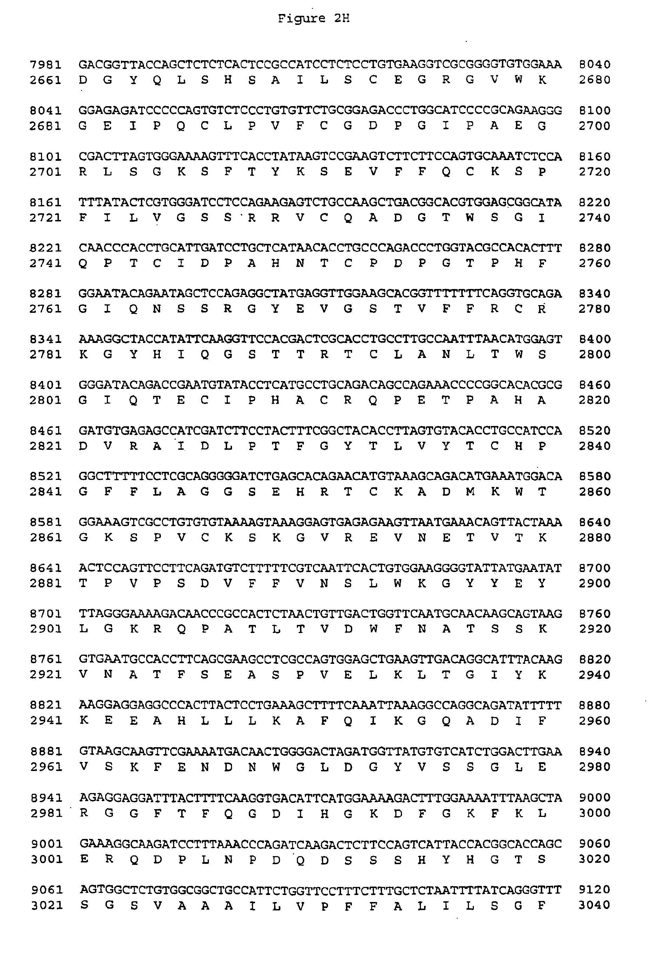

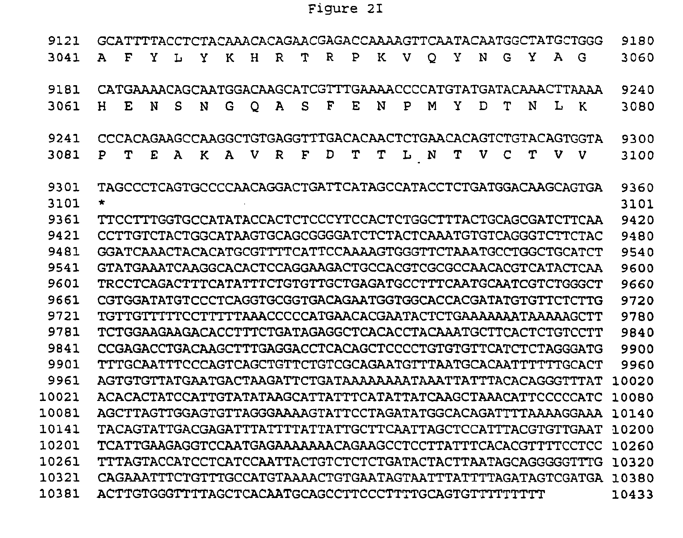

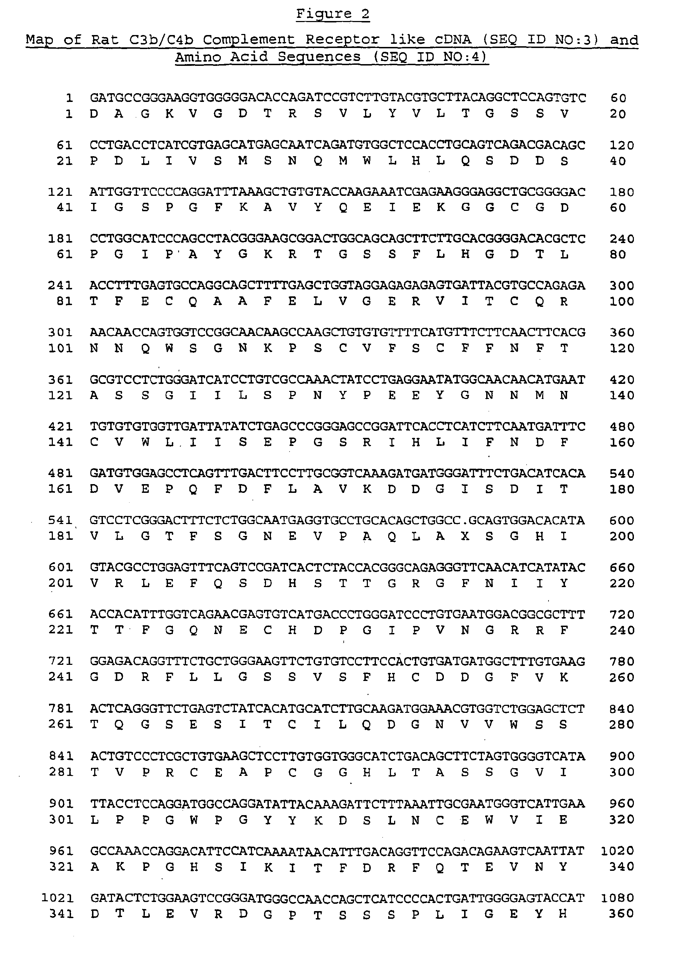

Figure 2 depicts a nucleic acid sequence (SEQ ID NO:6) encoding a second human C3b/C4b Complement Receptor-like polypeptide. Also depicted is the amino acid sequence (SEQ ID NO:7) of human C3B/C4b Complement Receptor-like polypeptide.

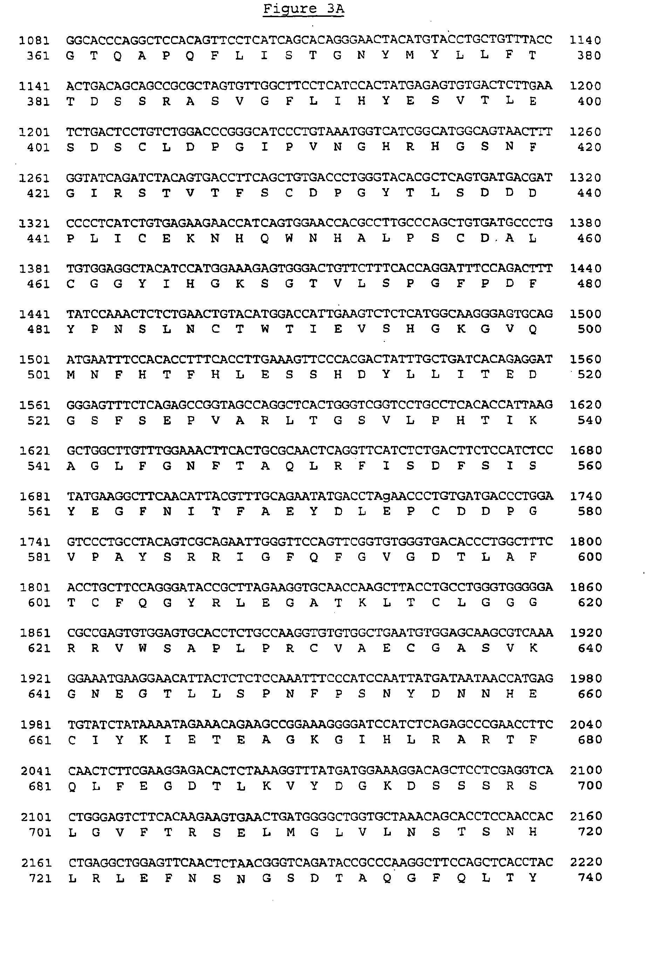

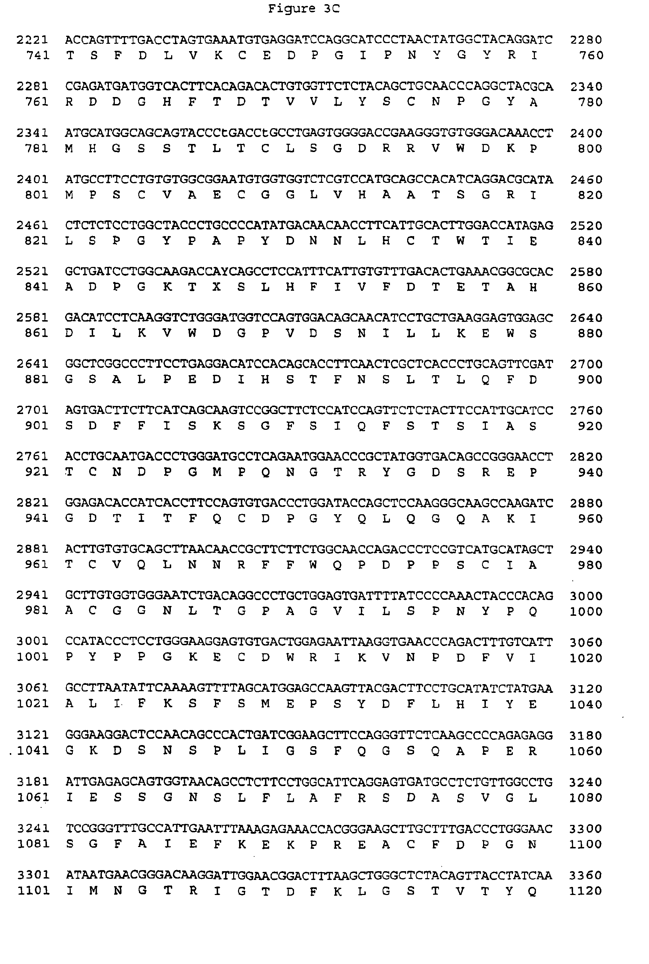

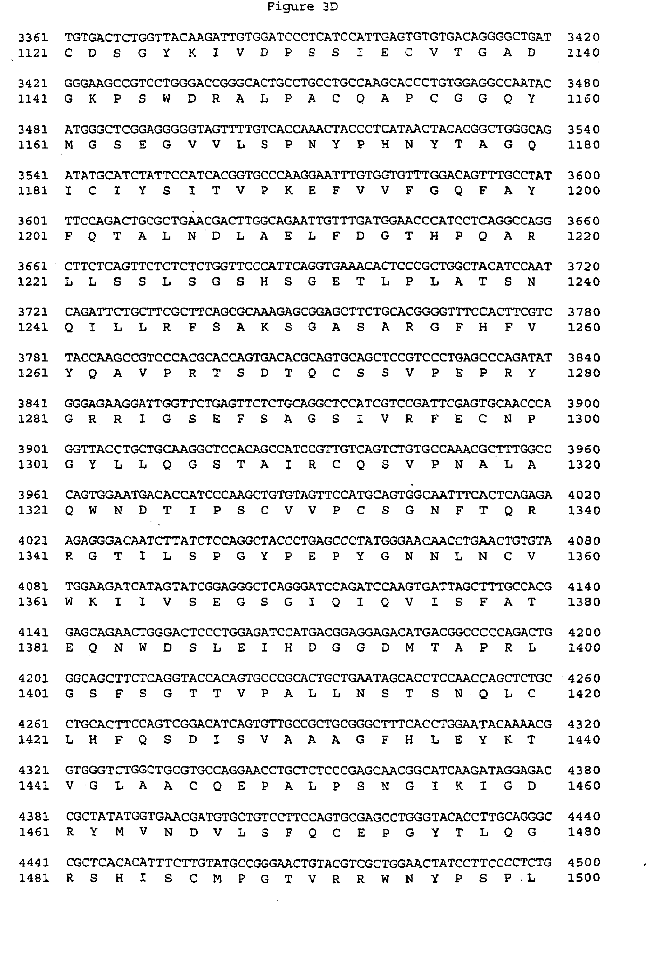

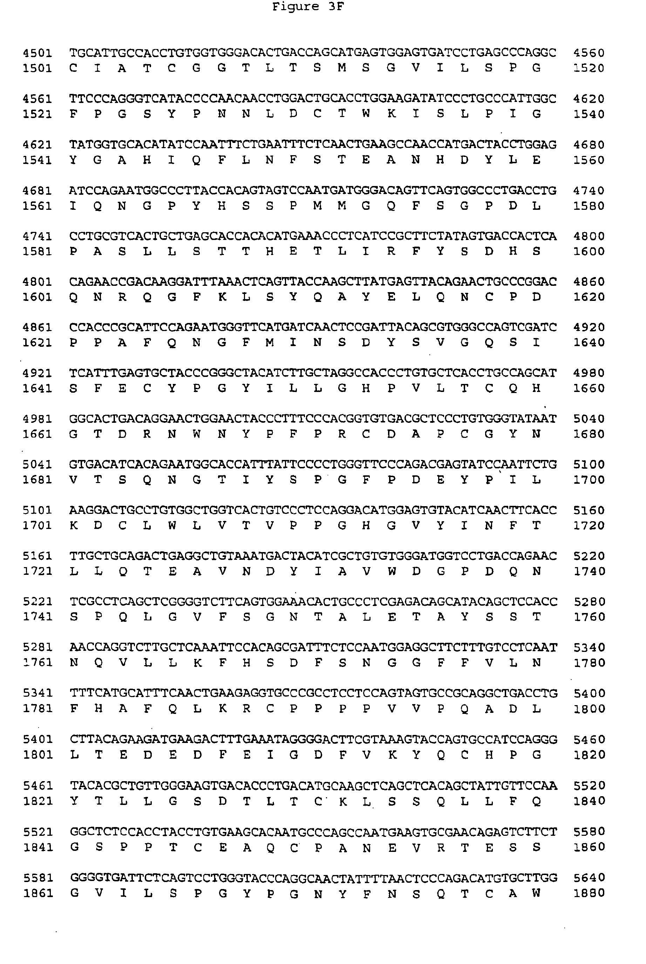

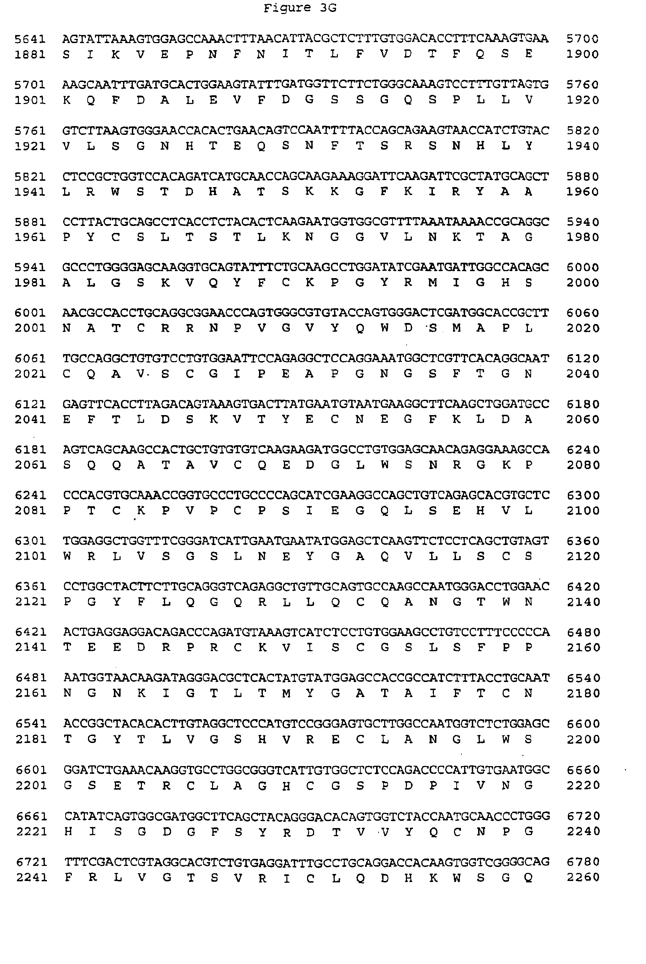

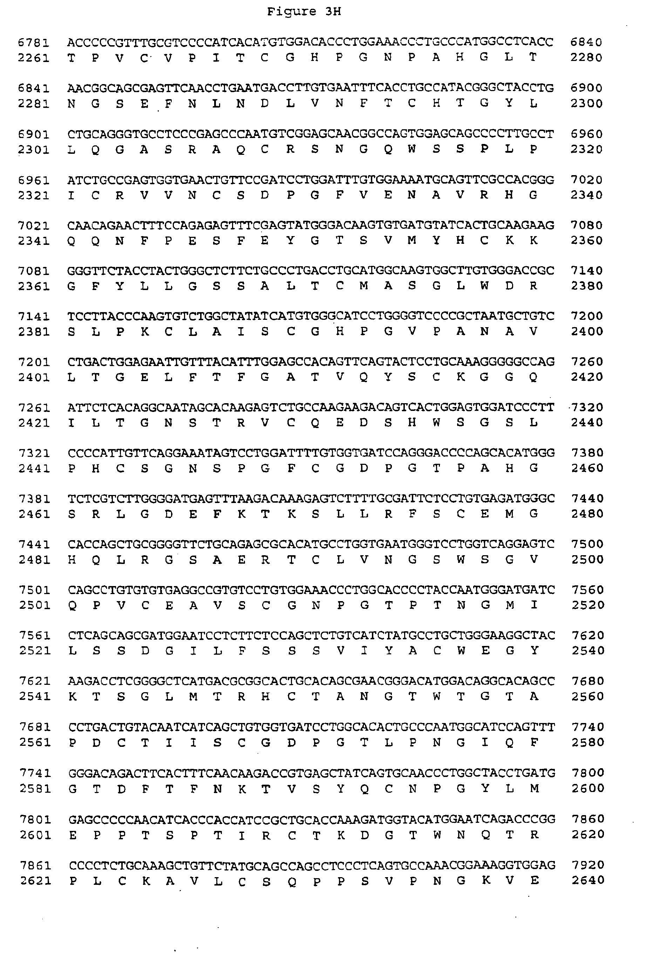

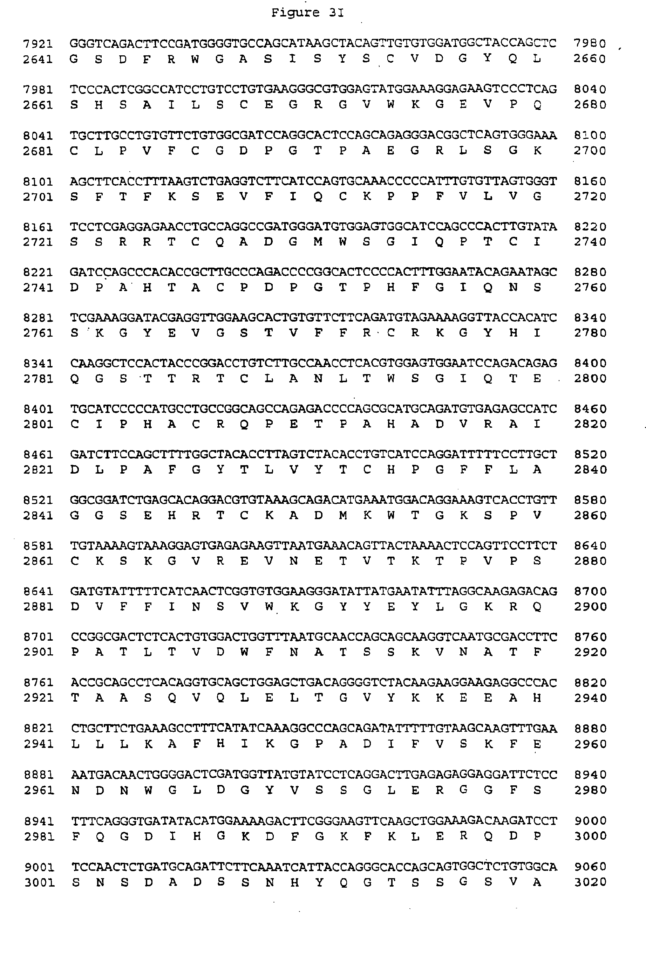

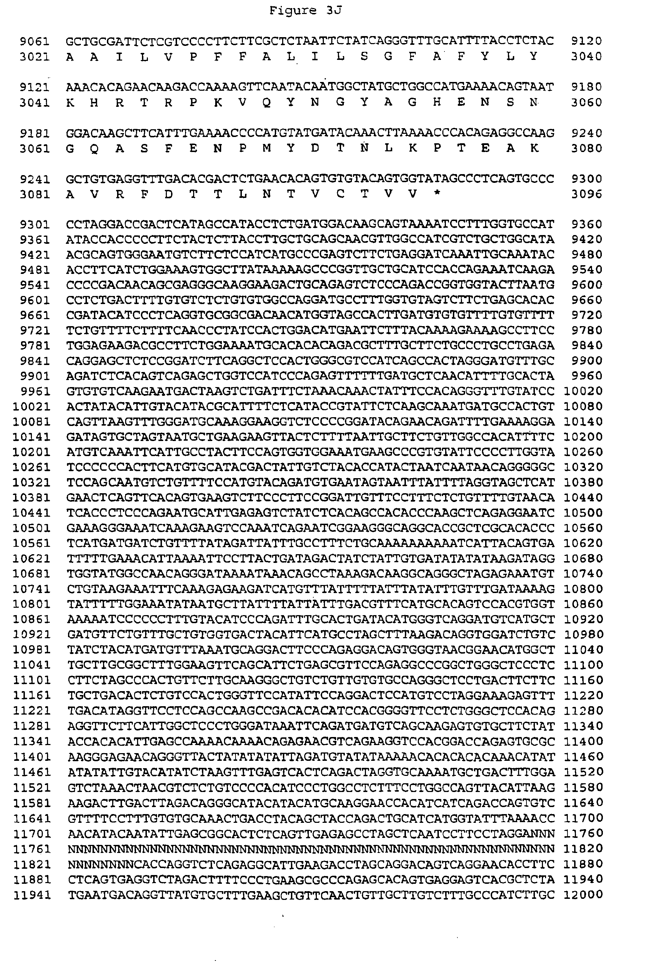

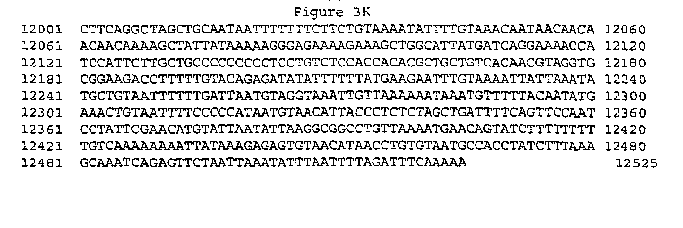

Figure 3 depicts a nucleic acid sequence (SEQ ID NO:3) encoding rat C3b/C4b Complement Receptor-like polypeptide. Also depicted is the amino acid sequence of rat C3b/C4b Complement Receptor-like polypeptide (SEQ ID NO:4).

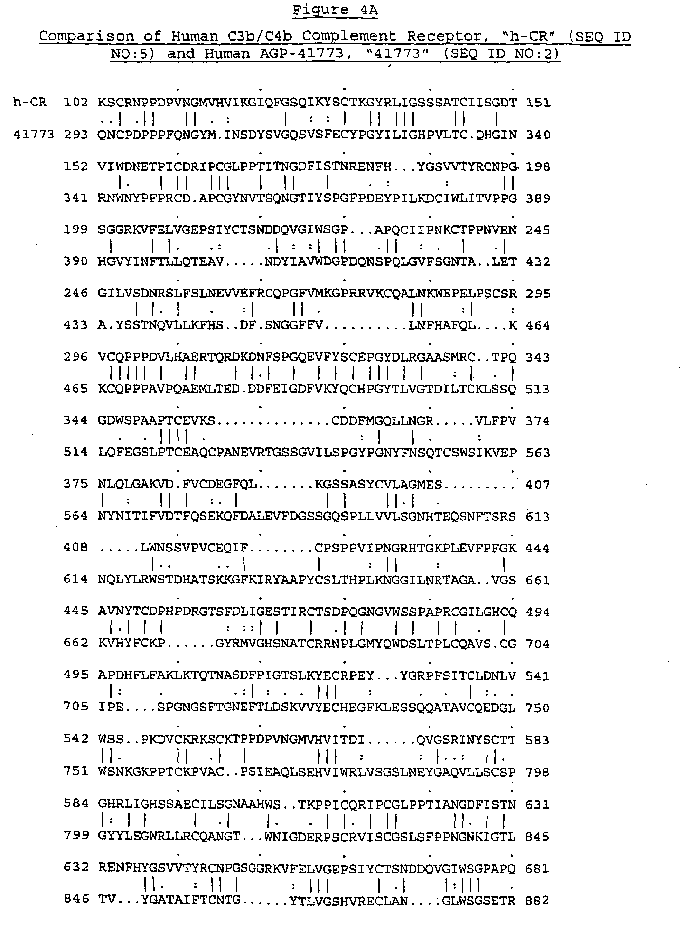

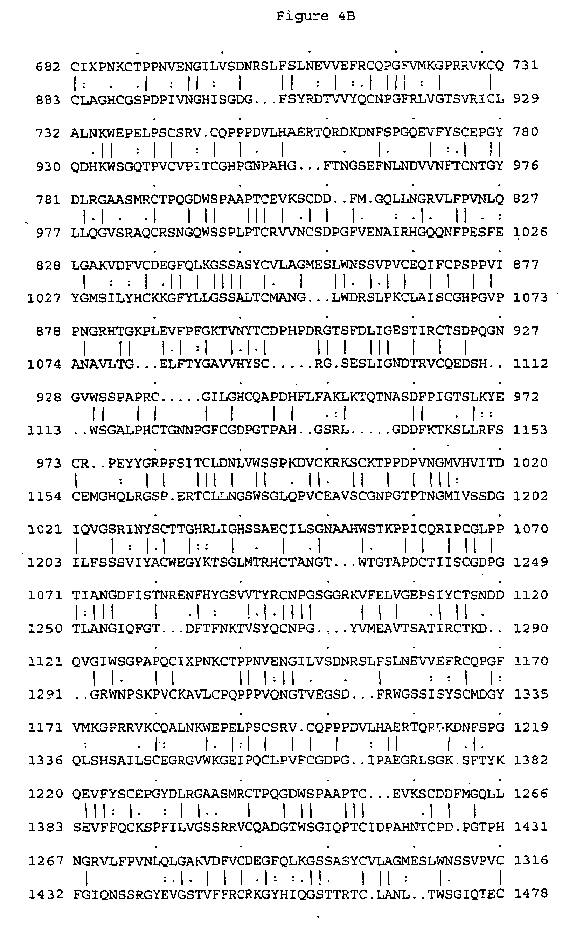

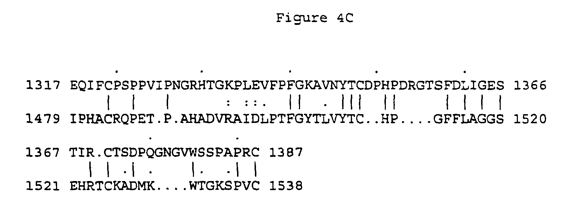

Figure 4 depicts an amino acid comparison of a known human C3b/C4b Complement Receptor (SEQ ID NO:5) and the human AGP-41773 (SEQ ID NO:2).

## Detailed Description of the Invention

The section headings used herein are for organizational purposes only and are not to be construed as limiting the subject matter described. All references cited in this application are expressly incorporated by reference herein.

## Definitions

The term "C3b/C4b Complement Receptor-like" is abbreviated herein as "C3b/C4b CR-like" and is also referred to as "AGP-41773". The terms "C3b/C4b CR-like gene" or "C3b/C4b CR-like nucleic acid molecule" or "polynucleotide" refers to a nucleic acid molecule comprising or consisting of a nucleotide sequence as set forth in SEQ ID NO:1, SEQ ID NO:3, or SEQ ID NO:6, a nucleotide sequence encoding the polypeptide as set forth in SEQ ID NO:2, SEQ ID NO: 4; or SEQ ID NO:7, and nucleic acid molecules as defined herein.

The term "C3b/C4b CR-like polypeptide" refers to a polypeptide comprising the amino acid sequence of SEQ ID NO:2, SEQ ID NO:4, or SEQ ID NO:7, and related polypeptides. Related polypeptides include: C3b/C4b CR-like polypeptide allelic variants, C3b/C4b CR-like polypeptide orthologs, C3b/C4b CR-like polypeptide splice variants, C3b/C4b CR-like polypeptide variants and C3b/C4b CR-like polypeptide derivatives. C3b/C4b CR-like polypeptides may be mature polypeptides, as defined herein, and may or may not have an amino terminal methionine residue, depending on the method by which they are prepared.

The term "C3b/C4b CR-like polypeptide allelic variant" refers to one of several possible naturally occurring alternate forms of a gene occupying a given locus on a chromosome of an organism or a population of organisms.

The term "C3b/C4b CR-like polypeptide derivatives" refers to the polypeptide as set forth in SEQ ID NO:2, SEQ ID NO:4, or SEQ ID NO:7, C3b/C4b CR-like polypeptide allelic variants, C3b/C4b CR-like polypeptide orthologs, C3b/C4b CR-like polypeptide splice variants, or C3b/C4b CR-like polypeptide variants, as defined herein, that have been chemically modified.

The term "C3b/C4b CR-like polypeptide fragment" refers to a polypeptide that comprises a truncation at the amino terminus (with or without a leader sequence) and/or a truncation at the carboxy terminus of the polypeptide as set forth in SEQ ID NO:2, SEQ ID NO:4, or SEQ ID NO:7, C3b/C4b CR-like polypeptide allelic variants, C3b/C4b CR-like polypeptide orthologs, C3b/C4b CR-like polypeptide splice variants and/or a C3b/C4b CR-like polypeptide variant having one or more amino acid additions or substitutions or internal deletions (wherein the resulting polypeptide is at least 6 amino acids or more in length) as compared to the C3b/C4b CR-like polypeptide amino acid sequence set forth in SEQ ID NO:2, SEQ ID NO:4, or SEQ ID NO:7. C3b/C4b CR-like polypeptide fragments may result from alternative RNA splicing or from *in vivo* protease activity. For transmembrane or membrane-bound forms of a C3b/C4b CR-like polypeptide, preferred fragments include soluble forms such as those lacking a transmembrane or membrane-binding domain. In preferred embodiments, truncations comprise about 10 amino acids, or about 20 amino acids, or about 50 amino acids, or about 75 amino acids, or about 100 amino acids, or more than about 100 amino acids. The polypeptide fragments so produced will comprise about 25 contiguous amino acids, or about 50 amino acids, or about 75 amino acids, or about 100 amino acids, or about 150 amino acids, or about 200 amino acids. Such C3b/C4b CR-like polypeptide fragments may optionally comprise an amino terminal methionine residue. It will be appreciated that such fragments can be used, for example, to generate antibodies to C3b/C4b CR-like polypeptides.

The term "C3b/C4b CR-like fusion polypeptide" refers to a fusion of one or more amino acids (such as a heterologous peptide or polypeptide) at the amino or carboxy terminus of the polypeptide as set forth in SEQ ID NO:2, SEQ ID NO:4, or SEQ ID NO:7, C3b/C4b CR-like polypeptide allelic variants, C3b/C4b CR-like polypeptide orthologs, C3b/C4b CR-like polypeptide splice variants, or C3b/C4b CR-like polypeptide variants having one or more amino acid deletions, substitutions or internal additions as compared to the C3b/C4b CR-like polypeptide amino acid sequence set forth in SEQ ID NO:2, SEQ ID NO:4, or SEQ ID NO:7.

The term "C3b/C4b CR-like polypeptide ortholog" refers to a polypeptide from another species that corresponds to C3b/C4b CR-like polypeptide amino acid sequence as set forth in SEQ ID NO:2, SEQ ID NO:4, or SEQ ID NO:7. For example, mouse and human C3b/C4b CR-like polypeptides are considered orthologs of each other.

The term "C3b/C4b CR-like polypeptide splice variant" refers to a nucleic acid molecule, usually RNA, which is generated by alternative processing of intron sequences in an RNA transcript of C3b/C4b CR-like polypeptide amino acid sequence as set forth in SEQ ID NO:2, SEQ ID NO:4, or SEQ ID NO:7.

The term "C3b/C4b CR-like polypeptide variants" refers to C3b/C4b CR-like polypeptides comprising amino acid sequences having one or more amino acid sequence substitutions, deletions (such as internal deletions and/or C3b/C4b CR-like polypeptide fragments), and/or additions (such as internal additions and/or C3b/C4b CR-like fusion polypeptides) as compared to the C3b/C4b CR-like polypeptide amino acid sequence set forth in SEQ ID NO:2, SEQ ID NO:4, or SEQ ID NO:7 (with or without a leader sequence). Variants may be naturally occurring (e.g., C3b/C4b CR-like polypeptide allelic variants, C3b/C4b CR-like polypeptide orthologs and C3b/C4b CR-like polypeptide splice variants) or artificially constructed. Such C3b/C4b CR-like polypeptide variants may be prepared from the corresponding nucleic acid molecules having a DNA sequence that varies accordingly from the DNA sequence as set forth in SEQ ID NO:1, SEQ ID NO:3, or SEQ ID NO:6. In preferred embodiments, the variants have from 1 to 3, or from 1 to 5, or from 1 to 10, or from 1 to 15, or from 1 to 20, or from 1 to 25, or from 1 to 50, or from 1 to 75, or from 1 to 100, or more than 100 amino acid substitutions, insertions, additions and/or deletions, wherein the substitutions may be conservative, or non-conservative, or any combination thereof.

The term "antigen" refers to a molecule or a portion of a molecule capable of being bound by a selective binding agent, such as an antibody, and additionally capable of being used in an animal to produce antibodies capable of binding to an epitope of that antigen. An antigen may have one or more epitopes.

The term "biologically active C3b/C4b CR-like polypeptides" refers to C3b/C4b CR-like polypeptides having at least one activity characteristic of the polypeptide comprising the amino acid sequence of SEQ ID NO:2, SEQ ID NO: 4, or SEQ ID NO:7.

The terms "effective amount" and "therapeutically effective amount" each refer to the amount of a C3b/C4b CR-like polypeptide or C3b/C4b CR-like nucleic acid molecule used to support an observable level of one or more biological activities of the C3b/C4b CR-like polypeptides as set forth herein.

The term "expression vector" refers to a vector which is suitable for use in a host cell and contains nucleic acid sequences which direct and/or control the expression of heterologous nucleic acid sequences. Expression includes, but is not limited to, processes such as transcription, translation, and RNA splicing, if introns are present.

The term "host cell" is used to refer to a cell which has been transformed, or is capable of being transformed with a nucleic acid sequence and then of expressing a selected gene of interest. The term includes the progeny of the parent cell, whether or not the progeny is identical in morphology or in genetic make-up to the original parent, so long as the selected gene is present.

The term "identity" as known in the art, refers to a relationship between the sequences of two or more polypeptide molecules or two or more nucleic acid molecules, as determined by comparing the sequences. In the art, "identity" also means the degree of sequence relatedness between nucleic acid molecules or polypeptides, as the case may be, as determined by the match between strings of two or more nucleotide or two or more amino acid sequences. "Identity" measures the percent of identical matches between the smaller of two or more sequences with gap alignments (if any) addressed by a particular mathematical model or computer program (i.e., "algorithms").

The term "similarity" is a related concept, but in contrast to "identity", refers to a measure of similarity which includes both identical matches and conservative substitution matches. If two polypeptide sequences have, for example, 10/20 identical amino acids, and the remainder are all non-conservative substitutions, then the percent identity and similarity would both be 50%. If in the same example, there are 5 more positions where there are conservative substitutions, then the percent identity remains 50%, but the per cent similarity would be 75% (15/20). Therefore, in cases where there are conservative substitutions, the degree of similarity between two polypeptides will be higher than the percent identity between those two polypeptides.

The term "isolated nucleic acid molecule" refers to a nucleic acid molecule of the invention that (1) has been separated from at least about 50 percent of proteins, lipids, carbohydrates or other materials with which it is naturally found when total DNA is isolated from the source cells, (2) is not linked to all or a portion of a polynucleotide to which the "isolated nucleic acid molecule" is linked in nature, (3) is operably linked to a polynucleotide which it is not linked to in nature, or (4) does not occur in nature as part of a larger polynucleotide sequence. Preferably, the isolated nucleic acid molecule of the present invention is substantially free from any other contaminating nucleic acid molecule(s) or other contaminants that are found in its natural environment that would interfere with its use in polypeptide production or its therapeutic, diagnostic, prophylactic or research use.

The term "isolated polypeptide" refers to a polypeptide of the present invention that (1) has been separated from at least about 50 percent of polynucleotides, lipids, carbohydrates or other materials with which it is naturally found when isolated from the source cell, (2) is not linked (by covalent or noncovalent interaction) to all or a portion of a polypeptide to which the "isolated polypeptide" is linked in nature, (3) is operably linked (by covalent or noncovalent interaction) to a polypeptide with which it is not linked in nature, or (4) does not occur in nature. Preferably, the isolated polypeptide is substantially free from any other contaminating polypeptides or other contaminants that are found in its natural environment that would interfere with its therapeutic, diagnostic, prophylactic or research use.

The term "mature C3b/C4b CR-like polypeptide" refers to a C3b/C4b CR-like polypeptide lacking a leader sequence. A mature C3b/C4b CR-like polypeptide may also include other modifications such as proteolytic processing of the amino terminus (with or without a leader sequence) and/or the carboxy terminus, cleavage of a smaller polypeptide from a larger precursor, N-linked and/or O-linked glycosylation, and the like.

The term "nucleic acid sequence" or "nucleic acid molecule" refers to a DNA or RNA sequence. The term encompasses molecules formed from any of the known base analogs of DNA and RNA such as, but not limited to 4-acetylcytosine, 8-hydroxy-N6-methyladenosine, aziridinyl-cytosine, pseudoisocytosine, 5-(carboxyhydroxylmethyl) uracil, 5-fluorouracil, 5-bromouracil, 5-carboxymethylaminomethyl-2-thiouracil, 5-carboxy-methylaminomethyluracil, dihydrouracil, inosine, N6-iso-pentenyladenine, 1-methyladenine, 1-methylpseudouracil, 1-methylguanine, 1-methylinosine, 2,2-dimethyl-guanine, 2-methyladenine, 2-methylguanine, 3-methylcytosine, 5-methylcytosine, N6-methyladenine, 7-methylguanine, 5-methylaminomethyluracil, 5-methoxyamino-methyl-2-thiouracil, beta-D-mannosylqueosine, 5' -methoxycarbonyl-methyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "naturally occurring" or "native" when used in connection with biological materials such as nucleic acid molecules, polypeptides, host cells, and the like, refers to materials which are found in nature and are mot manipulated by man. Similarly, "non-naturally occurring" or "non-native" as used herein refers to a material that is not found in nature or that has been structurally modified or synthesized by man.

The term "operably linked" is used herein to refer to an arrangement of flanking sequences wherein the flanking sequences so described are configured or assembled so as to perform their usual function. Thus, a flanking sequence operably linked to a coding sequence may be capable of effecting the replication, transcription and/or translation of the coding sequence. For example, a coding sequence is operably linked to a promoter when the promoter is capable of directing transcription of that coding sequence. A flanking sequence need not be contiguous with the coding sequence, so long as it functions correctly. Thus, for example, intervening untranslated yet transcribed sequences can be present between a promoter sequence and the coding sequence and the promoter sequence can still be considered "operably linked" to the coding sequence.

The term "pharmaceutically acceptable carrier" or "physiologically acceptable carrier" as used herein refers to one or more formulation materials suitable for accomplishing or enhancing the delivery of the C3b/C4b CR-like polypeptide, C3b/C4b CR-like nucleic acid molecule or C3b/C4b CR-like selective binding agent as a pharmaceutical composition.

The term "selective binding agent" refers to a molecule or molecules having specificity for a C3B/C4B CR-like polypeptide. As used herein, the terms, "specific" and "specificity" refer to the ability of the selective binding agents to bind to human C3b/C4b CR-like polypeptides and not to bind to human non-C3b/C4b CR-like polypeptides. It will be appreciated, however, that the selective binding agents may also bind orthologs of the polypeptide as set forth in SEQ ID NO:2, SEQ ID NO:4, or SEQ ID NO:7, that is, interspecies versions thereof, such as mouse and rat polypeptides.

The term "transduction" is used to refer to the transfer of genes from one bacterium to another, usually by a phage. "Transduction" also refers to the acquisition and transfer of eukaryotic cellular sequences by retroviruses.

The term "transfection" is used to refer to the uptake of foreign or exogenous DNA by a cell, and, a cell has been "transfected" when the exogenous DNA has been introduced inside the cell membrane. A number of transfection techniques are well known in the art and are disclosed herein. See, for example, Graham et al., Virology, 52:456 (1973); Sambrook et al., Molecular Cloning, a laboratory Manual, Cold Spring Harbor Laboratories (New York, 1989); Davis et al., Basic Methods in Molecular Biology, Elsevier, 1986; and Chu et al., Gene, 13:197 (1981). Such techniques can be used to introduce one or more exogenous DNA moieties into suitable host cells.

The term "transformation" as used herein refers to a change in a cell's genetic characteristics, and a cell has been transformed when it has been modified to contain a new DNA. For example, a cell is transformed where it is genetically modified from its native state. Following transfection or transduction, the transforming DNA may recombine with that of the cell by physically integrating into a chromosome of the cell, may be maintained transiently as an episomal element without being replicated, or may replicate independently as a plasmid. A cell is considered to have been stably transformed when the DNA is replicated with the division of the cell.

The term "vector" is used to refer to any molecule (e.g., nucleic acid, plasmid, or virus) used to transfer coding information to a host cell.

Relatedness of Nucleic Acid Molecules and/or Polypeptides

It is understood that related nucleic acid molecules include allelic or splice variants of the nucleic acid molecule of SEQ ID NO:1, SEQ ID NO:3, or SEQ ID NO:6, and include sequences which are complementary to any of the above nucleotide sequences. Related nucleic acid molecules also include a nucleotide sequence encoding a polypeptide comprising or consisting essentially of a substitution, modification, addition and/or a deletion of one or more amino acid residues compared to the polypeptide in SEQ ID NO:2, SEQ ID NO:4, or SEQ ID NO:7.

Fragments include molecules which encode a polypeptide of at least about 25 amino acid residues, or about 50, or about 75, or about 100, or greater than about 100 amino acid residues of the polypeptide of SEQ ID NO:2, SEQ ID NO:4, or SEQ ID NO:7.

In addition, related C3b/C4b CR-like nucleic acid molecules include those molecules which comprise nucleotide sequences which hybridize under moderately or highly stringent conditions as defined herein with the fully complementary sequence of the nucleic acid molecule of SEQ ID NO:1, SEQ ID NO:3, or SEQ ID NO:6, or of a molecule encoding a polypeptide, which polypeptide comprises the amino acid sequence as shown in SEQ ID NO:2, SEQ ID NO:4, or SEQ ID NO:7, or of a nucleic acid fragment as defined herein, or of a nucleic acid fragment encoding a polypeptide as defined herein. Hybridization probes may be prepared using the C3b/C4b CR-like sequences provided herein to screen cDNA, genomic or synthetic DNA libraries for related sequences. Regions of the DNA and/or amino acid sequence of C3b/C4b CR-like polypeptide that exhibit significant identity to known sequences are readily determined using sequence alignment algorithms as described herein and those regions may be used to design probes for screening.

The term "highly stringent conditions" refers to those conditions that are designed to permit hybridization of DNA strands whose sequences are highly complementary, and to exclude hybridization of significantly mismatched DNAs. Hybridization stringency is principally determined by temperature, ionic strength, and the concentration of denaturing agents such as formamide. Examples of "highly stringent conditions" for hybridization and washing are 0.015M sodium chloride, 0.0015M sodium citrate at 65-68°C or 0.015M sodium chloride, 0.0015M sodium citrate, and 50% formamide at 42°C. See Sambrook, Fritsch & Maniatis, Molecular Cloning: A Laboratory Manual, 2nd Ed., Cold Spring Harbor Laboratory, (Cold Spring Harbor, N.Y. 1989); Anderson et al., Nucleic Acid Hybridisation: a practical approach, Ch. 4, IRL Press Limited (Oxford, England).

More stringent conditions (such as higher temperature, lower ionic strength, higher formamide, or other denaturing agent) may also be used, however, the rate of hybridization will be affected. Other agents may be included in the hybridization and washing buffers for the purpose of reducing non-specific and/or background hybridization. Examples are 0.1% bovine serum albumin, 0.1% polyvinyl-pyrrolidone, 0.1% sodium pyrophosphate, 0.1% sodium dodecylsulfate ($NaDodSO_4$ or SDS), ficoll, Denhardt's solution, sonicated salmon sperm DNA (or other non-complementary DNA), and dextran sulfate, although other suitable agents can also be used. The concentration and types of these additives can be changed without substantially affecting the stringency of the hybridization conditions. Hybridization experiments are usually carried out at pH 6.8-7.4, however, at typical ionic strength conditions, the rate of hybridization is nearly independent of pH. See Anderson et al., Nucleic Acid Hybridisation: a Practical Approach, Ch. 4, IRL Press Limited (Oxford, England).

Factors affecting the stability of a DNA duplex include base composition, length, and degree of base pair mismatch. Hybridization conditions can be adjusted by one skilled in the art in order to accommodate these variables and allow DNAs of different sequence relatedness to form hybrids. The melting temperature of a perfectly matched DNA duplex can be estimated by the following equation:

$$T_m(^\circ C) = 81.5 + 16.6(\log[Na+]) + 0.41(\%G+C) - 600/N - 0.72(\%formamide)$$

where N is the length of the duplex formed, [Na+] is the molar concentration of the sodium ion in the hybridization or washing solution, %G+C is the percentage of (guanine+cytosine) bases in the hybrid. For imperfectly matched hybrids, the melting temperature is reduced by approximately 1°C for each 1% mismatch.

The term "moderately stringent conditions" refers to conditions under which a DNA duplex with a greater degree of base pair mismatching than could occur under "highly stringent conditions" is able to form. Examples of typical "moderately stringent conditions" are 0.015M sodium chloride, 0.0015M sodium citrate at 50-65°C or 0.015M sodium chloride, 0.0015M sodium citrate, and 20% formamide at 37-50°C. By way of example, a "moderately stringent" condition of 50°C in 0.015 M sodium ion will allow about a 21% mismatch.

It will be appreciated by those skilled in the art that there is no absolute distinction between "highly" and "moderately" stringent conditions. For example, at 0.01.5M sodium ion (no formamide), the melting temperature of perfectly matched long DNA is about 71°C. With a wash at 65°C (at the same ionic strength), this would allow for approximately a 6% mismatch. To capture more distantly related sequences, one skilled in the art can simply lower the temperature or raise the ionic strength.

A good estimate of the melting temperature in 1M NaCl* for oligonucleotide probes up to about 20nt is given by:

$$Tm = 2^\circ C \text{ per A-T base pair} + 4^\circ C \text{ per G-C base pair}$$

*The sodium ion concentration in 6X salt sodium citrate (SSC) is 1M. See Suggs et al., Developmental Biology Using Purified Genes, p. 683, Brown and Fox (eds.) (1981).

High stringency washing conditions for oligonucleotides are usually at a temperature of 0-5°C below the Tm of the oligonucleotide in 6X SSC, 0.1% SDS.

In another embodiment, related nucleic acid molecules comprise or consist of a nucleotide sequence that is about 70 percent identical to the nucleotide sequence as shown in SEQ ID NO:1, SEQ ID NO:3, or SEQ ID NO:6, or comprise or consist essentially of a nucleotide sequence encoding a polypeptide that is about 70 percent identical to the polypeptide as set forth in SEQ ID NO:2, SEQ ID NO:4, or SEQ ID NO:7. In preferred embodiments, the nucleotide sequences are about 75 percent, or about 80 percent, or about 85 percent, or about 90 percent, or about 95, 96, 97, 98, or 99 percent identical to the nucleotide sequence as shown in SEQ ID NO:1, SEQ ID NO:3, or SEQ ID NO:6, or the nucleotide sequences encode a polypeptide that is about 75 percent, or about 80 percent, or about 85 percent, or about 90 percent, or about 95, 96, 97, 98, or 99 percent identical to the polypeptide sequence as set forth in SEQ ID NO:2, SEQ ID NO:4, or SEQ ID NO:7.

Differences in the nucleic acid sequence may result in conservative and/or non-conservative modifications of the amino acid sequence relative to the amino acid sequence of SEQ ID NO:2, SEQ ID NO:4, or SEQ ID NO:7.

Conservative modifications to the amino acid sequence of SEQ ID NO:2, SEQ ID NO:4, or SEQ ID NO:7 (and the corresponding modifications to the encoding nucleotides) will produce C3b/C4b CR-like polypeptides having functional and chemical characteristics similar to those of naturally occurring C3b/C4b CR-like polypeptide. In contrast, substantial modifications in the functional and/or chemical characteristics of C3b/C4b CR-like polypeptides may be accomplished by selecting substitutions in the amino acid sequence of SEQ ID NO:2, SEQ ID NO:4, or SEQ ID NO:7 that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain.

For example, a "conservative amino acid substitution" may involve a substitution of a native amino acid residue with a nonnative residue such that there is little or no effect on the polarity or charge of the amino acid residue at that position. Furthermore, any native residue in the polypeptide may also be substituted with alanine, as has been previously described for "alanine scanning mutagenesis."

Conservative amino acid substitutions also encompass non-naturally occurring amino acid residues which are typically incorporated by chemical peptide synthesis rather than by synthesis in biological systems. These include peptidomimetics, and other reversed or inverted forms of amino acid moieties.

Naturally occurring residues may be divided into classes based on common side chain properties:

1) hydrophobic: norleucine, Met, Ala, Val, Leu, Ile;
2) neutral hydrophilic: Cys, Ser, Thr, Asn, Gln;
3) acidic: Asp, Glu;
4) basic: His, Lys, Arg;
5) residues that influence chain orientation: Gly, Pro; and
6) aromatic: Trp, Tyr, Phe.

For example, non-conservative substitutions may involve the exchange of a member of one of these classes for a member from another class. Such substituted residues may be introduced into regions of the human C3b/C4b CR-like polypeptide that are homologous with non-human C3b/C4b CR-like polypeptide orthologs, or into the non-homologous regions of the molecule.

In making such changes, the hydropathic index of amino acids may be considered. Each amino acid has been assigned a hydropathic index on the basis of their hydrophobicity and charge characteristics, these are: isoleucine (+4.5); valine (+4.2); leucine (+3.8); phenylalanine (+2.8) ; cysteine/cystine (+2.5) ; methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art. Kyte et al., J. Mol. Biol., 157:105-131 (1982). It is known that certain amino acids may be substituted for other amino acids having a similar hydropathic index or score and still retain a similar biological activity. In making changes based upon the hydropathic index, the substitution of amino acids whose hydropathic indices are within $\pm 2$ is preferred, those which are within $\pm 1$ are particularly preferred, and those within $\pm 0.5$ are even more particularly preferred.

It is also understood in the art that the substitution of like amino acids can be made effectively on the basis of hydrophilicity, particularly where the biologically functionally equivalent protein or peptide thereby created is intended for use in immunological embodiments, as in the present case. The greatest local average hydrophilicity of a protein, as governed by the hydrophilicity of its adjacent amino acids, correlates with its immunogenicity and antigenicity, i.e., with a biological property of the protein.

The following hydrophilicity values have been assigned to amino acid residues: arginine (+3.0); lysine (+3.0); aspartate (+3.0 $\pm$ 1); glutamate (+3.0 $\pm$ 1); serine (+0.3); asparagine (+0.2); glutamine (+0.2); glycine (0); threonine (-0.4); proline (-0.5 $\pm$ 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, the substitution of amino acids whose hydrophilicity values are within $\pm 2$ is preferred, those which are within $\pm 1$ are particularly preferred, and those within $\pm 0.5$ are even more particularly preferred. One may also identify epitopes from primary amino acid sequences on the basis of hydrophilicity. These regions are also referred to as "epitopic core regions."

Desired amino acid substitutions (whether conservative or non-conservative) can be determined by those skilled in the art at the time such substitutions are desired. For example, amino acid substitutions can be used to identify important residues of the C3b/C4b CR-like polypeptide, or to increase or decrease the affinity of the C3b/C4b CR-like polypeptides described herein.

Exemplary amino acid substitutions are set forth in Table I.

Table I

| Amino Acid Substitutions | | |
|---|---|---|
| Original Residues | Exemplary Substitutions | Preferred Substitutions |
| Ala | Val, Leu, Ile | Val |
| Arg | Lys, Gln, Asn | Lys |
| Asn | Gln | Gln |
| Asp | Glu | Glu |
| Cys | Ser, Ala | Ser |
| Gln | Asn | Asn |
| Glu | Asp | Asp |
| Gly | Pro, Ala | Ala |

(continued)

| Amino Acid Substitutions | | |
|---|---|---|
| Original Residues | Exemplary Substitutions | Preferred Substitutions |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric Acid, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

A skilled artisan will be able to determine suitable variants of the polypeptide as set forth in SEQ ID NO:2, SEQ ID NO:4, or SEQ ID NO:7 using well known techniques. For identifying suitable areas of the molecule that may be changed without destroying activity, one skilled in the art may target areas not believed to be important for activity. For example, when similar polypeptides with similar activities from the same species or from other species are known, one skilled in the art may compare the amino acid sequence of a C3b/C4b CR-like polypeptide to such similar polypeptides. With such a comparison, one can identify residues and portions of the molecules that are conserved among similar polypeptides. It will be appreciated that changes in areas of a C3b/C4b CR-like polypeptide that are not conserved relative to such similar polypeptides would be less likely to adversely affect the biological activity and/or structure of the C3b/C4b CR-like polypeptide. One skilled in the art would also know that, even in relatively conserved regions, one may substitute chemically similar amino acids for the naturally occurring residues while retaining activity (conservative amino acid residue substitutions). Therefore, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in a C3b/C4b CR-like polypeptide that correspond to amino acid residues that are important for activity or structure in similar polypeptides. One skilled in the art may opt for chemically similar amino acid substitutions for such predicted important amino acid residues of C3b/C4b CR-like polypeptides.

One skilled in the art can also analyze the three-dimensional structure and amino acid sequence in relation to that structure in similar polypeptides. In view of that information, one skilled in the art may predict the alignment of amino acid residues of a C3b/C4b CR-like polypeptide with respect to its three dimensional structure. One skilled in the art may choose not to make radical changes to amino acid residues predicted to be on the surface of the protein, since such residues may be involved in important interactions with other molecules. Moreover, one skilled in the art may generate test variants containing a single amino acid substitution at each desired amino acid residue. The variants can then be screened using activity assays know to those skilled in the art. Such variants could be used to gather information about suitable variants. For example, if one discovered that a change to a particular amino acid residue resulted in destroyed, undesirably reduced, or unsuitable activity, variants with such a change would be avoided. In other words, based on information gathered from such routine experiments, one skilled in the art can readily determine the amino acids where further substitutions should be avoided either alone or in combination with other mutations.

A number of scientific publications have been devoted to the prediction of secondary structure . See Moult J., Curr. Op. in Biotech., 7(4):422-427 (1996), Chou et al., Biochemistry, 13(2):222-245 (1974); Chou et al., Biochemistry, 113 (2):211-222 (1974); Chou et al., Adv. Enzymol. Relat. Areas Mol. Biol., 47:45-148 (1978); Chou et al., Ann. Rev. Biochem., 47:251-276 and Chou et al., Biophys. J., 26:367-384 (1979). Moreover, computer programs are currently available to assist with predicting secondary structure. One method of predicting secondary structure is based upon homology modeling. For example, two polypeptides or proteins which have a sequence identity of greater than 30%, or similarity greater than 40% often have similar structural topologies. The recent growth of the protein structural data base (PDB) has provided enhanced predictability of secondary structure, including the potential number of folds within a polypeptide's or protein's structure. *See* Holm et al., Nucl. Acid. Res., 27(1):244-247 (1999). It has been suggested (Brenner et al., Curr. Op. Struct. Biol., 7(3):369-376 (1997)) that there are a limited number of folds in a given polypeptide or protein and that once a critical number of structures have been resolved, structural prediction will gain dramatically in accuracy.

Additional methods of predicting secondary structure include "threading" (Jones, D., Curr. Opin. Struct. Biol., 7(3):377-87 (1997); Sippl et al., Structure, 4(1):15-9 (1996)), "profile analysis" (Bowie et al., Science, 253:164-170 (1991); Gribskov et al., Meth. Enzym., 183:146-159 (1990); Gribskov et al., Proc. Nat. Acad. Sci., 84(13):4355-4358 (1987)), and "evolutionary linkage" (See Home, *supra*, and Brenner, *supra*).

Preferred C3b/C4b CR-like polypeptide variants include glycosylation variants wherein the number and/or type of glycosylation sites has been altered compared to the amino acid sequence set forth in SEQ ID NO:2, SEQ ID NO:4, or SEQ ID NO:7. In one embodiment, C3b/C4b CR-like polypeptide variants comprise a greater or a lesser number of N-linked glycosylation sites than the amino acid sequence set forth in SEQ ID NO:2, SEQ ID NO:4, or SEQ ID NO:7. An N-linked glycosylation site is characterized by the sequence: Asn-X-Ser or Asn-X-Thr, wherein the amino acid residue designated as X may be any amino acid residue except proline. The substitution(s) of amino acid residues to create this sequence provides a potential new site for the addition of an N-linked carbohydrate chain. Alternatively, substitutions which eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N-linked sites are created. Additional preferred C3b/C4b CR-like variants include cysteine variants, wherein one or more cysteine residues are deleted from or substituted for another amino acid (e.g., serine) as compared to the amino acid sequence set forth in SEQ ID NO:2, SEQ ID NO:4, or SEQ ID NO:7. Cysteine variants are useful when C3b/C4b CR-like polypeptides must be refolded into a biologically active conformation such as after the isolation of insoluble inclusion bodies. Cysteine variants generally have fewer cysteine residues than the native protein, and typically have an even number to minimize interactions resulting from unpaired cysteines.

In addition, the polypeptide comprising the amino acid sequence of SEQ ID NO:2, SEQ ID NO:4, or SEQ ID NC:7 or a C3b/C4b CR-like polypeptide variant may be fused to a homologous polypeptide to form a homodimer or to a heterologous polypeptide to form a heterodimer. Heterologous peptides and polypeptides include, but are not limited to: an epitope to allow for the detection and/or isolation of a C3b/C4b CR-like fusion polypeptide; a transmembrane receptor protein or a portion thereof, such as an extracellular domain, or a transmembrane and intracellular domain; a ligand or a portion thereof which binds to a transmembrane receptor protein; an enzyme or portion thereof which is catalytically active; a polypeptide or peptide which promotes oligomerization, such as a leucine zipper domain; a polypeptide or peptide which increases stability, such as an immunoglobulin constant region; and a polypeptide which has a therapeutic activity different from the polypeptide comprising the amino acid sequence as set forth in SEQ ID NO:2, SEQ ID NO:4, or SEQ ID NO:7, or a C3b/C4b CR-like polypeptide variant.

Fusions can be made either at the amino terminus or at the carboxy terminus of the polypeptide comprising the amino acid sequence set forth in SEQ ID NO:2, SEQ ID NO:4, or SEQ ID NO:7, or a C3b/C4b CR-like polypeptide variant. Fusions may be direct with no linker or adapter molecule or indirect using a linker or adapter molecule. A linker or adapter molecule may be one or more amino acid residues, typically up to about 20 to about 50 amino acid residues. A linker or adapter molecule may also be designed with a cleavage site for a DNA restriction endonuclease or for a protease to allow for the separation of the fused moieties. It will be appreciated that once constructed, the fusion polypeptides can be derivatized according to the methods described herein.

In a further embodiment of the invention, the polypeptide comprising the amino acid sequence of SEQ ID NO: 2, SEQ ID NO:4, or SEQ ID NO:7, or a C3b/C4b CR-like polypeptide variant is fused to one or more domains of an Fc region of human IgG. Antibodies comprise two functionally independent parts, a variable domain known as "Fab", which binds antigen, and a constant domain known as "Fc", which is involved in effector functions such as complement activation and attack by phagocytic cells. An Fc has a long serum half-life, whereas an Fab is short-lived. Capon et al., Nature, 337:525-31 (1989). When constructed together with a therapeutic protein, an Fc domain can provide longer half-life or incorporate such functions as Fc receptor binding, protein A binding, complement fixation and perhaps even placental transfer. *Id*. Table II summarizes the use of certain Fc fusions known in the art.

TABLE II: FC FUSION WITH THERAPEUTIC PROTEINS

| Form of Fc | Fusion partner | Therapeutic implications | Reference |
|---|---|---|---|
| IgG1 | N-terminus of CD30-L | Hodgkin's disease; anaplastic lymphoma; T-cell leukemia | U.S. Patent No. 5,480,981 |

(continued)

| Form of Fc | Fusion partner | Therapeutic implications | Reference |
|---|---|---|---|
| Murine Fcγ2a | IL-10 | anti-inflammatory; transplant rejection | Zheng et al. (1995), J. Immunol., 154: 5590-5600 |
| IgG1 | TNF receptor | septic shock | Fisher et al. (1996), N. Engl. J. Med., 334: 1697-1702; Van Zee et al., (1996), J. Immunol., 156: 2221-2230 |
| IgG, IgA, IgM, or IgE (excluding the first domain) | TNF receptor | inflammation, autoimmune disorders | U.S. Pat. No. 5,808,029, issuec September 15, 1998 |
| IgG1 | CD4 receptor | AIDS | Capon et al. (1989), Nature 337: 525-531 |
| IgG1, IgG3 | N-terminus of IL-2 | anti-cancer, antiviral | Harvill et al. (1995), Immunotech., 1: 95-105 |
| IgG1 | C-terminus of OPG | osteoarthritis; bone density | WO 97/23614, published July 3, 1997 |
| IgG1 | N-terminus of leptin | anti-obesity | PCT/US 97/23183, filed December 11, 1997 |
| Human Ig Cγ1 | CTLA-4 | autoimmune disorders | Linsley (1991), J. Exp. Med., 174:561-569 |

In one example, all or a portion of the human IgG hinge, CH2 and CH3 regions may be fused at either the N-terminus or C-terminus of the C3b/C4b CR-like polypeptides using methods known to the skilled artisan. The resulting C3b/C4b CR-like fusion polypeptide may be purified by use of a Protein A affinity column. Peptides and proteins fused to an Fc region have been found to exhibit a substantially greater half-life in vivo than the unfused counterpart. Also, a fusion to an Fc region allows for dimerization/multimerization of the fusion polypeptide. The Fc region may be a naturally occurring Fc region, or may be altered to improve certain qualities, such as therapeutic qualities, circulation time, reduce aggregation, etc.

Identity and similarity of related nucleic acid molecules and polypeptides can be readily calculated by known methods. Such methods include, but are not limited to, those described in Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math., 48:1073 (1988).

Preferred methods to determine identity and/or similarity are designed to give the largest match between the sequences tested. Methods to determine identity and similarity are described in publicly available computer programs. Preferred computer program methods to determine identity and similarity between two sequences include, but are not limited to, the GCG program package, including GAP (Devereux et al., Nucl. Acid. Res., 12:387 (1984); Genetics Computer Group, University of Wisconsin, Madison, WI), BLASTP, BLASTN, and FASTA (Altschul et al., J. Mol. Biol., 215: 403-410 (1990)). The BLASTX program is publicly available from the National Center for Biotechnology Information (NCBI) and other sources (BLAST Manual, Altschul et al. NCB/NLM/NIH Bethesda, MD 20894; Altschul et *al.,* supra). The well known Smith Waterman algorithm may also be used to determine identity.

Certain alignment schemes for aligning two amino acid sequences may result in the matching of only a short region of the two sequences, and this small aligned region may have very high sequence identity even though there is no significant relationship between the two full length sequences. Accordingly, in a preferred embodiment, the selected alignment method (GAP program) will result in an alignment that spans at least 50 contiguous amino acids of the target polypeptide.

For example, using the computer algorithm GAP (Genetics Computer Group, University of Wisconsin, Madison, WI), two polypeptides for which the percent sequence identity is to be determined are aligned for optimal matching of their respective amino acids (the "matched span", as determined by the algorithm). A gap opening penalty (which is calculated as 3X the average diagonal; the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. A standard comparison matrix (see Dayhoff et al., Atlas of Protein Sequence and Structure, vol. 5, supp.3 (1978) for the PAM 250 comparison matrix; Henikoff et al., Proc. Natl. Acad. Sci USA, 89:10915-10919 (1992) for the BLOSUM 62 comparison matrix) is also used by the algorithm.

Preferred parameters for a polypeptide sequence comparison include the following:

Algorithm: Needleman et al., J. Mol. Biol., 48:443-453 (1970);

Comparison matrix: BLOSUM 62 from Henikoff et al., Proc. Natl. Acad. Sci. USA, 89:10915-10919 (1992);

Gap Penalty: 12

Gap Length Penalty: 4

Threshold of Similarity: 0

The GAP program is useful with the above parameters. The aforementioned parameters are the default parameters for polypeptide comparisons (along with no penalty for end gaps) using the GAP algorithm.

Preferred parameters for nucleic acid molecule sequence comparisons include the following:

Algorithm: Needleman et al., J. Mol Biol., 48:443-453 (1970);

Comparison matrix: matches = +10, mismatch = 0

Gap Penalty: 50

Gap Length Penalty: 3

The GAP program is also useful with the above parameters. The aforementioned parameters are the default parameters for nucleic acid molecule comparisons.

Other exemplary algorithms, gap opening penalties, gap extension penalties, comparison matrices, thresholds of similarity, etc. may be used, including those set forth in the Program Manual, Wisconsin Package, Version 9, September, 1997. The particular choices to be made will be apparent to those of skill in the art and will depend on the specific comparison to be made, such as DNA to DNA, protein to protein, protein to DNA; and additionally, whether the comparison is between given pairs of sequences (in which case GAP or BestFit are generally preferred) or between one sequence and a large database of sequences (in which case FASTA or BLASTA are preferred).

<u>Synthesis</u>

It will be appreciated by those skilled in the art the nucleic acid and polypeptide molecules described herein may be produced by recombinant and other means.

<u>Nucleic Acid Molecules</u>

The nucleic acid molecules encode a polypeptide comprising the amino acid sequence of a C3b/C4b CR-like polypeptide can readily be obtained in a variety of ways including, without limitation, chemical synthesis, cDNA or genomic library screening, expression library screening and/or PCR amplification of cDNA.

Recombinant DNA methods used herein are generally those set forth in Sambrook et al., Molecular Cloning: A Laboratory Manual, Cold Spring Harbor Laboratory Press, Cold Spring Harbor, NY (1989), and/or Ausubel et al., eds., Current Protocols in Molecular Biology, Green Publishers Inc. and Wiley and Sons, NY (1994). The present invention provides for nucleic acid molecules as described herein and methods for obtaining the molecules.

Where a gene encoding the amino acid sequence of a C3b/C4b CR-like polypeptide has been identified from one species, all or a portion of that gene may be used as a probe to identify orthologs or related genes from the same species. The probes or primers may be used to screen cDNA libraries from various tissue sources believed to express the C3b/C4b CR-like polypeptide. In addition, part or all of a nucleic acid molecule having the sequence as set forth in SEQ ID NO:1, SEQ ID NO:3, or SEQ ID NO:6 may be used to screen a genomic library to identify and isolate a gene encoding the amino acid sequence of a C3b/C4b CR-like polypeptide. Typically, conditions of moderate or high stringency will be employed for screening to minimize the number of false positives obtained from the screen.

Nucleic acid molecules encoding the amino acid sequence of C3b/C4b CR-like polypeptides may also be identified by expression cloning which employs the detection of positive clones based upon a property of the expressed protein. Typically, nucleic acid libraries are screened by the binding of an antibody or other binding partner (e.g., receptor or ligand) to cloned proteins which are expressed and displayed on a host cell surface. The antibody or binding partner is modified with a detectable label to identify those cells expressing the desired clone.

Recombinant expression techniques conducted in accordance with the descriptions set forth below may be followed to produce these polynucleotides and to express the encoded polypeptides. For example, by inserting a nucleic acid sequence which encodes the amino acid sequence of a C3b/C4b CR-like polypeptide into an appropriate vector, one skilled in the art can readily produce large quantities of the desired nucleotide sequence. The sequences can then be used to generate detection probes or amplification primers. Alternatively, a polynucleotide encoding the amino acid sequence of a C3b/C4b CR-like polypeptide can be inserted into an expression vector. By introducing the expression vector into an appropriate host, the encoded C3b/C4b CR-like polypeptide may be produced in large amounts.

Another method for obtaining a suitable nucleic acid sequence is the polymerase chain reaction (PCR). In this method, cDNA is prepared from poly(A)+RNA or total RNA using the enzyme reverse transcriptase. Two primers, typically complementary to two separate regions of cDNA (oligonucleotides) encoding the amino acid sequence of a C3b/C4b CR-like polypeptide, are then added to the cDNA along with a polymerase such as Taq polymerase, and the polymerase amplifies the cDNA region between the two primers.

Another means of preparing a nucleic acid molecule encoding the amino acid sequence of a C3b/C4b CR-like polypeptide is chemical synthesis using methods well known to the skilled artisan such as those described by Engels et al., Angew. Chem. Intl. Ed., 28:716-734 (1989). These methods include, inter alia, the phosphotriester, phosphoramidite, and H-phosphonate methods for nucleic acid synthesis. A preferred method for such chemical synthesis is polymer-supported synthesis using standard phosphoramidite chemistry. Typically, the DNA encoding the amino acid sequence of a C3b/C4b CR-like polypeptide will be several hundred nucleotides in length. Nucleic acids larger than about 100 nucleotides can be synthesized as several fragments using these methods. The fragments can then be ligated together to form the full length nucleotide sequence of a C3b/C4b CR-like polypeptide. Usually, the DNA fragment encoding the amino terminus of the polypeptide will have an ATG, which.encodes a methionine residue. This methionine may or may not be present on the mature form of the C3b/C4b CR-like polypeptide, depending on whether the polypeptide produced in the host cell is designed to be secreted from that cell. Other methods known to the skilled artisan may be used as well.

In certain embodiments, nucleic acid variants contain codons which have been altered for the optimal expression of a C3b/C4b CR-like polypeptide in a given host cell. Particular codon alterations will depend upon the C3b/C4b CR-like polypeptide(s) and host cell(s) selected for expression. Such "codon optimization" can be carried out by a variety of methods, for example, by selecting codons which are preferred for use in highly expressed genes in a given host cell. Computer algorithms which incorporate codon frequency tables such as "Ecohigh.cod" for codon preference of highly expressed bacterial genes may be used and are provided by the University of Wisconsin Package Version 9.0, Genetics Computer Group, Madison, WI. Other useful codon frequency tables include "Celegans_high.cod", "Celegans_low.cod", "Drosophila_high.cod", "Human_high.cod", "Maize_high.cod", and "Yeast_high.cod".

<u>Vectors and Host Cells</u>

A nucleic acid molecule encoding the amino acid sequence of a C3b/C4b CR-like polypeptide may be inserted into an appropriate expression vector using standard ligation techniques. The vector is typically selected to be functional in the particular host cell employed (i.e., the vector is compatible with the host cell machinery such that' amplification of the gene and/or expression of the gene can occur). A nucleic acid molecule encoding the amino acid sequence of a C3b/C4b CR-like polypeptide may be amplified/expressed in prokaryotic, yeast, insect (baculovirus systems), and/or eukaryotic host cells. Selection of the host cell will depend in part on whether a C3b/C4b CR-like polypeptide is to be post-translationally modified (e.g., glycosylated and/or phosphorylated). If so, yeast, insect, or mammalian host cells are preferable. For a review of expression vectors, see Meth. Enz., v.185, D.V. Goeddel, ed. Academic Press Inc., San Diego, CA (1990).

Typically, expression vectors used in any of the host cells will contain sequences for plasmid maintenance and for cloning and expression of exogenous nucleotide sequences. Such sequences, collectively referred to as "flanking sequences" in certain embodiments will typically include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element. Each of these sequences is discussed below.

Optionally, the vector may contain a "tag"-encoding sequence, i.e., an oligonucleotide molecule located at the 5' or 3' end of the C3b/C4b CR-like polypeptide coding sequence; the oligonucleotide sequence encodes polyHis (such as hexaHis), or other "tag" such as FLAG, HA (hemaglutinin Influenza virus) or *myc* for which commercially available antibodies exist. This tag is typically fused to the polypeptide upon expression of the polypeptide, and can serve as a means for affinity purification of the C3b/C4b CR-like polypeptide from the host cell. Affinity purification can be accomplished, for example, by column chromatography using antibodies against the tag as an affinity matrix. Optionally, the tag can subsequently be removed from the purified C3b/C4b CR-like polypeptide by various means such as using certain peptidases for cleavage.

Flanking sequences may be homologous (i.e., from the same species and/or strain as the host cell), heterologous (i.e., from a species other than the host cell species or strain), hybrid (i.e., a combination of flanking sequences from more than one source) or synthetic, or the flanking sequences may be native sequences which normally function to regulate C3b/C4b CR-like polypeptide expression. As such, the source of a flanking sequence may be any prokaryotic or eukaryotic organism, any vertebrate or invertebrate organism, or any plant, provided that the flanking sequence is functional in, and can be activated by, the host cell machinery.

The flanking sequences useful in the vectors of this invention may be obtained by any of several methods well known in the art. Typically, flanking sequences useful herein ether than the C3b/C4b CR-like gene flanking sequences will have been previously identified by mapping and/or by restriction endonuclease digestion and can thus be isolated from the proper tissue source using the appropriate restriction endonucleases. In some cases, the full nucleotide sequence of a flanking sequence may be known. Here, the flanking sequence may be synthesized using the methods described herein for nucleic acid synthesis or cloning.

Where all or only a portion of the flanking sequence is known, it may be obtained using PCR and/or by screening a genomic library with suitable oligonucleotide and/or flanking sequence fragments from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen® column chromatography (Chatsworth, CA), or other methods known to the skilled artisan. The' selection of suitable enzymes to accomplish this purpose will be readily apparent to one of ordinary skill in the art.

An origin of replication is typically a part of those prokaryotic expression vectors purchased commercially, and the origin aids in the amplification of the vector in a host cell. Amplification of the vector to a certain copy number can, in some cases, be important for the optimal expression of a C3b/C4b CR-like polypeptide. If the vector of choice does not contain an origin of replication site, one may be chemically synthesized based on a known sequence, and ligated into the vector. For example, the origin of replication from the plasmid pBR322 (Product No. 303-3s, New England Biolabs, Beverly, MA) is suitable for most Gram-negative bacteria and various origins (*e.g.,* SV40, polyoma, adenovirus, vesicular stomatitus virus (VSV) or papillomaviruses such as HPV or BPV) are useful for cloning vectors in mammalian cells. Generally, the origin of replication component is not needed for mammalian expression vectors (for example, the SV40 origin is often used only because it contains the early promoter).

A transcription termination sequence is typically located 3' of the end of a polypeptide coding region and serves to terminate transcription. Usually, a transcription termination sequence in prokaryotic cells is a G-C rich fragment followed by a poly T sequence. While the sequence is easily cloned from a library or even purchased commercially as part of a vector, it can also be readily synthesized using methods for nucleic acid synthesis such as those described herein.

A selectable marker gene element encodes a protein necessary for the survival and growth of a host cell grown in a selective culture medium. Typical selection marker genes encode proteins that (a) confer resistance to antibiotics or other toxins, *e.g.*, ampicillin, tetracycline, or kanamycin for prokaryotic host cells, (b) complement auxotrophic deficiencies of the cell; or (c) supply critical nutrients not available from complex media. Preferred selectable markers are the kanamycin resistance gene, the ampicillin resistance gene, and the tetracycline resistance gene. A neomycin resistance gene may also be used for selection in prokaryotic and eukaryotic host cells.

Other selection genes may be used to amplify the gene which will be expressed. Amplification is the process wherein genes which are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The mammalian cell transformants are placed under selection pressure which only the transformants are uniquely adapted to survive by virtue of the selection gene present in the vector. Selection pressure is imposed by culturing the transformed cells under conditions in which the concentration of selection agent in the medium is successively changed, thereby leading to the amplification of both the selection gene and the DNA that encodes a C3b/C4b CR-like polypeptide. As a result, increased quantities of C3b/C4b CR-like polypeptide are synthesized from the amplified DNA.

A ribosome binding site is usually necessary for translation initiation of mRNA and is characterized by a Shine-Dalgarno sequence (prokaryotes) or a Kozak sequence (eukaryotes). The element is typically located 3' to the promoter and 5' to the coding sequence of a C3b/C4b CR-like polypeptide to be expressed. The Shine-Dalgarno sequence is varied but is typically a polypurine (i.e., having a high A-G content). Many Shine-Dalgarno sequences have been identified, each of which can be readily synthesized using methods set forth herein and used in a prokaryotic vector.

A leader, or signal, sequence may be used to direct a C3b/C4b CR-like polypeptide out of the host cell. Typically, a nucleotide sequence encoding the signal sequence is positioned in the coding region of a C3b/C4b CR-like nucleic acid molecule, or directly at the 5' end of a C3b/C4b CR-like polypeptide coding region. Many signal sequences have been identified, and any of those that are functional in the selected host cell may be used in conjunction with a C3b/C4b CR-like nucleic acid molecule. Therefore, a signal sequence may be homologous (naturally occurring) or heterologous to a C3b/C4b CR-like gene or cDNA. Additionally, a signal sequence may be chemically synthesized using methods described herein. In most cases, the secretion of a C3b/C4b CR-like polypeptide from the host cell via the presence of a signal peptide will result in the removal of the signal peptide from the secreted C3b/C4b CR-like polypeptide. The signal sequence may be a component of the vector, or it may be a part of a C3b/C4b CR-like nucleic acid molecule that is inserted into the vector.

Included within the scope of this invention is the use of either a nucleotide sequence encoding a native C3b/C4b CR-like polypeptide signal sequence joined to a C3b/C4b CR-like polypeptide coding region or a nucleotide sequence encoding a heterologous signal sequence joined to a C3b/C4b CR-like polypeptide coding region. The heterologous signal sequence selected should be one that is recognized and processed, i.e., cleaved by a signal peptidase, by the host cell. For prokaryotic host cells that do not recognize and process the native C3B/C4B CR-like polypeptide signal sequence, the signal sequence is substituted by a prokaryotic signal sequence selected, for example, from the group of the alkaline phosphatase, penicillinase, or heat-stable enterotoxin II leaders. For yeast secretion, the native C3B/C4B CR-like polypeptide signal sequence may be substituted by the yeast invertase, alpha factor, or acid phosphatase leaders. In mammalian cell expression the native signal sequence is satisfactory, although other mammalian signal sequences may be suitable.

In some cases, such as where glycosylation is desired in a eukaryotic host cell expression system, one may manipulate the various presequences to improve glycosylation or yield. For example, one may alter the peptidase cleavage site of a particular signal peptide, or add presequences, which also may affect glycosylation. The final protein product may have, in the -1 position (relative to the first amino acid of the mature protein) one or more additional amino acids incident to expression, which may not have been totally removed. For example, the final protein product may have one or two amino acid residues found in the peptidase cleavage site, attached to the N-terminus. Alternatively, use of some enzyme cleavage sites may result in a slightly truncated form of the desired C3b/C4b CR-like polypeptide, if the enzyme cuts at such area within the mature polypeptide.

In many cases, transcription of a nucleic acid molecule is increased by the presence of one or more introns in the vector; this is particularly true where a polypeptide is produced in eukaryotic host cells, especially mammalian host cells. The introns used may be naturally occurring within the C3b/C4b CR-like gene, especially where the gene used is a full length genomic sequence or a fragment thereof. Where the intron is not naturally occurring within the gene (as for most cDNAs), the intron(s) may be obtained from another source. The position of the intron with respect to flanking sequences and the C3b/C4b CR-like gene is generally important, as the intron must be transcribed to be effective. Thus, when a C3b/C4b CR-like cDNA molecule is being transcribed, the preferred position for the intron is 3' to the transcription start site, and 5' to the polyA transcription termination sequence. Preferably, the intron or introns will be located on one side or the other (*i.e.*, 5', or 3') of the cDNA such that it does not interrupt the coding sequence. Any intron from any source, including any viral, prokaryotic and eukaryotic (plant or animal) organisms, may be used to practice this invention, provided that it is compatible with the host cell(s) into which it is inserted. Also included herein are synthetic introns. Optionally, more than one intron may be used in the vector.

The expression and cloning vectors of the present invention will each typically contain a promoter that is recognized by the host organism and operably linked to the molecule encoding a C3B/C4B CR-like polypeptide. Promoters are untranscribed sequences located upstream (5') to the start codon of a structural gene (generally within about 100 to 1000 bp) that control the transcription of the structural gene. Promoters are conventionally grouped into one of two classes, inducible promoters and constitutive promoters. Inducible promoters initiate increased levels of transcription from DNA under their control in response to some change in culture conditions, such as the presence or absence of a nutrient or a change in temperature. Constitutive promoters, on the other hand, initiate continual gene product production; that is, there is little or no control over gene expression. A large number of promoters, recognized by a variety of potential host cells, are well known. A suitable promoter is operably linked to the DNA encoding a C3B/C4B CR-like polypeptide by removing the promoter from the source DNA by restriction enzyme digestion and inserting the desired promoter sequence into the vector. The native C3B/C4B CR-like gene promoter sequence may be used to direct amplification and/or expression of a C3B/C4B CR-like nucleic acid molecule. A heterologous promoter is preferred, however, if it permits greater transcription and higher yields of the expressed protein as compared to the native promoter, and if it is compatible with the host cell system that has been selected for use.

Promoters suitable for use with prokaryotic hosts include the beta-lactamase and lactose promoter systems; alkaline phosphatase, a tryptophan (trp) promoter system; and hybrid promoters such as the tac promoter. Other known bacterial promoters are also suitable. Their sequences have been published, thereby enabling one skilled in the art to ligate them to the desired DNA sequence(s), using linkers or adapters as needed to supply any useful restriction sites.

Suitable promoters for use with yeast hosts are also well known in the art. Yeast enhancers are advantageously used with yeast promoters. Suitable promoters for use with mammalian host cells are well known and include, but are not limited to, those obtained from the genomes of viruses such as polyoma virus, fowlpox virus, adenovirus (such as Adenovirus 2), bovine papilloma virus, avian sarcoma virus, cytomegalovirus (CMV), a retrovirus, hepatitis-B virus and most preferably Simian Virus 40 (SV40). Other suitable mammalian promoters include heterologous mammalian promoters, e.g'., heat-shock promoters and the actin promoter.

Additional promoters which may be of interest in controlling C3B/C4B CR-lilte gene transcription include, but are not limited to: the SV40 early promoter region (Bernoist and Chambon, Nature, 290:304-310, 1981); the CMV promoter; the promoter contained in the 3' long terminal repeat of Rous sarcoma virus (Yamamoto et al., Cell, 22:

787-797, 1980); the herpes thymidine kinase promoter (Wagner et al., Proc. Natl. Acad. Sci. USA, 78:144-1445, 1981); the regulatory sequences of the metallothionine gene (Brinster et al., Nature, 296:39-42, 1982); prokaryotic expression vectors such as the beta-lactamase promoter (Villa-Kamaroff, et al., Proc. Natl. Acad. Sci. USA, 75:3727-3731, 1978); or the tac promoter (DeBoer, et al., Proc. Natl. Acad. Sci. USA, 80:21-25, 1983). Also of interest are the following animal transcriptional control regions, which exhibit tissue specificity and have been utilized in transgenic animals: the elastase I gene control region which is active in pancreatic acinar cells (Swift et al., Cell, 38:639-646, 1984; Ornitz et al., Cold Spring Harbor Symp. Quant. Biol., 50:399-409 (1986); MacDonald, Hepatology, 7:425-515, 1987); the insulin gene control region which is active in pancreatic beta cells (Hanahan, Nature, 315:115-122, 1985); the immunoglobulin gene control region which is active in lymphoid cells (Grosschedl et al., Cell, 38:647-658 (1984); Adames et al., Nature, 318: 533-538 (1985); Alexander et al., Mol. Cell. Biol., 7:1436-1444, 1987); the mouse mammary tumor virus control region which is active in testicular, breast, lymphoid and mast cells (Leder et al., Cell, 45:485-495, 1986); the albumin gene control region which is active in liver (Pinkert et al., Genes and Devel., 1:268-276, 1987); the alphafetcprotein gene control region which is active in liver (Krumlauf et al., Mol. Cell. Biol., 5:1639-1648, 1985; Hammer et al., Science, 235: 53-58, 1987); the alpha 1-antitrypsin gene control region which is active in the liver (Kelsey et al., Genes and Devel., 1: 161-171, 1987); the beta-globin gene control region which is active in myeloid cells (Mogram et al., Nature, 315:338-340, 1985; Kollias et al., Cell, 46:89-94, 1986); the myelin basic protein gene control region which is active in oligodendrocyte cells in the brain (Readhead et al., Cell, 48:703-712, 1987); the myosin light chain-2 gene control region which is active in skeletal muscle (Sani, Nature, 314:283-286, 1985); and the gonadotropic releasing hormone gene control region which is active in the hypothalamus ( Mason et al., Science, 234:1372-1378, 1986).

An enhancer sequence may be inserted into the vector to increase the transcription of a DNA encoding a C3B/C4B CR-like polypeptide of the present invention by higher eukaryotes. Enhancers are cis-acting elements of DNA, usually about 10-300 bp in length, that act on the promoter to increase transcription. Enhancers are relatively orientation and position independent. They have been found 5' and 3' to the transcription unit. Several enhancer sequences available from mammalian genes are known (e.g., globin, elastase, albumin, alpha-feto-protein and insulin). Typically, however, an enhancer from a virus will be used. The SV40 enhancer, the cytomegalovirus early promoter enhancer, the polyoma enhancer, and adenovirus enhancers are exemplary enhancing elements for the activation of eukaryotic promoters. While an enhancer may be spliced into the vector at a position 5' or 3' to a C3B/C4B CR-like nucleic acid molecule, it is typically located at a site 5' from the promoter.

Expression vectors of the invention may be constructed from a starting vector such as a commercially available vector. Such vectors may or may not contain all of the desired flanking sequences. Where one or more of the desired flanking sequences are not already present in the vector, they may be individually obtained and ligated into the vector. Methods used for obtaining each of the flanking sequences are well known to one skilled in the art.

Preferred vectors for practicing this invention are those which are compatible with bacterial, insect, and mammalian host cells. Such vectors include, inter *alia*, pCRII, pCR3, and pcDNA3.1 (Invitrogen Company, Carlsbad, CA), pBSII (Stratagene Company, La Jolla, CA), pET15□ (Novagen, Madison, WI), pGEX (Pharmacia Biotech, Piscataway, NJ), pEGFP-N2 (Clontech, Palo Alto, CA), pETL (BlueBacII; Invitrogen), pDSR-alpha (PCT Publication No. WO90/14363) and pFastBacDual (Gibco/BRL, Grand Island, NY).

Additional suitable vectors include, but are not limited to, cosmids, plasmids or modified viruses, but it will be appreciated that the vector system must be compatible with the selected host cell. Such vectors include, but are not limited to plasmids such as Bluescript® plasmid derivatives (a high copy number ColE1-based phagemid, Stratagene Cloning Systems Inc., La Jolla CA), PCR cloning plasmids designed for cloning Taq-amplified PCR products (*e.g.,* TOPO™ TA Cloning® Kit, PCR2.1® plasmid derivatives, Invitrogen, Carlsbad, CA), and mammalian, yeast, or virus vectors such as a baculovirus expression system (pBacPAK plasmid derivatives, Clontech, Palo Alto, CA).

After the vector has been constructed and a nucleic acid molecule encoding a C3b/C4b CR-like polypeptide has been inserted into the proper site of the vector, the completed vector may be inserted into a suitable host cell for amplification and/or polypeptide expression. The transformation of an expression vector for a C3b/C4b CR-like polypeptide into a selected host cell may be accomplished by well known methods including methods such as transfection, infection, calcium chloride, electroporation, microinjection, lipofection or the DEAE-dextran method or other known techniques. The method selected will in part be a function of the type of host cell to be used. These methods and other suitable methods are well known to the skilled artisan, and are set forth, for example, in Sambrook et al., *supra*.

Host cells may be prokaryotic host cells (such as *E. coli*) or eukaryotic host cells (such as a yeast cell, an insect cell or a vertebrate cell). The host cell, when cultured under appropriate conditions, synthesizes a C3b/C4b CR-like polypeptide which can subsequently be collected from the culture medium (if the host cell secretes it into the medium) or directly from the host cell producing it (if it is not secreted). The selection of an appropriate host cell will depend upon various factors, such as desired expression levels, polypeptide modifications that are desirable or necessary for activity, such as glycosylation or phosphorylation, and ease of folding into a biologically active molecule.

A number of suitable host cells are known in the art and many are available from the American Type Culture Collection (ATCC), 10801 University Boulevard, Manassas, VA 20110-2209. Examples include, but are not limited to, mammalian cells, such as Chinese hamster ovary cells (CHO) (ATCC No. CCL61) CHO DHFR-cells (Urlaub et al., Proc. Natl. Acad. Sci. USA, 97:4216-4220 (1980)), human embryonic kidney (HEK) 293 or 293T cells (ATCC No. CRL1573), or 3T3 cells (ATCC No. CCL92). The selection of suitable mammalian host cells and methods for transformation, culture, amplification, screening and product production and purification are known in the art. Other suitable mammalian cell lines, are the monkey COS-1 (ATCC No. CRL1650) and COS-7 cell lines (ATCC No. CRL1651), and the CV-1 cell line (ATCC No. CCL70). Further exemplary mammalian host cells include primate cell lines and rodent cell lines, including transformed cell lines. Normal diploid cells, cell strains derived from *in vitro* culture of primary tissue, as well as primary explants, are also suitable. Candidate cells may be genotypically deficient in the selection gene, or may contain a dominantly acting selection gene. Other suitable mammalian cell lines include but are not limited to, mouse neuroblastoma N2A cells, HeLa, mouse L-929 cells, 3T3 lines derived from Swiss, Balb-c or NIH mice, BHK or HaK hamster cell lines, which are available from the ATCC. Each of these cell lines is known by and available to those skilled in the art of protein expression.

Similarly useful as host cells suitable for the present invention are bacterial cells. For example, the various strains of *E. coli* (*e.g.,* HB101, (ATCC No. 33694) DH5$\alpha$, DH10, and MC1061 (ATCC No. 53338)) are well-known as host cells in the field of biotechnology. Various strains of *B. subtilis*, *Pseudomonas spp.,* other *Bacillus spp., Streptomyces spp*., and the like may also be employed in this method.

Many strains of yeast cells known to those skilled in the art are also available as host cells for the expression of the polypeptides of the present invention. Preferred yeast cells include, for example, *Saccharomyces cerivisae* and *Pichia pastoris*.

Additionally, where desired, insect cell systems may be utilized in the methods of the present invention. Such systems are described for example in Kitts et al., Biotechniques, 14:810-817 (1993); Lucklow, Curr. Opin. Biotechnol., 4:564-572 (1993); and Lucklow et al. (J. Virol., 67:4566-4579 (1993). Preferred insect cells are Sf-9 and Hi5 (Invitrogen, Carlsbad, CA).

One may also use transgenic animals to express glycosylated C3b/C4b CR-like polypeptides. For example, one may use a transgenic milk-producing animal (a cow or goat, for example) and obtain the present glycosylated polypeptide in the animal milk. One may also use plants to produce C3b/C4b CR-like polypeptides, however, in general, the glycosylation occurring in plants is different from that produced in mammalian cells, and may result in a glycosylated product which is not suitable for human therapeutic use.

Polypeptide Production

Host cells comprising a C3b/C4b CR-like polypeptide expression vector may be cultured using standard media well known to the skilled artisan. The media will usually contain all nutrients necessary for the growth and survival of the cells. Suitable media for culturing *E. coli* cells include, for example, Luria Broth (LB) and/or Terrific Broth (TB). Suitable media for culturing eukaryotic cells include Roswell Park Memorial Institute medium 1640 (RPMI 1640), Minimal Essential Medium (MEM) and/or Dulbecco's Modified Eagle Medium (DMEM), all of which may be supplemented with serum and/or growth factors as indicated by the particular cell line being cultured. A suitable medium for insect cultures is Grace's medium supplemented with yeastolate, lactalbumin hydrolysate and/or fetal calf serum, as necessary.

Typically, an antibiotic or other compound useful for selective growth of transformed cells is added as a supplement to the media. The compound to be used will be dictated by the selectable marker element present on the plasmid with which the host cell was transformed. For example, where the selectable marker element is kanamycin resistance, the compound added to the culture medium will be kanamycin. Other compounds for selective growth include ampicillin, tetracycline, and neomycin.

The amount of a C3b/C4b CR-like polypeptide produced by a host cell can be evaluated using standard methods known in the art. Such methods include, without limitation, Western blot analysis, SDS-polyacrylamide gel electrophoresis, non-denaturing gel electrophoresis, HPLC separation, immunoprecipitation, and/or activity assays such as DNA binding gel shift assays.

If a C3b/C4b CR-like polypeptide has been designed to be secreted from the host cells, the majority of polypeptide may be found in the cell culture medium. If however, the C3b/C4b CR-like polypeptide is not secreted from the host cells, it will be present in the cytoplasm and/or the nucleus (for eukaryotic host cells) or in the cytosol (for bacterial host cells).

For a C3b/C4b CR-like polypeptide situated in the host cell cytoplasm and/or the nucleus (for eukaryotic host cells) or in the cytosol (for bacterial host cells), intracellular material (including inclusion bodies for gram-negative bacteria) can be extracted from the host cell using any standard technique known to the skilled artisan. For example, the host cells can be lysed to release the contents of the periplasm/cytoplasm by French press, homogenization, and/or sonication followed by centrifugation.

If a C3b/C4b CR-like polypeptide has formed inclusion bodies in the cytosol, the inclusion bodies can often bind to the inner and/or outer cellular membranes and thus will be found primarily in the pellet material after centrifugation.

The pellet material can then be treated at pH extremes or with a chaotropic agent such as a detergent, guanidine, guanidine derivatives, urea, or urea derivatives in the presence of a reducing agent such as dithiothreitol at alkaline pH or tris carboxyethyl phosphine at acid pH to release, break apart, and solubilize the inclusion bodies. The C3b/C4b CR-like polypeptide in its now soluble form can then be analyzed using gel electrophoresis, immunoprecipitation or the like. If it is desired to isolate the C3b/C4b CR-like polypeptide, isolation may be accomplished using standard methods such as those described herein and in Marston et al., Meth. Enz. , 182:264-275 (1990).

In some cases, a C3b/C4b CR-like polypeptide may not be biologically active upon isolation. Various methods for "refolding" or converting the polypeptide to its tertiary structure and generating disulfide linkages can be used to restore biological activity. Such methods include exposing the solubilized polypeptide to a pH usually above 7 and in the presence of a particular concentration of a chaotrope. The selection of chaotrope is very similar to the choices used for inclusion body solubilization, but usually the chaotrope is used at a lower concentration and is not necessarily the same as chaotropes used for the solubilization. In most cases the refolding/oxidation solution will also contain a reducing agent or the reducing agent plus its oxidized form in a specific ratio to generate a particular redox potential allowing for disulfide shuffling to occur in the formation of the protein's cysteine bridge(s). Some of the commonly used redox couples include cysteine/cystamine, glutathione (GSH)/dithiobis GSH, cupric chloride, dithiothreitol(DTT)/ dithiane DTT, and 2-2mercaptoethanol (bME)/dithio-b (ME). A cosolvent may be used to increase the efficiency of the refolding, and the more common reagents used for this purpose include glycerol, polyethylene glycol of various molecular weights, arginine and the like.

If inclusion bodies are not formed to a significant degree upon expression of a C3b/C4b CR-like polypeptide, then the polypeptide will be found primarily in the supernatant after centrifugation of the cell homogenate. The polypeptide may be further isolated from the supernatant using methods such as those described herein.

The purification of a C3b/C4b CR-like polypeptide from solution can be accomplished using a variety of techniques. If the polypeptide has been synthesized such that it contains a tag such as Hexahistidine (C3b/C4b CR-like polypeptide/hexaHis) or other small peptide such as FLAG (Eastman Kodak Co., New Haven, CT) or myc (Invitrogen, Carlsbad, CA) at either its carboxyl or amino terminus, it may be purified in a one-step process by passing the solution through an affinity column where the column matrix has a high affinity for the tag.

For example, polyhistidine binds with great affinity and specificity to nickel, thus an affinity column of nickel (such as the Qiagen® nickel columns) can be used for purification of C3b/C4b CR-like polypeptide/polyHis. See for example, Ausubel et al., eds. , Current Protocols in Molecular Biology, Section 10.11.8, John Wiley & Sons, New York (1993).

Additionally, the C3B/C4B CR-like polypeptide may be purified through the use of a monoclonal antibody which is capable of specifically recognizing and binding to the C3B/C4B CR-like polypeptide.

Suitable procedures for purification thus include, without limitation, affinity chromatography, immunoaffinity chromatography, ion exchange chromatography, molecular sieve chromatography, High Performance Liquid Chromatography (HPLC), electrophoresis (including native gel electrophoresis) followed by gel elution, and preparative isoelectric focusing ("Isoprime" machine/technique, Hoefer Scientific, San Francisco, CA). In some cases, two or more purification techniques may be combined to achieve increased purity.

C3b/C4b CR-like polypeptides may also be prepared by chemical synthesis methods (such as solid phase peptide synthesis) using techniques known in the art, such as those set forth by Merrifield et al., J. Am. Chem. Soc., 85: 2149 (1963), Houghten et al., Proc Natl Acad. Sci. USA, 82:5132 (1985), and Stewart and Young, Solid Phase Peptide Synthesis, Pierce Chemical Co., Rockford, IL (1984).

Such polypeptides may be synthesized with or without a methionine on the amino terminus. Chemically synthesized C3b/C4b CR-like polypeptides may be oxidized using methods set forth in these references to form disulfide bridges. Chemically synthesized C3b/C4b CR-like polypeptides are expected to have comparable biological activity to the corresponding C3b/C4b CR-like polypeptides produced recombinantly or purified from natural sources, and thus may be used interchangeably with a recombinant or natural C3b/C4b CR-like polypeptide.

Another means of obtaining a C3b/C4b CR-like polypeptide is via purification from biological samples such as source tissues and/or fluids in which the C3b/C4b CR-like polypeptide is naturally found. Such purification can be conducted using methods for protein purification as described herein. The presence of the C3b/C4b CR--like polypeptide during purification may be monitored using, for example, an antibody prepared against recombinantly produced C3b/C4b CR-like polypeptide or peptide fragments thereof.

A number of additional methods for producing nucleic acids and polypeptides are known in the art, and can be used to produce polypeptides having specificity for C3b/C4b CR-like. See for example, Roberts et al., Proc. Natl. Acad. Sci., 94:12297-12303 (1997), which describes the production of fusion proteins between an mRNA and its encoded peptide. See also Roberts, R., Curr. Opin. Chem. Biol., 3:268-273 (1999). Additionally, U.S. patent No. 5,824,469 describes methods of obtaining oligonucleotides capable of carrying out a specific biological function. The procedure involves generating a heterogeneous pool of oligonucleotides, each having a 5' randomized sequence, a central preselected sequence, and a 3' randomized sequence. The resulting heterogeneous pool is introduced into a population of cells that do not exhibit the desired biological function. Subpopulations of the cells are then screened for those which exhibit a predetermined biological function. From that subpopulation, oligonucleotides capable of carrying out the desired biological function are isolated.

U.S. Patent Nos. 5,763,192, 5,814,476, 5,723,323, and 5,817,483 describe processes for producing peptides or polypeptides. This is done by producing stochastic genes or fragments thereof, and then introducing these genes into host cells which produce one or more proteins encoded by the stochastic genes. The host cells are then screened to identify those clones producing peptides or polypeptides having the desired activity.

Chemical Derivatives

Chemically modified derivatives of the C3b/C4b CR-like polypeptides may be prepared by one skilled in the art, given the disclosures set forth hereinbelow. C3b/C4b CR-like polypeptide derivatives are modified in a manner that is different, either in the type or location of the molecules naturally attached to the polypeptide. Derivatives may include molecules formed by the deletion of one or more naturally-attached chemical groups. The polypeptide comprising the amino acid sequence of SEQ ID NO:2, SEQ ID NO:4, or SEQ ID NO:7, or a C3b/C4b CR-like polypeptide variant may be modified by the covalent attachment of one or more polymers. For example, the polymer selected is typically water soluble so that the protein to which it is attached does not precipitate in an aqueous environment, such as a physiological environment. Included within the scope of suitable polymers is a mixture of polymers. Preferably, for therapeutic use of the end-product preparation, the polymer will be pharmaceutically acceptable.

The polymers each may be of any molecular weight and may be branched or unbranched. The polymers each typically have an average molecular weight of between about 2kDa to about 100kDa (the term "about" indicating that in preparations of a water soluble polymer, some molecules will weigh more, some less, than the stated molecular weight). The average molecular weight of each polymer preferably is between about 5kDa and about 50kDa; more preferably between about 12kDa and about 40kDa and most preferably between about 20kDa and about 35kDa.

Suitable water soluble polymers or mixtures thereof include, but are not limited to, N-linked or O-linked carbohydrates, sugars, phosphates, polyethylene glycol (PEG) (including the forms of PEG that have been used to derivatize proteins, including mono-($C_1$-$C_{10}$) alkoxy- or aryloxy-polyethylene glycol), monomethoxy-polyethylene glycol, dextran (such as low molecular weight dextran, of, for example about 6 kD), cellulose, or other carbohydrate based polymers, poly-(N-vinyl pyrrolidone) polyethylene glycol, propylene glycol homopolymers, a polypropylene oxide/ethylene oxide copolymer, polyoxyethylated polyols (e.g., glycerol) and polyvinyl alcohol. Also encompassed by the present invention are bifunctional crosslinking molecules which may be used to prepare covalently attached multimers of the polypeptide comprising the amino acid sequence of SEQ ID NO:2, SEQ ID NO:4, or SEQ ID NO:7, or a C3b/C4b CR-like polypeptide variant.

In general, chemical derivatization may be performed under any suitable condition used to react a protein with an activated polymer molecule. Methods for preparing chemical derivatives of polypeptides will generally comprise the steps of (a) reacting the polypeptide with the activated polymer molecule (such as a reactive ester or aldehyde derivative of the polymer molecule) under conditions whereby the polypeptide comprising the amino acid sequence of SEQ ID NO: 2, SEQ ID NO:4, or SEQ ID NO:7, or a C3b/C4b CR-like polypeptide variant becomes attached to one or more polymer molecules, and (b) obtaining the reaction product(s). The optimal reaction conditions will be determined based on known parameters and the desired result. For example, the larger the ratio of polymer molecules:protein, the greater the percentage of attached polymer molecule. In one embodiment, the C3b/C4b CR-like polypeptide derivative may have a single polymer molecule moiety at the amino terminus. See, for example, U.S. Patent No. 5,234,784.

The pegylation of the polypeptide specifically may be carried out by any of the pegylation reactions known in the art, as described for example in the following references: Francis et al., Focus on Growth Factors, 3:4-10 (1992); EP 0154316; EP 0401384 and U.S. Patent No. 4,179,337. For example, pegylation may be carried out via an acylation reaction or an alkylation reaction with a reactive polyethylene glycol molecule (or an analogous reactive water-soluble polymer) as described herein. For the acylation reactions, the polymer(s) selected should have a single reactive ester group. For reductive alkylation, the polymer(s) selected should have a single reactive aldehyde group. A reactive aldehyde is, for example, polyethylene glycol propionaldehyde, which is water stable, or mono $C_1$-$C_{10}$ alkoxy or aryloxy derivatives thereof (see U.S. Patent No. 5,252,714).

In another embodiment, C3b/C4b CR-like polypeptides may be chemically coupled to biotin, and the biotin/C3b/C4b,CR-like polypeptide molecules which are conjugated are then allowed to bind to avidin, resulting in tetravalent avidin/biotin/C3b/C4b CR-like polypeptide molecules. C3b/C4b CR-like polypeptides may also be covalently coupled to dinitrophenol (DNP) or trinitrophenol (TNP) and the resulting conjugates precipitated with anti-DNP or anti-TNP-IgM to form decameric conjugates with a valency of 10.

Generally, conditions which may be alleviated or modulated by the administration of the present C3b/C4b CR-like polypeptide derivatives include those described herein for C3b/C4b CR-like polypeptides. However, the C3b/C4b CR-like polypeptide derivatives disclosed herein may have additional activities, enhanced or reduced biological activity, or other characteristics, such as increased or decreased half-life, as compared to the non-derivatized molecules.

Genetically Engineered Non-Human Animals

Additionally included within the scope of the present invention are non-human animals such as mice, rats, or other rodents, rabbits, goats, or sheep, or other farm animals, in which the gene (or genes) encoding the native C3b/C4b CR-like polypeptide has (have) been disrupted ("knocked out") such that the level of expression of this gene or genes is (are) significantly decreased or completely abolished. Such animals may be prepared' using techniques and methods such as those described in U.S. Patent No. 5,557,032.

The present invention further includes non-human animals such as mice, rats, or other rodents, rabbits, goats, sheep, or other farm animals, in which either the native form of the C3b/C4b CR-like gene(s) for that animal or a heterologous C3b/C4b CR-like gene(s) is (are) over-expressed by the animal, thereby creating a "transgenic" animal. Such transgenic animals may be prepared using well known methods such as those described in U.S. Patent No 5,489,743 and PCT application No. WO94/28122.

The present invention further includes non-human animals in which the promoter for one or more of the C3b/C4b CR-like polypeptides of the present invention is either activated or inactivated (e.g., by using homologous recombination methods) to alter the level of expression of one or more of the native C3b/C4b CR-like polypeptides.

These non-human animals may be used for drug candidate screening. In such screening, the impact of a drug candidate on the animal may be measured. For example, drug candidates may decrease or increase the expression of the C3b/C4b CR-like gene. In certain embodiments, the amount of C3b/C4b CR-like polypeptide, that is produced may be measured after the exposure of the animal to the drug candidate. Additionally, in certain embodiments, one may detect the actual impact of the drug candidate on the animal. For example, the overexpression of a particular gene may result in, or be associated with, a disease or pathological condition. In such cases, one may test a drug candidate's ability to decrease expression of the gene or its ability to prevent or inhibit a pathological condition. In other examples, the production of a particular metabolic product such as a fragment of a polypeptide, may result in, or be associated with, a disease or pathological condition. In such cases, one may test a drug candidate's ability to decrease the production of such a metabolic product or its ability to prevent or inhibit a pathological condition.

Microarray

It will be appreciated that DNA microarray technology can be utilized in accordance with the present invention. DNA microarrays are miniature, high density arrays of nucleic acids positioned on a solid support, such as glass. Each cell or element within the array has numerous copies of a single species of DNA which acts as a target for hybridization for its cognate mRNA. In expression profiling using DNA microarray technology, mRNA is first extracted from a cell or tissue sample and then converted enzymatically to fluorescently labeled cDNA. This material is hybridized to the microarray and unbound cDNA is removed by washing. The expression of discrete genes represented on the array is then visualized by quantitating the amount of labeled cDNA which is specifically bound to each target DNA. In this way, the expression of thousands of genes can be quantitated in a high throughput, parallel manner from a single sample of biological material.

This high throughput expression profiling has a broad range of applications with respect to the C3b/C4b CR-like molecules of the invention, including, but not limited to: the identification and validation of C3b/C4b CR-like disease-related genes as targets for therapeutics; molecular toxicology of C3b/C4b CR-like molecules and inhibitors thereof; stratification of populations and generation of surrogate markers for clinical trials; and enhancing C3b/C4b CR-like-related small molecule drug discovery by aiding in the identification of selective compounds in high throughput screens (HTS).

Selective Binding Agents

As used herein, the term "selective binding agent" refers to a molecule which has specificity for one or more C3b/C4b CR-like polypeptides. Suitable selective binding agents include, but are not limited to, antibodies and derivatives thereof, polypeptides, and small molecules. Suitable selective binding agents may be prepared using methods known in the art. An exemplary C3B/C4B CR-like polypeptide selective binding agent of the present invention is capable of binding a certain portion of the C3B/C4B CR-like polypeptide thereby inhibiting the binding of the polypeptide to the C3B/C4B CR-like polypeptide receptor(s).

Selective binding agents such as antibodies and antibody fragments that bind C3b/C4b CR-like polypeptides are within the scope of the present invention. The antibodies may be polyclonal including monospecific polyclonal, monoclonal (MAbs), recombinant, chimeric, humanized such as CDR-grafted, human, single chain, and/or bispecific, as well as fragments, variants or derivatives thereof. Antibody fragments include those portions of the antibody which bind to an epitope on the C3B/C4B CR-like polypeptide. Examples of such fragments include Fab and F(ab') fragments generated by enzymatic cleavage of full-length antibodies. Other binding fragments include those generated by recombinant DNA techniques, such as the expression of recombinant plasmids containing nucleic acid sequences encoding antibody variable regions.

Polyclonal antibodies directed toward a C3b/C4b CR-like polypeptide generally are produced in animals (e.g., rabbits or mice) by means of multiple subcutaneous or intraperitoneal injections of C3b/C4b CR-like polypeptide and an adjuvant. It may be useful to conjugate a C3b/C4b CR-like polypeptide to a carrier protein that is immunogenic in the species to be immunized, such as keyhole limpet heocyanin, serum, albumin, bovine thyroglobulin, or soybean trypsin inhibitor. Also, aggregating agents such as alum are used to enhance the immune response. After immunization, the animals are bled and the serum is assayed for anti-C3b/C4b CR-like polypeptide antibody titer.

Monoclonal antibodies directed toward a C3b/C4b CR-like polypeptide are produced using any method which provides for the production of antibody molecules by continuous cell lines in culture. Examples of suitable methods for preparing monoclonal antibodies include the hybridoma methods of Kohler et al., Nature, 256:495-497 (1975) and the human B-cell hybridoma method, Kozbor, J. Immunol., 133:3001 (1984); Brodeur et al., Monoclonal Antibody Production Techniques and Applications, pp. 51-63 (Marcel Dekker, Inc., New York, 1987). Also provided by the invention are hybridoma cell lines which produce monoclonal antibodies reactive with C3b/C4b CR-like polypeptides.

Monoclonal antibodies of the invention may be modified for use as therapeutics. One embodiment is a "chimeric" antibody in which a portion of the heavy and/or light chain is identical with or homologous to a corresponding sequence in antibodies derived from a particular species or belonging to a particular antibody class or subclass, while the remainder of the chain(s) is identical with or homologous to a corresponding sequence in antibodies derived from another species or belonging to another antibody class or subclass. Also included are fragments of such antibodies, so long as they exhibit the desired biological activity. See, U.S. Patent No. 4,816,567; Morrison et al . , Proc. Natl. Acad. Sci., 81: 6851-6855 (1985).

In another embodiment, a monoclonal antibody of the invention is a "humanized" antibody. Methods for humanizing non-human antibodies are well known in the art. See U.S. Patent Nos. 5,585,089, and 5,693,762. Generally, a humanized antibody has one or more amino acid residues introduced into it from a source which is non-human. Humanization can be performed, for example, using methods described in the art (Jones et al., Nature 321:522-525 (1986); Riechmann et al., Nature, 332:323-327 (1988); Verhoeyen et al., Science 239:1534-1536 (1988)), by substituting at least a portion of a rodent complementarity-determining region (CDR) for the corresponding regions of a human antibody.

Also encompassed by the invention are human antibodies which bind C3b/C4b CR-like polypeptides. Using transgenic animals (*e.g.,* mice) that are capable of producing a repertoire of human antibodies in the absence of endogenous immunoglobulin production such antibodies are produced by immunization with a C3b/C4b CR-like antigen (i.e., having at least 6 contiguous amino acids), optionally conjugated to a carrier. See, for example, Jakobovits et al., Proc. Natl. Acad. Sci., 90:2551-2555 (1993); Jakobovits et al., Nature 362:255-258 (1993); Bruggemann et al., Year in Immuno., 7:33 (1993). In one method, such transgenic animals are produced by incapacitating the endogenous loci encoding the heavy and light immunoglobulin chains therein, and inserting loci encoding human heavy and light chain proteins into the genome thereof. Partially modified animals, that is those having less than the full complement of modifications, are then cross-bred to obtain an animal having all of the desired immune system modifications. When administered an immunogen, these transgenic animals produce antibodies with human (rather than *e.g.,* murine) amino acid sequences, including variable regions which are immunospecific for these antigens. See PCT application nos. PCT/US96/05928 and PCT/US93/06926. Additional methods are described in U.S. Patent No. 5,545,807, PCT application nos. PCT/US91/245, PCT/GB89/01207, and in EP 546073B1 and EP 546073A1. Human antibodies may also be produced by the expression of recombinant DNA in host cells or by expression in hybridoma cells as described herein.

In an alternative embodiment, human antibodies can be produced from phage-display libraries (Hoogenboom et al., J. Mol. Biol. 227:381 (1991); Marks et al., J. Mol. Biol. 222 : 581 (1991). These processes mimic immune selection through the display of antibody repertoires on the surface of filamentous bacteriophage, and subsequent selection of phage by their binding to an antigen of choice. One such technique is described in PCT Application no. PCT/US98/17364, which describes the isolation of high affinity and functional agonistic antibodies for MPL- and msk- receptors using such an approach.

Chimeric, CDR grafted, and humanized antibodies are typically produced by recombinant methods. Nucleic acids encoding the antibodies are introduced into host cells and expressed using materials and procedures described herein. In a preferred embodiment, the antibodies are produced in mammalian host cells, such as CHO cells. Monoclonal (e.g., human) antibodies may be produced by the expression of recombinant DNA in host cells or by expression in hybridoma cells as described herein.

The anti-C3b/C4b CR-like antibodies cf the invention may be employed in any known assay method, such as competitive binding assays, direct and indirect sandwich assays, and immunoprecipitation assays (Zola, Monoclonal Antibodies: A Manual of Techniques, pp. 147-158 (CRC Press, Inc., 1987)) for the detection and quantitation of C3b/C4b CR-like polypeptides. The antibodies will bind C3b/C4b CR-like polypeptides with an affinity which is appropriate for the assay method being employed.

For diagnostic applications, in certain embodiments, anti-C3b/C4b CR-like antibodies may be labeled with a detectable moiety. The detectable moiety can be any one which is capable of producing, either directly or indirectly, a detectable signal. For example, the detectable moiety may be a radioisotope, such as $^{3}H$, $^{14}C$, $^{32}P$, $^{35}S$, or $^{125}I$, a fluorescent or chemiluminescent compound, such as fluorescein isothiocyanate, rhodamine, or luciferin; or an enzyme, such as alkaline phosphatase, □-galactosidase, or horseradish peroxidase (Bayer et al., Meth. Enz., 184:138-163 (1990)).

Competitive binding assays rely on the ability of a labeled standard (e.g., a C3b/C4b CR-like polypeptide, or an immunologically reactive portion thereof) to compete with the test sample analyte (an C3b/C4b CR-like polypeptide) for binding with a limited amount of anti C3b/C4b CR-like antibody. The amount of a C3b/C4b CR-like polypeptide in the test sample is inversely proportional to the amount of standard that becomes bound to the antibodies. To facilitate determining the amount of standard that becomes bound, the antibodies typically are insolubilized before or after the competition, so that the standard and analyte that are bound to the antibodies may conveniently be separated from the standard and analyte which remain unbound.

Sandwich assays typically involve the use of two antibodies, each capable of binding to a different immunogenic portion, or epitope, of the protein to be detected and/or quantitated. In a sandwich assay, the test sample analyte is typically bound by a first antibody which is immobilized on a solid support, and thereafter a second antibody binds to the analyte, thus forming an insoluble three part complex. See, *e.g.,* U.S. Patent No. 4,376,110. The second antibody may itself be labeled with a detectable moiety (direct sandwich assays) or may be measured using an anti-immunoglobulin antibody that is labeled with a detectable moiety (indirect sandwich assays). For example, one type of sandwich assay is an enzyme-linked immunosorbent assay (ELISA), in which case the detectable moiety is an enzyme.

The selective binding agents, including antiC3b/C4b CR-like antibodies, also are useful for *in vivo* imaging. An antibody labeled with a detectable moiety may be administered to an animal, preferably into the bloodstream, and the presence and location of the labeled antibody in the host is assayed. The antibody may be labeled with any moiety that is detectable in an animal, whether by nuclear magnetic resonance, radiology, or other detection means known in the art.

Selective binding agents of the invention, including antibodies, may be used as therapeutics. These therapeutic agents are generally agonists or antagonists, in that they either enhance or reduce, respectively, at least one of the biological activities of a C3b/C4b CR-like polypeptide. In one embodiment, antagonist antibodies of the invention are antibodies or binding fragments thereof which are capable of specifically binding to a C3b/C4b CR-like polypeptide and which are capable of inhibiting or eliminating the functional activity of a C3b/C4b CR-like polypeptide in vivo or in vitro. In preferred embodiments, the selective binding agent, *e.g.,* an antagonist antibody, will inhibit the functional activity of a C3b/C4b CR-like polypeptide by at least about 50%, and preferably by at least about 80%. In another embodiment, the selective binding agent may be an antibody that is capable of interacting with a C3b/C4b CR-like binding partner (a ligand or receptor) thereby inhibiting or eliminating C3b/C4b CR-like activity *in vitro* or in vivo. Selective binding agents, including agonist and antagonist anti-C3b/C4b CR-like antibodies, are identified by screening assays which are well known in the art.

The invention also relates to a kit comprising C3b/C4b CR-like selective binding agents (such as antibodies) and other reagents useful for detecting C3b/C4b CR-like polypeptide levels in biological samples. Such reagents may include, a detectable label, blocking serum, positive and negative control samples, and detection reagents.

C3b/C4b CR-like polypeptides can be used to clone C3b/C4b CR-like ligand(s) using an "expression cloning" strategy. Radiolabeled (125-Iodine) C3b/C4b CR-like polypeptide or "affinity/activity-tagged" C3b/C4b CR-like polypeptide (such as an Fc fusion or an alkaline phosphatase fusion) can be used in binding assays to identify a cell type or cell line or tissue that expresses C3b/C4b CR-like ligand(s). RNA isolated from such cells or tissues can then be converted to cDNA, cloned into a mammalian expression vector, and transfected into mammalian cells (for example, COS, or 293) to create an expression library. Radiolabeled or tagged C3b/C4b CR-like polypeptide can then be used as an affinity reagent to identify and isolate the subset of cells in this library expressing C3b/C4b CR-like ligand(s). DNA is then isolated from these cells and transfected into mammalian cells to create a secondary expression library in which the fraction of cells expressing C3b/C4b CR-like ligand(s) would be many-fold higher than in the original library. This enrichment process can be repeated iteratively until a single recombinant clone containing a C3b/C4b CR-like ligand is isolated. Isolation of C3b/C4b CR-like ligand(s) is useful for identifying or developing novel agonists and antagonists of the C3b/C4b CR-like signaling pathway. Such agonists and antagonists include C3b/C4b CR-like ligand(s), anti-C3b/C4b CR-like ligand antibodies, small molecules or antisense oligonucleotides.

Assaying for other modulators of C3b/C4b CR-like Polypeptide activity

In some situations, it may be desirable to identify molecules that are modulators, i.e., agonists or antagonists, of the activity of C3b/C4b CR-like polypeptide. Natural or synthetic molecules that modulate C3b/C4b CR-like polypeptide may be identified using one or more screening assays, such as those described herein. Such molecules may be administered either in an ex vivo manner, or in an in vivo manner by injection, or by oral delivery, implantation device, or the like.

"Test molecule(s)" refers to the molecule(s) that is/are under evaluation for the ability to modulate (*i.e.,* increase or decrease) the activity of a C3b/C4b CR-like polypeptide. Most commonly, a test molecule will interact directly with a C3b/C4b CR-like polypeptide. However, it is also contemplated that a test molecule may also modulate C3b/C4b CR-like polypeptide activity indirectly, such as by affecting C3b/C4b CR-like gene expression, or by binding to a C3b/C4b CR-like binding partner (*e.g.,* receptor or ligand). In one embodiment, a.test molecule will bind to a C3b/C4b CR-like polypeptide with an affinity constant of at least about $10^{-6}$ M, preferably about $10^{-8}$ M, more preferably about $10^{-9}$ M, and even more preferably about $10^{-10}$ M.

Methods for identifying compounds which interact with C3b/C4b CR-like polypeptides are encompassed by the present invention. In certain embodiments, a C3b/C4b CR-like polypeptide is incubated with a test molecule under conditions which permit the interaction of the test molecule with a C3b/C4b CR-like polypeptide, and the extent of the interaction can be measured. The test molecule(s) can be screened in a substantially purified form or in a crude mixture.

In certain embodiments, a C3b/C4b CR-like polypeptide agonist or antagonist may be a protein, peptide, carbohydrate, lipid, or small molecular weight molecule which interacts with C3b/C4b CR-like polypeptide to regulate its activity. Molecules which regulate C3b/C4b CR-like polypeptide expression include nucleic acids which are complementary to nucleic acids encoding a C3b/C4b CR-like polypeptide, or are complementary to nucleic acids sequences which direct or control the expression of C3b/C4b CR-like polypeptide, and which act as anti-sense regulators of expression.

Once a set of test molecules has been identified as interacting with a C3b/C4b CR-like polypeptide, the molecules may be further evaluated for their ability to increase or decrease C3b/C4b CR-like polypeptide activity. The measurement of the interaction of test molecules with C3b/C4b CR-like polypeptides may be carried out in several formats, including cell-based binding assays, membrane binding assays, solution-phase assays and immunoassays. In general, test molecules are incubated with a C3b/C4b CR-like polypeptide for a specified period of time, and C3b/C4b CR-like polypeptide activity is determined by one or more assays for measuring biological activity.

The interaction of test molecules with C3b/C4b CR-like polypeptides may also be assayed directly using polyclonal or monoclonal antibodies in an immunoassay. Alternatively, modified forms of C3b/C4b CR-like polypeptides containing epitope tags as described herein may be used in immunoassays.

In the event that C3b/C4b CR-like polypeptides display biological activity through an interaction with a binding partner (e.g., a receptor or a ligand), a variety of in vitro assays may be used to measure the binding of a C3b/C4b CR-like polypeptide to the corresponding binding partner (such as a selective binding agent, receptor, or ligand). These assays may be used to screen test molecules for their ability to increase or decrease the rate and/or the extent of binding of a C3b/C4b CR-like polypeptide to its binding partner. In one assay, a C3b/C4b CR-like polypeptide is immobilized in the wells of a microtiter plate. Radiolabeled C3b/C4b CR-like binding partner (for example, iodinated C3b/C4b CR-like binding partner) and the test molecule(s) can then be added either one at a time (in either order) or simultaneously to the wells. After incubation, the wells can be washed and counted, using a scintillation counter, for radioactivity to determine the extent to which the binding partner bound to C3b/C4b CR-like polypeptide. Typically, the molecules will be tested over a range of concentrations, and a series of control wells lacking one or more elements of the test assays can be used for accuracy in the evaluation of the results. An alternative to this method involves reversing the "positions" of the proteins, i.e., immobilizing C3b/C4b CR-like binding partner to the microtiter plate wells, incubating with the test molecule and radiolabeled C3b/C4b CR-like polypeptide, and determining the extent of C3b/C4b CR-like polypeptide binding. See, for example, chapter 18, Current Protocols in Molecular Biology, Ausubel et al., eds., John Wiley & Sons, New York, NY (1995).

As an alternative to radiolabelling, a C3b/C4b CR-like polypeptide or its binding partner may be conjugated to biotin and the presence of biotinylated protein can then be detected using streptavidin linked to an enzyme, such as horseradish peroxidase (HRP) or alkaline phosphatase (AP), that can be detected colorometrically, or by fluorescent tagging of streptavidin. An antibody directed to a C3b/C4b CR-like polypeptide or to a C3b/C4b CR-like binding partner and conjugated to biotin may also be used and can be detected after incubation with enzyme-linked streptavidin linked to AP or HRP.

An C3b/C4b CR-like polypeptide or a C3b/C4b CR-like binding partner can also be immobilized by attachment to agarose beads, acrylic beads or other types of such inert solid phase substrates. The' substrate-protein complex can be placed in a solution containing the complementary protein and the test compound. After incubation, the beads can be precipitated by centrifugation, and the amount of binding between a C3b/C4b CR-like polypeptide and its binding partner can be assessed using the methods described herein. Alternatively, the substrate-protein complex can be immobilized in a column, and the test molecule and complementary protein are passed through the column. The formation of a complex between a C3b/C4b CR-like polypeptide and its binding partner can then be assessed using any of the techniques set forth herein, i.e., radiolabelling, antibody binding, or the like.

Another in vitro assay that is useful for identifying a test molecule which increases or decreases the formation of a complex between a C3b/C4b Complement Receptor polypeptide and a C3b/C4b CR-like binding partner is a surface plasmon resonance detector system such as the BIAcore assay system (Pharmacia, Piscataway, NJ). The BIAcore system may be carried out using the manufacturer's protocol. This assay essentially involves the covalent binding of either C3b/C4b CR-like polypeptide or a C3b/C4b CR-like binding partner to a dextran-coated sensor chip which is located in a detector. The test compound and the other complementary protein can then be injected, either simultaneously or sequentially, into the chamber containing the sensor chip. The amount of complementary protein that binds can be assessed based on the change in molecular mass' which is physically associated with the dextran-coated side of the sensor chip; the change in molecular mass can be measured by the detector system.

In some cases, it may be desirable to evaluate two or more test compounds together for their ability to increase or decrease the formation of a complex between a C3b/C4b CR-like polypeptide and a C3b/C4b CR-like binding partner. In these cases, the assays set forth herein can be readily modified by adding such additional test compound(s) either simultaneous with, or subsequent to, the first test compound. The remainder of the steps in the assay are as set forth herein.

In vitro assays such as those described herein may be used advantageously to screen large numbers of compounds for effects on complex formation by C3b/C4b CR-like polypeptide and C3b/C4b CR-like binding partner. The assays may be automated to screen compounds generated in phage display, synthetic peptide, and chemical synthesis libraries.

Compounds which increase or decrease the formation of a complex between a C3b/C4b CR-like polypeptide and a C3b/C4b CR-like binding partner may also be screened in cell culture using cells and cell lines expressing either C3b/C4b CR-like polypeptide or C3b/C4b CR-like binding partner. Cells and cell lines may be obtained from any mammal, but preferably will be from human or other primate, canine, or rodent sources. The binding of a C3b/C4b CR-like polypeptide to cells expressing C3b/C4b CR-like binding partner at the surface is evaluated in the presence or absence of test molecules, and the extent of binding may be determined by, for example, flow cytometry using a biotinylated antibody to a C3b/C4b CR-like binding partner. Cell culture assays can be used advantageously to further evaluate compounds that score positive in protein binding assays described herein.

Cell cultures can also be used to screen the impact of a drug candidate. For example, drug candidates may decrease or increase the expression of the C3b/C4b CR-like gene. In certain embodiments, the amount of C3b/C4b CR-like polypeptide that is produced may be measured after exposure of the cell culture to the drug candidate. In certain embodiments, one may detect the actual impact of the drug candidate on the cell culture. For example, the overexpression of a particular gene may have a particular impact on the cell culture. In such cases, one may test a drug candidate's ability to increase or decrease the expression of the gene or its ability to prevent or inhibit a particular impact on the cell culture. In other examples, the production of a particular metabolic product such as a fragment of a polypeptide, may result in, or be associated with, a disease or pathological condition. In such cases, one may test a drug candidate's ability to decrease the production of such a metabolic product in a cell culture.

A yeast two hybrid system (Chien et al., Proc. Natl. Acad. Sci. USA, 88:9578-9583 (1991)) can be used to identify novel polypeptides that bind to, or interact with, C3b/C4b CR-like polypeptides. As an example, hybrid constructs comprising DNA encoding a cytoplasmic domain of a C3b/C4b CR-like polypeptide fused to a yeast GAL4-DNA binding domain may be used as a two-hybrid bait plasmid. Positive clones emerging from the screening may be characterized further to identify interacting proteins.

## Internalizing Proteins

The tat protein sequence (from HIV) can be used to internalize proteins into a cell. See *e.g.*, Falwell et al., Proc. Natl. Acad. Sci., 91:664-668 (1994). For example, an 11 amino acid sequence (YGRKKRRQRRR) of the HIV tat protein (termed the "protein transduction domain", or TAT PDT) has been described as mediating delivery across the cytoplasmic membrane and the nuclear membrane of a cell. *See* Schwarze et al., Science, 285:1569-1572 (1999); and Nagahara et al., Nature Medicine, 4:1449-1452 (1998). In these procedures, FITC-constructs (FITC-GGGGYGRKKRRQRRR) are prepared which bind to cells as observed by fluorescence-activated cell sorting (FACS) analysis, and these constructs penetrate tissues after i.p. administration. Next, tat-bgal fusion proteins are constructed. Cells treated with this construct demonstrated b-gal activity. Following injection, a number of tissues, including liver, kidney, lung, heart, and brain tissue have been found to demonstrate expression using these procedures. It is believed that these constructions underwent some degree of unfolding in order to enter the cell; as such, refolding may be required after entering the cell.

It will thus be appreciated that the tat protein sequence may be used to internalize a desired protein or polypeptide into a cell. For example, using the tat protein sequence, a C3b/C4b CR-like antagonist (such as an anti-C3b/C4b CR-like selective binding agent, small molecule, soluble receptor, or antisense oligonucleotide) can be administered intracellularly to inhibit the activity of a C3b/C4b CR-like molecule. As used herein, the term "C3b/C4b CR-like molecule" refers to both C3b/C4b CR-like nucleic acid molecules and C3b/C4b CR-like polypeptides as defined herein. Where desired, the C3b/C4b CR-like protein itself may also be internally administered to a cell using these procedures. *See also,* Strauss, E., "Introducing Proteins Into the Body's Cells", Science, 285:1466-1467 (1999) .

Therapeutic Uses

A non-exclusive list of acute and chronic diseases which can be treated, diagnosed, ameliorated, or prevented with the polypeptides and nucleic acids of the invention is set forth below.

C3b/C4b CR-related polypeptides may act to stimulate the activation of the complement system, which acts alone and in conjunction with antibodies to destroy cells that are foreign to the host and is a main defense against bacterial and viral infections. The ability of a binding partner to bind to and activate C3b/C4b CR-related polypeptide or protein may lead to complement activation. Such a binding partner can be an agonist of C3b/C4b-CR related polypeptide or protein, such as antibody, peptibody, peptide, carbohydrate, polynucleotide, or small molecular weight organic molecule. Agonists of C3b/C4b CR-related polypeptides or proteins may be used to prevent and treat conditions characterized by insufficient or defective complement activation, such as bacterial and viral infections.

Alternatively, it may be desirable to use an antagonist of C3b/C4b CR-related polypeptide or protein to block complement activation. An antagonist would be useful for preventing and treating conditions characterized by excessive complement activation, particularly immune system disorders such as rheumatoid arthritis, psioriatic arthritis, inflammatory arthritis, osteoarthritis, inflammatory joint disease, autoimmune disease, multiple sclerosis, lupus, diabetes, inflammatory bowel disease, transplant rejection, and graft versus host disease. Antagonists would also be useful for prevent or treating undesired complement-mediated damage to cells and tissues. In one embodiment, an ant agonist comprises a soluble domain of a C3b/C4b CR-related polypeptide or protein.

Other uses for agonists and antagonists of C3b/C4b CR-like molecules include the diagnosis, prevention and treatment of nervous system disorders, such as stroke, Alzheimer's disease, brain injury, and Parkinson's disease; damaged tissues, such as by wounds and burns; ischemic conditions, such as atherosclerosis, restenosis, myocardial infarction, angioplasty, hypertension, and ischemia; metabolic disorders, such as obesity, diabetes, and cachexia; and reproductive disorders, infertility, miscarriage, preterm labor and delivery, and endometriosis.

C3b/C4b CR-like Compositions and Administration

Therapeutic compositions are within the scope of the present invention. Such C3B/C4B CR-like pharmaceutical compositions may comprise a therapeutically effective amount of a C3b/C4b CR-like polypeptide or a C3b/C4b CR-like nucleic acid molecule in admixture with a pharmaceutically or physiologically acceptable formulation agent selected for suitability with the mode of administration. Pharmaceutical compositions may comprise a therapeutically effective amount of one or more C3b/C4b CR-like selective binding agents in admixture with a pharmaceutically or physiologically acceptable formulation agent selected for suitability with the mode of administration.

Acceptable formulation materials preferably are nontoxic to recipients at the dosages and concentrations employed.

The pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. Suitable formulation materials include, but are not limited to, amino acids (such as glycine, glutamine, asparagine, arginine or lysine), antimicrobials, antioxidants (such as ascorbic acid, sodium sulfite or sodium hydrogensulfite), buffers (such as borate, bicarbonate, Tris-HCl, citrates, phosphates, other organic acids), bulking agents (such as mannitol or glycine), chelating agents (such as ethylenediamine tetraacetic acid (EDTA)), complexing agents (such as caffeine, polyvinylpyrrolidone, beta-cyclodextrin or hydroxypropyl-beta-cyclodextrin), fillers, monosaccharides, disaccharides, and other carbohydrates (such as glucose, mannose, or dextrins), proteins (such as serum albumin, gelatin or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid or hydrogen peroxide), solvents (such as glycerin, propylene glycol or polyethylene glycol), sugar alcohols (such as mannitol or sorbitcl), suspending agents, surfactants or wetting agents (such as pluronics, PEG, sorbitan esters, polysorbates such as polysorbate 20, polysorbate 80, triton, tromethamine, lecithin, cholesterol, tyloxapal), stability enhancing agents (sucrose or sorbitol), tonicity enhancing agents (such as alkali metal halides (preferably sodium or potassium chloride), mannitol sorbitol), delivery vehicles, diluents, excipients and/or pharmaceutical adjuvants. (Remington's Pharmaceutical Sciences, 18th Edition, A.R. Gennaro, ed., Mack Publishing Company [1990]).

The optimal pharmaceutical composition will be determined by one skilled in the art depending upon, for example, the intended route of administration, delivery format, and desired dosage. See for example, *Remington's* Pharmaceutical *Sciences, supra.* Such compositions may influence the physical state, stability, rate of *in vivo* release, and rate of in vivo clearance of the C3b/C4b CR-like molecule.

The primary vehicle or carrier in a pharmaceutical composition may be either aqueous or non-aqueous in nature. For example, a suitable vehicle or carrier may be water for injection, physiological saline solution, or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which may further include sorbitol or a suitable substitute therefor. In one embodiment of the present invention, C3b/C4b CR-like polypeptide compositions may be prepared for storage by mixing the selected composition having the desired degree of purity with optional formulation agents (*Remington's Pharmaceutical Sciences, supra)* in the form of a lyophilized cake or an aqueous solution. Further, the C3b/C4b CR-like polypeptide product may be formulated as a lyophilizate using appropriate excipients such as sucrose.

The C3b/C4b CR-like pharmaceutical compositions can be selected for parenteral delivery. Alternatively, the compositions may be selected for inhalation or for delivery through the digestive tract, such as orally. The preparation of such pharmaceutically acceptable compositions is within the skill of the art.

The formulation components are present in concentrations that are acceptable to the site of administration. For example, buffers are used to maintain the composition at physiological pH or at slightly lower pH, typically within a pH range of from about 5 to about 8.

When parenteral administration is contemplated, the therapeutic compositions for use in this invention may be in the form of a pyrogen-free, parenterally acceptable aqueous solution comprising the desired C3b/C4b CR-like molecule in a pharmaceutically acceptable vehicle. A particularly suitable vehicle for parenteral injection is sterile distilled water in which a C3b/C4b CR-like molecule is formulated as a sterile, isotonic solution, properly preserved. Yet another preparation can involve the formulation of the desired molecule with an agent, such as injectable microspheres, bio-erodible particles, polymeric compounds (polylactic acid, polyglycolic acid), or beads, or liposomes, that provides for the controlled or sustained release of the product which may then be delivered as a depot injection. Hyaluronic acid may also be used, and this may have the effect of promoting sustained duration in the circulation. Other suitable means for the introduction of the desired molecule include implantable drug delivery devices.

In one embodiment, a pharmaceutical composition may be formulated for inhalation. For example, a C3b/C4b CR-like molecule may be formulated as a dry powder for inhalation. C3b/C4b CR-like polypeptide or C3b/C4b CR-like nucleic acid molecule inhalation solutions may also be formulated with a propellant for aerosol delivery. In yet another embodiment, solutions may be nebulized. Pulmonary administration is further described in PCT application no. PCT/US94/001875, which describes pulmonary delivery of chemically modified proteins.

It is also contemplated that certain formulations may be administered orally. In one embodiment of the present invention, C3b/C4b CR-like molecules which are administered in this fashion can be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents can be included to facilitate absorption of the C3b/C4b CR-like molecule. Diluents, flavorings, low melting point waxes, vegetable oils, lubricants, suspending agents, tablet disintegrating agents, and binders may also be employed.

Another pharmaceutical composition may involve an effective quantity of C3b/C4b CR-like molecules in a mixture with non-toxic excipients which are suitable for the manufacture of tablets. By dissolving the tablets in sterile water, or other appropriate vehicle, solutions can be prepared in unit dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional C3b/C4b CR-like pharmaceutical compositions will be evident to those skilled in the art, including formulations involving C3b/C4b CR-like polypeptides in sustained- or controlled-delivery formulations. Techniques for foi-mulating a variety of other sustained- or controlled-delivery means, such as liposome carriers, bio-erodible microparticles or porous beads and depot injections, are also known to those skilled in the art. See for example, PCT/US93/00829 which describes controlled release of porous polymeric microparticles for the delivery of pharmaceutical compositions. Additional examples of sustained-release preparations include semipermeable polymer matrices in the form of shaped articles, e.g. films, or microcapsules. Sustained release matrices may include polyesters, hydrogels, polylactides (U.S. 3,773,919, EP 58,481), copolymers of L-glutamic acid and gamma ethyl-L-glutamate (Sidman et al., Biopolymers, 22:547-556 (1983)), poly (2-hydroxyethylmethacrylate) (Langer et al., J. Biomed. Mater. Res., 15:167-277 (1981) and Langer, Chem. Tech., 12:98-105 (1982)), ethylene vinyl acetate (Langer *et al., supra)* or poly-D(-)-3-hydroxybutyric acid (EP 133,988). Sustained-release compositions also may include liposomes, which can be prepared by any of several methods known in the art. See *e.g.*, Eppstein et al., Proc. Natl. Acad. Sci. USA, 82:3688-3692 (1985); EP 36,676; EP 88,046; EP 143,949.

The C3b/C4b CR-like pharmaceutical composition to be used for in *vivo* administration typically must be sterile. This may be accomplished by filtration through sterile filtration membranes. Where the composition is lyophilized, sterilization using these methods may be conducted either prior to, or following, lyophilization and reconstitution. The composition for parenteral administration may be stored in lyophilized form or in solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form (e.g., lyophilized) requiring reconstitution prior to administration.

In a specific embodiment, the present invention is directed to kits for producing a single-dose administration unit. The kits may each contain both a first container having a dried protein and a second container having an aqueous formulation. Also included within the scope of this invention are kits containing single and multi-chambered pre-filled syringes (e.g., liquid syringes and lyosyringes).

An effective amount of a C3b/C4b CR-like pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the C3b/C4b CR-like molecule is being used, the route of administration, and the size (body weight, body surface or organ size) and condition (the age and general health) of the patient. Accordingly, the clinician may titer the dosage and modify the route of administration to obtain the optimal therapeutic effect. A typical dosage may range from about 0.1 μg/kg to up to about 100 mg/kg or more, depending on the factors mentioned above. In other embodiments, the dosage may range from 0.1 μg/kg up to about 100 mg/kg; or 1 μg/kg up to about 100 mg/kg; or 5 μg/kg up to about 100 mg/kg.

The frequency of dosing will depend upon the pharmacokinetic parameters of the C3b/C4b CR-like molecule in the formulation used. Typically, a clinician will administer the composition until a dosage is reached that achieves the desired effect. The composition may therefore be administered as a single dose, or as two or more doses (which may or may not contain the same amount of the desired molecule) over time, or as a continuous infusion via implantation device or catheter. Further refinement of the appropriate dosage is routinely made by those of ordinary skill in the art and is within the ambit of tasks routinely performed by them. Appropriate dosages may be ascertained through use of appropriate dose-response data.

The route of administration of the pharmaceutical composition is in accord with known methods, e.g. oral, injection by intravenous, intraperitoneal, intracerebral (intra-parenchymal), intracerebroventricular, intramuscular, intra-ocular, intraarterial, intraportal, or intralesional routes, or by sustained release systems or implantation device. Where desired, the compositions may be administered by bolus injection or continuously by infusion, or by implantation device.

Alternatively or additionally, the composition may be administered locally via implantation of a membrane, sponge, or other appropriate material on to which the desired molecule has been absorbed or encapsulated. Where an implantation device is used, the device may be implanted into any suitable tissue or organ, and delivery of the desired molecule may be via diffusion, timed release bolus, or continuous administration.

In some cases, it may be desirable to use C3b/C4b CR-like pharmaceutical compositions in an *ex vivo* manner. In such instances, cells, tissues, or organs that have been removed from the patient are exposed to C3b/C4b CR-like pharmaceutical compositions after which the cells, tissues and/or organs are subsequently implanted back into the patient.

In other cases, a C3b/C4b CR-like polypeptide can be delivered by implanting certain cells that have been genetically engineered, using methods such as those described herein, to express and secrete the polypeptide. Such cells may be animal or human cells, and may be autologous, heterologous, or xenogeneic. Optionally, the cells may be immortalized. In order to decrease the chance of an immunological response, the cells may be encapsulated to avoid infiltration of surrounding tissues. The encapsulation materials are typically biocompatible, semi-permeable polymeric enclosures or membranes that allow the release of the protein product(s) but prevent the destruction of the cells by the patient's immune system or by other detrimental factors from the surrounding tissues.

Additional embodiments of the present invention relate to cells and methods (*e.g.,* homologous recombination and/or other recombinant production methods) for both the *in vitro* production of therapeutic polypeptides and for the production and delivery of therapeutic polypeptides by gene therapy or cell therapy. Homologous and other recombination methods may be used to modify a cell that contains a normally transcriptionally silent C3b/C4b CR-like gene, or an under expressed gene, and thereby produce a cell which expresses therapeutically efficacious amounts of C3b/C4b CR-like polypeptides.

Homologous recombination is a technique originally developed for targeting genes to induce or correct mutations in transcriptionally active genes (Kucherlapati, Prog. in Nucl. Acid Res. & Mol. Biol., 36:301, 1989). The basic technique was developed as a method for introducing specific mutations into specific regions of the mammalian genome (Thomas et al., Cell, 44:419-428, 1986; Thomas and Capecchi, Cell, 51:503-512, 1987; Doetschman et al., Proc. Natl. Acad. Sci., 85:8583-8587, 1988) or to correct specific mutations within defective genes (Doetschman et al., Nature, 330:576-578, 1987). Exemplary homologous recombination techniques are described in U.S. Patent No. 5,272,071 (EP 9193051, EP Publication No. 505500; PCT/US90/07642, International Publication No. WO 91/09955).

Through homologous recombination, the DNA sequence to be inserted into the genome can be directed to a specific region of the gene of interest by attaching it to targeting DNA. The targeting DNA is a nucleotide sequence that is complementary (homologous) to a region of the genomic DNA. Small pieces of targeting DNA that are complementary to a specific region of the genome are put in contact with the parental strand during the DNA replication process. It is a general property of DNA that has been inserted into a cell to hybridize, and therefore, recombine with other pieces of endogenous DNA through shared homologous regions. If this complementary strand is attached to an oligonucleotide that contains a mutation or a different sequence or an additional nucleotide, it too is incorporated into the newly synthesized strand as a result of the recombination. As a result of the proofreading function, it is possible for the new sequence of DNA to serve as the template. Thus, the transferred DNA is incorporated into the genome.

Attached to these pieces of targeting DNA are regions of DNA which may interact with or control the expression of a C3b/C4b CR-like polypeptide, *e.g.,* flanking sequences. For example, a promoter/enhancer element, a suppresser, or an exogenous transcription modulatory element is inserted in the genome of the intended host cell in proximity and orientation sufficient to influence the transcription of DNA encoding the desired C3b/C4b CR-like polypeptide. The control element controls a portion of the DNA present in the host cell genome. Thus', the expression of the desired C3b/C4b CR-like polypeptide may be achieved not by transfection of DNA that encodes the C3b/C4b CR-like gene itself, but rather by the use of targeting DNA (containing regions of homology with the endogenous gene of interest) coupled with DNA regulatory segments that provide the endogenous gene sequence with recognizable signals for transcription of a C3b/C4b CR-like polypeptide.

In an exemplary method, the expression of a desired targeted gene in a cell (i.e., a desired endogenous cellular gene) is altered via homologous recombination into the cellular genome at a preselected site, by the introduction of DNA which includes at least a regulatory sequence, an exon and a splice donor site. These components are introduced into the chromosomal (genomic) DNA in such a manner that this, in effect, results in the production of a new transcription unit (in which the regulatory sequence, the exon and the splice donor site present in the DNA construct are operatively linked to the endogenous gene). As a result of the introduction of these components into the chromosomal DNA, the expression of the desired endogenous gene is altered.

Altered gene expression, as described herein, encompasses activating (or causing to be expressed) a gene which is normally silent (unexpressed) in the cell as obtained, as well as increasing the expression of a gene which is not expressed at physiologically significant levels in the cell as obtained. The embodiments further encompass changing the pattern of regulation or induction such that it is different from the pattern of regulation or induction that occurs in the cell as obtained, and reducing (including eliminating) the expression of a. gene which is expressed in the cell as obtained.

One method by which homologous recombination can be used to increase, or cause, C3b/C4b CR-like polypeptide production from a cell's endogenous C3b/C4b CR-like gene involves first using homologous recombination to place a recombination sequence from a site-specific recombination system (*e.g.,* Cre/loxP, FLP/FRT) (Sauer, Current Opinion In Biotechnology, 5:521-527, 1994; Sauer, Methods In Enzymology, 225:890-900, 1993) upstream (that is, 5' to) of the cell's endogenous genomic C3b/C4b CR-like polypeptide coding region. A plasmid containing a recombination site homologous to the site that was placed just upstream of the genomic C3b/C4b CR-like polypeptide coding region is introduced into the modified cell line along with the appropriate recombinase enzyme. This recombinase causes the plasmid to integrate, via the plasmid's recombination site, into the recombination site located just upstream of the genomic C3b/C4b CR-like polypeptide coding region in the cell line (Baubonis and Sauer, Nucleic Acids Res., 21:2025-2029, 1993; O'Gorman et al., Science, 251:1351-1355, 1991). Any flanking sequences known to increase transcription (e.g., enhancer/promoter, intron, translational enhancer), if properly positioned in this plasmid, would integrate in such a manner as to create a new or modified transcriptional unit resulting in de novo or increased C3b/C4b CR-like polypeptide production from the cell's endogenous C3b/C4b CR-like gene.

A further method to use the cell line in which the site specific recombination sequence had been placed just upstream of the cell's endogenous genomic C3b/C4b CR-like polypeptide coding region is to use homologous recombination to introduce a second recombination site elsewhere in the cell line's genome. The appropriate recombinase enzyme is then introduced into the two-recombination-site cell line, causing a recombination event (deletion, inversion, translocation) (Sauer, *Current Opinion In Biotechnology, supra,* 1994; Sauer, *Methods In Enzymology, supra,* 1993) that would create a new or modified transcriptional unit resulting in *de novo* or increased C3b/C4b CR-like polypeptide production from the cell's endogenous C3b/C4b CR-like gene.

An additional approach for increasing, or causing, the expression of C3b/C4b CR-like polypeptide from a cell's endogenous C3b/C4b CR-like gene involves increasing, or causing, the expression of a gene or genes (e.g., transcription factors) and/or decreasing the expression of a gene or genes (e.g., transcriptional repressors) in a manner which results in de novo or increased C3b/C4b CR-like polypeptide production from the cell's endogenous C3b/C4b CR-like gene. This method includes the introduction of a non-naturally occurring polypeptide (e.g., a polypeptide comprising a site specific DNA binding domain fused to a transcriptional factor domain) into the cell such that *de novo* or increased C3b/C4b CR-like polypeptide production from the cell's endogenous C3b/C4b CR-like gene results.

The present invention further relates to DNA constructs useful in the method of altering expression of a target gene. In certain embodiments, the exemplary DNA constructs comprise: (a) one or more targeting sequences; (b) a regulatory sequence; (c) an exon; and (d) an unpaired splice-donor site. The targeting sequence in the DNA construct directs the integration of elements (a)-(d) into a target gene in a cell such that the elements (b)-(d) are operatively linked to sequences of the endogenous target gene. In another embodiment, the DNA constructs comprise: (a) one or more targeting sequences, (b) a regulatory sequence, (c) an exon, (d) a splice-donor site, (e) an intron, and (f) a splice-acceptor site, wherein the targeting sequence directs the integration of elements (a)-(f) such that the elements of (b)-(f) are operatively linked to the endogenous gene. The targeting sequence is homologous to the preselected site in the cellular chromosomal DNA with which homologous recombination is to occur. In the construct, the exon is generally 3' of the regulatory sequence and the splice-donor site is 3' of the exon.

If the sequence of a particular gene is known, such as the nucleic acid sequence of C3b/C4b CR-like polypeptide presented herein, a piece of DNA that is complementary to a selected region of the gene can be synthesized or otherwise obtained, such as by appropriate restriction of the native DNA at specific recognition sites bounding the region of interest. This piece serves as a targeting sequence(s) upon insertion into the cell and will hybridize to its homologous region within the genome. If this hybridization occurs during DNA replication, this piece of DNA, and any additional sequence attached thereto, will act as an Okazaki fragment and will be incorporated into the newly synthesized daughter strand of DNA. The present invention, therefore, includes nucleotides encoding a C3b/C4b CR-like polypeptide, which nucleotides may be used as targeting sequences.

C3b/C4b CR-like polypeptide cell therapy, *e.g.*, the implantation of cells producing C3b/C4b CR-like polypeptides, is also contemplated. This embodiment involves implanting cells capable of synthesizing and secreting a biologically active form of C3b/C4b CR-like polypeptide. Such C3b/C4b CR-like polypeptide-producing cells can be cells that are natural producers of C3b/C4b CR-like polypeptides or may be recombinant cells whose ability to produce C3b/C4b CR-like polypeptides has been augmented by transformation with a gene encoding the desired C3b/C4b CR-like polypeptide or with a gene augmenting the expression of C3b/C4b CR-like polypeptide. Such a modification may be accomplished by means of a vector suitable for delivering the gene as well as promoting its expression and secretion. In order to minimize a potential immunological reaction in patients being administered a C3b/C4b CR-like polypeptide, as may occur with the administration of a polypeptide of a foreign species, it is preferred that the natural cells producing C3b/C4b CR-like polypeptide be of human origin and produce human C3b/C4b CR-like polypeptide. Likewise, it is preferred that the recombinant cells producing C3b/C4b CR-like polypeptide be transformed with an expression vector containing a gene encoding a human C3b/C4b CR-like polypeptide.

Implanted cells may be encapsulated to avoid the infiltration of surrounding tissue. Human or non-human animal cells may be implanted in patients in biocompatible, semipermeable polymeric enclosures or membranes that allow the release of C3b/C4b CR-like polypeptide, but that prevent the destruction of the' cells by the patient's immune system or by other detrimental factors from the surrounding tissue. Alternatively, the patient's own cells, transformed to produce C3b/C4b CR-like polypeptides *ex vivo,* may be implanted directly into the patient without such encapsulation.

Techniques for the encapsulation of living cells are known in the art, and the preparation of the encapsulated cells and their implantation in patients may be routinely accomplished. For example, Baetge et *al.* (WO95/05452; PCT/US94/09299) describe membrane capsules containing genetically engineered cells for the effective delivery of biologically active molecules. The capsules are biocompatible and are easily retrievable. The capsules encapsulate cells transfected with recombinant DNA molecules comprising DNA sequences coding for biologically active molecules operatively linked to promoters that are not subject to down regulation in vivo upon implantation into a mammalian host. The devices provide for the delivery of the molecules from living cells to specific sites within a recipient. In addition, see U.S. Patent Nos. 4,892,538, 5,011,472, and 5,106,627. A system for encapsulating living cells is described in PCT Application no. PCT/US91/00157 of Aebischer et *al.* See also, PCT Application no. PCT/US91/00155 of Aebischer et al., Winn et al., Exper. Neurol., 113:322-329 (1991), Aebischer et al., Exper. Neurol., 111:269-275 (1991); and Tresco et al., ASAIO, 38:17-23 (1992).

In vivo and *in vitro* gene therapy delivery of C3b/C4b CR-like polypeptides is also envisioned. One example of a gene therapy technique is to use the C3b/C4b CR-like gene (either genomic DNA, cDNA, and/or synthetic DNA) encoding a C3b/C4b CR-like polypeptide which may be operably linked to a constitutive or inducible promoter to form a "gene therapy DNA construct". The promoter may be homologous or heterologous to the endogenous C3b/C4b CR-like gene, provided that it is active in the cell or tissue type into which the' construct will be inserted. Other components of the gene therapy DNA construct may optionally include, DNA molecules designed for site-specific integration (*e.g.,* endogenous sequences useful for homologous recombination), tissue-specific promoter, enhancer(s) or silencer(s), DNA molecules capable of providing a selective advantage over the parent cell, DNA molecules useful as labels to identify transformed cells, negative selection systems, cell specific binding agents (as, for example, for cell targeting), cell-specific internalization factors, and transcription factors to enhance expression by a vector as well as factors to enable vector manufacture.

A gene therapy DNA construct can then be introduced into cells (either ex vivo or in vivo) using viral or non-viral vectors. One means for introducing the gene therapy DNA construct is by means of viral vectors as described herein. Certain vectors, such as retroviral vectors, will deliver the DNA construct to the chromosomal DNA of the cells, and the gene can integrate into the chromosomal DNA. Other vectors will function as episomes, and the gene therapy DNA construct will remain in the cytoplasm.

In yet other embodiments, regulatory elements can be included for the controlled expression of the C3b/C4b CR-like gene in the target cell. Such elements are turned on in response to an appropriate effector. In this way, a therapeutic polypeptide can be expressed when desired. One conventional control means involves the use of small molecule dimerizers or rapalogs (as described in WO9641865 (PCT/US96/099486); WO9731898 (PCT/US97/03137) and WO9731899 (FCT/US95/03157) used to dimerize chimeric proteins which contain a small molecule-binding domain and a domain capable of initiating biological process, such as a DNA-binding protein or transcriptional activation protein. The dimerization of the proteins can be used to initiate transcription of the transgene.

An alternative regulation technology uses a method of storing proteins expressed from the gene of interest inside the cell as an aggregate or cluster. The gene of interest is expressed as a fusion protein that includes a conditional aggregation domain which results in the retention of the aggregated protein in the endoplasmic reticulum. The stored proteins are stable and inactive inside the cell. The proteins can be released, however, by administering a drug (e.g., small molecule ligand) that removes the conditional aggregation domain and thereby specifically breaks apart the aggregates or clusters so that the proteins may be secreted from the cell. See, Science 287:816-817, and 826-830 (2000).

Other suitable control means or gene switches include, but are not limited to, the following systems. Mifepristone (RU486) is used as a progesterone antagonist. The binding of a modified progesterone receptor ligand-binding domain to the progesterone antagonist activates transcription by forming a dimer of two transcription factors which then pass into the nucleus to bind DNA. The ligand binding domain is modified to eliminate the ability of the receptor to bind to the natural ligand. The modified steroid hormone receptor system is further described in U.S. 5,364,791; WO9640911, and WO9710337.

Yet another control system uses ecdysone (a fruit fly steroid hormone) which binds to and activates an ecdysone receptor (cytoplasmic receptor). The receptor then translocates to the nucleus to bind a specific DNA response element (promoter from ecdysone-responsive ; gene). The ecdysone receptor includes a transactivation domain/DNA-binding domain/ligand-binding domain to initiate transcription. The ecdysone system is further described in U.S. 5,514,578; WO9738117; WO9637609; and WO9303162.

Another control means uses a positive tetracycline-controllable transactivator. This system involves a mutated tet repressor protein DNA-binding domain (mutated tet R-4 amino acid changes which resulted in a reverse tetracycline-regulated transactivator protein, i.e., it binds to a tet operator in the presence of tetracycline) linked to a polypeptide which activates transcription. Such systems are described in U.S. Patent Nos. 5,464,758; 5,650,298 and 5,654,168.

Additional expression control systems and nucleic acid constructs are described in U.S. Patent Nos. 5,741,679 and 5,834,186, to Innovir Laboratories Inc.

*In vivo* gene therapy may be accomplished by introducing the gene encoding a C3b/C4b CR-like polypeptide into cells via local injection of a C3b/C4b CR-like nucleic acid molecule or by other appropriate viral or non-viral delivery vectors. Hefti, Neurobiology, 25:1418-1435 (1994). For example, a nucleic acid molecule encoding a C3b/C4b CR-like polypeptide may be contained in an adeno-associated virus (AAV) vector for delivery to the targeted cells *(e.g.,* Johnson, International Publication No. WO95/34670; International Application No. PCT/US95/07178). The recombinant AAV genome typically contains AAV inverted terminal repeats flanking a DNA sequence encoding a C3b/C4b CR-like polypeptide operably linked to functional promoter and polyadenylation sequences.

Alternative suitable viral vectors include, but are not limited to, retrovirus, adenovirus, herpes simplex virus, lentivirus, hepatitis virus, parvovirus, papovavirus, poxvirus, alphavirus, coronavirus, rhabdovirus, paramyxovirus, and papilloma virus vectors. U.S. Patent No. 5,672,344 describes an in *vivo* viral-mediated gene transfer system involving a recombinant neurotrophic HSV-1 vector. U.S. Patent No. 5,399,346 provides examples of a process for providing a patient with a therapeutic protein by the delivery of human cells which have been treated in vitro to insert a DNA segment encoding a therapeutic protein. Additional methods and materials for the practice of gene therapy techniques are described in U.S. Patent No. 5,631,236 involving adenoviral vectors; U.S. Patent No. 5,672,510 involving retroviral vectors; and U.S. 5,635,399 involving retroviral vectors expressing cytokines.

Nonviral delivery methods include, but are not limited to, liposome-mediated transfer, naked DNA delivery (direct injection), receptor-mediated transfer (ligand-DNA complex), electroporation, calcium phosphate precipitation, and microparticle bombardment (e.g., gene gun). Gene therapy materials and methods may also include the use of inducible promoters, tissue-specific enhancer-promoters, DNA sequences designed for site-specific integration, DNA sequences capable of providing a selective advantage over the parent cell, labels to identify transformed cells, negative selection systems and expression control systems (safety measures), cell-specific binding agents (for cell targeting), cell-specific internalization factors, and transcription factors to enhance expression by a vector as well as methods of vector manufacture. Such additional methods and materials for the practice of gene therapy techniques are described in U.S. Patent No. 4,970,154 involving electroporation techniques; WO96/40958 involving nuclear ligands; U.S. Patent No. 5,679,559 describing a lipoprotein-containing system for gene delivery; U.S. Patent No. 5,676,954 involving liposome carriers; U.S. Patent No. 5,593,875 concerning methods for calcium phosphate transfection; and U.S. Patent No. 4,945,050 wherein biologically active particles are propelled at cells at a speed whereby the particles penetrate the surface of the cells and become incorporated into the interior of the cells.

It is also contemplated that C3b/C4b CR-like gene therapy or cell therapy can further include the delivery of one or more additional polypeptide(s) in the same or a different cell(s). Such cells may be separately introduced into the patient, or the cells may be contained in a single implantable device, such as the encapsulating membrane described above, or the cells may be separately modified by means of viral vectors.

A means to increase endogenous C3b/C4b CR-like polypeptide expression in a cell via gene therapy is to insert one or more enhancer elements into the C3b/C4b CR-like polypeptide promoter, where the enhancer element(s) can serve to increase transcriptional activity of the C3b/C4b CR-like gene. The enhancer element(s) used will be selected based on the tissue in which one desires to activate the gene(s); enhancer elements known to confer promoter activation in that tissue will be selected. For example, if a gene encoding a C3b/C4b CR-like polypeptide is to be "turned on" in T-cells, the lck promoter enhancer element may be used. Here, the functional portion of the transcriptional element to be added may be inserted into a fragment of DNA containing the C3b/C4b CR-like polypeptide promoter (and optionally, inserted into a vector and/or 5' and/or 3' flanking sequence(s), etc.) using standard cloning techniques. This construct, known as a "homologous recombination construct", can then be introduced into the desired cells either *ex vivo* or *in vivo.*

Gene therapy also can be used to decrease C3b/C4b CR-like polypeptide expression by modifying the nucleotide sequence of the endogenous promoter(s) . Such modification is typically accomplished via homologous recombination methods. For example, a DNA molecule containing all or a portion of the promoter of the C3b/C4b CR-like gene (s) selected for inactivation can be engineered to remove and/or replace pieces of the promoter that regulate transcription. For example the TATA box and/or the binding site of a transcriptional activator of the promoter may be deleted using standard molecular biology techniques; such deletion can inhibit promoter activity thereby repressing the transcription of the corresponding C3b/C4b CR-like gene. The deletion of the TATA box or the transcription activator binding site in the promoter may be accomplished by generating a DNA construct comprising all or the relevant portion of the C3b/C4b CR-like polypeptide promoter(s) (from the same or a related species as the C3b/C4b CR-like gene(s) to be regulated) in which one or more of the TATA box and/or transcriptional activator binding site nucleotides are mutated via substitution, deletion and/or insertion of one or more nucleotides. As a result, the TATA box and/or activator binding site has decreased activity or is rendered completely inactive. The construct will typically contain at least about 500 bases of DNA that correspond to the native (endogenous) 5' and 3' DNA sequences adjacent to the promoter segment that has been modified. The construct may be introduced into the appropriate cells (either ex *vivo* or in vivo) either directly or via a viral vector as described herein. Typically, the integration of the construct into the genomic DNA of the cells will be via homologous recombination, where the 5' and 3' DNA sequences in the promoter construct can serve to help integrate the modified promoter region via hybridization to the endogenous chromosomal DNA.

Additional Uses of C3b/C4b CR-like Nucleic Acids and Polypeptides

Nucleic acid molecules of the present invention (including those that do not themselves encode biologically active polypeptides) may be used to map the locations of the C3b/C4b CR-like gene and related genes on chromosomes. Mapping may be done by techniques known in the art, such as. PCR amplification and in situ hybridization.

C3b/C4b CR-like nucleic acid molecules (including those that do not themselves encode biologically active polypeptides), may be useful as hybridization probes in diagnostic assays to test, either qualitatively or quantitatively, for the presence of a C3b/C4b CR-like DNA or corresponding RNA in mammalian tissue or bodily fluid samples.

The C3b/C4b CR-like polypeptides may be used (simultaneously or sequentially) in combination with one or more cytokines, growth factors, antibiotics, anti-inflammatories, and/or chemotherapeutic agents as is appropriate for the indication being treated.

Other methods may also be employed where it is desirable to inhibit the activity of one or more C3b/C4b CR-like polypeptides. Such inhibition may be effected by nucleic acid molecules which are complementary to and hybridize to expression control sequences (triple helix formation) or to C3b/C4b CR-like mRNA. For example, antisense DNA or RNA molecules, which have a sequence that is complementary to at least a portion of the selected C3b/C4b CR-like gene(s) can be introduced into the cell. Anti-sense probes may be designed by available techniques using the sequence of C3b/C4b CR-like polypeptide disclosed herein. Typically, each such antisense molecule will be complementary to the start site (5' end) of each selected C3b/C4b CR-like gene. When the antisense molecule then hybridizes to the corresponding C3b/C4b CR-like mRNA, translation of this mRNA is prevented or reduced. Anti-sense inhibitors provide information relating to the decrease or absence of a C3b/C4b CR-like polypeptide in a cell or organism.

Alternatively, gene therapy may be employed to create a dominant-negative inhibitor of one or more C3b/C4b CR-like polypeptides. In this situation, the DNA encoding a mutant polypeptide of each selected C3b/C4b CR-like polypeptide can be prepared and introduced into the cells of a patient using either viral or non-viral methods as described herein. Each such mutant is typically designed to compete with endogenous polypeptide in its biological role.

In addition, a C3b/C4b CR-like polypeptide, whether biologically active or not, may be used as an immunogen, that is, the polypeptide contains at least one epitope to which antibodies may be raised. Selective binding agents that bind to a C3b/C4b CR-like polypeptide (as described herein) may be used for in vivo and in vitro diagnostic purposes, including, but not limited to, use in labeled form to detect the presence of C3b/C4b CR-like polypeptide in a body fluid or cell sample. The antibodies may also be used to prevent, treat, or diagnose a number of diseases and disorders, including those recited herein. The antibodies may bind to a C3b/C4b CR-like polypeptide so as to diminish or block at least one activity characteristic of a C3b/C4b CR-like polypeptide, or may bind to a polypeptide to increase at least one activity characteristic of a C3b/C4b CR-like polypeptide (including by increasing the pharmacokinetics of the C3b/C4b CR-like polypeptide).

## Claims

**1.** An isolated nucleic acid molecule comprising a nucleotide sequence selected from the group consisting of:

(a) the nucleotide sequence as set forth in SEQ ID NO:1, SEQ ID NO:3, or SEQ ID NO:6;
(b) a nucleotide sequence encoding the polypeptide as set forth in SEQ ID NO:2, SEQ ID NO:4, or SEQ ID NO:7;
(c) a nucleotide sequence which hybridizes under moderately or highly stringent conditions to the complement of (a) or (b), wherein the encoded polypeptide has an activity of the polypeptide as set forth in SEQ ID NO:2, SEQ ID NO:4, or SEQ ID NO:7; and
(d) a nucleotide sequence complementary to any of (a)-(c).

**2.** An isolated nucleic acid molecule comprising a nucleotide sequence selected from the group consisting of:

(a) a nucleotide sequence encoding a polypeptide that is at least about 70, 75, 80, 85, 90, 95, 96, 97, 98, or 99 percent identical to the polypeptide as set forth in SEQ ID NO:2, SEQ ID NO:4, or SEQ ID NO:7, wherein the polypeptide has an activity of the polypeptide as set forth in SEQ ID NO:2, SEQ ID NO:4, or SEQ ID NO:7;
(b) a nucleotide sequence encoding an allelic variant or splice variant of the nucleotide sequence as set forth in SEQ ID NO:1, SEQ ID NO:3, or SEQ ID NO:6, wherein the encoded polypeptide has an activity of the polypeptide as set forth in SEQ ID NO:2, SEQ ID NO:4, or SEQ ID NO:7;
(c) a nucleotide sequence of SEQ ID NO:1, SEQ ID NO:3, or SEQ ID NO:6; (a); or (b) encoding a polypeptide fragment of at least about 25 amino acid residues, wherein the polypeptide has an activity of the polypeptide as set forth in SEQ ID NO:2, SEQ ID NO:4, or SEQ ID NO:7;
(d) a nucleotide sequence of SEQ ID NO:1, SEQ ID NO:3, or SEQ ID NO:6, or (a)-(c) comprising a fragment of at least about 16 nucleotides;
(e) a nucleotide sequence which hybridizes under moderately or highly stringent conditions to the complement of any of (a)-(d), wherein the polypeptide has an activity of the polypeptide as set forth in SEQ ID NO:2, SEQ ID NO:4, or SEQ ID NO:7; and
(f) a nucleotide sequence complementary to any of (a)-(c) .

**3.** An isolated nucleic acid molecule comprising a nucleotide sequence selected from the group consisting of:

(a) a nucleotide sequence encoding a polypeptide as set forth in SEQ ID NO:2, SEQ ID NO: 4, or SEQ ID NO: 7, with at least one conservative amino acid substitution, wherein the polypeptide has an activity of the polypeptide as set forth in SEQ ID NO:2, SEQ ID NO:4, or SEQ ID NO:7;
(b) a nucleotide sequence encoding a polypeptide as set forth in SEQ ID NO:2, SEQ ID NO:4, or SEQ ID NO: 7 with at least one amino acid insertion, wherein the polypeptide has an activity of the polypeptide as set forth in SEQ ID NO:2, SEQ ID NO:4, or SEQ ID NO:7;
(c) a nucleotide sequence encoding a polypeptide as set forth in SEQ ID NO:2, SEQ ID NO:4, or SEQ ID NO: 7 with at least one amino acid deletion, wherein the polypeptide has an activity of the polypeptide as set forth in SEQ ID NO:2, SEQ ID NO:4, or SEQ ID-NO:7;
(d) a nucleotide sequence encoding a polypeptide as set forth in SEQ ID NO:2, SEQ ID NO:4, or SEQ ID NO: 7 which has a C- and/or N- terminal truncation, wherein the polypeptide has an activity of the polypeptide as set forth in SEQ ID NO:2, SEQ ID NO:4, or SEQ ID NO:7;
(e) a nucleotide sequence encoding a polypeptide as set forth in SEQ ID NO:2, SEQ ID NO:4, or SEQ ID NO: 7 with at least one'modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2, SEQ ID NO:4, or SEQ ID NO:7;
(f) a nucleotide sequence of (a)-(e) comprising a fragment of at least about 16 nucleotides;
(g) a nucleotide sequence which hybridizes under moderately or highly stringent conditions to the complement of any of (a)-(f), wherein the polypeptide has an activity of the polypeptide as set forth in SEQ ID NO:2, SEQ ID NO:4, or SEQ ID NO:7; and
(h) a nucleotide sequence complementary to any of (a) - (e).

**4.** A vector comprising the nucleic acid molecule of Claims 1, 2, or 3.

**5.** A host cell comprising the vector of Claim 4.

**6.** The host cell of Claim 5 that is a eukaryotic cell.

**7.** The host cell of Claim 5 that is a prokaryotic cell.

**8.** A process of producing a C3b/C4b CR-like polypeptide comprising culturing the host cell of Claim 5 under suitable conditions to express the polypeptide, and optionally isolating the polypeptide from the culture.

**9.** A polypeptide produced by the process of Claim 8.

**10.** The process of Claim 8, wherein the nucleic acid molecule comprises promoter DNA other than the promoter DNA for the native C3b/C4b CR-like polypeptide operatively linked to the DNA encoding the C3b/C4b CR-like polypeptide.

**11.** The isolated nucleic acid molecule according to Claim 2 wherein the percent identity is determined using a computer program selected from the group consisting of GAP, BLASTP, BLASTN, FASTA, BLASTA, BLASTX, BestFit, and the Smith-Waterman algorithm.

**12.** A process for determining whether a compound inhibits C3b/C4b CR-like polypeptide activity or production comprising exposing a cell according to Claims 5, 6, or 7 to the compound, and measuring C3b/C4b CR-like polypeptide activity or production in said cell.

**13.** An isolated polypeptide comprising the amino acid sequence set forth in SEQ ID NO:2, SEQ ID NO:4, or SEQ ID NO:7.

**14.** An isolated polypeptide comprising the amino acid sequence selected from the group consisting of:

(a) an amino acid sequence of the mature C3b/C4b CR-like polypeptide wherein the mature polypeptide comprises the amino acid sequence contained within SEQ ID NO:2, SEQ ID NO:4, or SEQ ID NO:7, and optionally further comprises an amino-terminal methionine;
(b) an amino acid sequence for an ortholog of SEQ ID NO:2, SEQ ID NO:4, or SEQ ID NO:7, wherein the encoded polypeptide has an activity of the polypeptide as set forth in SEQ ID NO:2, SEQ ID NO:4, or SEQ ID NO:7;
(c) an amino acid sequence that is at least about 70, 80, 85, 90, 95, 96, 97, 98, or 99 percent identical to the amino acid sequence of SEQ ID NO:2, SEQ ID NO:4, or SEQ ID NO:7, wherein the polypeptide has an activity of the polypeptide as set forth in SEQ ID NO:2, SEQ ID NO:4, or SEQ ID NO:7;
(d) a fragment of the amino acid sequence set forth in SEQ ID NO:2, SEQ ID NO:4, or SEQ ID NO:7 comprising at least about 25 amino acid residues,
wherein the polypeptide has an activity of the polypeptide as set forth in SEQ ID NO:2, SEQ ID NO:4, or SEQ ID NO:7;
(e) an amino acid sequence for an allelic variant or splice variant of either the amino acid sequence as set forth in SEQ ID NO:2, SEQ ID NO:4, or SEQ ID NO: 7, or at least one of (a)-(c) wherein the polypeptide has an activity of the polypeptide as set forth in SEQ ID NO:2, SEQ ID NO:4, or SEQ ID NO:7.

**15.** An isolated polypeptide comprising the amino acid sequence selected from the group consisting of:

(a) the amino acid sequence as set forth in SEQ ID NO:2, SEQ ID NO:4, or SEQ ID NO:7 with at least one conservative amino acid substitution, wherein the polypeptide has an activity of the polypeptide as set forth in SEQ ID NO:2, SEQ ID NO:4, or SEQ ID NO:7;
(b) the amino acid sequence as set forth in SEQ ID NO:2, SEQ ID NO:4, or SEQ ID NO:7 with at least one amino acid insertion, wherein the polypeptide has an activity of the polypeptide as set forth in SEQ ID NO:2, SEQ ID NO:4, or SEQ ID NO:7;
(c) the amino acid sequence as set forth in SEQ ID NO:2, SEQ ID NO:4, or SEQ ID NO:7 with at least one amino acid deletion, wherein the polypeptide has an activity of the polypeptide as set forth in SEQ ID NO:2, SEQ ID NO:4, or SEQ ID NO:7;
(d) the amino acid sequence as set forth in SEQ ID NO:2, SEQ ID NO:4, or SEQ ID NO:7 which has a C- and/or N-terminal truncation, wherein the polypeptide has an activity of the polypeptide as set forth in SEQ ID NO:2, SEQ ID NO:4, or SEQ ID NO:7; and
(e) the amino acid sequence as set forth in SEQ ID NO:2, SEQ ID NO:4, or SEQ ID NO:7, with at least one modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2, SEQ ID NO:4, or SEQ ID NO:7.

**16.** An isolated polypeptide encoded by the nucleic acid molecule of.Claims 1, 2, or 3.

**17.** The isolated polypeptide according to Claim 14 wherein the percent identity is determined using a computer program selected from the group consisting of GAP, BLASTP, BLASTN, FASTA, BLASTA, BLASTX, BestFit, and the Smith-Waterman algorithm.

**18.** An antibody produced by immunizing an animal with a peptide comprising an amino acid sequence of SEQ ID NO: 2, SEQ ID NO:4, or SEQ ID NO:7.

**19.** An antibody or fragment thereof that specifically binds the polypeptide of Claims 13, 14, or 15.

**20.** The antibody of Claim 19 that is a monoclonal antibody.

**21.** A hybridoma that produces a monoclonal antibody that binds to a peptide comprising an amino acid sequence of SEQ ID NO:2, SEQ ID NO:4, or SEQ ID NO:7.

**22.** A method of detecting or quantitating the amount of C3b/C4b CR-like polypeptide using the antiC3b/C4b CR-like antibody or fragment of Claims 18, 19, or 20.

**23.** A selective binding agent or fragment thereof that specifically binds at least one polypeptide wherein said polypeptide comprises the amino acid' sequence selected from the group consisting of:

a) the amino acid sequence as set forth in SEQ ID NO:2, SEQ ID NO:4, or SEQ ID NO:7; and
b) a fragment of the amino acid sequence set forth in at least one of SEQ ID NO:2, SEQ ID NO:4, or SEQ ID NO:7; and
c) a naturally occurring variant of (a) or (b).

**24.** The selective binding agent of Claim 23 that is an antibody or fragment thereof.

**25.** The selective binding agent of Claim 23 that is a humanized antibody.

**26.** The selective binding agent of Claim 23 that is a human antibody or fragment thereof.

**27.** The selective binding agent of Claim 23 that is a polyclonal antibody or fragment thereof.

**28.** The selective binding agent Claim 23 that is a monoclonal antibody or fragment thereof.

**29.** The selective binding agent of Claim 23 that is a chimeric antibody or fragment thereof.

**30.** The selective binding agent of Claim 23 that is a CDR-grafted antibody or fragment thereof.

**31.** The selective binding agent of Claim 23 that is an antiidiotypic antibody or fragment thereof.

**32.** The selective binding agent of Claim 23 which is a variable region fragment.

**33.** The variable region fragment of Claim 32 which is a Fab or a Fab' fragment.

**34.** A selective binding agent or fragment thereof comprising at least one complementarity determining region with specificity for a polypeptide having the amino acid sequence of SEQ ID NO:2, SEQ ID NO:4, or SEQ ID NO:7.

**35.** The selective binding agent of Claim 23 which is bound to a detectable label.

**36.** The selective binding agent of Claim 23 which antagonizes C3b/C4b CR-like polypeptide biological activity.

**37.** A method for treating, preventing, or ameliorating a disease, condition, or disorder comprising administering to a patient an effective amount of a selective binding agent according to Claim 23.

**38.** A selective binding agent produced by immunizing an animal with a polypeptide comprising an amino acid sequence selected from the group consisting of SEQ ID NO:2, SEQ ID NO:4, or SEQ ID NO:7.

**39.** A hybridoma that produces a selective binding agent capable of binding a polypeptide according to Claims 1, 2, or 3.

**40.** A composition comprising the polypeptide of Claims 13, 14, or 15 and a pharmaceutically acceptable formulation agent.

**41.** The composition of Claim 40 wherein the pharmaceutically acceptable formulation agent is a carrier, adjuvant, solubilizer, stabilizer, or antioxidant.

**42.** The composition of Claim 40 wherein the polypeptide comprises the mature amino acid sequence portion of SEQ ID NO:2, SEQ ID NO:4, or SEQ ID NO:7.

**43.** A polypeptide comprising a derivative of the polypeptide of Claims 13, 14, or 15.

**44.** The polypeptide of Claim 43 which is covalently modified with a water-soluble polymer.

**45.** The polypeptide ot Claim 44 wherein the water-soluble polymer is selected from the group consisting of polyethylene glycol, monomethoxy-polyethylene glycol, dextran, cellulose, poly-(N-vinyl pyrrolidone) polyethylene glycol, propylene glycol homopolymers, polypropylene oxide/ethylene oxide co-polymers, polyoxyethylated polyols, and polyvinyl alcohol.

**46.** A composition comprising a nucleic acid molecule of Claims 1, 2, or 3 and a pharmaceutically acceptable formulation agent.

**47.** A composition of Claim 46 wherein said nucleic acid molecule is contained in a viral vector.

**48.** A viral vector comprising a nucleic acid molecule of Claims 1, 2, or 3.

**49.** A fusion polypeptide comprising the polypeptide of Claims 13, 14, or 15 fused to a heterologous amino acid sequence.

**50.** The fusion polypeptide of Claim 49 wherein the heterologous amino acid sequence is an IgG constant domain or fragment thereof.

**51.** A method for treating, preventing or ameliorating a medical condition comprising administering to a patient the polypeptide of Claims 13, 14, or 15 or the polypeptide encoded by the nucleic acid of Claims 1, 2, or 3.

**52.** A method of diagnosing a pathological condition or a susceptibility to a pathological condition in a subject comprising:

(a) determining the presence or amount of expression of the polypeptide of Claims 13, 14, or 15 or the polypeptide encoded by the nucleic acid molecule of Claims 1, 2, or 3 in a sample; and
(b) diagnosing a pathological condition or a susceptibility to a pathological condition based on the presence or amount of expression of the polypeptide.

**53.** A device, comprising:

(a) a membrane suitable for implantation; and
(b) cells encapsulated within said membrane, wherein said cells secrete a protein of Claims 13, 14, or 15, and wherein said membrane is permeable to said protein and impermeable to materials detrimental to said cells.

**54.** A method of identifying a compound which binds to a polypeptide comprising:

(a) contacting the polypeptide of Claims 13, 14, or 15 with a compound; and
(b) determining the extent of binding of the polypeptide to the compound.

**55.** A method of modulating levels of a polypeptide in an animal comprising administering to the animal the nucleic acid molecule of Claims 1, 2, or 3.

**56.** A transgenic non-human mammal comprising the nucleic acid molecule of Claims 1, 2, or 3.

# Figure 1A

## Map of Human C3b/C4b Complement Receptor like cDNA (SEQ ID NO:1) and Amino Acid Sequences (SEQ ID NO:2)

```
   1  CCTGGGGAAGCCTCTCGGTTCCAGGAAAATGGGATGGTTGATTGCCCTAAATTGATTTTT   60
  61  TAAAAGAAAATTCACGAATTGGCAGCCATAGAATAGAGTAATTTCTGTAAAGCACCAGTG  120
 121  ATAGTGATGTTTGAATATTAATATAATGGACCAGAGGCTGTACAGTCTTTGAAAGAGGGT  180
 181  CTTGCTACCTATATATCTAGGGTTTGGCTGTTTAAAGCAGCAAGACCCTCCTTTCAGGTG  240
 241  GAAGTCGATGTACTTGTTGCCTTACCTAAAAGCTTTGACATTTCTCTTTCTTGCAGGCTC  300
 301  ACGGGATCCAGTGTTCCTGACCTCATTGTGAGCATGAGCAACCAGATGTGGCTACATCTG  360
   1                                  M  S  N  Q  M  W  L  H  L    9

 361  CAGTCGGATGATAGCATTGGCTCACCTGGGTTTAAAGCTGTTTACCAAGAAATTGAAAAG  420
  10  Q  S  D  D  S  I  G  S  P  G  F  K  A  V  Y  Q  E  I  E  K   29

 421  GGAGGGTGTGGGGATCCTGGAATCCCCGCCTATGGGAAGCGGACGGGCAGCAGTTTCCTC  480
  30  G  G  C  G  D  P  G  I  P  A  Y  G  K  R  T  G  S  S  F  L   49

 481  CATGGAGATACACTCACCTTTGAATGCCCGGCGGCCTTTGAGCTGGTGGGGGAGAGAGTT  540
  50  H  G  D  T  L  T  F  E  C  P  A  A  F  E  L  V  G  E  R  V   69

 541  ATCACCTGTCAGCAGAACAATCAGTGGTCTGGCAACAAGCCCAGCTGTGTATTTTCATGT  600
  70  I  T  C  Q  Q  N  N  Q  W  S  G  N  K  P  S  C  V  F  S  C   89

 601  TTCTTCAACTTTACGGCATCATCTGGGATTATTCTGTCACCAAATTATCCAGAGGAATAT  660
  90  F  F  N  F  T  A  S  S  G  I  I  L  S  P  N  Y  P  E  E  Y  109

 661  GGGAACAACATGAACTGTGTCTGGTTGATTATCTCGGAGCCAGGAAGTCGAATTCACCTA  720
 110  G  N  N  M  N  C  V  W  L  I  I  S  E  P  G  S  R  I  H  L  129

 721  ATCTTTAATGATTTTGATGTTGAGCCTCAATTTGACTTTCTCGCGGTCAAGGATGATGGC  780
 130  I  F  N  D  F  D  V  E  P  Q  F  D  F  L  A  V  K  D  D  G  149

 781  ATTTCTGACATAACTGTCCTGGGTACTTTTTCTGGCAATGAAGTGCCTTCCCAGCTGGCC  840
 150  I  S  D  I  T  V  L  G  T  F  S  G  N  E  V  P  S  Q  L  A  169

 841  AGCAGTGGGCATATAGTTCGCTTGGAATTTCAGTCTGACCATTCCACTACTGGCAGAGGG  900
 170  S  S  G  H  I  V  R  L  E  F  Q  S  D  H  S  T  T  G  R  G  189

 901  TTCAACATCACTTACACCACATTTGGTCAGAATGAGTGCCATGATCCTGGCATTCCTATA  960
 190  F  N  I  T  Y  T  T  F  G  Q  N  E  C  H  D  P  G  I  P  I  209

 961  AACGGACGACGTTTTGGTGACAGGTTTCTACTCGGGAGCTCGGTTTCTTTCCACTGTGAT 1020
 210  N  G  R  R  F  G  D  R  F  L  L  G  S  S  V  S  F  H  C  D  229

1021  GATGGCTTTGTCAAGACCCAGGGATCCGAGTCCATTACCTGCATACTGCAAGACGGGAAC 1080
 230  D  G  F  V  K  T  Q  G  S  E  S  I  T  C  I  L  Q  D  G  N  249

1081  GTGGTCTGGAGCTCCACCGTGCCCCGCTGTGAAGCTCCATGTGGTGGACATCTGACAGCG 1140
 250  V  V  W  S  S  T  V  P  R  C  E  A  P  C  G  G  H  L  T  A  269

1141  TCCAGCGGAGTCATTTTGCCTCCTGGATGGCCAGGATATTATAAGGATTCTTTACATTGT 1200
 270  S  S  G  V  I  L  P  P  G  W  P  G  Y  Y  K  D  S  L  H  C  289

1201  GAATGGATAATTGAAGCAAAACCAGGCCACTCTATCAAAATAACTTTTGACAGATTTCAG 1260
 290  E  W  I  I  E  A  K  P  G  H  S  I  K  I  T  F  D  R  F  Q  309
```

Figure 1B

```
1261  ACAGAGGTCAATTATGACACCTTGGAGGTCAGAGATGGGCCAGCCAGTTCGTCCCCACTG  1320
 310  T  E  V  N  Y  D  T  L  E  V  R  D  G  P  A  S  S  S  P  L    329

1321  ATCGGCGAGTACCACGGCACCCAGGCACCCCAGTTCCTCATCAGCACCGGGAACTTCATG  1380
 330  I  G  E  Y  H  G  T  Q  A  P  Q  F  L  I  S  T  G  N  F  M    349

1381  TACCTGCTATTCACCACTGACAACAGCCGCTCCAGCATCGGCTTCCTCATCCACTATGAG  1440
 350  Y  L  L  F  T  T  D  N  S  R  S  S  I  G  F  L  I  H  Y  E    369

1441  AGTGTGACGCTTGAGTCGGATTCCTGCCTGGACCCGGGCATCCCTGTGAACGRCCATCGC  1500
 370  S  V  T  L  E  S  D  S  C  L  D  P  G  I  P  V  N  X  H  R    389

1501  CACGGTGGAGACTTTGGCATCAGGTCCACAGTGACTTTCAGCTGTGACCCGGGGTACACA  1560
 390  H  G  G  D  F  G  I  R  S  T  V  T  F  S  C  D  P  G  Y  T    409

1561  CTAAGTGACGACGAGCCCCTCGTCTGTGAGAGGAACCACCAGTGGAACCACGCCTTGCCC  1620
 410  L  S  D  D  E  P  L  V  C  E  R  N  H  Q  W  N  H  A  L  P    429

1621  AGCTGCGACGCTCTATGTGGAGGCTACATCCAAGGGAAGAGTGGAACAGTCCTTTCTCCT  1680
 430  S  C  D  A  L  C  G  G  Y  I  Q  G  K  S  G  T  V  L  S  P    449

1681  GGGTTTCCAGATTTTTATCCAAACTCTCTAAACYGCACGTGGACCATTGAAGTGTCTCAT  1740
 450  G  F  P  D  F  Y  P  N  S  L  N  X  T  W  T  I  E  V  S  H    469

1741  GGGAAAGGAGTTCAAATGATCTTTCACACCTTTCATCTTGAGAGTTCCCACGACTATTTA  1800
 470  G  K  G  V  Q  M  I  F  H  T  F  H  L  E  S  S  H  D  Y  L    489

1801  CTGATCACAGAGGATGGAAGTTTTTCCGAGCCCGTTGCCAGGCTCACCGGGTCGGTGTTG  1860
 490  L  I  T  E  D  G  S  F  S  E  P  V  A  R  L  T  G  S  V  L    509

1861  CCTCATACGATCAAGGCAGGCCTGTTTGGAAACTTCACTGCCCAGCTTCGGTTTATATCA  1920
 510  P  H  T  I  K  A  G  L  F  G  N  F  T  A  Q  L  R  F  I  S    529

1921  GACTTCTCAATTTCGTACGAGGGCTTCAATATCACATTTTCAGAATATGACCTGGAGCCA  1980
 530  D  F  S  I  S  Y  E  G  F  N  I  T  F  S  E  Y  D  L  E  P    549

1981  TGTGATGATCCTGGAGTCCCTGCCTTCAGCCGAAGAATTGGTTTTCACTTTGGTGTGGGA  2040
 550  C  D  D  P  G  V  P  A  F  S  R  R  I  G  F  H  F  G  V  G    569

2041  GACTCTCTGACGTTTTCCTGCTTCCTGGGATATCGTTTAGAAGGTGCCRCCAAGCTTACC  2100
 570  D  S  L  T  F  S  C  F  L  G  Y  R  L  E  G  A  X  K  L  T    589

2101  TGCCTGGGTGGGGGCCGCCGTGTGTGGAGTGCACCTCTGCCAAGGTGTGTGGCCGAATGT  2160
 590  C  L  G  G  G  R  R  V  W  S  A  P  L  P  R  C  V  A  E  C    609

2161  GGAGCAAGTGTCAAAGGAAATGAAGGAACATTACTGTCTCCAAATTTTCCATCCAATTAT  2220
 610  G  A  S  V  K  G  N  E  G  T  L  L  S  P  N  F  P  S  N  Y    629

2221  GATAATAACCATGAGTGTATCTATAAAATAGAAACAGAAGCCGGCAAGGGCATCCACCTT  2280
 630  D  N  N  H  E  C  I  Y  K  I  E  T  E  A  G  K  G  I  H  L    649

2281  AGAACACGAAGCTTCCAGCTGTTTGAAGGAGATACTCTAAAGGTATATGATGGAAAAGAC  2340
 650  R  T  R  S  F  Q  L  F  E  G  D  T  L  K  V  Y  D  G  K  D    669

2341  AGTTCCTCACGTCCACTGGGCACGTTCACTAAAAATGAACTTCTGGGGCTGATCCTAAAC  2400
 670  S  S  S  R  P  L  G  T  F  T  K  N  E  L  L  G  L  I  L  N    689
```

Figure 1C

2401 AGCACATCCAATCACCTRTGGCTAGAGTTCAACACCAATGGATCTGACACCGACCAAGGT 2460
690 S T S N H L W L E F N T N G S D T D Q G 709

2461 TTTCAACTCACCTATACCAGTTTTGATCTGGTAAAATGTGAGGATCCGGGCATCCCTAAC 2520
710 F Q L T Y T S F D L V K C E D P G I P N 729

2521 TACGGCTATAGGATCCGTGATGAAGGCCACTTTACCGACACTGTAGTTCTGTACAGTTGC 2580
730 Y G Y R I R D E G H F T D T V V L Y S C 749

2581 AACCCGGGGTACGCCATGCATGGCAGCAACACCCTGACCTGTTTGAGTGGAGACAGGAGA 2640
750 N P G Y A M H G S N T L T C L S G D R R 769

2641 GTGTGGGACAAACCACTACCTTCGTGCATAGCGGAATGTGGTGGTCAGATCCATGCAGCC 2700
770 V W D K P L P S C I A E C G G Q I H A A 789

2701 ACATCAGGACGAATATTGTCCCCTGGCTATCCAGCTCCGTATGACAACAACCTCCACTGC 2760
790 T S G R I L S P G Y P A P Y D N N L H C 809

2761 ACCTGGATTATAGAGGCAGACCCAGGAAAGACCATTAGCCTCCATTTCATTGTTTTCGAC 2820
810 T W I I E A D P G K T I S L H F I V F D 829

2821 ACGGAGATGGCTCACGACATCCTCAAGGTCTGGGACGGGCCGGTGGACAGTGACATCCTG 2880
830 T E M A H D I L K V W D G P V D S D I L 849

2881 CTGAAGGAGTGGAGTGGCTCCGCCCTTCCGGAGGACATCCACAGCACCTTCAACTCACTC 2940
850 L K E W S G S A L P E D I H S T F N S L 869

2941 ACCCTGCAGTTCGACAGCGACTTCTTCATCAGCAAGTCTGGCTTCTCCATCCAGTTCTCC 3000
870 T L Q F D S D F F I S K S G F S I Q F S 889

3001 ACCTCAATTGCAGCCACCTGTAACGATCCAGGTATGCCCCAAAATGGCACCCGCTATGGA 3060
890 T S I A A T C N D P G M P Q N G T R Y G 909

3061 GACAGCAGAGAGGCTGGAGACACCGTCACATTCCAGTGTGACCCTGGCTATCAGCTCCAA 3120
910 D S R E A G D T V T F Q C D P G Y Q L Q 929

3121 GGACAAGCCAAAATCACCTGTGTGCAGCTGAATAACCGGTTCTTTTGGCAACCAGACCCT 3180
930 G Q A K I T C V Q L N N R F F W Q P D P 949

3181 CCTACATGCATAGCTGCTTGTGGAGGGAATCTGACGGGCCCAGCAGGTGTTATTTTGTCA 3240
950 P T C I A A C G G N L T G P A G V I L S 969

3241 CCCAACTACCCACAGCCGTATCCTCCTGGGAAGGAATGTGACTGGAGAGTAAAAGTGAAC 3300
970 P N Y P Q P Y P P G K E C D W R V K V N 989

3301 CCGGACTTTGTCATCGCCTTGATATTCAAAAGTTTCAACATGGAGCCCAGCTATGACTTC 3360
990 P D F V I A L I F K S F N M E P S Y D F 1009

3361 CTACACATCTATGAAGGGGAAGATTCCAACAGCCCCCTCATTGGGAGTTACCAGGGCTCT 3420
1010 L H I Y E G E D S N S P L I G S Y Q G S 1029

3421 CAGGCCCCAGAAAGAATAGAGAGTAGCGGAAACAGCCTGTTTCTGGCATTTCGGAGTGAT 3480
1030 Q A P E R I E S S G N S L F L A F R S D 1049

3481 GCCTCCGTGGGCCTTTCAGGGTTCGCCATTGAATTTAAAGAGAAACCACGGGAAGCTTGT 3540
1050 A S V G L S G F A I E F K E K P R E A C 1069

Figure 1D

3541 TTTGACCCAGGAAATATAATGAATGGGACAAGAGTTGGAACAGACTTCAAGCTTGGCTCC 3600
1070 F D P G N I M N G T R V G T D F K L G S 1089

3601 ACCATCACCTACCAGTGTGACTCTGGCTATAAGATTCTTGACCCCTCATCCATCACCTGT 3660
1090 T I T Y Q C D S G Y K I L D P S S I T C 1109

3661 GTGATTGGGGCTGATGGGAAACCCTCCTGGGACCAAGTGCTGCCCTCCTGCAATGCTCCC 3720
1110 V I G A D G K P S W D Q V L P S C N A P 1129

3721 TGTGGAGGCCAGTACACGGGATCAGAAGGGGTAGTTTTATCACCAAACTACCCCCATAAT 3780
1130 C G G Q Y T G S E G V V L S P N Y P H N 1149

3781 TACACAGCTGGTCAAATATGCCTCTATTCCATCACGGTACCAAAGGAATTCGTGGTCTTT 3840
1150 Y T A G Q I C L Y S I T V P K E F V V F 1169

3841 GGACAGTTTGCCTATTTCCAGACAGCCCTGAATGATTTGGCAGAATTATTTGATGGAACC 3900
1170 G Q F A Y F Q T A L N D L A E L F D G T 1189

3901 CATGCACAGGCCAGACTTCTCAGCTCACTCTCGGGGTCTCACTCAGGGGAAACATTGCCC 3960
1190 H A Q A R L L S S L S G S H S G E T L P 1209

3961 TTGGCTACGTCAAATCAAATTCTGCTCCGATTCAGTGCAAAGAGCGGTGCCTCTGCCCGC 4020
1210 L A T S N Q I L L R F S A K S G A S A R 1229

4021 GGCTTCCACTTCGTGTATCAAGCTGTTCCTCGTACCAGTGACACCCAATGCAGCTCTGTC 4080
1230 G F H F V Y Q A V P R T S D T Q C S S V 1249

4081 CCCGAGCCCAGATACGGAAGGAGAATTGGTTCTGAGTTTTCTGCCGGCTCCATCGTCCGA 4140
1250 P E P R Y G R R I G S E F S A G S I V R 1269

4141 TTCGAGTRCAACCCGGGATACCTGCTTCAGGGTTCCACGGCGCTCCACTGCCAGTCCGTG 4200
1270 F E X N P G Y L L Q G S T A L H C Q S V 1289

4201 CCCAACGCCTTGGCACAGTGGAACGACACGATCCCCAGCTGTGTGGTACCCTGCAGTGGC 4260
1290 P N A L A Q W N D T I P S C V V P C S G 1309

4261 AATTTCACTCAACGAAGAGGTACAATCCTGTCCCCCGGCTACCCTGAGCCATACGGAAAC 4320
1310 N F T Q R R G T I L S P G Y P E P Y G N 1329

4321 AACTTGAACTGTATATGGAAGATCATAGTTACGGAGGGCTCGGGAATTCAGATCCAAGTG 4380
1330 N L N C I W K I I V T E G S G I Q I Q V 1349

4381 ATCAGTTTTGCCACGGAGCAGAACTGGGACTCCCTTGAGATCCACGATGGTGGGGATGTG 4440
1350 I S F A T E Q N W D S L E I H D G G D V 1369

4441 ACCGCACCCAGACTGGGAAGCTTCTCAGGCACCACAGTACCGGCACTGCTGAACAGTACT 4500
1370 T A P R L G S F S G T T V P A L L N S T 1389

4501 TCCAACCAACTCTACCTGCATTTCCAGTCTGACATTAGTGTGGCAGCTGCTGGTTTCCAC 4560
1390 S N Q L Y L H F Q S D I S V A A A G F H 1409

4561 CTGGAATACAAAACTGTAGGTCTTGCTGCATGCCAAGAACCAGCCCTCCCCAGCAACAGC 4620
1410 L E Y K T V G L A A C Q E P A L P S N S 1429

4621 ATCAAAATCGGAGATCGGTACATGGTGAACGACGTGCTCTCCTTCCAGTGCGAGCCCGGG 4680
1430 I K I G D R Y M V N D V L S F Q C E P G 1449

Figure 1E

```
4681  TACACCCTGCAGGGCCGTTCCCACATTTCCTGTATGCCAGGGACCGTTCGCCGTTGGAAC  4740
1450  Y  T  L  Q  G  R  S  H  I  S  C  M  P  G  T  V  R  R  W  N   1469

4741  TATCCGTCTCCCCTGTGCATTGCAACCTGTGGAGGGACGCTGAGCACCTTGGGTGGTGTG  4800
1470  Y  P  S  P  L  C  I  A  T  C  G  G  T  L  S  T  L  G  G  V   1489

4801  ATCCTGAGCCCCGGCTTCCCAGGTTCTTACCCCAACAACTTAGACTGCACCTGGAGGATC  4860
1490  I  L  S  P  G  F  P  G  S  Y  P  N  N  L  D  C  T  W  R  I   1509

4861  TCATTACCCATCGGCTATGGTGCACATATTCAGTTTCTGAATTTTTCTACCGAAGCTAAT  4920
1510  S  L  P  I  G  Y  G  A  H  I  Q  F  L  N  F  S  T  E  A  N   1529

4921  CATGACTTCCTTGAAATTCAAAATGGACCTTACCACACCAGCCCCATGATTGGACAATTT  4980
1530  H  D  F  L  E  I  Q  N  G  P  Y  H  T  S  P  M  I  G  Q  F   1549

4981  AGCGGCACGGATCTCCCCGCGGCCCTGCTGAGCACAACGCATGAAACCCTCATCCACTTT  5040
1550  S  G  T  D  L  P  A  A  L  L  S  T  T  H  E  T  L  I  H  F   1569

5041  TATAGTGACCATTCGCAAAACCGGCAAGGATTTAAACTTGCTTACCAAGCCTATGAATTA  5100
1570  Y  S  D  H  S  Q  N  R  Q  G  F  K  L  A  Y  Q  A  Y  E  L   1589

5101  CAGAACTGTCCAGATCCACCCCCATTTCAGAATGGGTACATGATCAACTCGGATTACAGC  5160
1590  Q  N  C  P  D  P  P  P  F  Q  N  G  Y  M  I  N  S  D  Y  S   1609

5161  GTGGGGCAATCAGTATCTTTCGAGTGTTATCCTGGGTACATTCTAATAGGCCATCCTGTC  5220
1610  V  G  Q  S  V  S  F  E  C  Y  P  G  Y  I  L  I  G  H  P  V   1629

5221  CTCACTTGTCAGCATGGGATCAACAGAAACTGGAACTACCCTTTTCCAAGATGTGATGCC  5280
1630  L  T  C  Q  H  G  I  N  R  N  W  N  Y  P  F  P  R  C  D  A   1649

5281  CCTTGTGGGTACAACGTAACTTCTCAGAACGGCACCATCTACTCCCCTGGCTTTCCTGAT  5340
1650  P  C  G  Y  N  V  T  S  Q  N  G  T  I  Y  S  P  G  F  P  D   1669

5341  GAGTATCCGATCCTGAAGGACTGCATTTGGCTCATCACGGTGCCTCCAGGGCACGGAGTT  5400
1670  E  Y  P  I  L  K  D  C  I  W  L  I  T  V  P  P  G  H  G  V   1689

5401  TACATCAACTTCACCCTGTTACAGACGGAAGCTGTCAACGATTACATTGCTGTTTGGGAC  5460
1690  Y  I  N  F  T  L  L  Q  T  E  A  V  N  D  Y  I  A  V  W  D   1709

5461  GGTCCCGATCAGAACTCACCCCAGCTGGGAGTTTTCAGTGGCAACACAGCCCTCGAAACG  5520
1710  G  P  D  Q  N  S  P  Q  L  G  V  F  S  G  N  T  A  L  E  T   1729

5521  GCGTATAGCTCCACCAACCAAGTCCTGCTCAAGTTCCACAGCGACTTTTCAAATGGAGGC  5580
1730  A  Y  S  S  T  N  Q  V  L  L  K  F  H  S  D  F  S  N  G  G   1749

5581  TTCTTTGTCCTCAATTTCCACGCATTTCAGCTCAAGAAATGTCAACCTCCCCCAGCGGTT  5640
1750  F  F  V  L  N  F  H  A  F  Q  L  K  K  C  Q  P  P  P  A  V   1769

5641  CCACAGGCAGAAATGCTTACTGAGGATGATGATTTCGAGATAGGAGATTTTGTGAAGTAC  5700
1770  P  Q  A  E  M  L  T  E  D  D  D  F  E  I  G  D  F  V  K  Y   1789

5701  CAGTGCCACCCCGGGTACACCTTGGTGGGGACCGACATTCTGACTTGCAAGCTCAGTTCC  5760
1790  Q  C  H  P  G  Y  T  L  V  G  T  D  I  L  T  C  K  L  S  S   1809

5761  CAGTTGCAGTTTGAGGGTTCTCTCCCAACATGTGAAGCACAATGCCCAGCAAATGAAGTC  5820
1810  Q  L  Q  F  E  G  S  L  P  T  C  E  A  Q  C  P  A  N  E  V   1829
```

Figure 1F

```
5821  CGGACTGGATCATCGGGAGTCATTCTCAGTCCAGGGTATCCGGGTAATTATTTTAACTCC  5880
1830  R  T  G  S  S  G  V  I  L  S  P  G  Y  P  G  N  Y  F  N  S   1849

5881  CAGACTTGCTCTTGGAGTATTAAAGTGGAACCAAACTACAACATTACCATCTTTGTGGAC  5940
1850  Q  T  C  S  W  S  I  K  V  E  P  N  Y  N  I  T  I  F  V  D   1869

5941  ACATTTCAAAGTGAAAAGCAGTTTGATGCACTGGAAGTGTTTGATGGTTCTTCTGGGCAA  6000
1870  T  F  Q  S  E  K  Q  F  D  A  L  E  V  F  D  G  S  S  G  Q   1889

6001  AGTCCTCTGCTAGTAGTCTTAAGTGGGAATCATACTGAACAATCAAATTTTACAAGCAGG  6060
1890  S  P  L  L  V  V  L  S  G  N  H  T  E  Q  S  N  F  T  S  R   1909

6061  AGTAATCAGTTATATCTCCGCTGGTCCACTGACCATGCCACCAGTAAGAAAGGATTCAAG  6120
1910  S  N  Q  L  Y  L  R  W  S  T  D  H  A  T  S  K  K  G  F  K   1929

6121  ATTCGCTATGCAGCACCTTACTGCAGTTTGACCCACCCCCTGAAGAATGGGGGTATTCTA  6180
1930  I  R  Y  A  A  P  Y  C  S  L  T  H  P  L  K  N  G  G  I  L   1949

6181  AACAGGACTGCAGGAGCGGTTGGAAGCAAAGTGCATTATTTTTGCAAGCCTGGATACCGA  6240
1950  N  R  T  A  G  A  V  G  S  K  V  H  Y  F  C  K  P  G  Y  R   1969

6241  ATGGTCGGCCACAGCAATGCAACCTGTAGACGAAACCCACTTGGCATGTACCAGTGGGAC  6300
1970  M  V  G  H  S  N  A  T  C  R  R  N  P  L  G  M  Y  Q  W  D   1989

6301  TCCCTCACGCCACTCTGCCAGGCTGTGTCCTGTGGAATCCCAGAATCCCCAGGAAACGGT  6360
1990  S  L  T  P  L  C  Q  A  V  S  C  G  I  P  E  S  P  G  N  G   2009

6361  TCATTTACCGGGAACGAGTTCACTTTGGACAGTAAAGTGGTCTATGAATGTCATGAGGGC  6420
2010  S  F  T  G  N  E  F  T  L  D  S  K  V  V  Y  E  C  H  E  G   2029

6421  TTCAAGCTTGAATCCAGCCAGCAAGCAACAGCCGTGTGTCAAGAAGATGGGCTGTGGAGT  6480
2030  F  K  L  E  S  S  Q  Q  A  T  A  V  C  Q  E  D  G  L  W  S   2049

6481  AACAAGGGGAAGCCGCCCACGTGTAAGCCGGTCGCTTGCCCCAGCATTGAAGCTCAGCTC  6540
2050  N  K  G  K  P  P  T  C  K  P  V  A  C  P  S  I  E  A  Q  L   2069

6541  TCAGAACATGTCATCTGGAGGCTGGTTTCAGGATCCTTGAATGAGTACGGTGCTCAAGTA  6600
2070  S  E  H  V  I  W  R  L  V  S  G  S  L  N  E  Y  G  A  Q  V   2089

6601  TTGCTGAGCTGCAGTCCTGGTTACTACTTAGAAGGCTGGAGGCTCCTGCGGTGCCAGGCC  6660
2090  L  L  S  C  S  P  G  Y  Y  L  E  G  W  R  L  L  R  C  Q  A   2109

6661  AATGGGACGTGGAACATAGGAGATGAGAGGCCAAGCTGTCGAGTTATCTCGTGTGGAAGC  6720
2110  N  G  T  W  N  I  G  D  E  R  P  S  C  R  V  I  S  C  G  S   2129

6721  CTTTCCTTTCCCCCAAATGGCAACAAGATTGGAACGTTGACAGTTTATGGGGCCACAGCT  6780
2130  L  S  F  P  P  N  G  N  K  I  G  T  L  T  V  Y  G  A  T  A   2149

6781  ATATTTACGTGCAACACCGGCTACACGCTTGTGGGGTCTCATGTCAGAGAGTGCTTGGCA  6840
2150  I  F  T  C  N  T  G  Y  T  L  V  G  S  H  V  R  E  C  L  A   2169

6841  AATGGGCTCTGGAGCGGCAGCGAAACTCGATGTCTGGCTGGCCACTGCGGTTCCCCAGAC  6900
2170  N  G  L  W  S  G  S  E  T  R  C  L  A  G  H  C  G  S  P  D   2189

6901  CCGATTGTGAACGGTCACATTAGTGGAGATGGCTTCAGTTACAGAGACACGGTGGTTTAC  6960
2190  P  I  V  N  G  H  I  S  G  D  G  F  S  Y  R  D  T  V  V  Y   2209
```

Figure 1G

```
6961  CAGTGCAATCCTGGTTTCCGGCTTGTGGGAACTTCCGTGAGGATATGCCTGCAAGACCAC  7020
2210  Q  C  N  P  G  F  R  L  V  G  T  S  V  R  I  C  L  Q  D  H   2229

7021  AAGTGGTCTGGACAAACGCCTGTCTGTGTCCCCATCACATGTGGTCACCCTGGAAACCCT  7080
2230  K  W  S  G  Q  T  P  V  C  V  P  I  T  C  G  H  P  G  N  P   2249

7081  GCCCACGGATTCACTAATGGCAGTGAGTTCAACCTGAATGATGTCGTGAATTTCACCTGC  7140
2250  A  H  G  F  T  N  G  S  E  F  N  L  N  D  V  V  N  F  T  C   2269

7141  AACACGGGCTATTTGCTGCAGGGCGTGTCTCGAGCCCAGTGTCGGAGCAACGGCCAGTGG  7200
2270  N  T  G  Y  L  L  Q  G  V  S  R  A  Q  C  R  S  N  G  Q  W   2289

7201  AGTAGCCCTCTGCCCACGTGTCGAGTGGTGAACTGTTCTGATCCAGGCTTTGTGGAAAAT  7260
2290  S  S  P  L  P  T  C  R  V  V  N  C  S  D  P  G  F  V  E  N   2309

7261  GCCATTCGTCACGGGCAACAGAACTTCCCTGAGAGTTTTGAGTATGGAATGAGTATCCTG  7320
2310  A  I  R  H  G  Q  Q  N  F  P  E  S  F  E  Y  G  M  S  I  L   2329

7321  TACCATTGCAAGAAGGGATTTTACTTGCTGGGATCTTCAGCCTTGACCTGTATGGCAAAT  7380
2330  Y  H  C  K  K  G  F  Y  L  L  G  S  S  A  L  T  C  M  A  N   2349

7381  GGCTTATGGGACCGATCCCTGCCCAAGTGTTTGGCTATATCGTGTGGACACCCAGGGGTC  7440
2350  G  L  W  D  R  S  L  P  K  C  L  A  I  S  C  G  H  P  G  V   2369

7441  CCTGCCAACGCCGTCCTCACTGGAGAGCTGTTTACCTATGGCGCCGTCGTGCACTACTCC  7500
2370  P  A  N  A  V  L  T  G  E  L  F  T  Y  G  A  V  V  H  Y  S   2389

7501  TGCAGAGGGAGCGAGAGCCTCATAGGCAACGACACGAGAGTGTGCCAGGAAGACAGTCAC  7560
2390  C  R  G  S  E  S  L  I  G  N  D  T  R  V  C  Q  E  D  S  H   2409

7561  TGGAGCGGGGCACTGCCCCACTGCACAGGAAATAATCCTGGATTCTGTGGTGATCCGGGG  7620
2410  W  S  G  A  L  P  H  C  T  G  N  N  P  G  F  C  G  D  P  G   2429

7621  ACCCCAGCACATGGGTCTCGGCTTGGTGATGACTTTAAGACAAAGAGTCTTCTCCGCTTC  7680
2430  T  P  A  H  G  S  R  L  G  D  D  F  K  T  K  S  L  L  R  F   2449

7681  TCCTGTGAAATGGGGCACCAGCTGAGGGGCTCCCCTGAACGCACGTGTTTGCTCAATGGG  7740
2450  S  C  E  M  G  H  Q  L  R  G  S  P  E  R  T  C  L  L  N  G   2469

7741  TCATGGTCAGGACTGCAGCCGGTGTGTGAGGCCGTGTCCTGTGGCAACCCTGGCACACCC  7800
2470  S  W  S  G  L  Q  P  V  C  E  A  V  S  C  G  N  P  G  T  P   2489

7801  ACCAACGGAATGATTGTCAGTAGTGATGGCATTCTGTTCTCCAGCTCGGTCATCTATGCC  7860
2490  T  N  G  M  I  V  S  S  D  G  I  L  F  S  S  S  V  I  Y  A   2509

7861  TGCTGGGAAGGCTACAAGACCTCAGGGCTCATGACACGGCATTGCACAGCCAATGGGACC  7920
2510  C  W  E  G  Y  K  T  S  G  L  M  T  R  H  C  T  A  N  G  T   2529

7921  TGGACAGGCACTGCTCCCGACTGCACAATTATAAGTTGTGGGGATCCAGGCACACTAGCA  7980
2530  W  T  G  T  A  P  D  C  T  I  I  S  C  G  D  P  G  T  L  A   2549

7981  AATGGCATCCAGTTTGGGACCGACTTCACCTTCAACAAGACTGTGAGCTATCAGTGTAAC  8040
2550  N  G  I  Q  F  G  T  D  F  T  F  N  K  T  V  S  Y  Q  C  N   2569
```

Figure 1H

```
8041 CCAGGCTATGTCATGGAAGCAGTCACATCCGCCACTATTCGCTGTACCAAAGACGGCAGG 8100
2570 P   G   Y   V   M   E   A   V   T   S   A   T   I   R   C   T   K   D   G   R   2589

8101 TGGAATCCGAGCAAACCTGTCTGCAAAGCCGTGCTGTGTCCTCAGCCGCCGCCGGTGCAG 8160
2590 W   N   P   S   K   P   V   C   K   A   V   L   C   P   Q   P   P   P   V   Q   2609

8161 AATGGAACAGTGGAGGGAAGTGATTTCCGCTGGGGCTCCAGCATAAGTTACAGCTGCATG 8220
2610 N   G   T   V   E   G   S   D   F   R   W   G   S   S   I   S   Y   S   C   M   2629

8221 GACGGTTACCAGCTCTCTCACTCCGCCATCCTCTCCTGTGAAGGTCGCGGGGTGTGGAAA 8280
2630 D   G   Y   Q   L   S   H   S   A   I   L   S   C   E   G   R   G   V   W   K   2649

8281 GGAGAGATCCCCCAGTGTCTCCCTGTGTTCTGCGGAGACCCTGGCATCCCCGCAGAAGGG 8340
2650 G   E   I   P   Q   C   L   P   V   F   C   G   D   P   G   I   P   A   E   G   2669

8341 CGACTTAGTGGGAAAAGTTTCACCTATAAGTCCGAAGTCTTCTTCCAGTGCAAATCTCCA 8400
2670 R   L   S   G   K   S   F   T   Y   K   S   E   V   F   F   Q   C   K   S   P   2689

8401 TTTATACTCGTGGGATCCTCCAGAAGAGTCTGCCAAGCTGACGGCACGTGGAGCGGCATA 8460
2690 F   I   L   V   G   S   S   R   R   V   C   Q   A   D   G   T   W   S   G   I   2709

8461 CAACCCACCTGCATTGATCCTGCTCATAACACCTGCCCAGACCCTGGTACGCCACACTTT 8520
2710 Q   P   T   C   I   D   P   A   H   N   T   C   P   D   P   G   T   P   H   F   2729

8521 GGAATACAGAATAGCTCCAGAGGCTATGAGGTTGGAAGCACGGTTTTTTTCAGGTGCAGA 8580
2730 G   I   Q   N   S   S   R   G   Y   E   V   G   S   T   V   F   F   R   C   R   2749

8581 AAAGGCTACCATATTCAAGGTTCCACGACTCGCACCTGCCTTGCCAATTTAACATGGAGT 8640
2750 K   G   Y   H   I   Q   G   S   T   T   R   T   C   L   A   N   L   T   W   S   2769

8641 GGGATACAGACCGAATGTATACCTCATGCCTGCAGACAGCCAGAAACCCCGGCACACGCG 8700
2770 G   I   Q   T   E   C   I   P   H   A   C   R   Q   P   E   T   P   A   H   A   2789

8701 GATGTGAGAGCCATCGATCTTCCTACTTTCGGCTACACCTTAGTGTACACCTGCCATCCA 8760
2790 D   V   R   A   I   D   L   P   T   F   G   Y   T   L   V   Y   T   C   H   P   2809

8761 GGCTTTTTCCTCGCAGGGGGATCTGAGCACAGAACATGTAAAGCAGACATGAAATGGACA 8820
2810 G   F   F   L   A   G   G   S   E   H   R   T   C   K   A   D   M   K   W   T   2829

8821 GGAAAGTCGCCTGTGTGTAAAAGTAAAGGAGTGAGAGAAGTTAATGAAACAGTTACTAAA 8880
2830 G   K   S   P   V   C   K   S   K   G   V   R   E   V   N   E   T   V   T   K   2849

8881 ACTCCAGTTCCTTCAGATGTCTTTTTCGTCAATTCACTGTGGAAGGGGTATTATGAATAT 8940
2850 T   P   V   P   S   D   V   F   F   V   N   S   L   W   K   G   Y   Y   E   Y   2869

8941 TTAGGGAAAAGACAACCCGCCACTCTAACTGTTGACTGGTTCAATGCAACAAGCAGTAAG 9000
2870 L   G   K   R   Q   P   A   T   L   T   V   D   W   F   N   A   T   S   S   K   2889

9001 GTGAATGCCACCTTCAGCGAAGCCTCGCCAGTGGAGCTGAAGTTGACAGGCATTTACAAG 9060
2890 V   N   A   T   F   S   E   A   S   P   V   E   L   K   L   T   G   I   Y   K   2909

9061 AAGGAGGAGGCCCACTTACTCCTGAAAGCTTTTCAAATTAAAGGCCAGGCAGATATTTTT 9120
2910 K   E   E   A   H   L   L   L   K   A   F   Q   I   K   G   Q   A   D   I   F   2929

9121 GTAAGCAAGTTCGAAAATGACAACTGGGGACTAGATGGTTATGTGTCATCTGGACTTGAA 9180
2930 V   S   K   F   E   N   D   N   W   G   L   D   G   Y   V   S   S   G   L   E   2949
```

Figure 1I

```
9181  AGAGGAGGATTTACTTTTCAAGGTGACATTCATGGAAAAGACTTTGGAAAATTTAAGCTA  9240
2950  R  G  G  F  T  F  Q  G  D  I  H  G  K  D  F  G  K  F  K  L   2969

9241  GAAAGGCAAGATCCTTTAAACCCAGATCAAGACTCTTCCAGTCATTACCACGGCACCAGC  9300
2970  E  R  Q  D  P  L  N  P  D  Q  D  S  S  S  H  Y  H  G  T  S   2989

9301  AGTGGCTCTGTGGCGGCTGCCATTCTGGTTCCTTTCTTTGCTCTAATTTTATCAGGGTTT  9360
2990  S  G  S  V  A  A  A  I  L  V  P  F  F  A  L  I  L  S  G  F

9361  GCATTTTACCTCTACAAACACAGAACGAGACCAAAAGTTCAATACAATGGCTATGCTGGG  9420
3010  A  F  Y  L  Y  K  H  R  T  R  P  K  V  Q  Y  N  G  Y  A  G   3029

9421  CATGAAAACAGCAATGGACAAGCATCGTTTGAAAACCCCATGTATGATACAAACTTAAAA  9480
3030  H  E  N  S  N  G  Q  A  S  F  E  N  P  M  Y  D  T  N  L  K   3049

9481  CCCACAGAAGCCAAGGCTGTGAGGTTTGACACAACTCTGAACACAGTCTGTACAGTGGTA  9540
3050  P  T  E  A  K  A  V  R  F  D  T  T  L  N  T  V  C  T  V  V   3069

9541  TAGCCCTCAGTGCCCCAACAGGACTGATTCATAGCCATACCTCTGATGGACAAGCAGTGA  9600
3070  *                                                             3070
9601  TTCCTTTGGTGCCATATACCACTCTCCCYTCCACTCTGGCTTTACTGCAGCGATCTTCAA  9660
9661  CCTTGTCTACTGGCATAAGTGCAGCGGGGATCTCTACTCAAATGTGTCAGGGTCTTCTAC  9720
9721  GGATCAAACTACACATGCGTTTTCATTCCAAAAGTGGGTTCTAAATGCCTGGCTGCATCT  9780
9781  GTATGAAATCAAGGCACACTCCAGGAAGACTGCCACGTCGCGCCAACACGTCATACTCAA  9840
9841  TRCCTCAGACTTTCATATTTCTGTGTTGCTGAGATGCCTTTCAATGCAATCGTCTGGGCT  9900
9901  CGTGGATATGTCCCTCAGGTGCGGTGACAGAATGGTGGCACCACGATATGTGTTCTCTTG  9960
9961  TGTTGTTTTTCCTTTTTAAACCCCCATGAACACGAATACTCTGAAAAAAATAAAAAGCTT 10020
10021 TCTGGAAGAAGACACCTTTCTGATAGAGGCTCACACCTACAAATGCTTCACTCTGTCCTT 10080
10081 CCGAGACCTGACAAGCTTTGAGGACCTCACAGCTCCCCTGTGTGTTCATCTCTAGGGATG 10140
10141 TTTGCAATTTCCCAGTCAGCTGTTCTGTCGCAGAATGTTTAATGCACAATTTTTTGCACT 10200
10201 AGTGTGTTATGAATGACTAAGATTCTGATAAAAAAAATAAATTATTTACACAGGGTTTAT 10260
10261 ACACACTATCCATTGTATATAAGCATTATTTCATATTATCAAGCTAAACATTCCCCCATC 10320
10321 AGCTTAGTTGGAGTGTTAGGGAAAAGTATTCCTAGATATGGCACAGATTTTAAAAGGAAA 10380
10381 TACAGTATTGACGAGATTTATTTTATTATTGCTTCAATTAGCTCCATTTACGTGTTGAAT 10440
10441 TCATTGAAGAGGTCCAATGAGAAAAAAACAGAAGCCTCCTTATTTCACACGTTTTCCTCC 10500
10501 TTTAGTACCATCCTCATCCAATTACTGTCTCTCTGATACTACTTAATAGCAGGGGGTTTG 10560
10561 CAGAAATTTCTGTTTGCCATGTAAAACTGTGAATAGTAATTTATTTTAGATAGTCGATGA 10620
10621 ACTTGTGGGTTTTAGCTCACAATGCAGCCTTCCCTTTTGCAGTGTTTTTTTTT  10673
```

Figure 2A

Map of Second Human C3b/C4b Complement Receptor like cDNA (SEQ ID NO:6) and Amino Acid Sequences (SEQ ID NO:7)

```
   1  ACCCTGACGGTTGGTGATGCTGGGAAGGTGGGAGACACCAGATCGGTCTTGTACGTGCTC    60
   1  T  L  T  V  G  D  A  G  K  V  G  D  T  R  S  V  L  Y  V  L     20

  61  ACGGGATCCAGTGTTCCTGACCTCATTGTGAGCATGAGCAACCAGATGTGGCTACATCTG   120
  21  T  G  S  S  V  P  D  L  I  V  S  M  S  N  Q  M  W  L  H  L     40

 121  CAGTCGGATGATAGCATTGGCTCACCTGGGTTTAAAGCTGTTTACCAAGAAATTGAAAAG   180
  41  Q  S  D  D  S  I  G  S  P  G  F  K  A  V  Y  Q  E  I  E  K     60

 181  GGAGGGTGTGGGGATCCTGGAATCCCCGCCTATGGAAGCGGACGGGCAGCAGTTTCCTC   240
  61  G  G  C  G  D  P  G  I  P  A  Y  G  K  R  T  G  S  S  F  L     80

 241  CATGGAGATACACTCACCTTTGAATGCCCGGCGGCCTTTGAGCTGGTGGGGGAGAGAGTT   300
  81  H  G  D  T  L  T  F  E  C  P  A  A  F  E  L  V  G  E  R  V    100

 301  ATCACCTGTCAGCAGAACAATCAGTGGTCTGGCAACAAGCCCAGCTGTGTATTTTCATGT   360
 101  I  T  C  Q  Q  N  N  Q  W  S  G  N  K  P  S  C  V  F  S  C    120

 361  TTCTTCAACTTTACGGCATCATCTGGGATTATTCTGTCACCAAATTATCCAGAGGAATAT   420
 121  F  F  N  F  T  A  S  S  G  I  I  L  S  P  N  Y  P  E  E  Y    140

 421  GGGAACAACATGAACTGTGTCTGGTTGATTATCTCGGAGCCAGGAAGTCGAATTCACCTA   480
 141  G  N  N  M  N  C  V  W  L  I  I  S  E  P  G  S  R  I  H  L    160

 481  ATCTTTAATGATTTTGATGTTGAGCCTCAATTTGACTTTCTCGCGGTCAAGGATGATGGC   540
 161  I  F  N  D  F  D  V  E  P  Q  F  D  F  L  A  V  K  D  D  G    180

 541  ATTTCTGACATAACTGTCCTGGGTACTTTTTCTGGCAATGAAGTGCCTTCCCAGCTGGCC   600
 181  I  S  D  I  T  V  L  G  T  F  S  G  N  E  V  P  S  Q  L  A    200

 601  AGCAGTGGGCATATAGTTCGCTTGGAATTTCAGTCTGACCATTCCACTACTGGCAGAGGG   660
 201  S  S  G  H  I  V  R  L  E  F  Q  S  D  H  S  T  T  G  R  G    220

 661  TTCAACATCACTTACACCACATTTGGTCAGAATGAGTGCCATGATCCTGGCATTCCTATA   720
 221  F  N  I  T  Y  T  T  F  G  Q  N  E  C  H  D  P  G  I  P  I    240

 721  AACGGACGACGTTTTGGTGACAGGTTTCTACTCGGGAGCTCGGTTTCTTTCCACTGTGAT   780
 241  N  G  R  R  F  G  D  R  F  L  L  G  S  S  V  S  F  H  C  D    260

 781  GATGGCTTTGTCAAGACCCAGGGATCCGAGTCCATTACCTGCATACTGCAAGACGGGAAC   840
 261  D  G  F  V  K  T  Q  G  S  E  S  I  T  C  I  L  Q  D  G  N    280

 841  GTGGTCTGGAGCTCCACCGTGCCCCGCTGTGAAGCTCCATGTGGTGGACATCTGACAGCG   900
 281  V  V  W  S  S  T  V  P  R  C  E  A  P  C  G  G  H  L  T  A    300

 901  TCCAGCGGAGTCATTTTGCCTCCTGGATGGCCAGGATATTATAAGGATTCTTTACATTGT   960
 301  S  S  G  V  I  L  P  P  G  W  P  G  Y  Y  K  D  S  L  H  C    320

 961  GAATGGATAATTGAAGCAAAACCAGGCCACTCTATCAAAATAACTTTTGACAGATTTCAG  1020
 321  E  W  I  I  E  A  K  P  G  H  S  I  K  I  T  F  D  R  F  Q    340

1021  ACAGAGGTCAATTATGACACCTTGGAGGTCAGAGATGGGCCAGCCAGTTCGTCCCCACTG  1080
 341  T  E  V  N  Y  D  T  L  E  V  R  D  G  P  A  S  S  S  P  L    360
```

Figure 2B

1081 ATCGGCGAGTACCACGGCACCCAGGCACCCCAGTTCCTCATCAGCACCGGGAACTTCATG 1140
361 I G E Y H G T Q A P Q F L I S T G N F M 380

1141 TACCTGCTATTCACCACTGACAACAGCCGCTCCAGCATCGGCTTCCTCATCCACTATGAG 1200
381 Y L L F T T D N S R S S I G F L I H Y E 400

1201 AGTGTGACGCTTGAGTCGGATTCCTGCCTGGACCCGGGCATCCCTGTGAACGRCCATCGC 1260
401 S V T L E S D S C L D P G I P V N X H R 420

1261 CACGGTGGAGACTTTGGCATCAGGTCCACAGTGACTTTCAGCTGTGACCCGGGGTACACA 1320
421 H G G D F G I R S T V T F S C D P G Y T 440

1321 CTAAGTGACGACGAGCCCCTCGTCTGTGAGAGGAACCACCAGTGGAACCACGCCTTGCCC 1380
441 L S D D E P L V C E R N H Q W N H A L P 460

1381 AGCTGCGACGCTCTATGTGGAGGCTACATCCAAGGGAAGAGTGGAACAGTCCTTTCTCCT 1440
461 S C D A L C G G Y I Q G K S G T V L S P 480

1441 GGGTTTCCAGATTTTTATCCAAACTCTCTAAACYGCACGTGGACCATTGAAGTGTCTCAT 1500
481 G F P D F Y P N S L N X T W T I E V S H 500

1501 GGGAAAGGAGTTCAAATGATCTTTCACACCTTTCATCTTGAGAGTTCCCACGACTATTTA 1560
501 G K G V Q M I F H T F H L E S S H D Y L 520

1561 CTGATCACAGAGGATGGAAGTTTTTCCGAGCCCGTTGCCAGGCTCACCGGGTCGGTGTTG 1620
521 L I T E D G S F S E P V A R L T G S V L 540

1621 CCTCATACGATCAAGGCAGGCCTGTTTGGAAACTTCACTGCCCAGCTTCGGTTTATATCA 1680
541 P H T I K A G L F G N F T A Q L R F I S 560

1681 GACTTCTCAATTTCGTACGAGGGCTTCAATATCACATTTTCAGAATATGACCTGGAGCCA 1740
561 D F S I S Y E G F N I T F S E Y D L E P 580

1741 TGTGATGATCCTGGAGTCCCTGCCTTCAGCCGAAGAATTGGTTTTCACTTTGGTGTGGGA 1800
581 C D D P G V P A F S R R I G F H F G V G 600

1801 GACTCTCTGACGTTTTCCTGCTTCCTGGGATATCGTTTAGAAGGTGCCRCCAAGCTTACC 1860
601 D S L T F S C F L G Y R L E G A X K L T 620

1861 TGCCTGGGTGGGGGCCGCCGTGTGTGGAGTGCACCTCTGCCAAGGTGTGTGGCCGAATGT 1920
621 C L G G G R R V W S A P L P R C V A E C 640

1921 GGAGCAAGTGTCAAAGGAAATGAAGGAACATTACTGTCTCCAAATTTTCCATCCAATTAT 1980
641 G A S V K G N E G T L L S P N F P S N Y 660

1981 GATAATAACCATGAGTGTATCTATAAAATAGAAACAGAAGCCGGCAAGGGCATCCACCTT 2040
661 D N N H E C I Y K I E T E A G K G I H L 680

2041 AGAACACGAAGCTTCCAGCTGTTTGAAGGAGATACTCTAAAGGTATATGATGGAAAAGAC 2100
681 R T R S F Q L F E G D T L K V Y D G K D 700

2101 AGTTCCTCACGTCCACTGGGCACGTTCACTAAAAATGAACTTCTGGGGCTGATCCTAAAC 2160
701 S S S R P L G T F T K N E L L G L I L N 720

2161 AGCACATCCAATCACCTRTGGCTAGAGTTCAACACCAATGGATCTGACACCGACCAAGGT 2220
721 S T S N H L W L E F N T N G S D T D Q G 740

Figure 2C

```
2221  TTTCAACTCACCTATACCAGTTTTGATCTGGTAAAATGTGAGGATCCGGGCATCCCTAAC  2280
 741  F  Q  L  T  Y  T  S  F  D  L  V  K  C  E  D  P  G  I  P  N   760

2281  TACGGCTATAGGATCCGTGATGAAGGCCACTTTACCGACACTGTAGTTCTGTACAGTTGC  2340
 761  Y  G  Y  R  I  R  D  E  G  H  F  T  D  T  V  V  L  Y  S  C   780

2341  AACCCGGGGTACGCCATGCATGGCAGCAACACCCTGACCTGTTTGAGTGGAGACAGGAGA  2400
 781  N  P  G  Y  A  M  H  G  S  N  T  L  T  C  L  S  G  D  R  R   800

2401  GTGTGGGACAAACCACTACCTTCGTGCATAGCGGAATGTGGTGGTCAGATCCATGCAGCC  2460
 801  V  W  D  K  P  L  P  S  C  I  A  E  C  G  G  Q  I  H  A  A   820

2461  ACATCAGGACGAATATTGTCCCCTGGCTATCCAGCTCCGTATGACAACAACCTCCACTGC  2520
 821  T  S  G  R  I  L  S  P  G  Y  P  A  P  Y  D  N  N  L  H  C   840

2521  ACCTGGATTATAGAGGCAGACCCAGGAAAGACCATTAGCCTCCATTTCATTGTTTTCGAC  2580
 841  T  W  I  I  E  A  D  P  G  K  T  I  S  L  H  F  I  V  F  D   860

2581  ACGGAGATGGCTCACGACATCCTCAAGGTCTGGGACGGGCCGGTGGACAGTGACATCCTG  2640
 861  T  E  M  A  H  D  I  L  K  V  W  D  G  P  V  D  S  D  I  L   880

2641  CTGAAGGAGTGGAGTGGCTCCGCCCTTCCGGAGGACATCCACAGCACCTTCAACTCACTC  2700
 881  L  K  E  W  S  G  S  A  L  P  E  D  I  H  S  T  F  N  S  L   900

2701  ACCCTGCAGTTCGACAGCGACTTCTTCATCAGCAAGTCTGGCTTCTCCATCCAGTTCTCC  2760
 901  T  L  Q  F  D  S  D  F  F  I  S  K  S  G  F  S  I  Q  F  S   920

2761  ACCTCAATTGCAGCCACCTGTAACGATCCAGGTATGCCCCAAAATGGCACCCGCTATGGA  2820
 921  T  S  I  A  A  T  C  N  D  P  G  M  P  Q  N  G  T  R  Y  G   940

2821  GACAGCAGAGAGGCTGGAGACACCGTCACATTCCAGTGTGACCCTGGCTATCAGCTCCAA  2880
 941  D  S  R  E  A  G  D  T  V  T  F  Q  C  D  P  G  Y  Q  L  Q   960

2881  GGACAAGCCAAAATCACCTGTGTGCAGCTGAATAACCGGTTCTTTTGGCAACCAGACCCT  2940
 961  G  Q  A  K  I  T  C  V  Q  L  N  N  R  F  F  W  Q  P  D  P   980

2941  CCTACATGCATAGCTGCTTGTGGAGGGAATCTGACGGGCCCAGCAGGTGTTATTTTGTCA  3000
 981  P  T  C  I  A  A  C  G  G  N  L  T  G  P  A  G  V  I  L  S  1000

3001  CCCAACTACCCACAGCCGTATCCTCCTGGGAAGGAATGTGACTGGAGAGTAAAAGTGAAC  3060
1001  P  N  Y  P  Q  P  Y  P  P  G  K  E  C  D  W  R  V  K  V  N  1020

3061  CCGGACTTTGTCATCGCCTTGATATTCAAAAGTTTCAACATGGAGCCCAGCTATGACTTC  3120
1021  P  D  F  V  I  A  L  I  F  K  S  F  N  M  E  P  S  Y  D  F  1040

3121  CTACACATCTATGAAGGGGAAGATTCCAACAGCCCCCTCATTGGGAGTTACCAGGGCTCT  3180
1041  L  H  I  Y  E  G  E  D  S  N  S  P  L  I  G  S  Y  Q  G  S  1060

3181  CAGGCCCCAGAAAGAATAGAGAGTAGCGGAAACAGCCTGTTTCTGGCATTTCGGAGTGAT  3240
1061  Q  A  P  E  R  I  E  S  S  G  N  S  L  F  L  A  F  R  S  D  1080

3241  GCCTCCGTGGGCCTTTCAGGGTTCGCCATTGAATTTAAAGAGAAACCACGGGAAGCTTGT  3300
1081  A  S  V  G  L  S  G  F  A  I  E  F  K  E  K  P  R  E  A  C  1100

3301  TTTGACCCAGGAAATATAATGAATGGGACAAGAGTTGGAACAGACTTCAAGCTTGGCTCC  3360
1101  F  D  P  G  N  I  M  N  G  T  R  V  G  T  D  F  K  L  G  S  1120

3361  ACCATCACCTACCAGTGTGACTCTGGCTATAAGATTCTTGACCCCTCATCCATCACCTGT  3420
1121  T  I  T  Y  Q  C  D  S  G  Y  K  I  L  D  P  S  S  I  T  C  1140
```

Figure 2D

```
3421  GTGATTGGGGCTGATGGGAAACCCTCCTGGGACCAAGTGCTGCCCTCCTGCAATGCTCCC  3480
1141  V  I  G  A  D  G  K  P  S  W  D  Q  V  L  P  S  C  N  A  P   1160

3481  TGTGGAGGCCAGTACACGGGATCAGAAGGGGTAGTTTTATCACCAAACTACCCCCATAAT  3540
1161  C  G  G  Q  Y  T  G  S  E  G  V  V  L  S  P  N  Y  P  H  N   1180

3541  TACACAGCTGGTCAAATATGCCTCTATTCCATCACGGTACCAAAGGAATTCGTGGTCTTT  3600
1181  Y  T  A  G  Q  I  C  L  Y  S  I  T  V  P  K  E  F  V  V  F   1200

3601  GGACAGTTTGCCTATTTCCAGACAGCCCTGAATGATTTGGCAGAATTATTTGATGGAACC  3660
1201  G  Q  F  A  Y  F  Q  T  A  L  N  D  L  A  E  L  F  D  G  T   1220

3661  CATGCACAGGCCAGACTTCTCAGCTCACTCTCGGGGTCTCACTCAGGGGAAACATTGCCC  3720
1221  H  A  Q  A  R  L  L  S  S  L  S  G  S  H  S  G  E  T  L  P   1240

3721  TTGGCTACGTCAAATCAAATTCTGCTCCGATTCAGTGCAAAGAGCGGTGCCTCTGCCCGC  3780
1241  L  A  T  S  N  Q  I  L  L  R  F  S  A  K  S  G  A  S  A  R   1260

3781  GGCTTCCACTTCGTGTATCAAGCTGTTCCTCGTACCAGTGACACCCAATGCAGCTCTGTC  3840
1261  G  F  H  F  V  Y  Q  A  V  P  R  T  S  D  T  Q  C  S  S  V   1280

3841  CCCGAGCCCAGATACGGAAGGAGAATTGGTTCTGAGTTTTCTGCCGGCTCCATCGTCCGA  3900
1281  P  E  P  R  Y  G  R  R  I  G  S  E  F  S  A  G  S  I  V  R   1300

3901  TTCGAGTRCAACCCGGGATACCTGCTTCAGGGTTCCACGGCGCTCCACTGCCAGTCCGTG  3960
1301  F  E  X  N  P  G  Y  L  L  Q  G  S  T  A  L  H  C  Q  S  V   1320

3961  CCCAACGCCTTGGCACAGTGGAACGACACGATCCCCAGCTGTGTGGTACCCTGCAGTGGC  4020
1321  P  N  A  L  A  Q  W  N  D  T  I  P  S  C  V  V  P  C  S  G   1340

4021  AATTTCACTCAACGAAGAGGTACAATCCTGTCCCCCGGCTACCCTGAGCCATACGGAAAC  4080
1341  N  F  T  Q  R  R  G  T  I  L  S  P  G  Y  P  E  P  Y  G  N   1360

4081  AACTTGAACTGTATATGGAAGATCATAGTTACGGAGGGCTCGGGAATTCAGATCCAAGTG  4140
1361  N  L  N  C  I  W  K  I  I  V  T  E  G  S  G  I  Q  I  Q  V   1380

4141  ATCAGTTTTGCCACGGAGCAGAACTGGGACTCCCTTGAGATCCACGATGGTGGGGATGTG  4200
1381  I  S  F  A  T  E  Q  N  W  D  S  L  E  I  H  D  G  G  D  V   1400

4201  ACCGCACCCAGACTGGGAAGCTTCTCAGGCACCACAGTACCGGCACTGCTGAACAGTACT  4260
1401  T  A  P  R  L  G  S  F  S  G  T  T  V  P  A  L  L  N  S  T   1420

4261  TCCAACCAACTCTACCTGCATTTCCAGTCTGACATTAGTGTGGCAGCTGCTGGTTTCCAC  4320
1421  S  N  Q  L  Y  L  H  F  Q  S  D  I  S  V  A  A  A  G  F  H   1440

4321  CTGGAATACAAAACTGTAGGTCTTGCTGCATGCCAAGAACCAGCCCTCCCCAGCAACAGC  4380
1441  L  E  Y  K  T  V  G  L  A  A  C  Q  E  P  A  L  P  S  N  S   1460

4381  ATCAAAATCGGAGATCGGTACATGGTGAACGACGTGCTCTCCTTCCAGTGCGAGCCCGGG  4440
1461  I  K  I  G  D  R  Y  M  V  N  D  V  L  S  F  Q  C  E  P  G   1480

4441  TACACCCTGCAGGGCCGTTCCCACATTTCCTGTATGCCAGGGACCGTTCGCCGTTGGAAC  4500
1481  Y  T  L  Q  G  R  S  H  I  S  C  M  P  G  T  V  R  R  W  N   1500

4501  TATCCGTCTCCCCTGTGCATTGCAACCTGTGGAGGGACGCTGAGCACCTTGGGTGGTGTG  4560
1501  Y  P  S  P  L  C  I  A  T  C  G  G  T  L  S  T  L  G  G  V   1520
```

Figure 2E

```
4561  ATCCTGAGCCCCGGCTTCCCAGGTTCTTACCCCAACAACTTAGACTGCACCTGGAGGATC  4620
1521  I   L   S   P   G   F   P   G   S   Y   P   N   N   L   D   C   T   W   R   I   1540

4621  TCATTACCCATCGGCTATGGTGCACATATTCAGTTTCTGAATTTTTCTACCGAAGCTAAT  4680
1541  S   L   P   I   G   Y   G   A   H   I   Q   F   L   N   F   S   T   E   A   N   1560

4681  CATGACTTCCTTGAAATTCAAAATGGACCTTACCACACCAGCCCCATGATTGGACAATTT  4740
1561  H   D   F   L   E   I   Q   N   G   P   Y   H   T   S   P   M   I   G   Q   F   1580

4741  AGCGGCACGGATCTCCCCGCGGCCCTGCTGAGCACAACGCATGAAACCCTCATCCACTTT  4800
1581  S   G   T   D   L   P   A   A   L   L   S   T   T   H   E   T   L   I   H   F   1600

4801  TATAGTGACCATTCGCAAAACCGGCAAGGATTTAAACTTGCTTACCAAGCCTATGAATTA  4860
1601  Y   S   D   H   S   Q   N   R   Q   G   F   K   L   A   Y   Q   A   Y   E   L   1620

4861  CAGAACTGTCCAGATCCACCCCCATTTCAGAATGGGTACATGATCAACTCGGATTACAGC  4920
1621  Q   N   C   P   D   P   P   P   F   Q   N   G   Y   M   I   N   S   D   Y   S   1640

4921  GTGGGGCAATCAGTATCTTTCGAGTGTTATCCTGGGTACATTCTAATAGGCCATCCTGTC  4980
1641  V   G   Q   S   V   S   F   E   C   Y   P   G   Y   I   L   I   G   H   P   V   1660

4981  CTCACTTGTCAGCATGGGATCAACAGAAACTGGAACTACCCTTTTCCAAGATGTGATGCC  5040
1661  L   T   C   Q   H   G   I   N   R   N   W   N   Y   P   F   P   R   C   D   A   1680

5041  CCTTGTGGGTACAACGTAACTTCTCAGAACGGCACCATCTACTCCCCTGGCTTTCCTGAT  5100
1681  P   C   G   Y   N   V   T   S   Q   N   G   T   I   Y   S   P   G   F   P   D   1700

5101  GAGTATCCGATCCTGAAGGACTGCATTTGGCTCATCACGGTGCCTCCAGGGCACGGAGTT  5160
1701  E   Y   P   I   L   K   D   C   I   W   L   I   T   V   P   P   G   H   G   V   1720

5161  TACATCAACTTCACCCTGTTACAGACGGAAGCTGTCAACGATTACATTGCTGTTTGGGAC  5220
1721  Y   I   N   F   T   L   L   Q   T   E   A   V   N   D   Y   I   A   V   W   D   1740

5221  GGTCCCGATCAGAACTCACCCCAGCTGGGAGTTTTCAGTGGCAACACAGCCCTCGAAACG  5280
1741  G   P   D   Q   N   S   P   Q   L   G   V   F   S   G   N   T   A   L   E   T   1760

5281  GCGTATAGCTCCACCAACCAAGTCCTGCTCAAGTTCCACAGCGACTTTTCAAATGGAGGC  5340
1761  A   Y   S   S   T   N   Q   V   L   L   K   F   H   S   D   F   S   N   G   G   1780

5341  TTCTTTGTCCTCAATTTCCACGCATTTCAGCTCAAGAAATGTCAACCTCCCCCAGCGGTT  5400
1781  F   F   V   L   N   F   H   A   F   Q   L   K   K   C   Q   P   P   P   A   V   1800

5401  CCACAGGCAGAAATGCTTACTGAGGATGATGATTTCGAGATAGGAGATTTTGTGAAGTAC  5460
1801  P   Q   A   E   M   L   T   E   D   D   D   F   E   I   G   D   F   V   K   Y   1820

5461  CAGTGCCACCCCGGGTACACCTTGGTGGGGACCGACATTCTGACTTGCAAGCTCAGTTCC  5520
1821  Q   C   H   P   G   Y   T   L   V   G   T   D   I   L   T   C   K   L   S   S   1840

5521  CAGTTGCAGTTTGAGGGTTCTCTCCCAACATGTGAAGCACAATGCCCAGCAAATGAAGTC  5580
1841  Q   L   Q   F   E   G   S   L   P   T   C   E   A   Q   C   P   A   N   E   V   1860

5581  CGGACTGGATCATCGGGAGTCATTCTCAGTCCAGGGTATCCGGGTAATTATTTTAACTCC  5640
1861  R   T   G   S   S   G   V   I   L   S   P   G   Y   P   G   N   Y   F   N   S   1880

5641  CAGACTTGCTCTTGGAGTATTAAAGTGGAACCAAACTACAACATTACCATCTTTGTGGAC  5700
1881  Q   T   C   S   W   S   I   K   V   E   P   N   Y   N   I   T   I   F   V   D   1900
```

Figure 2F

```
5701  ACATTTCAAAGTGAAAAGCAGTTTGATGCACTGGAAGTGTTTGATGGTTCTTCTGGGCAA  5760
1901  T  F  Q  S  E  K  Q  F  D  A  L  E  V  F  D  G  S  S  G  Q   1920

5761  AGTCCTCTGCTAGTAGTCTTAAGTGGGAATCATACTGAACAATCAAATTTTACAAGCAGG  5820
1921  S  P  L  L  V  V  L  S  G  N  H  T  E  Q  S  N  F  T  S  R   1940

5821  AGTAATCAGTTATATCTCCGCTGGTCCACTGACCATGCCACCAGTAAGAAAGGATTCAAG  5880
1941  S  N  Q  L  Y  L  R  W  S  T  D  H  A  T  S  K  K  G  F  K   1960

5881  ATTCGCTATGCAGCACCTTACTGCAGTTTGACCCACCCCCTGAAGAATGGGGGTATTCTA  5940
1961  I  R  Y  A  A  P  Y  C  S  L  T  H  P  L  K  N  G  G  I  L   1980

5941  AACAGGACTGCAGGAGCGGTTGGAAGCAAAGTGCATTATTTTGCAAGCCTGGATACCGA  6000
1981  N  R  T  A  G  A  V  G  S  K  V  H  Y  F  C  K  P  G  Y  R   2000

6001  ATGGTCGGCCACAGCAATGCAACCTGTAGACGAAACCCACTTGGCATGTACCAGTGGGAC  6060
2001  M  V  G  H  S  N  A  T  C  R  R  N  P  L  G  M  Y  Q  W  D   2020

6061  TCCCTCACGCCACTCTGCCAGGCTGTGTCCTGTGGAATCCCAGAATCCCCAGGAAACGGT  6120
2021  S  L  T  P  L  C  Q  A  V  S  C  G  I  P  E  S  P  G  N  G   2040

6121  TCATTTACCGGGAACGAGTTCACTTTGGACAGTAAAGTGGTCTATGAATGTCATGAGGGC  6180
2041  S  F  T  G  N  E  F  T  L  D  S  K  V  V  Y  E  C  H  E  G   2060

6181  TTCAAGCTTGAATCCAGCCAGCAAGCAACAGCCGTGTGTCAAGAAGATGGGCTGTGGAGT  6240
2061  F  K  L  E  S  S  Q  Q  A  T  A  V  C  Q  E  D  G  L  W  S   2080

6241  AACAAGGGGAAGCCGCCCACGTGTAAGCCGGTCGCTTGCCCCAGCATTGAAGCTCAGCTC  6300
2081  N  K  G  K  P  P  T  C  K  P  V  A  C  P  S  I  E  A  Q  L   2100

6301  TCAGAACATGTCATCTGGAGGCTGGTTTCAGGATCCTTGAATGAGTACGGTGCTCAAGTA  6360
2101  S  E  H  V  I  W  R  L  V  S  G  S  L  N  E  Y  G  A  Q  V   2120

6361  TTGCTGAGCTGCAGTCCTGGTTACTACTTAGAAGGCTGGAGGCTCCTGCGGTGCCAGGCC  6420
2121  L  L  S  C  S  P  G  Y  Y  L  E  G  W  R  L  L  R  C  Q  A   2140

6421  AATGGGACGTGGAACATAGGAGATGAGAGGCCAAGCTGTCGAGTTATCTCGTGTGGAAGC  6480
2141  N  G  T  W  N  I  G  D  E  R  P  S  C  R  V  I  S  C  G  S   2160

6481  CTTTCCTTTCCCCCAAATGGCAACAAGATTGGAACGTTGACAGTTTATGGGGCCACAGCT  6540
2161  L  S  F  P  P  N  G  N  K  I  G  T  L  T  V  Y  G  A  T  A   2180

6541  ATATTTACGTGCAACACCGGCTACACGCTTGTGGGGTCTCATGTCAGAGAGTGCTTGGCA  6600
2181  I  F  T  C  N  T  G  Y  T  L  V  G  S  H  V  R  E  C  L  A   2200

6601  AATGGGCTCTGGAGCGGCAGCGAAACTCGATGTCTGGCTGGCCACTGCGGTTCCCCAGAC  6660
2201  N  G  L  W  S  G  S  E  T  R  C  L  A  G  H  C  G  S  P  D   2220

6661  CCGATTGTGAACGGTCACATTAGTGGAGATGGCTTCAGTTACAGAGACACGGTGGTTTAC  6720
2221  P  I  V  N  G  H  I  S  G  D  G  F  S  Y  R  D  T  V  V  Y   2240

6721  CAGTGCAATCCTGGTTTCCGGCTTGTGGGAACTTCCGTGAGGATATGCCTGCAAGACCAC  6780
2241  Q  C  N  P  G  F  R  L  V  G  T  S  V  R  I  C  L  Q  D  H   2260

6781  AAGTGGTCTGGACAAACGCCTGTCTGTGTCCCCATCACATGTGGTCACCCTGGAAACCCT  6840
2261  K  W  S  G  Q  T  P  V  C  V  P  I  T  C  G  H  P  G  N  P   2280
```

Figure 2G

```
6841  GCCCACGGATTCACTAATGGCAGTGAGTTCAACCTGAATGATGTCGTGAATTTCACCTGC  6900
2281  A  H  G  F  T  N  G  S  E  F  N  L  N  D  V  V  N  F  T  C   2300

6901  AACACGGGCTATTTGCTGCAGGGCGTGTCTCGAGCCCAGTGTCGGAGCAACGGCCAGTGG  6960
2301  N  T  G  Y  L  L  Q  G  V  S  R  A  Q  C  R  S  N  G  Q  W   2320

6961  AGTAGCCCTCTGCCCACGTGTCGAGTGGTGAACTGTTCTGATCCAGGCTTTGTGGAAAAT  7020
2321  S  S  P  L  P  T  C  R  V  V  N  C  S  D  P  G  F  V  E  N   2340

7021  GCCATTCGTCACGGGCAACAGAACTTCCCTGAGAGTTTTGAGTATGGAATGAGTATCCTG  7080
2341  A  I  R  H  G  Q  Q  N  F  P  E  S  F  E  Y  G  M  S  I  L   2360

7081  TACCATTGCAAGAAGGGATTTTACTTGCTGGGATCTTCAGCCTTGACCTGTATGGCAAAT  7140
2361  Y  H  C  K  K  G  F  Y  L  L  G  S  S  A  L  T  C  M  A  N   2380

7141  GGCTTATGGGACCGATCCCTGCCCAAGTGTTTGGCTATATCGTGTGGACACCCAGGGGTC  7200
2381  G  L  W  D  R  S  L  P  K  C  L  A  I  S  C  G  H  P  G  V   2400

7201  CCTGCCAACGCCGTCCTCACTGGAGAGCTGTTTACCTATGGCGCCGTCGTGCACTACTCC  7260
2401  P  A  N  A  V  L  T  G  E  L  F  T  Y  G  A  V  V  H  Y  S   2420

7261  TGCAGAGGGAGCGAGAGCCTCATAGGCAACGACACGAGAGTGTGCCAGGAAGACAGTCAC  7320
2421  C  R  G  S  E  S  L  I  G  N  D  T  R  V  C  Q  E  D  S  H   2440

7321  TGGAGCGGGGCACTGCCCCACTGCACAGGAAATAATCCTGGATTCTGTGGTGATCCGGGG  7380
2441  W  S  G  A  L  P  H  C  T  G  N  N  P  G  F  C  G  D  P  G   2460

7381  ACCCCAGCACATGGGTCTCGGCTTGGTGATGACTTTAAGACAAAGAGTCTTCTCCGCTTC  7440
2461  T  P  A  H  G  S  R  L  G  D  D  F  K  T  K  S  L  L  R  F   2480

7441  TCCTGTGAAATGGGGCACCAGCTGAGGGGCTCCCCTGAACGCACGTGTTTGCTCAATGGG  7500
2481  S  C  E  M  G  H  Q  L  R  G  S  P  E  R  T  C  L  L  N  G   2500

7501  TCATGGTCAGGACTGCAGCCGGTGTGTGAGGCCGTGTCCTGTGGCAACCCTGGCACACCC  7560
2501  S  W  S  G  L  Q  P  V  C  E  A  V  S  C  G  N  P  G  T  P   2520

7561  ACCAACGGAATGATTGTCAGTAGTGATGGCATTCTGTTCTCCAGCTCGGTCATCTATGCC  7620
2521  T  N  G  M  I  V  S  S  D  G  I  L  F  S  S  S  V  I  Y  A   2540

7621  TGCTGGGAAGGCTACAAGACCTCAGGGCTCATGACACGGCATTGCACAGCCAATGGGACC  7680
2541  C  W  E  G  Y  K  T  S  G  L  M  T  R  H  C  T  A  N  G  T   2560

7681  TGGACAGGCACTGCTCCCGACTGCACAATTATAAGTTGTGGGGATCCAGGCACACTAGCA  7740
2561  W  T  G  T  A  P  D  C  T  I  I  S  C  G  D  P  G  T  L  A   2580

7741  AATGGCATCCAGTTTGGGACCGACTTCACCTTCAACAAGACTGTGAGCTATCAGTGTAAC  7800
2581  N  G  I  Q  F  G  T  D  F  T  F  N  K  T  V  S  Y  Q  C  N   2600

7801  CCAGGCTATGTCATGGAAGCAGTCACATCCGCCACTATTCGCTGTACCAAAGACGGCAGG  7860
2601  P  G  Y  V  M  E  A  V  T  S  A  T  I  R  C  T  K  D  G  R   2620

7861  TGGAATCCGAGCAAACCTGTCTGCAAAGCCGTGCTGTGTCCTCAGCCGCCGCCGGTGCAG  7920
2621  W  N  P  S  K  P  V  C  K  A  V  L  C  P  Q  P  P  P  V  Q   2640

7921  AATGGAACAGTGGAGGGAAGTGATTTCCGCTGGGGCTCCAGCATAAGTTACAGCTGCATG  7980
2641  N  G  T  V  E  G  S  D  F  R  W  G  S  S  I  S  Y  S  C  M   2660
```

Figure 2H

```
7981  GACGGTTACCAGCTCTCTCACTCCGCCATCCTCTCCTGTGAAGGTCGCGGGGTGTGGAAA  8040
2661  D  G  Y  Q  L  S  H  S  A  I  L  S  C  E  G  R  G  V  W  K   2680

8041  GGAGAGATCCCCCAGTGTCTCCCTGTGTTCTGCGGAGACCCTGGCATCCCCGCAGAAGGG  8100
2681  G  E  I  P  Q  C  L  P  V  F  C  G  D  P  G  I  P  A  E  G   2700

8101  CGACTTAGTGGGAAAAGTTTCACCTATAAGTCCGAAGTCTTCTTCCAGTGCAAATCTCCA  8160
2701  R  L  S  G  K  S  F  T  Y  K  S  E  V  F  F  Q  C  K  S  P   2720

8161  TTTATACTCGTGGGATCCTCCAGAAGAGTCTGCCAAGCTGACGGCACGTGGAGCGGCATA  8220
2721  F  I  L  V  G  S  S  R  R  V  C  Q  A  D  G  T  W  S  G  I   2740

8221  CAACCCACCTGCATTGATCCTGCTCATAACACCTGCCCAGACCCTGGTACGCCACACTTT  8280
2741  Q  P  T  C  I  D  P  A  H  N  T  C  P  D  P  G  T  P  H  F   2760

8281  GGAATACAGAATAGCTCCAGAGGCTATGAGGTTGGAAGCACGGTTTTTTTCAGGTGCAGA  8340
2761  G  I  Q  N  S  S  R  G  Y  E  V  G  S  T  V  F  F  R  C  R   2780

8341  AAAGGCTACCATATTCAAGGTTCCACGACTCGCACCTGCCTTGCCAATTTAACATGGAGT  8400
2781  K  G  Y  H  I  Q  G  S  T  T  R  T  C  L  A  N  L  T  W  S   2800

8401  GGGATACAGACCGAATGTATACCTCATGCCTGCAGACAGCCAGAAACCCCGGCACACGCG  8460
2801  G  I  Q  T  E  C  I  P  H  A  C  R  Q  P  E  T  P  A  H  A   2820

8461  GATGTGAGAGCCATCGATCTTCCTACTTTCGGCTACACCTTAGTGTACACCTGCCATCCA  8520
2821  D  V  R  A  I  D  L  P  T  F  G  Y  T  L  V  Y  T  C  H  P   2840

8521  GGCTTTTTCCTCGCAGGGGGATCTGAGCACAGAACATGTAAAGCAGACATGAAATGGACA  8580
2841  G  F  F  L  A  G  G  S  E  H  R  T  C  K  A  D  M  K  W  T   2860

8581  GGAAAGTCGCCTGTGTGTAAAAGTAAAGGAGTGAGAGAAGTTAATGAAACAGTTACTAAA  8640
2861  G  K  S  P  V  C  K  S  K  G  V  R  E  V  N  E  T  V  T  K   2880

8641  ACTCCAGTTCCTTCAGATGTCTTTTTCGTCAATTCACTGTGGAAGGGGTATTATGAATAT  8700
2881  T  P  V  P  S  D  V  F  F  V  N  S  L  W  K  G  Y  Y  E  Y   2900

8701  TTAGGGAAAAGACAACCCGCCACTCTAACTGTTGACTGGTTCAATGCAACAAGCAGTAAG  8760
2901  L  G  K  R  Q  P  A  T  L  T  V  D  W  F  N  A  T  S  S  K   2920

8761  GTGAATGCCACCTTCAGCGAAGCCTCGCCAGTGGAGCTGAAGTTGACAGGCATTTACAAG  8820
2921  V  N  A  T  F  S  E  A  S  P  V  E  L  K  L  T  G  I  Y  K   2940

8821  AAGGAGGAGGCCCACTTACTCCTGAAAGCTTTTCAAATTAAAGGCCAGGCAGATATTTTT  8880
2941  K  E  E  A  H  L  L  L  K  A  F  Q  I  K  G  Q  A  D  I  F   2960

8881  GTAAGCAAGTTCGAAAATGACAACTGGGGACTAGATGGTTATGTGTCATCTGGACTTGAA  8940
2961  V  S  K  F  E  N  D  N  W  G  L  D  G  Y  V  S  S  G  L  E   2980

8941  AGAGGAGGATTTACTTTTCAAGGTGACATTCATGGAAAAGACTTTGGAAAATTTAAGCTA  9000
2981  R  G  G  F  T  F  Q  G  D  I  H  G  K  D  F  G  K  F  K  L   3000

9001  GAAAGGCAAGATCCTTTAAACCCAGATCAAGACTCTTCCAGTCATTACCACGGCACCAGC  9060
3001  E  R  Q  D  P  L  N  P  D  Q  D  S  S  S  H  Y  H  G  T  S   3020

9061  AGTGGCTCTGTGGCGGCTGCCATTCTGGTTCCTTTCTTTGCTCTAATTTTATCAGGGTTT  9120
3021  S  G  S  V  A  A  A  I  L  V  P  F  F  A  L  I  L  S  G  F   3040
```

Figure 2I

```
9121   GCATTTTACCTCTACAAACACAGAACGAGACCAAAAGTTCAATACAATGGCTATGCTGGG   9180
3041   A  F  Y  L  Y  K  H  R  T  R  P  K  V  Q  Y  N  G  Y  A  G     3060

9181   CATGAAAACAGCAATGGACAAGCATCGTTTGAAAACCCCATGTATGATACAAACTTAAAA   9240
3061   H  E  N  S  N  G  Q  A  S  F  E  N  P  M  Y  D  T  N  L  K     3080

9241   CCCACAGAAGCCAAGGCTGTGAGGTTTGACACAACTCTGAACACAGTCTGTACAGTGGTA   9300
3081   P  T  E  A  K  A  V  R  F  D  T  T  L  N  T  V  C  T  V  V     3100

9301   TAGCCCTCAGTGCCCCAACAGGACTGATTCATAGCCATACCTCTGATGGACAAGCAGTGA   9360
3101   *                                                              3101
9361   TTCCTTTGGTGCCATATACCACTCTCCCYTCCACTCTGGCTTTACTGCAGCGATCTTCAA   9420
9421   CCTTGTCTACTGGCATAAGTGCAGCGGGATCTCTACTCAAATGTGTCAGGGTCTTCTAC   9480
9481   GGATCAAACTACACATGCGTTTTCATTCCAAAAGTGGGTTCTAAATGCCTGGCTGCATCT   9540
9541   GTATGAAATCAAGGCACACTCCAGGAAGACTGCCACGTCGCGCCAACACGTCATACTCAA   9600
9601   TRCCTCAGACTTTCATATTTCTGTGTTGCTGAGATGCCTTTCAATGCAATCGTCTGGGCT   9660
9661   CGTGGATATGTCCCTCAGGTGCGGTGACAGAATGGTGGCACCACGATATGTGTTCTCTTG   9720
9721   TGTTGTTTTTCCTTTTTAAACCCCCATGAACACGAATACTCTGAAAAAAATAAAAAGCTT   9780
9781   TCTGGAAGAAGACACCTTTCTGATAGAGGCTCACACCTACAAATGCTTCACTCTGTCCTT   9840
9841   CCGAGACCTGACAAGCTTTGAGGACCTCACAGCTCCCCTGTGTGTTCATCTCTAGGGATG   9900
9901   TTTGCAATTTCCCAGTCAGCTGTTCTGTCGCAGAATGTTTAATGCACAATTTTTTGCACT   9960
9961   AGTGTGTTATGAATGACTAAGATTCTGATAAAAAAAATAAATTATTTACACAGGGTTTAT  10020
10021  ACACACTATCCATTGTATATAAGCATTATTTCATATTATCAAGCTAAACATTCCCCCATC  10080
10081  AGCTTAGTTGGAGTGTTAGGGAAAAGTATTCCTAGATATGGCACAGATTTTAAAAGGAAA  10140
10141  TACAGTATTGACGAGATTTATTTTATTATTGCTTCAATTAGCTCCATTTACGTGTTGAAT  10200
10201  TCATTGAAGAGGTCCAATGAGAAAAAAACAGAAGCCTCCTTATTTCACACGTTTTCCTCC  10260
10261  TTTAGTACCATCCTCATCCAATTACTGTCTCTCTGATACTACTTAATAGCAGGGGGTTTG  10320
10321  CAGAAATTTCTGTTTGCCATGTAAAACTGTGAATAGTAATTTATTTTAGATAGTCGATGA  10380
10381  ACTTGTGGGTTTTAGCTCACAATGCAGCCTTCCCTTTTGCAGTGTTTTTTTTT         10433
```

Figure 2

Map of Rat C3b/C4b Complement Receptor like cDNA (SEQ ID NO:3) and Amino Acid Sequences (SEQ ID NO:4)

```
   1  GATGCCGGGAAGGTGGGGACACCAGATCCGTCTTGTACGTGCTTACAGGCTCCAGTGTC   60
   1  D  A  G  K  V  G  D  T  R  S  V  L  Y  V  L  T  G  S  S  V   20

  61  CCTGACCTCATCGTGAGCATGAGCAATCAGATGTGGCTCCACCTGCAGTCAGACGACAGC  120
  21  P  D  L  I  V  S  M  S  N  Q  M  W  L  H  L  Q  S  D  D  S   40

 121  ATTGGTTCCCCAGGATTTAAAGCTGTGTACCAAGAAATCGAGAAGGGAGGCTGCGGGGAC  180
  41  I  G  S  P  G  F  K  A  V  Y  Q  E  I  E  K  G  G  C  G  D   60

 181  CCTGGCATCCCAGCCTACGGGAAGCGGACTGGCAGCAGCTTCTTGCACGGGGACACGCTC  240
  61  P  G  I  P  A  Y  G  K  R  T  G  S  S  F  L  H  G  D  T  L   80

 241  ACCTTTGAGTGCCAGGCAGCTTTTGAGCTGGTAGGAGAGAGAGTGATTACGTGCCAGAGA  300
  81  T  F  E  C  Q  A  A  F  E  L  V  G  E  R  V  I  T  C  Q  R  100

 301  AACAACCAGTGGTCCGGCAACAAGCCAAGCTGTGTGTTTTCATGTTTCTTCAACTTCACG  360
 101  N  N  Q  W  S  G  N  K  P  S  C  V  F  S  C  F  F  N  F  T  120

 361  GCGTCCTCTGGGATCATCCTGTCGCCAAACTATCCTGAGGAATATGGCAACAACATGAAT  420
 121  A  S  S  G  I  I  L  S  P  N  Y  P  E  E  Y  G  N  N  M  N  140

 421  TGTGTGTGGTTGATTATATCTGAGCCCGGGAGCCGGATTCACCTCATCTTCAATGATTTC  480
 141  C  V  W  L  I  I  S  E  P  G  S  R  I  H  L  I  F  N  D  F  160

 481  GATGTGGAGCCTCAGTTTGACTTCCTTGCGGTCAAAGATGATGGGATTTCTGACATCACA  540
 161  D  V  E  P  Q  F  D  F  L  A  V  K  D  D  G  I  S  D  I  T  180

 541  GTCCTCGGGACTTTCTCTGGCAATGAGGTGCCTGCACAGCTGGCC.GCAGTGGACACATA  600
 181  V  L  G  T  F  S  G  N  E  V  P  A  Q  L  A  X  S  G  H  I  200

 601  GTACGCCTGGAGTTTCAGTCCGATCACTCTACCACGGGCAGAGGGTTCAACATCATATAC  660
 201  V  R  L  E  F  Q  S  D  H  S  T  T  G  R  G  F  N  I  I  Y  220

 661  ACCACATTTGGTCAGAACGAGTGTCATGACCCTGGGATCCCTGTGAATGGACGGCGCTTT  720
 221  T  T  F  G  Q  N  E  C  H  D  P  G  I  P  V  N  G  R  R  F  240

 721  GGAGACAGGTTTCTGCTGGGAAGTTCTGTGTCCTTCCACTGTGATGATGGCTTTGTGAAG  780
 241  G  D  R  F  L  L  G  S  S  V  S  F  H  C  D  D  G  F  V  K  260

 781  ACTCAGGGTTCTGAGTCTATCACATGCATCTTGCAAGATGGAAACGTGGTCTGGAGCTCT  840
 261  T  Q  G  S  E  S  I  T  C  I  L  Q  D  G  N  V  V  W  S  S  280

 841  ACTGTCCCTCGCTGTGAAGCTCCTTGTGGTGGGCATCTGACAGCTTCTAGTGGGGTCATA  900
 281  T  V  P  R  C  E  A  P  C  G  G  H  L  T  A  S  S  G  V  I  300

 901  TTACCTCCAGGATGGCCAGGATATTACAAAGATTCTTTAAATTGCGAATGGGTCATTGAA  960
 301  L  P  P  G  W  P  G  Y  Y  K  D  S  L  N  C  E  W  V  I  E  320

 961  GCCAAACCAGGACATTCCATCAAAATAACATTTGACAGGTTCCAGACAGAAGTCAATTAT 1020
 321  A  K  P  G  H  S  I  K  I  T  F  D  R  F  Q  T  E  V  N  Y  340

1021  GATACTCTGGAAGTCCGGGATGGGCCAACCAGCTCATCCCCACTGATTGGGGAGTACCAT 1080
 341  D  T  L  E  V  R  D  G  P  T  S  S  S  P  L  I  G  E  Y  H  360
```

Figure 3A

```
1081 GGCACCCAGGCTCCACAGTTCCTCATCAGCACAGGGAACTACATGTACCTGCTGTTTACC 1140
 361 G  T  Q  A  P  Q  F  L  I  S  T  G  N  Y  M  Y  L  L  F  T   380

1141 ACTGACAGCAGCCGCGCTAGTGTTGGCTTCCTCATCCACTATGAGAGTGTGACTCTTGAA 1200
 381 T  D  S  S  R  A  S  V  G  F  L  I  H  Y  E  S  V  T  L  E   400

1201 TCTGACTCCTGTCTGGACCCGGGCATCCCTGTAAATGGTCATCGGCATGGCAGTAACTTT 1260
 401 S  D  S  C  L  D  P  G  I  P  V  N  G  H  R  H  G  S  N  F   420

1261 GGTATCAGATCTACAGTGACCTTCAGCTGTGACCCTGGGTACACGCTCAGTGATGACGAT 1320
 421 G  I  R  S  T  V  T  F  S  C  D  P  G  Y  T  L  S  D  D  D   440

1321 CCCCTCATCTGTGAGAAGAACCATCAGTGGAACCACGCCTTGCCCAGCTGTGATGCCCTG 1380
 441 P  L  I  C  E  K  N  H  Q  W  N  H  A  L  P  S  C  D  A  L   460

1381 TGTGGAGGCTACATCCATGGAAAGAGTGGGACTGTTCTTTCACCAGGATTTCCAGACTTT 1440
 461 C  G  G  Y  I  H  G  K  S  G  T  V  L  S  P  G  F  P  D  F   480

1441 TATCCAAACTCTCTGAACTGTACATGGACCATTGAAGTCTCTCATGGCAAGGGAGTGCAG 1500
 481 Y  P  N  S  L  N  C  T  W  T  I  E  V  S  H  G  K  G  V  Q   500

1501 ATGAATTTCCACACCTTTCACCTTGAAAGTTCCCACGACTATTTGCTGATCACAGAGGAT 1560
 501 M  N  F  H  T  F  H  L  E  S  S  H  D  Y  L  L  I  T  E  D   520

1561 GGGAGTTTCTCAGAGCCGGTAGCCAGGCTCACTGGGTCGGTCCTGCCTCACACCATTAAG 1620
 521 G  S  F  S  E  P  V  A  R  L  T  G  S  V  L  P  H  T  I  K   540

1621 GCTGGCTTGTTTGGAAACTTCACTGCGCAACTCAGGTTCATCTCTGACTTCTCCATCTCC 1680
 541 A  G  L  F  G  N  F  T  A  Q  L  R  F  I  S  D  F  S  I  S   560

1681 TATGAAGGCTTCAACATTACGTTTGCAGAATATGACCTAgAACCCTGTGATGACCCTGGA 1740
 561 Y  E  G  F  N  I  T  F  A  E  Y  D  L  E  P  C  D  D  P  G   580

1741 GTCCCTGCCTACAGTCGCAGAATTGGGTTCCAGTTCGGTGTGGGTGACACCCTGGCTTTC 1800
 581 V  P  A  Y  S  R  R  I  G  F  Q  F  G  V  G  D  T  L  A  F   600

1801 ACCTGCTTCCAGGGATACCGCTTAGAAGGTGCAACCAAGCTTACCTGCCTGGGTGGGGGA 1860
 601 T  C  F  Q  G  Y  R  L  E  G  A  T  K  L  T  C  L  G  G  G   620

1861 CGCCGAGTGTGGAGTGCACCTCTGCCAAGGTGTGTGGCTGAATGTGGAGCAAGCGTCAAA 1920
 621 R  R  V  W  S  A  P  L  P  R  C  V  A  E  C  G  A  S  V  K   640

1921 GGAAATGAAGGAACATTACTCTCTCCAAATTTCCCATCCAATTATGATAATAACCATGAG 1980
 641 G  N  E  G  T  L  L  S  P  N  F  P  S  N  Y  D  N  N  H  E   660

1981 TGTATCTATAAAATAGAAACAGAAGCCGGAAAGGGGATCCATCTCAGAGCCCGAACCTTC 2040
 661 C  I  Y  K  I  E  T  E  A  G  K  G  I  H  L  R  A  R  T  F   680

2041 CAACTCTTCGAAGGAGACACTCTAAAGGTTTATGATGGAAAGGACAGCTCCTCGAGGTCA 2100
 681 Q  L  F  E  G  D  T  L  K  V  Y  D  G  K  D  S  S  S  R  S   700

2101 CTGGGAGTCTTCACAAGAAGTGAACTGATGGGGCTGGTGCTAAACAGCACCTCCAACCAC 2160
 701 L  G  V  F  T  R  S  E  L  M  G  L  V  L  N  S  T  S  N  H   720

2161 CTGAGGCTGGAGTTCAACTCTAACGGGTCAGATACCGCCCAAGGCTTCCAGCTCACCTAC 2220
 721 L  R  L  E  F  N  S  N  G  S  D  T  A  Q  G  F  Q  L  T  Y   740
```

Figure 3C

```
2221  ACCAGTTTTGACCTAGTGAAATGTGAGGATCCAGGCATCCCTAACTATGGCTACAGGATC  2280
 741  T  S  F  D  L  V  K  C  E  D  P  G  I  P  N  Y  G  Y  R  I    760

2281  CGAGATGATGGTCACTTCACAGACACTGTGGTTCTCTACAGCTGCAACCCAGGCTACGCA  2340
 761  R  D  D  G  H  F  T  D  T  V  V  L  Y  S  C  N  P  G  Y  A    780

2341  ATGCATGGCAGCAGTACCCtGACCtGCCTGAGTGGGGACCGAAGGGTGTGGGACAAACCT  2400
 781  M  H  G  S  S  T  L  T  C  L  S  G  D  R  R  V  W  D  K  P    800

2401  ATGCCTTCCTGTGTGGCGGAATGTGGTGGTCTCGTCCATGCAGCCACATCAGGACGCATA  2460
 801  M  P  S  C  V  A  E  C  G  G  L  V  H  A  A  T  S  G  R  I    820

2461  CTCTCTCCTGGCTACCCTGCCCCATATGACAACAACCTTCATTGCACTTGGACCATAGAG  2520
 821  L  S  P  G  Y  P  A  P  Y  D  N  N  L  H  C  T  W  T  I  E    840

2521  GCTGATCCTGGCAAGACCAYCAGCCTCCATTTCATTGTGTTTGACACTGAAACGGCGCAC  2580
 841  A  D  P  G  K  T  X  S  L  H  F  I  V  F  D  T  E  T  A  H    860

2581  GACATCCTCAAGGTCTGGGATGGTCCAGTGGACAGCAACATCCTGCTGAAGGAGTGGAGC  2640
 861  D  I  L  K  V  W  D  G  P  V  D  S  N  I  L  L  K  E  W  S    880

2641  GGCTCGGCCCTTCCTGAGGACATCCACAGCACCTTCAACTCGCTCACCCTGCAGTTCGAT  2700
 881  G  S  A  L  P  E  D  I  H  S  T  F  N  S  L  T  L  Q  F  D    900

2701  AGTGACTTCTTCATCAGCAAGTCCGGCTTCTCCATCCAGTTCTCTACTTCCATTGCATCC  2760
 901  S  D  F  F  I  S  K  S  G  F  S  I  Q  F  S  T  S  I  A  S    920

2761  ACCTGCAATGACCCTGGGATGCCTCAGAATGGAACCCGCTATGGTGACAGCCGGGAACCT  2820
 921  T  C  N  D  P  G  M  P  Q  N  G  T  R  Y  G  D  S  R  E  P    940

2821  GGAGACACCATCACCTTCCAGTGTGACCCTGGATACCAGCTCCAAGGGCAAGCCAAGATC  2880
 941  G  D  T  I  T  F  Q  C  D  P  G  Y  Q  L  Q  G  Q  A  K  I    960

2881  ACTTGTGTGCAGCTTAACAACCGCTTCTTCTGGCAACCAGACCCTCCGTCATGCATAGCT  2940
 961  T  C  V  Q  L  N  N  R  F  F  W  Q  P  D  P  P  S  C  I  A    980

2941  GCTTGTGGTGGGAATCTGACAGGCCCTGCTGGAGTGATTTTATCCCCAAACTACCCACAG  3000
 981  A  C  G  G  N  L  T  G  P  A  G  V  I  L  S  P  N  Y  P  Q   1000

3001  CCATACCCTCCTGGGAAGGAGTGTGACTGGAGAATTAAGGTGAACCCAGACTTTGTCATT  3060
1001  P  Y  P  P  G  K  E  C  D  W  R  I  K  V  N  P  D  F  V  I   1020

3061  GCCTTAATATTCAAAAGTTTTAGCATGGAGCCAAGTTACGACTTCCTGCATATCTATGAA  3120
1021  A  L  I  F  K  S  F  S  M  E  P  S  Y  D  F  L  H  I  Y  E   1040

3121  GGGAAGGACTCCAACAGCCCACTGATCGGAAGCTTCCAGGGTTCTCAAGCCCCAGAGAGG  3180
1041  G  K  D  S  N  S  P  L  I  G  S  F  Q  G  S  Q  A  P  E  R   1060

3181  ATTGAGAGCAGTGGTAACAGCCTCTTCCTGGCATTCAGGAGTGATGCCTCTGTTGGCCTG  3240
1061  I  E  S  S  G  N  S  L  F  L  A  F  R  S  D  A  S  V  G  L   1080

3241  TCCGGGTTTGCCATTGAATTTAAAGAGAAACCACGGGAAGCTTGCTTTGACCCTGGGAAC  3300
1081  S  G  F  A  I  E  F  K  E  K  P  R  E  A  C  F  D  P  G  N   1100

3301  ATAATGAACGGGACAAGGATTGGAACGGACTTTAAGCTGGGCTCTACAGTTACCTATCAA  3360
1101  I  M  N  G  T  R  I  G  T  D  F  K  L  G  S  T  V  T  Y  Q   1120
```

Figure 3D

3361 TGTGACTCTGGTTACAAGATTGTGGATCCCTCATCCATTGAGTGTGTGACAGGGGCTGAT 3420
1121 C D S G Y K I V D P S S I E C V T G A D 1140

3421 GGGAAGCCGTCCTGGGACCGGGCACTGCCTGCCTGCCAAGCACCCTGTGGAGGCCAATAC 3480
1141 G K P S W D R A L P A C Q A P C G G Q Y 1160

3481 ATGGGCTCGGAGGGGGTAGTTTTGTCACCAAACTACCCTCATAACTACACGGCTGGGCAG 3540
1161 M G S E G V V L S P N Y P H N Y T A G Q 1180

3541 ATATGCATCTATTCCATCACGGTGCCCAAGGAATTTGTGGTGTTTGGACAGTTTGCCTAT 3600
1181 I C I Y S I T V P K E F V V F G Q F A Y 1200

3601 TTCCAGACTGCGCTGAACGACTTGGCAGAATTGTTTGATGGAACCCATCCTCAGGCCAGG 3660
1201 F Q T A L N D L A E L F D G T H P Q A R 1220

3661 CTTCTCAGTTCTCTCTCTGGTTCCCATTCAGGTGAAACACTCCCGCTGGCTACATCCAAT 3720
1221 L L S S L S G S H S G E T L P L A T S N 1240

3721 CAGATTCTGCTTCGCTTCAGCGCAAAGAGCGGAGCTTCTGCACGGGGTTTCCACTTCGTC 3780
1241 Q I L L R F S A K S G A S A R G F H F V 1260

3781 TACCAAGCCGTCCCACGCACCAGTGACACGCAGTGCAGCTCCGTCCCTGAGCCCAGATAT 3840
1261 Y Q A V P R T S D T Q C S S V P E P R Y 1280

3841 GGGAGAAGGATTGGTTCTGAGTTCTCTGCAGGCTCCATCGTCCGATTCGAGTGCAACCCA 3900
1281 G R R I G S E F S A G S I V R F E C N P 1300

3901 GGTTACCTGCTGCAAGGCTCCACAGCCATCCGTTGTCAGTCTGTGCCAAACGCTTTGGCC 3960
1301 G Y L L Q G S T A I R C Q S V P N A L A 1320

3961 CAGTGGAATGACACCATCCCAAGCTGTGTAGTTCCATGCAGTGGCAATTTCACTCAGAGA 4020
1321 Q W N D T I P S C V V P C S G N F T Q R 1340

4021 AGAGGGACAATCTTATCTCCAGGCTACCCTGAGCCCTATGGGAACAACCTGAACTGTGTA 4080
1341 R G T I L S P G Y P E P Y G N N L N C V 1360

4081 TGGAAGATCATAGTATCGGAGGGCTCAGGGATCCAGATCCAAGTGATTAGCTTTGCCACG 4140
1361 W K I I V S E G S G I Q I Q V I S F A T 1380

4141 GAGCAGAACTGGGACTCCCTGGAGATCCATGACGGAGGAGACATGACGGCCCCCAGACTG 4200
1381 E Q N W D S L E I H D G G D M T A P R L 1400

4201 GGCAGCTTCTCAGGTACCACAGTGCCCGCACTGCTGAATAGCACCTCCAACCAGCTCTGC 4260
1401 G S F S G T T V P A L L N S T S N Q L C 1420

4261 CTGCACTTCCAGTCGGACATCAGTGTTGCCGCTGCGGGCTTTCACCTGGAATACAAAACG 4320
1421 L H F Q S D I S V A A A G F H L E Y K T 1440

4321 GTGGGTCTGGCTGCGTGCCAGGAACCTGCTCTCCCGAGCAACGGCATCAAGATAGGAGAC 4380
1441 V G L A A C Q E P A L P S N G I K I G D 1460

4381 CGCTATATGGTGAACGATGTGCTGTCCTTCCAGTGCGAGCCTGGGTACACCTTGCAGGGC 4440
1461 R Y M V N D V L S F Q C E P G Y T L Q G 1480

4441 CGCTCACACATTTCTTGTATGCCGGGAACTGTACGTCGCTGGAACTATCCTTCCCCTCTG 4500
1481 R S H I S C M P G T V R R W N Y P S P L 1500

Figure 3F

```
4501 TGCATTGCCACCTGTGGTGGGACACTGACCAGCATGAGTGGAGTGATCCTGAGCCCAGGC 4560
1501 C  I  A  T  C  G  G  T  L  T  S  M  S  G  V  I  L  S  P  G  1520

4561 TTCCCAGGGTCATACCCCAACAACCTGGACTGCACCTGGAAGATATCCCTGCCCATTGGC 4620
1521 F  P  G  S  Y  P  N  N  L  D  C  T  W  K  I  S  L  P  I  G  1540

4621 TATGGTGCACATATCCAATTTCTGAATTTCTCAACTGAAGCCAACCATGACTACCTGGAG 4680
1541 Y  G  A  H  I  Q  F  L  N  F  S  T  E  A  N  H  D  Y  L  E  1560

4681 ATCCAGAATGGCCCTTACCACAGTAGTCCAATGATGGGACAGTTCAGTGGCCCTGACCTG 4740
1561 I  Q  N  G  P  Y  H  S  S  P  M  M  G  Q  F  S  G  P  D  L  1580

4741 CCTGCGTCACTGCTGAGCACCACACATGAAACCCTCATCCGCTTCTATAGTGACCACTCA 4800
1581 P  A  S  L  L  S  T  T  H  E  T  L  I  R  F  Y  S  D  H  S  1600

4801 CAGAACCGACAAGGATTTAAACTCAGTTACCAAGCTTATGAGTTACAGAACTGCCCGGAC 4860
1601 Q  N  R  Q  G  F  K  L  S  Y  Q  A  Y  E  L  Q  N  C  P  D  1620

4861 CCACCCGCATTCCAGAATGGGTTCATGATCAACTCCGATTACAGCGTGGGCCAGTCGATC 4920
1621 P  P  A  F  Q  N  G  F  M  I  N  S  D  Y  S  V  G  Q  S  I  1640

4921 TCATTTGAGTGCTACCCGGGCTACATCTTGCTAGGCCACCCTGTGCTCACCTGCCAGCAT 4980
1641 S  F  E  C  Y  P  G  Y  I  L  L  G  H  P  V  L  T  C  Q  H  1660

4981 GGCACTGACAGGAACTGGAACTACCCTTTCCCACGGTGTGACGCTCCCTGTGGGTATAAT 5040
1661 G  T  D  R  N  W  N  Y  P  F  P  R  C  D  A  P  C  G  Y  N  1680

5041 GTGACATCACAGAATGGCACCATTTATTCCCCTGGGTTCCCAGACGAGTATCCAATTCTG 5100
1681 V  T  S  Q  N  G  T  I  Y  S  P  G  F  P  D  E  Y  P  I  L  1700

5101 AAGGACTGCCTGTGGCTGGTCACTGTCCCTCCAGGACATGGAGTGTACATCAACTTCACC 5160
1701 K  D  C  L  W  L  V  T  V  P  P  G  H  G  V  Y  I  N  F  T  1720

5161 TTGCTGCAGACTGAGGCTGTAAATGACTACATCGCTGTGTGGGATGGTCCTGACCAGAAC 5220
1721 L  L  Q  T  E  A  V  N  D  Y  I  A  V  W  D  G  P  D  Q  N  1740

5221 TCGCCTCAGCTCGGGGTCTTCAGTGGAAACACTGCCCTCGAGACAGCATACAGCTCCACC 5280
1741 S  P  Q  L  G  V  F  S  G  N  T  A  L  E  T  A  Y  S  S  T  1760

5281 AACCAGGTCTTGCTCAAATTCCACAGCGATTTCTCCAATGGAGGCTTCTTTGTCCTCAAT 5340
1761 N  Q  V  L  L  K  F  H  S  D  F  S  N  G  G  F  F  V  L  N  1780

5341 TTTCATGCATTTCAACTGAAGAGGTGCCCGCCTCCTCCAGTAGTGCCGCAGGCTGACCTG 5400
1781 F  H  A  F  Q  L  K  R  C  P  P  P  P  V  V  P  Q  A  D  L  1800

5401 CTTACAGAAGATGAAGACTTTGAAATAGGGGACTTCGTAAAGTACCAGTGCCATCCAGGG 5460
1801 L  T  E  D  E  D  F  E  I  G  D  F  V  K  Y  Q  C  H  P  G  1820

5461 TACACGCTGTTGGGAAGTGACACCCTGACATGCAAGCTCAGCTCACAGCTATTGTTCCAA 5520
1821 Y  T  L  L  G  S  D  T  L  T  C  K  L  S  S  Q  L  L  F  Q  1840

5521 GGCTCTCCACCTACCTGTGAAGCACAATGCCCAGCCAATGAAGTGCGAACAGAGTCTTCT 5580
1841 G  S  P  P  T  C  E  A  Q  C  P  A  N  E  V  R  T  E  S  S  1860

5581 GGGGTGATTCTCAGTCCTGGGTACCCAGGCAACTATTTTAACTCCCAGACATGTGCTTGG 5640
1861 G  V  I  L  S  P  G  Y  P  G  N  Y  F  N  S  Q  T  C  A  W  1880
```

Figure 3G

```
5641 AGTATTAAAGTGGAGCCAAACTTTAACATTACGCTCTTTGTGGACACCTTTCAAAGTGAA 5700
1881 S  I  K  V  E  P  N  F  N  I  T  L  F  V  D  T  F  Q  S  E  1900

5701 AAGCAATTTGATGCACTGGAAGTATTTGATGGTTCTTCTGGGCAAAGTCCTTTGTTAGTG 5760
1901 K  Q  F  D  A  L  E  V  F  D  G  S  S  G  Q  S  P  L  L  V  1920

5761 GTCTTAAGTGGGAACCACACTGAACAGTCCAATTTTACCAGCAGAAGTAACCATCTGTAC 5820
1921 V  L  S  G  N  H  T  E  Q  S  N  F  T  S  R  S  N  H  L  Y  1940

5821 CTCCGCTGGTCCACAGATCATGCAACCAGCAAGAAAGGATTCAAGATTCGCTATGCAGCT 5880
1941 L  R  W  S  T  D  H  A  T  S  K  K  G  F  K  I  R  Y  A  A  1960

5881 CCTTACTGCAGCCTCACCTCTACACTCAAGAATGGTGGCGTTTTAAATAAAACCGCAGGC 5940
1961 P  Y  C  S  L  T  S  T  L  K  N  G  G  V  L  N  K  T  A  G  1980

5941 GCCCTGGGGAGCAAGGTGCAGTATTTCTGCAAGCCTGGATATCGAATGATTGGCCACAGC 6000
1981 A  L  G  S  K  V  Q  Y  F  C  K  P  G  Y  R  M  I  G  H  S  2000

6001 AACGCCACCTGCAGGCGGAACCCAGTGGGCGTGTACCAGTGGGACTCGATGGCACCGCTT 6060
2001 N  A  T  C  R  R  N  P  V  G  V  Y  Q  W  D  S  M  A  P  L  2020

6061 TGCCAGGCTGTGTCCTGTGGAATTCCAGAGGCTCCAGGAAATGGCTCGTTCACAGGCAAT 6120
2021 C  Q  A  V  S  C  G  I  P  E  A  P  G  N  G  S  F  T  G  N  2040

6121 GAGTTCACCTTAGACAGTAAAGTGACTTATGAATGTAATGAAGGCTTCAAGCTGGATGCC 6180
2041 E  F  T  L  D  S  K  V  T  Y  E  C  N  E  G  F  K  L  D  A  2060

6181 AGTCAGCAAGCCACTGCTGTGTGTCAAGAAGATGGCCTGTGGAGCAACAGAGGAAAGCCA 6240
2061 S  Q  Q  A  T  A  V  C  Q  E  D  G  L  W  S  N  R  G  K  P  2080

6241 CCCACGTGCAAACCGGTGCCCTGCCCCAGCATCGAAGGCCAGCTGTCAGAGCACGTGCTC 6300
2081 P  T  C  K  P  V  P  C  P  S  I  E  G  Q  L  S  E  H  V  L  2100

6301 TGGAGGCTGGTTTCGGGATCATTGAATGAATATGGAGCTCAAGTTCTCCTCAGCTGTAGT 6360
2101 W  R  L  V  S  G  S  L  N  E  Y  G  A  Q  V  L  L  S  C  S  2120

6361 CCTGGCTACTTCTTGCAGGGTCAGAGGCTGTTGCAGTGCCAAGCCAATGGGACCTGGAAC 6420
2121 P  G  Y  F  L  Q  G  Q  R  L  L  Q  C  Q  A  N  G  T  W  N  2140

6421 ACTGAGGAGGACAGACCCAGATGTAAAGTCATCTCCTGTGGAAGCCTGTCCTTTCCCCCA 6480
2141 T  E  E  D  R  P  R  C  K  V  I  S  C  G  S  L  S  F  P  P  2160

6481 AATGGTAACAAGATAGGGACGCTCACTATGTATGGAGCCACCGCCATCTTTACCTGCAAT 6540
2161 N  G  N  K  I  G  T  L  T  M  Y  G  A  T  A  I  F  T  C  N  2180

6541 ACCGGCTACACACTTGTAGGCTCCCATGTCCGGGAGTGCTTGGCCAATGGTCTCTGGAGC 6600
2181 T  G  Y  T  L  V  G  S  H  V  R  E  C  L  A  N  G  L  W  S  2200

6601 GGATCTGAAACAAGGTGCCTGGCGGGTCATTGTGGCTCTCCAGACCCCATTGTGAATGGC 6660
2201 G  S  E  T  R  C  L  A  G  H  C  G  S  P  D  P  I  V  N  G  2220

6661 CATATCAGTGGCGATGGCTTCAGCTACAGGGACACAGTGGTCTACCAATGCAACCCTGGG 6720
2221 H  I  S  G  D  G  F  S  Y  R  D  T  V  V  Y  Q  C  N  P  G  2240

6721 TTTCGACTCGTAGGCACGTCTGTGAGGATTTGCCTGCAGGACCACAAGTGGTCGGGGCAG 6780
2241 F  R  L  V  G  T  S  V  R  I  C  L  Q  D  H  K  W  S  G  Q  2260
```

Figure 3H

```
6781  ACCCCCGTTTGCGTCCCCATCACATGTGGACACCCTGGAAACCCTGCCCATGGCCTCACC  6840
2261  T  P  V  C  V  P  I  T  C  G  H  P  G  N  P  A  H  G  L  T   2280

6841  AACGGCAGCGAGTTCAACCTGAATGACCTTGTGAATTTCACCTGCCATACGGGCTACCTG  6900
2281  N  G  S  E  F  N  L  N  D  L  V  N  F  T  C  H  T  G  Y  L   2300

6901  CTGCAGGGTGCCTCCCGAGCCCAATGTCGGAGCAACGGCCAGTGGAGCAGCCCCTTGCCT  6960
2301  L  Q  G  A  S  R  A  Q  C  R  S  N  G  Q  W  S  S  P  L  P   2320

6961  ATCTGCCGAGTGGTGAACTGTTCCGATCCTGGATTTGTGGAAAATGCAGTTCGCCACGGG  7020
2321  I  C  R  V  V  N  C  S  D  P  G  F  V  E  N  A  V  R  H  G   2340

7021  CAACAGAACTTTCCAGAGAGTTTCGAGTATGGGACAAGTGTGATGTATCACTGCAAGAAG  7080
2341  Q  Q  N  F  P  E  S  F  E  Y  G  T  S  V  M  Y  H  C  K  K   2360

7081  GGGTTCTACCTACTGGGCTCTTCTGCCCTGACCTGCATGGCAAGTGGCTTGTGGGACCGC  7140
2361  G  F  Y  L  L  G  S  S  A  L  T  C  M  A  S  G  L  W  D  R   2380

7141  TCCTTACCCAAGTGTCTGGCTATATCATGTGGGCATCCTGGGGTCCCCGCTAATGCTGTC  7200
2381  S  L  P  K  C  L  A  I  S  C  G  H  P  G  V  P  A  N  A  V   2400

7201  CTGACTGGAGAATTGTTTACATTTGGAGCCACAGTTCAGTACTCCTGCAAAGGGGGCCAG  7260
2401  L  T  G  E  L  F  T  F  G  A  T  V  Q  Y  S  C  K  G  G  Q   2420

7261  ATTCTCACAGGCAATAGCACAAGAGTCTGCCAAGAAGACAGTCACTGGAGTGGATCCCTT  7320
2421  I  L  T  G  N  S  T  R  V  C  Q  E  D  S  H  W  S  G  S  L   2440

7321  CCCCATTGTTCAGGAAATAGTCCTGGATTTTGTGGTGATCCAGGGACCCCAGCACATGGG  7380
2441  P  H  C  S  G  N  S  P  G  F  C  G  D  P  G  T  P  A  H  G   2460

7381  TCTCGTCTTGGGGATGAGTTTAAGACAAAGAGTCTTTTGCGATTCTCCTGTGAGATGGGC  7440
2461  S  R  L  G  D  E  F  K  T  K  S  L  L  R  F  S  C  E  M  G   2480

7441  CACCAGCTGCGGGGTTCTGCAGAGCGCACATGCCTGGTGAATGGGTCCTGGTCAGGAGTC  7500
2481  H  Q  L  R  G  S  A  E  R  T  C  L  V  N  G  S  W  S  G  V   2500

7501  CAGCCTGTGTGTGAGGCCGTGTCCTGTGGAAACCCTGGCACCCCTACCAATGGGATGATC  7560
2501  Q  P  V  C  E  A  V  S  C  G  N  P  G  T  P  T  N  G  M  I   2520

7561  CTCAGCAGCGATGGAATCCTCTTCTCCAGCTCTGTCATCTATGCCTGCTGGGAAGGCTAC  7620
2521  L  S  S  D  G  I  L  F  S  S  S  V  I  Y  A  C  W  E  G  Y   2540

7621  AAGACCTCGGGGCTCATGACGCGGCACTGCACAGCGAACGGGACATGGACAGGCACAGCC  7680
2541  K  T  S  G  L  M  T  R  H  C  T  A  N  G  T  W  T  G  T  A   2560

7681  CCTGACTGTACAATCATCAGCTGTGGTGATCCTGGCACACTGCCCAATGGCATCCAGTTT  7740
2561  P  D  C  T  I  I  S  C  G  D  P  G  T  L  P  N  G  I  Q  F   2580

7741  GGGACAGACTTCACTTTCAACAAGACCGTGAGCTATCAGTGCAACCCTGGCTACCTGATG  7800
2581  G  T  D  F  T  F  N  K  T  V  S  Y  Q  C  N  P  G  Y  L  M   2600

7801  GAGCCCCCAACATCACCCACCATCCGCTGCACCAAAGATGGTACATGGAATCAGACCCGG  7860
2601  E  P  P  T  S  P  T  I  R  C  T  K  D  G  T  W  N  Q  T  R   2620

7861  CCCCTCTGCAAAGCTGTTCTATGCAGCCAGCCTCCCTCAGTGCCAAACGGAAAGGTGGAG  7920
2621  P  L  C  K  A  V  L  C  S  Q  P  P  S  V  P  N  G  K  V  E   2640
```

Figure 3I

7921 GGGTCAGACTTCCGATGGGGTGCCAGCATAAGCTACAGTTGTGTGGATGGCTACCAGCTC 7980
2641 G S D F R W G A S I S Y S C V D G Y Q L 2660

7981 TCCCACTCGGCCATCCTGTCCTGTGAAGGGCGTGGAGTATGGAAAGGAGAAGTCCCTCAG 8040
2661 S H S A I L S C E G R G V W K G E V P Q 2680

8041 TGCTTGCCTGTGTTCTGTGGCGATCCAGGCACTCCAGCAGAGGGACGGCTCAGTGGGAAA 8100
2681 C L P V F C G D P G T P A E G R L S G K 2700

8101 AGCTTCACCTTTAAGTCTGAGGTCTTCATCCAGTGCAAACCCCCATTTGTGTTAGTGGGT 8160
2701 S F T F K S E V F I Q C K P P F V L V G 2720

8161 TCCTCGAGGAGAACCTGCCAGGCCGATGGGATGTGGAGTGGCATCCAGCCCACTTGTATA 8220
2721 S S R R T C Q A D G M W S G I Q P T C I 2740

8221 GATCCAGCCCACACCGCTTGCCCAGACCCCGGCACTCCCCACTTTGGAATACAGAATAGC 8280
2741 D P A H T A C P D P G T P H F G I Q N S 2760

8281 TCGAAAGGATACGAGGTTGGAAGCACTGTGTTCTTCAGATGTAGAAAAGGTTACCACATC 8340
2761 S K G Y E V G S T V F F R C R K G Y H I 2780

8341 CAAGGCTCCACTACCCGGACCTGTCTTGCCAACCTCACGTGGAGTGGAATCCAGACAGAG 8400
2781 Q G S T T R T C L A N L T W S G I Q T E 2800

8401 TGCATCCCCCATGCCTGCCGGCAGCCAGAGACCCCAGCGCATGCAGATGTGAGAGCCATC 8460
2801 C I P H A C R Q P E T P A H A D V R A I 2820

8461 GATCTTCCAGCTTTTGGCTACACCTTAGTCTACACCTGTCATCCAGGATTTTTCCTTGCT 8520
2821 D L P A F G Y T L V Y T C H P G F F L A 2840

8521 GGCGGATCTGAGCACAGGACGTGTAAAGCAGACATGAAATGGACAGGAAAGTCACCTGTT 8580
2841 G G S E H R T C K A D M K W T G K S P V 2860

8581 TGTAAAAGTAAAGGAGTGAGAGAAGTTAATGAAACAGTTACTAAAACTCCAGTTCCTTCT 8640
2861 C K S K G V R E V N E T V T K T P V P S 2880

8641 GATGTATTTTTCATCAACTCGGTGTGGAAGGGATATTATGAATATTTAGGCAAGAGACAG 8700
2881 D V F F I N S V W K G Y Y E Y L G K R Q 2900

8701 CCGGCGACTCTCACTGTGGACTGGTTTAATGCAACCAGCAGCAAGGTCAATGCGACCTTC 8760
2901 P A T L T V D W F N A T S S K V N A T F 2920

8761 ACCGCAGCCTCACAGGTGCAGCTGGAGCTGACAGGGGTCTACAAGAAGGAAGAGGCCCAC 8820
2921 T A A S Q V Q L E L T G V Y K K E E A H 2940

8821 CTGCTTCTGAAAGCCTTTCATATCAAAGGCCCAGCAGATATTTTTGTAAGCAAGTTTGAA 8880
2941 L L L K A F H I K G P A D I F V S K F E 2960

8881 AATGACAACTGGGGACTCGATGGTTATGTATCCTCAGGACTTGAGAGAGGAGGATTCTCC 8940
2961 N D N W G L D G Y V S S G L E R G G F S 2980

8941 TTTCAGGGTGATATACATGGAAAAGACTTCGGGAAGTTCAAGCTGGAAAGACAAGATCCT 9000
2981 F Q G D I H G K D F G K F K L E R Q D P 3000

9001 TCCAACTCTGATGCAGATTCTTCAAATCATTACCAGGGCACCAGCAGTGGCTCTGTGGCA 9060
3001 S N S D A D S S N H Y Q G T S S G S V A 3020

Figure 3J

```
9061   GCTGCGATTCTCGTCCCCTTCTTCGCTCTAATTCTATCAGGGTTTGCATTTTACCTCTAC   9120
3021   A  A  I  L  V  P  F  F  A  L  I  L  S  G  F  A  F  Y  L  Y     3040

9121   AAACACAGAACAAGACCAAAAGTTCAATACAATGGCTATGCTGGCCATGAAAACAGTAAT   9180
3041   K  H  R  T  R  P  K  V  Q  Y  N  G  Y  A  G  H  E  N  S  N     3060

9181   GGACAAGCTTCATTTGAAAACCCCATGTATGATACAAACTTAAAACCCACAGAGGCCAAG   9240
3061   G  Q  A  S  F  E  N  P  M  Y  D  T  N  L  K  P  T  E  A  K     3080

9241   GCTGTGAGGTTTGACACGACTCTGAACACAGTGTGTACAGTGGTATAGCCCTCAGTGCCC   9300
3081   A  V  R  F  D  T  T  L  N  T  V  C  T  V  V  *                 3096

9301   CCTAGGACCGACTCATAGCCATACCTCTGATGGACAAGCAGTAAAATCCTTTGGTGCCAT   9360
9361   ATACCACCCCCTTCTACTCTTACCTTGCTGCAGCAACGTTGGCCATCGTCTGCTGGCATA   9420
9421   ACGCAGTGGGAATGTCTTCTCCATCATGCCCGAGTCTTCTGAGGATCAAATTGCAAATAC   9480
9481   ACCTTCATCTGGAAAGTGGCTTATAAAAAGCCCGGTTGCTGCATCCACCAGAAATCAAGA   9540
9541   CCCCGACAACAGCGAGGGCAAGGAAGACTGCAGAGTCTCCCAGACCGGTGGTACTTAATG   9600
9601   CCTCTGACTTTTGTGTCTCTGTGTGGCCAGGATGCCTTTGGTGTAGTCTTCTGAGCACAC   9660
9661   CGATACATCCCTCAGGTGCGGCGACAACATGGTAGCCACTTGATGTGTGTTTTGTGTTTT   9720
9721   TCTGTTTTCTTTTCAACCCTATCCACTGGACATGAATTCTTTACAAAAGAAAAGCCTTCC   9780
9781   TGGAGAAGACGCCTTCTGGAAAATGCACACACAGACGCTTTGCTTCTGCCCTGCCTGAGA   9840
9841   CAGGAGCTCTCCGGATCTTCAGGCTCCACTGGGCGTCCATCAGCCACTAGGGATGTTTGC   9900
9901   AGATCTCACAGTCAGAGCTGGTCCATCCCAGAGTTTTTTGATGCTCAACATTTTGCACTA   9960
9961   GTGTGTCAAGAATGACTAAGTCTGATTTCTAAACAAACTATTTCCACAGGGTTTGTATCC  10020
10021  ACTATACATTGTACATACGCATTTTCTCATACCGTATTCTCAAGCAAATGATGCCACTGT  10080
10081  CAGTTAAGTTTGGGATGCAAAGGAAGGTCTCCCCGGATACAGAACAGATTTTGAAAAGGA  10140
10141  GATAGTGCTAGTAATGCTGAAGAAGTTACTCTTTTAATTGCTTCTGTTGGCCACATTTTC  10200
10201  ATGTCAAATTCATTGCCTACTTCCAGTGGTGGAAATGAAGCCCGTGTATTCCCCTTGGTA  10260
10261  TCCCCCCACTTCATGTGCATACGACTATTGTCTACACCATACTAATCAATAACAGGGGGC  10320
10321  TCCAGCAATGTCTGTTTTCCATGTACAGATGTGAATAGTAATTTATTTTAGGTAGCTCAT  10380
10381  GAACTCAGTTCACAGTGAAGTCTTCCCTTCCGGATTGTTTCCTTTCTCTGTTTTGTAACA  10440
10441  TCACCCTCCCAGAATGCATTGAGAGTCTATCTCACAGCCACACCCAAGCTCAGAGGAATC  10500
10501  GAAAGGGAAATCAAAGAAGTCCAAATCAGAATCGGAAGGGCAGGCACCGCTCGCACACCC  10560
10561  TCATGATGATCTGTTTTATAGATTATTTGCCTTTCTGCAAAAAAAAAATCATTACAGTGA  10620
10621  TTTTTGAAACATTAAAATTCCTTACTGATAGACTATCTATTGTGATATATATAAGATAGG  10680
10681  TGGTATGGCCAACAGGGATAAAATAAACAGCCTAAAGACAAGGCAGGGCTAGAGAAATGT  10740
10741  CTGTAAGAAATTTCAAAGAGAAGATCATGTTTATTTTTATTTATATTTGTTTGATAAAAG  10800
10801  TATTTTTGGAAATATAATGCTTATTTTATTATTTGACGTTTCATGCACAGTCCACGTGGT  10860
10861  AAAAATCCCCCCTTTGTACATCCCAGATTTGCACTGATACATGGGTCAGGATGTCATGCT  10920
10921  GATGTTCTGTTTGCTGTGGTGACTACATTCATGCCTAGCTTTAAGACAGGTGGATCTGTC  10980
10981  TATCTACATGATGTTTAAATGCAGGACTTCCCAGAGGACAGTGGGTAACGGAACATGGCT  11040
11041  TGCTTGCGGCTTTGGAAGTTCAGCATTCTGAGCGTTCCAGAGGCCCGGCTGGGCTCCCTC  11100
11101  CTTCTAGCCCACTGTTCTTGCAAGGGCTGTCTGTTGTGTGCCAGGGCTCCTGACTTCTTC  11160
11161  TGCTGACACTCTGTCCACTGGGTTCCATATTCCAGGACTCCATGTCCTAGGAAAGAGTTT  11220
11221  TGACATAGGTTCCTCCAGCCAAGCCGACACACATCCACGGGGTTCCTCTGGGCTCCACAG  11280
11281  AGGTTCTTCATTGGCTCCCTGGGATAAATTCAGATGATGTCAGCAAGAGTGTGCTTCTAT  11340
11341  ACCACACATTGAGCCAAAACAAAACAGAGAACGTCAGAAGGTCCACGGACCAGAGTGCGC  11400
11401  AAGGGAGAACAGGGTTACTATATATATTAGATGTATATAAAAACACACACACAAACATAT  11460
11461  ATATATTGTACATATCTAAGTTTGAGTCACTCAGACTAGGTGCAAAATGCTGACTTTGGA  11520
11521  GTCTAAACTAACGTCTCTGTCCCCACATCCCTGGCCTCTTTCCTGGCCAGTTACATTAAG  11580
11581  AAGACTTGACTTAGACAGGGCATACATACATGCAAGGAACCACATCATCAGACCAGTGTC  11640
11641  GTTTTCCTTTGTGTGCAAACTGACCTACAGCTACCAGACTGCATCATGGTATTTAAAACC  11700
11701  AACATACAATATTGAGCGGCACTCTCAGTTGAGAGCCTAGCTCAATCCTTCCTAGGANNN  11760
11761  NNNNNNNNNNNNNNNNNNNNNNNNNNNNNNNNNNNNNNNNNNNNNNNNNNNNNNNNNNNN  11820
11821  NNNNNNNNCACCAGGTCTCAGAGGCATTGAAGACCTAGCAGGACAGTCAGGAACACCTTC  11880
11881  CTCAGTGAGGTCTAGACTTTTCCCTGAAGCGCCCAGAGCACAGTGAGGAGTCACGCTCTA  11940
11941  TGAATGACAGGTTATGTGCTTTGAAGCTGTTCAACTGTTGCTTGTCTTTGCCCATCTTGC  12000
```

Figure 3K

```
12001   CTTCAGGCTAGCTGCAATAATTTTTTTCTTCTGTAAAATATTTTGTAAACAATAACAACA 12060
12061   ACAACAAAAGCTATTATAAAAAGGGAGAAAAGAAAGCTGGCATTATGATCAGGAAAACCA 12120
12121   TCCATTCTTGCTGCCCCCCCCCTCCTGTCTCCACCACACGCTGCTGTCACAACGTAGGTG 12180
12181   CGGAAGACCTTTTTGTACAGAGATATATTTTTTATGAAGAATTTGTAAAATTATTAAATA 12240
12241   TGCTGTAATTTTTTGATTAATGTAGGTAAATTGTTAAAAAATAAATGTTTTTACAATATG 12300
12301   AAACTGTAATTTTCCCCCATAATGTAACATTACCCTCTCTAGCTGATTTTCAGTTCCAAT 12360
12361   CCTATTCGAACATGTATTAATATTAAGGCGGCCTGTTAAAATGAACAGTATCTTTTTTTT 12420
12421   TGTCAAAAAAAATTATAAAGAGAGTGTAACATAACCTGTGTAATGCCACCTATCTTTAAA 12480
12481   GCAAATCAGAGTTCTAATTAAATATTTAATTTTAGATTTCAAAAA                12525
```

Figure 4A

Comparison of Human C3b/C4b Complement Receptor, "h-CR" (SEQ ID NO:5) and Human AGP-41773, "41773" (SEQ ID NO:2)

```
h-CR   102 KSCRNPPDPVNGMVHVIKGIQFGSQIKYSCTKGYRLIGSSSATCIISGDT 151
41773  293 QNCPDPPPFQNGYM.INSDYSVGQSVSFECYPGYILIGHPVLTC.QHGIN 340

       152 VIWDNETPICDRIPCGLPPTITNGDFISTNRENFH...YGSVVTYRCNPG 198
       341 RNWNYPFPRCD.APCGYNVTSQNGTIYSPGFPDEYPILKDCIWLITVPPG 389

       199 SGGRKVFELVGEPSIYCTSNDDQVGIWSGP...APQCIIPNKCTPPNVEN 245
       390 HGVYINFTLLQTEAV.....NDYIAVWDGPDQNSPQLGVFSGNTA..LET 432

       246 GILVSDNRSLFSLNEVVEFRCQPGFVMKGPRRVKCQALNKWEPELPSCSR 295
       433 A.YSSTNQVLLKFHS..DF.SNGGFFV..........LNFHAFQL....K 464

       296 VCQPPPDVLHAERTQRDKDNFSPGQEVFYSCEPGYDLRGAASMRC..TPQ 343
       465 KCQPPPAVPQAEMLTED.DDFEIGDFVKYQCHPGYTLVGTDILTCKLSSQ 513

       344 GDWSPAAPTCEVKS..............CDDFMGQLLNGR.....VLFPV 374
       514 LQFEGSLPTCEAQCPANEVRTGSSGVILSPGYPGNYFNSQTCSWSIKVEP 563

       375 NLQLGAKVD.FVCDEGFQL.......KGSSASYCVLAGMES......... 407
       564 NYNITIFVDTFQSEKQFDALEVFDGSSGQSPLLVVLSGNHTEQSNFTSRS 613

       408 .....LWNSSVPVCEQIF........CPSPPVIPNGRHTGKPLEVFPFGK 444
       614 NQLYLRWSTDHATSKKGFKIRYAAPYCSLTHPLKNGGILNRTAGA..VGS 661

       445 AVNYTCDPHPDRGTSFDLIGESTIRCTSDPQGNGVWSSPAPRCGILGHCQ 494
       662 KVHYFCKP......GYRMVGHSNATCRRNPLGMYQWDSLTPLCQAVS.CG 704

       495 APDHFLFAKLKTQTNASDFPIGTSLKYECRPEY...YGRPFSITCLDNLV 541
       705 IPE....SPGNGSFTGNEFTLDSKVVYECHEGFKLESSQQATAVCQEDGL 750

       542 WSS..PKDVCKRKSCKTPPDPVNGMVHVITDI......QVGSRINYSCTT 583
       751 WSNKGKPPTCKPVAC..PSIEAQLSEHVIWRLVSGSLNEYGAQVLLSCSP 798

       584 GHRLIGHSSAECILSGNAAHWS..TKPPICQRIPCGLPPTIANGDFISTN 631
       799 GYYLEGWRLLRCQANGT...WNIGDERPSCRVISCGSLSFPPNGNKIGTL 845

       632 RENFHYGSVVTYRCNPGSGGRKVFELVGEPSIYCTSNDDQVGIWSGPAPQ 681
       846 TV...YGATAIFTCNTG......YTLVGSHVRECLAN....GLWSGSETR 882
```

Figure 4B

```
682 CIXPNKCTPPNVENGILVSDNRSLFSLNEVVEFRCQPGFVMKGPRRVKCQ 731
883 CLAGHCGSPDPIVNGHISGDG...FSYRDTVVYQCNPGFRLVGTSVRICL 929

732 ALNKWEPELPSCSRV.CQPPPDVLHAERTQRDKDNFSPGQEVFYSCEPGY 780
930 QDHKWSGQTPVCVPITCGHPGNPAHG...FTNGSEFNLNDVVNFTCNTGY 976

781 DLRGAASMRCTPQGDWSPAAPTCEVKSCDD..FM.GQLLNGRVLFPVNLQ 827
977 LLQGVSRAQCRSNGQWSSPLPTCRVVNCSDPGFVENAIRHGQQNFPESFE 1026

828 LGAKVDFVCDEGFQLKGSSASYCVLAGMESLWNSSVPVCEQIFCPSPPVI 877
1027 YGMSILYHCKKGFYLLGSSALTCMANG...LWDRSLPKCLAISCGHPGVP 1073

878 PNGRHTGKPLEVFPFGKTVNYTCDPHPDRGTSFDLIGESTIRCTSDPQGN 927
1074 ANAVLTG...ELFTYGAVVHYSC.....RG.SESLIGNDTRVCQEDSH.. 1112

928 GVWSSPAPRC.....GILGHCQAPDHFLFAKLKTQTNASDFPIGTSLKYE 972
1113 ..WSGALPHCTGNNPGFCGDPGTPAH..GSRL.....GDDFKTKSLLRFS 1153

973 CR..PEYYGRPFSITCLDNLVWSSPKDVCKRKSCKTPPDPVNGMVHVITD 1020
1154 CEMGHQLRGSP.ERTCLLNGSWSGLQPVCEAVSCGNPGTPTNGMIVSSDG 1202

1021 IQVGSRINYSCTTGHRLIGHSSAECILSGNAAHWSTKPPICQRIPCGLPP 1070
1203 ILFSSSVIYACWEGYKTSGLMTRHCTANGT...WTGTAPDCTIISCGDPG 1249

1071 TIANGDFISTNRENFHYGSVVTYRCNPGSGGRKVFELVGEPSIYCTSNDD 1120
1250 TLANGIQFGT...DFTFNKTVSYQCNPG....YVMEAVTSATIRCTKD.. 1290

1121 QVGIWSGPAPQCIXPNKCTPPNVENGILVSDNRSLFSLNEVVEFRCQPGF 1170
1291 ..GRWNPSKPVCKAVLCPQPPPVQNGTVEGSD...FRWGSSISYSCMDGY 1335

1171 VMKGPRRVKCQALNKWEPELPSCSRV.CQPPPDVLHAERTQRDKDNFSPG 1219
1336 QLSHSAILSCEGRGVWKGEIPQCLPVFCGDPG..IPAEGRLSGK.SFTYK 1382

1220 QEVFYSCEPGYDLRGAASMRCTPQGDWSPAAPTC...EVKSCDDFMGQLL 1266
1383 SEVFFQCKSPFILVGSSRRVCQADGTWSGIQPTCIDPAHNTCPD.PGTPH 1431

1267 NGRVLFPVNLQLGAKVDFVCDEGFQLKGSSASYCVLAGMESLWNSSVPVC 1316
1432 FGIQNSSRGYEVGSTVFFRCRKGYHIQGSTTRTC.LANL..TWSGIQTEC 1478
```

Figure 4C

```
                .         .         .         .         .
1317 EQIFCPSPPVIPNGRHTGKPLEVFPFGKAVNYTCDPHPDRGTSFDLIGES 1366
         |  |   |       : ::.  ||  . |||  ||      | | | |
1479 IPHACRQPET.P.AHADVRAIDLPTFGYTLVYTC..HP....GFFLAGGS 1520
                .         .
1367 TIR.CTSDPQGNGVWSSPAPRC 1387
       | | .| .     |.  .| |
1521 EHRTCKADMK....WTGKSPVC 1538
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 22250400 P
- US 72878700 A
- US 5480981 A
- US 5808029 A
- WO 9723614 A
- US 9723183 W
- WO 9014363 A
- US 5824469 A
- US 5763192 A
- US 5814476 A
- US 5723323 A
- US 5817483 A
- US 5234784 A
- EP 0154316 A
- EP 0401384 A
- US 4179337 A
- US 5252714 A
- US 5557032 A
- US 5489743 A
- WO 9428122 A
- US 4816567 A
- US 5585089 A
- US 5693762 A
- US 9605928 W
- US 9306926 W
- US 5545807 A
- US 91245 W
- GB 8901207 W
- EP 546073 B1
- EP 546073 A1
- US 9817364 W
- US 4376110 A
- US 94001875 W
- US 9300829 W
- US 3773919 A
- EP 58481 A
- EP 133988 A
- EP 36676 A
- EP 88046 A
- EP 143949 A
- US 5272071 A
- EP 9193051 A
- EP 505500 A
- US 9007642 W
- WO 9109955 A
- WO 9505452 A
- US 9409299 W
- US 4892538 A
- US 5011472 A
- US 5106627 A
- US 9100157 W
- US 9100155 W
- WO 9641865 A
- US 96099486 W
- WO 9731898 A
- US 9703137 W
- WO 9731899 A
- US 9503157 B
- US 5364791 A
- WO 9640911 A
- WO 9710337 A
- US 5514578 A
- WO 9738117 A
- WO 9637609 A
- WO 9303162 A
- US 5464758 A
- US 5650298 A
- US 5654168 A
- US 5741679 A
- US 5834186 A
- WO 9534670 A
- US 9507178 W
- US 5672344 A
- US 5399346 A
- US 5631236 A
- US 5672510 A
- US 5635399 A
- US 4970154 A
- WO 9640958 A
- US 5679559 A
- US 5676954 A
- US 5593875 A
- US 4945050 A


### Non-patent literature cited in the description


- **DAVIS et al.** *Cell,* 1996, vol. 87, 1161-1169
- **GRAHAM et al.** *Virology,* 1973, vol. 52, 456
- **SAMBROOK et al.** Molecular Cloning, a laboratory Manual. Cold Spring Harbor Laboratories, 1989

- **DAVIS et al.** Basic Methods in Molecular Biology. Elsevier, 1986
- **CHU et al.** *Gene,* 1981, vol. 13, 197
- **SAMBROOK ; FRITSCH ; MANIATIS.** Molecular Cloning: A Laboratory Manual. Cold Spring Harbor Laboratory, 1989
- **ANDERSON et al.** Nucleic Acid Hybridisation: a practical approach. IRL Press Limited
- **ANDERSON et al.** Nucleic Acid Hybridisation: a Practical Approach. IRL Press Limited
- **SUGGS et al.** Developmental Biology Using Purified Genes. 1981, 683
- **KYTE et al.** *J. Mol. Biol.,* 1982, vol. 157, 105-131
- **MOULT J.** *Curr. Op. in Biotech.,* 1996, vol. 7 (4), 422-427
- **CHOU et al.** *Biochemistry,* 1974, vol. 13 (2), 222-245
- **CHOU et al.** *Biochemistry,* 1974, vol. 113 (2), 211-222
- **CHOU et al.** *Adv. Enzymol. Relat. Areas Mol. Biol.,* 1978, vol. 47, 45-148
- **CHOU et al.** *Ann. Rev. Biochem.,* vol. 47, 251-276
- **CHOU et al.** *Biophys. J.,* 1979, vol. 26, 367-384
- **HOLM et al.** *Nucl. Acid. Res.,* 1999, vol. 27 (1), 244-247
- **BRENNER et al.** *Curr. Op. Struct. Biol.,* 1997, vol. 7 (3), 369-376
- **JONES, D.** *Curr. Opin. Struct. Biol.,* 1997, vol. 7 (3), 377-87
- **SIPPL et al.** *Structure,* 1996, vol. 4 (1), 15-9
- **BOWIE et al.** *Science,* 1991, vol. 253, 164-170
- **GRIBSKOV et al.** *Meth. Enzym.,* 1990, vol. 183, 146-159
- **GRIBSKOV et al.** *Proc. Nat. Acad. Sci.,* 1987, vol. 84 (13), 4355-4358
- **CAPON et al.** *Nature,* 1989, vol. 337, 525-31
- **ZHENG et al.** *J. Immunol.,* 1995, vol. 154, 5590-5600
- **FISHER et al.** *N. Engl. J. Med.,* 1996, vol. 334, 1697-1702
- **VAN ZEE et al.** *J. Immunol.,* 1996, vol. 156, 2221-2230
- **CAPON et al.** *Nature,* 1989, vol. 337, 525-531
- **HARVILL et al.** *Immunotech.,* 1995, vol. 1, 95-105
- **LINSLEY.** *J. Exp. Med.,* 1991, vol. 174, 561-569
- Computational Molecular Biology. Oxford University Press, 1988
- Biocomputing: Informatics and Genome Projects. Academic Press, 1993
- Computer Analysis of Sequence Data. Humana Press, 1994
- Sequence Analysis in Molecular Biology. Academic Press, 1987
- Sequence Analysis Primer. M. Stockton Press, 1991
- **CARILLO et al.** *SIAM J. Applied Math.,* 1988, vol. 48, 1073
- **DEVEREUX et al.** *Nucl. Acid. Res.,* 1984, vol. 12, 387
- **ALTSCHUL et al.** *J. Mol. Biol.,* 1990, vol. 215, 403-410
- **ALTSCHUL et al.** BLAST Manual. NCB/NLM/NIH
- **DAYHOFF et al.** *Atlas of Protein Sequence and Structure,* 1978, vol. 5
- **HENIKOFF et al.** *Proc. Natl. Acad. Sci USA,* 1992, vol. 89, 10915-10919
- **NEEDLEMAN et al.** *J. Mol. Biol.,* 1970, vol. 48, 443-453
- **HENIKOFF et al.** *Proc. Natl. Acad. Sci. USA,* 1992, vol. 89, 10915-10919
- **NEEDLEMAN et al.** *J. Mol Biol.,* 1970, vol. 48, 443-453
- **SAMBROOK et al.** Molecular Cloning: A Laboratory Manual. Cold Spring Harbor Laboratory Press, 1989
- Current Protocols in Molecular Biology. Green Publishers Inc. and Wiley and Sons, 1994
- **ENGELS et al.** *Angew. Chem. Intl. Ed.,* 1989, vol. 28, 716-734
- Meth. Enz. Academic Press Inc, 1990, vol. 185
- **BERNOIST ; CHAMBON.** *Nature,* 1981, vol. 290, 304-310
- **YAMAMOTO et al.** *Cell,* 1980, vol. 22, 787-797
- **WAGNER et al.** *Proc. Natl. Acad. Sci. USA,* 1981, vol. 78, 144-1445
- **BRINSTER et al.** *Nature,* 1982, vol. 296, 39-42
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **SWIFT et al.** *Cell,* 1984, vol. 38, 639-646
- **ORNITZ et al.** *Cold Spring Harbor Symp. Quant. Biol.,* 1986, vol. 50, 399-409
- **MACDONALD.** *Hepatology,* 1987, vol. 7, 425-515
- **HANAHAN.** *Nature,* 1985, vol. 315, 115-122
- **GROSSCHEDL et al.** *Cell,* 1984, vol. 38, 647-658
- **ADAMES et al.** *Nature,* 1985, vol. 318, 533-538
- **ALEXANDER et al.** *Mol. Cell. Biol.,* 1987, vol. 7, 1436-1444
- **LEDER et al.** *Cell,* 1986, vol. 45, 485-495
- **PINKERT et al.** *Genes and Devel.,* 1987, vol. 1, 268-276

- **KRUMLAUF et al.** *Mol. Cell. Biol.,* 1985, vol. 5, 1639-1648
- **HAMMER et al.** *Science,* vol. 235, 53-58
- **KELSEY et al.** *Genes and Devel.,* 1987, vol. 1, 161-171
- **MOGRAM et al.** *Nature,* 1985, vol. 315, 338-340
- **KOLLIAS et al.** *Cell,* 1986, vol. 46, 89-94
- **READHEAD et al.** *Cell,* 1987, vol. 48, 703-712
- **SANI.** *Nature,* 1985, vol. 314, 283-286
- **MASON et al.** *Science,* 1986, vol. 234, 1372-1378
- **URLAUB et al.** *Proc. Natl. Acad. Sci. USA,* 1980, vol. 97, 4216-4220
- **KITTS et al.** *Biotechniques,* 1993, vol. 14, 810-817
- **LUCKLOW.** *Curr. Opin. Biotechnol.,* 1993, vol. 4, 564-572
- **LUCKLOW et al.** *J. Virol.,* 1993, vol. 67, 4566-4579
- **MARSTON et al.** *Meth. Enz.,* 1990, vol. 182, 264-275
- Current Protocols in Molecular Biology. John Wiley & Sons, 1993
- **MERRIFIELD et al.** *J. Am. Chem. Soc.,* 1963, vol. 85, 2149
- **HOUGHTEN et al.** *Proc Natl Acad. Sci. USA,* 1985, vol. 82, 5132
- **STEWART ; YOUNG.** Solid Phase Peptide Synthesis. Pierce Chemical Co, 1984
- **ROBERTS et al.** *Proc. Natl. Acad. Sci.,* 1997, vol. 94, 12297-12303
- **ROBERTS, R.** *Curr. Opin. Chem. Biol.,* 1999, vol. 3, 268-273
- **FRANCIS et al.** *Focus on Growth Factors,* 1992, vol. 3, 4-10
- **KOHLER et al.** *Nature,* 1975, vol. 256, 495-497
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MORRISON et al.** *Proc. Natl. Acad. Sci.,* 1985, vol. 81, 6851-6855
- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **JAKOBOVITS et al.** *Proc. Natl. Acad. Sci.,* 1993, vol. 90, 2551-2555
- **JAKOBOVITS et al.** *Nature,* 1993, vol. 362, 255-258
- **BRUGGEMANN et al.** *Year in Immuno.,* 1993, vol. 7, 33
- **HOOGENBOOM et al.** *J. Mol. Biol.,* 1991, vol. 227, 381
- **MARKS et al.** *J. Mol. Biol.,* 1991, vol. 222, 581
- **ZOLA.** Monoclonal Antibodies: A Manual of Techniques. CRC Press, Inc, 1987, 147-158
- **BAYER et al.** *Meth. Enz.,* 1990, vol. 184, 138-163
- Current Protocols in Molecular Biology. John Wiley & Sons, 1995
- **CHIEN et al.** *Proc. Natl. Acad. Sci.,* 1991, vol. 88, 9578-9583
- **FALWELL et al.** *Proc. Natl. Acad. Sci.,* 1994, vol. 91, 664-668
- **SCHWARZE et al.** *Science,* 1999, vol. 285, 1569-1572
- **NAGAHARA et al.** *Nature Medicine,* 1998, vol. 4, 1449-1452
- **STRAUSS, E.** Introducing Proteins Into the Body's Cells. *Science,* 1999, vol. 285, 1466-1467
- Remington's Pharmaceutical Sciences. Mack Publishing Company, 1990
- **SIDMAN et al.** *Biopolymers,* 1983, vol. 22, 547-556
- **LANGER et al.** *J. Biomed. Mater. Res.,* 1981, vol. 15, 167-277
- **LANGER.** *Chem. Tech.,* 1982, vol. 12, 98-105
- **EPPSTEIN et al.** *Proc. Natl. Acad. Sci. USA,* 1985, vol. 82, 3688-3692
- **KUCHERLAPATI.** *Prog. in Nucl. Acid Res. & Mol. Biol.,* 1989, vol. 36, 301
- **THOMAS et al.** *Cell,* 1986, vol. 44, 419-428
- **THOMAS ; CAPECCHI.** *Cell,* 1987, vol. 51, 503-512
- **DOETSCHMAN et al.** *Proc. Natl. Acad. Sci.,* 1988, vol. 85, 8583-8587
- **DOETSCHMAN et al.** *Nature,* 1987, vol. 330, 576-578
- **SAUER.** *Current Opinion In Biotechnology,* 1994, vol. 5, 521-527
- **SAUER.** *Methods In Enzymology,* 1993, vol. 225, 890-900
- **BAUBONIS ; SAUER.** *Nucleic Acids Res.,* 1993, vol. 21, 2025-2029
- **O'GORMAN et al.** *Science,* 1991, vol. 251, 1351-1355
- **WINN et al.** *Exper. Neurol.,* 1991, vol. 113, 322-329
- **AEBISCHER et al.** *Exper. Neurol.,* 1991, vol. 111, 269-275
- **TRESCO et al.** *ASAIO,* 1992, vol. 38, 17-23
- *Science,* 2000, vol. 287, 816-817826-830
- **HEFTI.** *Neurobiology,* 1994, vol. 25, 1418-1435